(19) **Europäisches Patentamt / European Patent Office / Office européen des brevets**

(11) **EP 2 673 375 B1**

(12) **FASCICULE DE BREVET EUROPEEN**

(45) Date de publication et mention de la délivrance du brevet:
**17.10.2018 Bulletin 2018/42**

(21) Numéro de dépôt: **12702842.1**

(22) Date de dépôt: **09.02.2012**

(51) Int Cl.:
*C12Q 1/6883* (2018.01)      *C12Q 1/6876* (2018.01)
*G01N 33/576* (2006.01)      *C12Q 1/70* (2006.01)

(86) Numéro de dépôt international:
**PCT/EP2012/052234**

(87) Numéro de publication internationale:
**WO 2012/107530 (16.08.2012 Gazette 2012/33)**

(54) **COMBINAISON DE BIOMARQUEURS POUR LA DÉTECTION ET L'ÉVALUATION D'UNE FIBROSE HÉPATIQUE**

KOMBINATION VON BIOMARKERN ZUR DETEKTION UND EVALUIERUNG EINER LEBERFIBROSE

COMBINATION OF BIOMARKERS FOR DETECTING AND EVALUATING AN HEPATIC FIBROSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.02.2011 FR 1151022**
           **09.02.2011 US 201161440986 P**

(43) Date de publication de la demande:
**18.12.2013 Bulletin 2013/51**

(73) Titulaires:
- **Bio-Rad Innovations**
  **92430 Marnes-La-Coquette (FR)**
- **Ariana Pharmaceuticals**
  **75015 Paris (FR)**
- **Institut National de la Santé et de la Recherche Médicale**
  **75654 Paris Cedex 13 (FR)**
- **Assistance Publique - Hôpitaux de Paris**
  **75004 Paris (FR)**
- **Centre National de la Recherche Scientifique**
  **75794 Paris Cedex 16 (FR)**

(72) Inventeurs:
- **WATELET, Bénédicte**
  **34980 Saint Clement De Riviere (FR)**
- **ASSELAH, Tarik**
  **75012 Paris (FR)**
- **BIECHE, Ivan**
  **92150 Suresnes (FR)**
- **BATXELLI, Isabelle, Catherine**
  **30670 Aigues-Vives (FR)**
- **JULLIAN, Nathalie**
  **92120 Montrouge (FR)**
- **VIDAUD, Michel**
  **94120 Fontenay Sous Bois (FR)**
- **MARCELLIN, Patrick**
  **75017 Paris (FR)**
- **LAUNE, Daniel**
  **34790 Grabels (FR)**
- **AFSHAR, Mohammad**
  **75016 Paris (FR)**
- **MATHIEU, Eve, Laure**
  **34080 Montpellier (FR)**

(74) Mandataire: **Desaix, Anne et al**
**Ernest Gutmann - Yves Plasseraud S.A.S.**
**3, rue Auber**
**75009 Paris (FR)**

(56) Documents cités:
**US-A1- 2007 172 907      US-A1- 2010 041 069**
**US-A1- 2010 136 579      US-A1- 2010 203 553**
**US-B1- 6 631 330**

- **SHIN HYOUNG DOO ET AL: "SPP1 polymorphisms associated with HBV clearance and HCC occurrence", INTERNATIONAL JOURNAL OF EPIDEMIOLOGY, vol. 36, no. 5, octobre 2007 (2007-10), pages 1001-1008, XP002668356, ISSN: 0300-5771, DOI: 10.1093/IJE/DYM093**

**(Cont. page suivante)**

EP 2 673 375 B1

- **SCHMILOVITZ-WEISS HEMDA ET AL: "Serum globulin levels in predicting the extent of hepatic fibrosis in patients with recurrent post-transplant hepatitis C infection.", CLINICAL TRANSPLANTATION, vol. 21, no. 3, mai 2007 (2007-05), pages 391-397, XP002668362, ISSN: 0902-0063**

**Description**

**DOMAINE DE L'INVENTION**

L'invention est relative aux fibroses hépatiques, plus particulièrement aux fibroses hépatiques qui peuvent être présentes chez un sujet infecté par un ou plusieurs virus de l'hépatite. L'invention fournit des moyens qui permettent de détecter de telles fibroses hépatiques. Les moyens de l'invention sont plus particulièrement adaptés à la détermination fiable du stade d'atteinte tissulaire hépatique, notamment du score de fibrose hépatique.

**ARRIÈRE-PLAN DE L'INVENTION**

Diverses pathologies causent, ou conduisent à, des lésions tissulaires du foie, connues sous le nom de fibrose hépatique. Une fibrose hépatique résulte notamment d'une accumulation excessive de composés moléculaires de la matrice extracellulaire altérée dans le parenchyme hépatique.

Le stade d'atteinte tissulaire du foie, plus particulièrement la nature et l'étendue des lésions tissulaires hépatiques, est évalué par un score de fibrose hépatique, notamment par le système de score F Metavir, qui comprend 5 stades de F0 à F4 (*cf.* tableau 1 ci-dessous). La détermination du score de fibrose hépatique revêt un caractère primordial pour le praticien, puisqu'il s'agit d'un score pronostique.

En effet, c'est sur la base de cette détermination que le praticien décide d'administrer ou de ne pas administrer de traitement en vue de traiter ces lésions, ou à tout le moins en vue de pallier leurs effets. C'est également sur cette base que le praticien décide d'initier un traitement. Notamment, lorsque le score de fibrose hépatique est d'au plus F1, le praticien décide généralement de ne pas administrer de traitement, alors que, lorsque le score est d'au moins F2, il est préconisé d'administrer un traitement, et cela quelque soit le degré d'activité nécrotico-inflammatoire.

Or, les traitements susceptibles d'être administrés entraînent des effets secondaires majeurs pour le patient. Par exemple, le traitement à ce jour consensuel pour le traitement des patients infectés par le virus de l'hépatite C (VHC) comprend notamment l'administration d'interféron standard ou pégylé sur une durée pouvant aller jusqu'à 48 semaines ou plus. Concernant l'interféron, les effets secondaires sont fréquents et nombreux. L'effet secondaire le plus fréquent est le syndrome pseudo-grippal (fièvre, arthralgies, céphalées, frissons). Les autres effets secondaires possibles sont : une asthénie, un amaigrissement, une perte modérée de cheveux, des troubles du sommeil, des troubles de l'humeur avec une irritabilité qui peut avoir des répercussions dans la vie quotidienne, des difficultés de concentration et une sécheresse cutanée. Certains effets secondaires rares peuvent être graves et doivent être anticipés comme les troubles psychiatriques. Une dépression peut survenir dans environ 10 % des cas. Celle ci doit être dépistée et traitée car elle peut avoir des conséquences graves (tentative de suicide). Une dysthyroïdie peut se déclarer. Le traitement par interféron est en outre contre-indiqué pendant la grossesse.

Concernant la ribavirine, le principal effet secondaire est l'anémie hémolytique. Une anémie conduit à un arrêt du traitement dans environ 5 % des cas. Une décompensation d'une coronaropathie ou d'une cardiopathie sous jacente, liée à l'anémie, peut survenir.

Une neutropénie est observée chez environ 20% des malades recevant l'association interféron pégylé et ribavirine, et représente la cause majeure de réduction de la dose d'interféron pégylé.

Le coût de ces traitements est également très élevé.

Dans ce contexte, pouvoir déterminer de manière fiable le score de fibrose hépatique d'un patient donné, et plus particulièrement pouvoir discriminer de manière fiable, pour un patient donné, un score de fibrose hépatique d'au plus F1 d'un score de fibrose hépatique d'au moins F2, est d'une importance cruciale pour le patient.

Les moyens actuellement disponibles pour déterminer le score de fibrose hépatique d'un patient comprennent notamment l'examen anatomo-pathologique d'une ponction-biopsique hépatique (PBH). Cet examen permet une détermination suffisamment fiable du niveau de fibrose mais nécessite toutefois des précautions particulières liées au mode invasif du prélèvement. Pour être suffisamment fiable pour un patient donné, cet examen nécessite à tout le moins d'être réalisé sur un prélèvement de quantité suffisante (prélèvement d'une longueur de 15 mm à l'aide d'une aiguille à PBH), et requiert une lecture par un anatomo-pathologiste confirmé. La PBH est un acte invasif et douloureux, et est associée à un taux de morbidité de 0,57%. Elle ne permet pas de suivre les patients de façon régulière pour évaluer la progression de la fibrose.

Il existe par ailleurs dans l'art antérieur des moyens qui ont l'avantage d'être non invasifs, tels que :

- Fibroscan™, qui est un système d'imagerie du foie par élastographie transitoire, et tels que
- Fibrotest™, Fibrometer™ et Hepascore™, qui sont des algorithmes de classification multivariée combinant les valeurs de dosage de protéines sériques et éventuellement les valeurs de certains facteurs cliniques ;
- *cf.* WO 02/16949 A1 (au nom de Epigene), WO 2006/103570 A2 (au nom de Assistance Publique - Hôpitaux de Paris), WO 2006/082522 A1 (au nom de Assistance Publique - Hôpitaux de Paris), ainsi que leurs contreparties nationales et régionales.

Le Fibrotest™ (commercialisé par BioPredictive ; Paris, France) met en oeuvre les dosages de l'alpha-2-macroglobuline (A2M), de l'haptoglobine, de l'apolipoprotéine A1, de la bilirubinémie totale et de la gamma-glutamyl transpeptidase.

Le Fibrometer™ (commercialisé par BioLiveScale ; Angers, France) met en oeuvre les mesures des plaquettes, de l'index de prothrombine, de l'aspartate amino-transférase, de l'alpha-2-macroglobuline (A2M), de l'acide hyaluronique, et de l'urée.

L'Hepascore™ met en oeuvre les dosages de l'alpha-2-macroglobuline (A2M), de l'acide hyaluronique, de la bilirubine totale, de la gamma-glutamyl transpeptidase, et les facteurs cliniques âge et sexe.

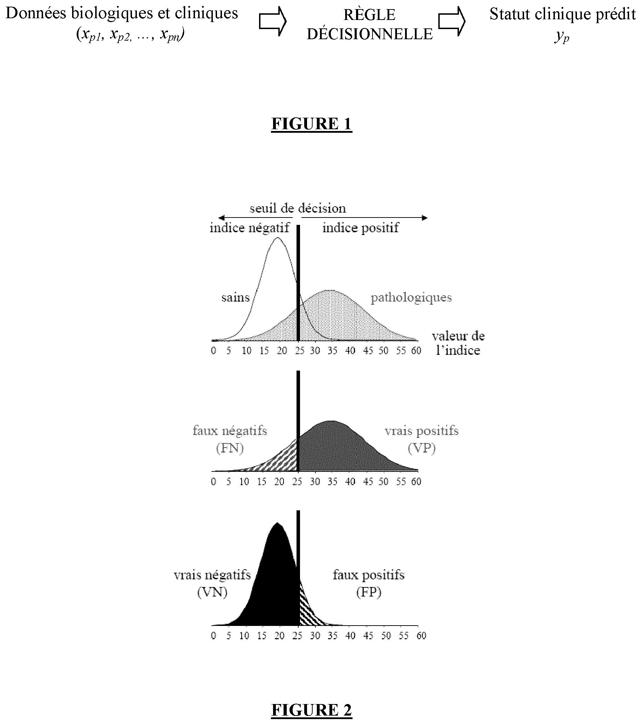

Fibroscan™ n'a pas la sensibilité suffisante pour différencier un score F1 d'un score F2 de fibrose (*cf.* par exemple, Castera *et al.* 2005, plus particulièrement la Figure 1A de cet article).

Par ailleurs, s'il semble maintenant établi que les tests, tels que Fibrotest™, Fibrometer™, Hepascore™, permettent d'identifier de manière fiable une cirrhose hépatique, notamment celle liée au VHC, ces tests n'ont pas la capacité d'identifier de manière précise et fiable les stades plus précoces de fibrose, et n'ont notamment pas la capacité à différencier de manière fiable le stade F1 du stade F2 de fibrose pour un patient donné (*cf.* par exemple, Shaheen *et al.* 2007).

Il existe donc toujours un besoin pour des moyens permettant de déterminer de manière précise et fiable le stade d'atteinte tissulaire hépatique, plus particulièrement le score de fibrose hépatique d'un patient donné. Il existe plus particulièrement toujours un besoin clinique pour des moyens permettant de distinguer de manière fiable, pour un patient donné, une fibrose absente, minime ou cliniquement non significative (score Metavir F0 ou F1), d'une fibrose modérée ou cliniquement significative (score Metavir F2 ou plus), plus particulièrement pour distinguer de manière fiable, pour un patient donné, une fibrose F1 (fibrose sans *septa*) d'une fibrose F2 (fibrose avec quelques septas). Il existe notamment toujours un besoin clinique pour des moyens permettant de détecter de manière fiable l'apparition des premiers septas.

L'invention propose des moyens qui permettent notamment de répondre à ces besoins.

## RÉSUMÉ DE L'INVENTION

L'invention est relative aux fibroses hépatiques, notamment aux fibroses hépatiques qui peuvent être présentes chez un sujet qui est, ou a été, infecté par un ou plusieurs virus de l'hépatite, tels que notamment le virus de l'hépatite C (VHC), le virus de l'hépatite B (VHB), le virus de l'hépatite D (VHD).

Les inventeurs ont identifié des gènes dont les niveaux d'expression sont des biomarqueurs du stade d'atteinte tissulaire hépatique, plus particulièrement du score de fibrose hépatique. Plus particulièrement, les inventeurs proposent d'établir le profil d'expression de ces gènes, et d'utiliser ce profil à titre de signature du stade d'atteinte tissulaire hépatique, plus particulièrement du score de fibrose hépatique.

L'invention fournit des moyens qui sont spécialement adaptés à cet effet. Les moyens de l'invention mettent notamment en oeuvre le dosage ou la mesure des niveaux d'expression de gènes choisis, lesdits gènes choisis étant :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

Les moyens de l'invention comprennent notamment :

- des méthodes qui comprennent le dosage ou la mesure des niveaux d'expression de gènes choisis ;
- des produits ou réactifs qui sont spécialement adaptés au dosage ou à la mesure de ces niveaux d'expression de gènes ;
- des articles manufacturés, des compositions, des compositions pharmaceutiques, des kits, des tubes, des supports solides comprenant de tels produits ou réactifs, ainsi que
- des systèmes informatiques (notamment, produit programme d'ordinateur et dispositif informatique), qui sont spécialement adaptés à la mise en oeuvre des moyens de l'invention.

## BRÈVE DESCRIPTION DES FIGURES

**Figure 1 :** Processus de classification du patient *p*, à partir des données cliniques et biologiques ($x_p$) prédictrices du statut clinique $y_p$.

**Figure 2 :** Distribution d'un biomarqueur d'intérêt de l'invention, discrimination des deux populations cliniques (population des patients "sains" *versus* population des patients "pathologiques"), et représentation des caractéristiques diagnostiques associées (faux négatifs (FN), faux positifs (FP), vrais positifs (VP) et vrais négatifs (VN)), qui sont fixées en fonction du seuil de décision choisi par l'utilisateur.

**Figure 3 :** Tracé de la courbe ROC (*Receiver Operating Characteristic*) pour un biomarqueur d'intérêt de l'invention. Chaque valeur du seuil fixé (seuil A, seuil B) génère un couple de valeurs (Se, 1-Sp) que l'on reporte dans un graphique sur un plan orthonormé où les abscisses (x) représentent (*1-Sp*), variant de 0 à 1, et où les ordonnées (y) représentent (*Se*).

**Figure 4 :** Représentation graphique sous forme de courbe ROC du test diagnostique parfait (aire sous la courbe où AUC = 1) et du test diagnostique non informatif (aire sous la courbe ou AUC = 0,5).

**Figure 5 :** distribution des concentrations sériques des protéines A2M, CXCL10, IL8, SPP1 et S100A4 (*cf.* exemple 3).

## DESCRIPTION DÉTAILLÉE DE L'INVENTION

L'invention est relative aux objets définis dans les revendications

Dans la demande, à moins que cela ne soit autrement spécifié, ou que le contexte n'en dicte autrement, tous les termes ont leur sens habituel dans le(s) domaine(s) concerné(s). L'invention est relative à des moyens pour détecter ou diagnostiquer une atteinte tissulaire du foie, notamment une fibrose hépatique. Les moyens de l'invention sont notamment adaptés à la détermination du stade ou degré d'atteinte tissulaire hépatique, plus particulièrement à la détermination du score de fibrose hépatique.

Les moyens de l'invention sont plus particulièrement adaptés aux fibroses hépatiques qui peuvent être présentes chez un sujet qui est, ou a été, infecté par un ou plusieurs virus de l'hépatite, tels que notamment par le virus de l'hépatite C (VHC) et/ou le virus de l'hépatite B (VHB) et/ou le virus de l'hépatite D (VHD), plus particulièrement par au moins le VHC (et, optionnellement, par le VHB et/ou le VHD).

La fibrose est la transformation fibreuse de certains tissus, à l'origine d'une augmentation du tissu conjonctif (tissu de soutien et de remplissage). Généralement, la fibrose intervient comme conséquence de l'inflammation chronique.

Le terme 'score de fibrose hépatique' reflète le stade d'avancement de la fibrose hépatique. Le score de fibrose hépatique quantifie l'atteinte tissulaire du foie, notamment la nature, le nombre et l'intensité des lésions fibreuses du foie.

Les moyens de l'invention sont donc des moyens qui permettent de détecter, de quantifier, ou à tout le moins d'évaluer, l'atteinte tissulaire du foie d'un sujet.

Dans le domaine des fibroses hépatiques, différents systèmes de scores ont été établis et sont connus de la personne du métier, par exemple le score Metavir (notamment le score F de Metavir) ou le score d'Ishak (*cf.* Goodman 2007).

**Tableau 1 :** correspondance entre les scores de fibrose Metavir et les scores de fibrose Ishak

| Stade de fibrose | Score de fibrose | |
|---|---|---|
| | Metavir | Ishak |
| Absence de fibrose | F0 | F0 |
| **Fibrose portale sans septa** | **F1** | **F1/F2** |
| **Fibrose portale et quelques septas** | **F2** | **F3** |
| Fibrose septale sans cirrhose | F3 | F4 |
| Cirrhose | F4 | F5/F6 |

Sauf indication contraire, ou sauf à ce que le contexte n'en dicte autrement, les scores de fibrose hépatique indiqués dans l'invention sont des scores F établis selon le système Metavir, et les termes « score », « score fibrotique », « score de fibrose », « score de fibrose hépatique » et termes similaires ont la signification clinique d'un score F Metavir, c'est-à-dire qu'ils qualifient, voire quantifient, l'atteinte tissulaire, plus particulièrement les lésions (ou fibrose), d'un foie.

Dans l'invention, l'expression « au plus F1» inclut un score de F1 ou F0, plus particulièrement un score de F1, et l'expression « au moins F2 » inclut un score de F2, F3 ou F4.

Les moyens de l'invention permettent de distinguer de manière fiable :

- une fibrose hépatique dont le score fibrotique selon le système Metavir est d'au plus F1 (absence de fibrose ou fibrose portale sans septa),
- d'une fibrose hépatique dont le score fibrotique selon le système Metavir est d'au moins F2 (fibrose portale avec quelques septas, fibrose septale sans cirrhose ou cirrhose).

Plus particulièrement, les moyens de l'invention permettent de distinguer de manière fiable :

- une fibrose hépatique dont le score fibrotique selon le système Metavir est F1 (fibrose portale sans septa),
- d'une fibrose hépatique dont le score fibrotique selon le système Metavir est F2 (fibrose portale avec quelques septas).

D'un point de vue clinique, les moyens de l'invention permettent de déterminer de manière fiable si la fibrose hépatique ne présente pas de septa, ou si cette fibrose comprend déjà des septas.

Le distinguo auquel les moyens de l'invention permettent d'accéder est cliniquement très utile.

En effet, lorsque la fibrose hépatique est absente ou n'en est à un stade où les septas ne sont pas encore apparus (score Metavir F0 ou F1), le praticien peut choisir de ne pas administrer de traitement au patient, jugeant par exemple qu'à ce stade de la fibrose hépatique, le rapport bénéfice/risque du traitement médicamenteux susceptible de lui être administré ne lui est pas favorable, alors que, lorsque la fibrose hépatique a atteint le stade septal (score Metavir F2, F3 ou F4), le praticien recommandera l'administration d'un traitement médicamenteux pour bloquer ou au moins ralentir la progression de cette fibrose hépatique, afin de diminuer le risque d'évolution vers la cirrhose.

En permettant d'opérer ces distinguos de manière fiable, les moyens de l'invention permettent d'administrer à bon escient les traitements médicamenteux qui sont actuellement disponibles pour tenter de lutter contre, ou à tout le moins pallier, une fibrose hépatique. Comme ces traitements médicamenteux entraînent le plus souvent des effets secondaires majeurs pour le patient, les moyens de l'invention apportent des avantages très nets vis-à-vis de la santé générale du patient. C'est par exemple le cas lorsque ce traitement comprend l'administration d'interféron standard ou pégylé, soit en monothérapie (par exemple dans le cas de l'hépatite chronique virale B et de l'hépatite D), soit en association à la ribavirine (par exemple dans le cas de l'hépatite chronique C).

C'est aussi le cas, lorsque le traitement doit être administré à long terme comme c'est le cas pour les analogues nucléosidiques et nucléotidiques dans le traitement de l'hépatite chronique B.

Les moyens de l'invention comprennent notamment :

- des méthodes qui comprennent le dosage ou la mesure des niveaux d'expression de gènes choisis (niveaux de transcription ou de traduction);
- des produits ou réactifs qui sont spécialement adaptés au dosage ou à la mesure de ces niveaux d'expression de gènes ;
- des articles manufacturés, des compositions, des compositions pharmaceutiques, des kits, des tubes, des supports solides comprenant de tels produits ou réactifs.

Selon un aspect de l'invention, une **méthode** de l'invention est une méthode pour détecter ou diagnostiquer une fibrose hépatique chez un sujet, notamment une méthode pour déterminer le score de fibrose hépatique de ce sujet.

La méthode de l'invention est plus particulièrement adaptée pour les sujets qui sont, ou ont été, infectés par un ou plusieurs virus de l'hépatite, tels que notamment par le virus de l'hépatite C (VHC) et/ou le virus de l'hépatite B (VHB) et/ou le virus de l'hépatite D (VHD), notamment par au moins le VHC.

Une méthode de l'invention est une méthode pour déterminer si le score fibrotique d'une fibrose hépatique est d'au plus F1 (score de F1 ou F0, plus particulièrement de F1) ou bien d'au moins F2 (score de F2, F3 ou F4), plus particulièrement si ce score est F1 ou F2 (scores exprimés selon le système Metavir).

Comme indiqué ci-dessus, il est préférable de n'administrer un traitement qu'aux patients présentant un score fibrotique Metavir supérieur à F1. Pour les autres patients, une simple surveillance est préférable à moyen terme (plusieurs mois à quelques années).

Par suite, la méthode de l'invention peut être considérée comme une méthode de traitement, plus particulièrement comme une méthode pour déterminer le moment auquel un traitement doit être administré au sujet. Ledit traitement peut notamment être un traitement visant à bloquer ou ralentir la progression de la fibrose hépatique, en éliminant le virus (notamment, dans le cas de l'hépatite C) et/ou en bloquant le virus (notamment dans le cas de l'hépatite B).

En effet, les moyens de l'invention permettent de déterminer de manière fiable le degré d'atteinte tissulaire du foie du sujet, plus particulièrement de déterminer la nature de ces lésions (fibrose absente ou sans septa *versus* fibrose septale). L'invention propose donc une méthode, comprenant le fait de :

- déterminer le score de fibrose hépatique d'un sujet à l'aide des moyens de l'invention, et

- si le score ainsi déterminé est un score fibrotique d'au moins F2 (selon le système de score Metavir), administrer à ce sujet un traitement visant à bloquer ou ralentir la progression de la fibrose hépatique (tel que interféron standard ou pégylé, en monothérapie, ou en plurithérapie, par exemple en association avec ribavirine).

Si le score ainsi déterminé est d'au plus F1 (score exprimé selon le système de score Metavir), le praticien peut choisir de ne pas administrer ce traitement.

Une caractéristique d'une méthode de l'invention est qu'elle comprend le fait de doser (ou mesurer) le niveau auquel des gènes choisis s'expriment dans l'organisme dudit sujet.

L'expression « niveau d'expression d'un gène » ou expression équivalente désigne ici tant le niveau auquel ce gène est transcrit en ARN, plus particulièrement en ARNm, que le niveau auquel est exprimée une protéine codée par ce gène.

Le terme « doser » ou « mesurer » ou terme équivalent est entendu selon son sens commun dans le domaine, et fait référence au fait de quantifier.

On peut doser le niveau de transcription (ARN) de chacun des desdits gènes, ou doser le niveau de traduction (protéine) de chacun desdits gènes, ou bien encore doser le niveau de transcription pour certains desdits gènes choisis et le niveau de traduction pour les autres de ces gènes choisis. Selon un mode de réalisation de l'invention, on dose soit le niveau de transcription soit le niveau de traduction de chacun desdits gènes choisis.

Le fait de doser (ou mesurer) le niveau de transcription d'un gène comprend le fait de quantifier des ARN transcrits de ce gène, plus particulièrement de déterminer la concentration d'ARN transcrits par ce gène (par exemple, la quantité de ces ARN rapportée à la quantité totale d'ARN initialement présente dans l'échantillon, telle que par exemple une valeur de Ct normalisée par la méthode du $2^{-\Delta Ct}$ ; *cf.* ci-dessous).

Le fait de doser (ou mesurer) le niveau de traduction d'un gène comprend le fait de quantifier des protéines codées par ce gène, plus particulièrement de déterminer la concentration en protéines codées par ce gène (par exemple, la quantité de cette protéine par volume de fluide biologique).

Certaines protéines codées par un gène de mammifère, notamment un gène humain, peuvent parfois subir des modifications post-traductionnelles telles que, par exemple, un clivage en polypeptides et/ou peptides. Le cas échéant, le fait de doser (ou mesurer) le niveau de traduction d'un gène peut alors comprendre le fait de quantifier ou de déterminer la concentration non pas de la ou des protéines elles-mêmes, mais d'une ou de formes post-traductionnelles de cette ou ces protéines, telles que par exemple, des polypeptides et/ou peptides qui sont des fragments spécifiques de cette ou ces protéines. Pour doser ou mesurer le niveau d'expression d'un gène, l'on peut donc quantifier :

- des ARN transcrits de ce gène, ou
- des protéines exprimées par ce gène, ou des formes post-traductionnelles de telles protéines, telles que par exemple des polypeptides ou peptides qui sont des fragments spécifiques de ces protéines.

Conformément à l'invention, les gènes choisis sont :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

Les gènes ainsi choisis constituent une combinaison de gènes conforme à l'invention.

Des exemples de combinaisons de gènes conformes à l'invention sont présentés dans le tableau 3 ci-dessous.

Chacun de ces gènes est individuellement connu de la personne du métier, et est entendu conformément à sa signification dans le domaine. Un rappel de leurs identités respectives est présenté à titre indicatif dans le tableau 2 ci-dessous.

Aucun de ces gènes n'est un gène de virus de l'hépatite. Il s'agit de gènes de mammifères, plus particulièrement de gènes humains.

Chacun de ces gènes code pour une protéine non-membranaire, c'est-à-dire une protéine qui n'est pas ancrée dans une membrane cellulaire. La localisation *in vivo* de ces protéines est donc intracellulaire et/ou extracellulaire. Ces protéines sont potentiellement présentes dans un fluide biologique du sujet, tel que par exemple dans le sang, le sérum, le plasma ou l'urine, notamment dans le sang ou le sérum ou le plasma.

En sus des niveaux d'expression de gènes choisis parmi la liste de vingt-deux gènes de l'invention (SPP1, A2M, VIM, IL8, CXCL10, ENG, IL6ST, p14ARF, MMP9, ANGPT2, CXCL11, MMP2, MMP7, S100A4, TIMP1, CHI3L1, COL1A1, CXCL1, CXCL6, IHH, IRF9, MMP1), une méthode conforme à l'invention peut en outre comprendre le dosage d'un ou plusieurs facteurs biologiques autres que le niveau d'expression desdits choisis, tel que :

- le dosage de métabolites intracorporels (par exemple, le cholestérol), et/ou le dosage d'éléments figurés du sang (par exemple les plaquettes), et/ou
- le dosage de la quantité de fer circulant, et/ou
- le dosage du niveau d'expression d'autres gènes de mammifères (plus particulièrement de gènes humains), par exemple pour doser le niveau de transcription de gènes qui sont ci-dessous listés en tant que « autres facteurs biologiques », tels que le gène codant l'alanine-amino-transférase (mesure de la concentration en ALT). Il s'agit toutefois de dosages optionnels.

Dans une méthode conforme à l'invention, le nombre de gènes de mammifères (plus particulièrement de gènes humains), dont le niveau d'expression est dosé, et qui ne sont pas des gènes choisis parmi ladite liste de vingt-deux gènes de l'invention (par exemple ALT), est de préférence au maximum de 18, plus particulièrement de 14 ou moins, plus particulièrement de 11 ou moins, plus particulièrement de 6 ou moins, plus particulièrement de 4 ou 3 ou 2, plus particulièrement de 1 ou 0.

Par suite, en comptabilisant ces « autres » gènes de mammifères (plus particulièrement ces gènes humains), dont le niveau d'expression peut être éventuellement dosé, ainsi que le nombre maximal des vingt-deux gènes qui peuvent être des gènes choisis parmi ladite liste de vingt-deux gènes de l'invention, le nombre total de gènes dont le niveau d'expression est dosé dans une méthode conforme à l'invention, est de préférence de 3 à 40 gènes, plus particulièrement de 3 à 36, plus particulièrement de 3 à 33, plus particulièrement de 3 à 28, plus particulièrement de 3 à 26, plus particulièrement de 3 à 25, plus particulièrement de 3 à 24, plus particulièrement de 3 à 23, plus particulièrement de 3 à 22, plus particulièrement de 3 à 20, plus particulièrement de 3 à 21, plus particulièrement de 3 à 20, plus particulièrement de 3 à 19, plus particulièrement de 3 à 18, plus particulièrement de 3 à 17, plus particulièrement de 3 à 16, plus particulièrement de 3 à 15, plus particulièrement de 3 à 14, plus particulièrement de 3 à 13, plus particulièrement de 3 à 12, plus particulièrement de 3 à 11, plus particulièrement de 3 à 10, plus particulièrement de 3 à 9, plus particulièrement de 3 à 8, plus particulièrement de 3 à 7, plus particulièrement de 3 à 6, plus particulièrement de 3 à 5, par exemple de 3, 4 ou 5, notamment de 4 ou 5.

En outre, comme cela est présenté plus en détail ci-dessous, et comme cela est illustré en exemples, le nombre de gènes parmi ladite liste de vingt-deux gènes de l'invention peut avantageusement être inférieur à 22 : ce nombre peut plus particulièrement être de 3 à 10, plus particulièrement de 3 à 9, plus particulièrement de 3 à 8, plus particulièrement de 3 à 7, plus particulièrement de 3 à 6, plus particulièrement de 3 à 5, par exemple de 3, 4 ou 5, notamment de 4 ou 5.

La méthode de l'invention peut optionnellement comprendre le dosage du produit d'expression d'un ou plusieurs gènes non humains, plus particulièrement de gènes viraux tels que des gènes de virus de l'hépatite (plus particulièrement de VHC et/ou VHB et/ou VHD).

La méthode de l'invention peut optionnellement comprendre la détermination du ou des génotypes du ou des virus de l'hépatite dont le sujet est infecté.

La méthode de l'invention peut optionnellement comprendre la détermination d'un ou plusieurs facteurs cliniques dudit sujet tels que l'indice de sensibilité à l'insuline.

**Tableau 2 :**

| Symbole | Nom (en français) de la protéine codée | Nom (en anglais) de la protéine codée | Alias | Numéro d'accession NM |
|---------|------------------|------------------|-------|---------------|
| SPP1 | phosphoprotéine 1 sécrétée | secreted phosphoprotein 1 | OPN ; BNSP ; BSPI ; ETA-1 ; MGC110940 | NM_000582 |
| A2M | alpha 2 macroglobuline | alpha-2-macroglobulin | CPAMD5 ; FWP007 ; S863-7 ; DKFZp779B086 | NM_000014 |
| VIM | vimentine | vimentin | FLJ36606 | NM_003380 |
| IL8 | interleukine 8 | interleukin-8 | IL-8 ; CXCL8 ; GCP-1 ; GCP1 ; LECT ; LUCT ; LYNAP ; MDNCF ; MONAP ; NAF ; NAP-1 ; NAP1 | NM_000584 |
| CXCL10 | ligand 10 à chémokine (motif CXC) | C-X-C motif chemokine 10 | C7 ; IFI10 ; INP10 ; IP-10 ; SCYB10 ; crg-2 ; gIP-10 ; mob-1 | NM_001565 |
| ENG | endogline | endoglin | CD105 ; ORW | NM_000118 |

(suite)

| Symbole | Nom (en français) de la protéine codée | Nom (en anglais) de la protéine codée | Alias | Numéro d'accession NM |
|---|---|---|---|---|
| IL6ST | transducteur de signal interleukin-6 | interleukin-6 signal transducer | CD130 ; GP130 ; CDw130 ; IL6R-beta ; GP130-RAPS | NM_002184 |
| p14ARF transcrit n°4 du gène CDKN2A | inhibiteur de kinase 2A cycline dépendent | cyclin-dependent kinase 2A inhibitor | CDKN2A (codant pour pl4 et p16) ; CDKN2 ; MLM ; ARF ; p14 ; p16 ; p19 ; CMM2 ; INK4 ; MTS1 ; TP16 ; CD4I ; INK4a ; p16INK4 ; p16INK4a | NM_058195 |
| MMP9 | métallopeptidase 9 de matrice | matrix metallopeptidase 9 | CLG4B ; GELB ; MANDP2 | NM_004994 |
| ANGPT2 | angiopoïétine 2 | angiopoietin-2 | ANG2 ; AGPT2 | NM_001147 |
| CXCL11 | ligand 11 à chémokine (motif CXC) | C-X-C motif chemokine 11 | IP9 ; SCYB11 ; ITAC; SCYB9B ; H174 ; IP-9; b-R1; I-TAC ; MGC 102770 | NM_005409 |
| MMP2 | métallopeptidase 2 de matrice | matrix metallopeptidase 2 | CLG4; MONA; TBE1; CLG4A; MMPII | NM_004530 |
| MMP7 | métalloprotéinase 7 de matrice | matrix metallopeptidase 7 | MPSL1 ; PUMP1 ; MMP-7 ; PUMP-1 | NM_002423 |
| S100A4 | protéine A4 liant le calcium S100 | protein S100-A4 | FSP1 | NM_019554 |
| TIMP1 | inhibiteur 1 de métalloprotéinase | metalloproteinase inhibitor 1 | RP1-230G1.3; CLGI; EPA; EPO; FLJ90373 ; HCI ; TIMP | NM_003254 |
| CHI3L1 | protéine 1 de type chitinase-3 | chitinase-3-like protein | GP39 ; ASRT7 ; YKL40 ; YYL-40 ; HC-gp39 ; HCGP-3P ; FLJ38139 ; DKFZp686N19119 | NM_001276 |
| COL1A1 | chaîne alpha-1(I) du collagène | collagen alpha-1(I) chain | OI4 | NM_000088 |
| CXCL1 | chimiokine 1 de la protéine alpha régulant la croissance (motif CXC) | growth-regulated alpha protein C-X-C motif chemokine 1 | GRO ; GRO1 ; GROA ; MGSA ; SCYB1FS ; NAP-3 ; SCYB1 ; MGSA-a | NM_001511 |
| CXCL6 | ligand 6 à chémokine (motif CXC) | C-X-C motif chemokine 6 | CKA-3 ; GCP-2 ; GCP2 ; SCYB6 | NM_002993 |
| IHH | protéine « Indian Hedgehog » | Indian hedgehog protein | BDA1 ; HHG2 | NM_002181 |
| IRF9 | facteur de transcription 3G stimulé par interféron | interferon regulatory factor 9 | ISGF3G ; p48 ; ISGF3 | NM_ 006084 |
| MMP1 | métalloprotéinase 1 de matrice | matrix metalloproteinase-1 | CLG ; CLGN | NM_002421 |

Le dosage (ou la mesure) du niveau d'expression desdits gènes choisis peut être fait dans un échantillon préalablement obtenu à partir dudit sujet, tel que :

- un échantillon biologique prélevé ou collecté dudit sujet, ou
- un échantillon comprenant des acides nucléiques (notamment des ARN) et/ou protéines et/ou polypeptides et/ou peptides dudit échantillon biologique, notamment un échantillon comprenant des acides nucléiques et/ou protéines et/ou polypeptides et/ou peptides qui ont été, ou sont susceptibles d'avoir été extraits et/ou purifiés dudit échantillon biologique, ou
- un échantillon comprenant des ADNc qui ont été, ou sont susceptibles d'avoir été obtenus par transcription inverse desdits ARN.

Un échantillon biologique collecté ou prélevé dudit sujet peut par exemple être un échantillon prélevé ou collecté, ou susceptible d'être prélevé ou collecté, à partir :

- d'un organe ou tissu interne dudit sujet, notamment de son foie ou de son parenchyme hépatique, ou
- d'un fluide biologique dudit sujet, tel que le sang, le sérum, le plasma ou l'urine, notamment d'un fluide intracorporel tel que le sang.

Un échantillon biologique collecté ou prélevé dudit sujet peut par exemple être un échantillon comprenant une portion de tissu dudit sujet, notamment une portion de tissu hépatique, plus particulièrement une portion de parenchyme hépatique.

Un échantillon biologique collecté ou prélevé dudit sujet peut par exemple être un échantillon comprenant des cellules qui ont été, ou sont susceptibles d'être, prélevées ou collectées d'un tissu dudit sujet, notamment d'un tissu hépatique, plus particulièrement des cellules hépatiques.

Un échantillon biologique collecté ou prélevé dudit sujet peut par exemple être un échantillon de fluide biologique, tel qu'un échantillon de sang, de sérum, de plasma ou d'urine, plus particulièrement un échantillon de sang ou de sérum ou de plasma. En effet, comme les gènes choisis parmi ladite liste de vingt-deux gènes de l'invention codent tous pour des protéines non-membranaires, le produit de leur expression peut notamment avoir une localisation extracellulaire.

Le prélèvement ou la collecte dudit échantillon biologique peut être fait par insertion d'un instrument de prélèvement, notamment par insertion d'une aiguille ou d'un cathéter, dans le corps dudit sujet. Cet instrument peut par exemple être inséré :

- dans un organe ou tissu interne dudit sujet, notamment dans son foie ou dans parenchyme hépatique, par exemple,

  - pour prélever un échantillon de foie ou de parenchyme hépatique, ce prélèvement pouvant par exemple être fait par ponction biopsique hépatique (PBH), plus particulièrement par PBH par voie transjugulaire ou par voie transpariétale, ou
  - pour prélever ou collecter des cellules à partir du compartiment hépatique (prélèvement de cellules, et non de tissu), plus particulièrement à partir du parenchyme hépatique, notamment pour prélever des cellules hépatiques, ce prélèvement ou cette collecte pouvant par exemple être fait par cyto-ponction hépatique ; et/ou

- dans une veine, une artère ou un vaisseau dudit sujet pour prélever un fluide biologique dudit sujet, tel que du sang.

Les moyens de l'invention ne sont pas limités à une mise en oeuvre sur une biopsie de tissu, notamment de tissu hépatique. Ils peuvent être mis en oeuvre sur un échantillon obtenu, ou susceptible d'être obtenu par un prélèvement de taille ou volume nettement plus réduit qu'un échantillon de tissu, à savoir un échantillon qui se limite à quelques cellules. Les moyens de l'invention peuvent notamment être mis en oeuvre sur un échantillon obtenu, ou susceptible d'être obtenu par cyto-ponction hépatique.

La quantité ou le volume de matière prélevé par cyto-ponction hépatique est beaucoup plus réduit que celui prélevé par PBH. Outre le gain immédiat pour le patient en termes de diminution d'invasivité de la technique de prélèvement et de diminution de la morbidité associée, la cyto-ponction hépatique présente l'avantage de pouvoir être répétée à des temps distincts sur le même patient (par exemple, pour déterminer l'évolution de la fibrose hépatique entre deux instants), alors que la PBH ne peut être raisonnablement répétée sur le même patient. La cyto-ponction hépatique présente donc, contrairement à la PBH, l'avantage de permettre de suivre l'évolution clinique du patient.

Ainsi, conformément à l'invention, ledit échantillon biologique peut avantageusement être :

- un prélèvement ou une collecte de cellules à partir du compartiment hépatique (prélèvement ou collecte de cellules, et non de tissu), plus particulièrement à partir du parenchyme hépatique, c'est-à-dire un échantillon biologique obtenu, ou susceptible d'être obtenu par cyto-ponction hépatique ; et/ou

- un prélèvement ou une collecte de fluide biologique dudit sujet, tel que du sang ou de l'urine, notamment du sang.

Le dosage (ou la mesure) peut être fait dans un échantillon biologique qui a été collecté ou prélevé dudit sujet, et qui a été plus avant transformé, par exemple :

- par extraction et/ou purification des acides nucléiques, notamment des ARN, plus particulièrement des ARNm, et/ou par transcription inverse de ces ARN, notamment de ces ARNm, ou
- par extraction et/ou purification des protéines et/ou polypeptides et/ou peptides, ou par extraction et/ou purification d'une fraction protéique, telle que le sérum ou le plasma extrait du sang.

Par exemple, lorsque l'échantillon biologique collecté ou prélevé est un fluide biologique tel que du sang ou de l'urine, cet échantillon peut, avant de réaliser le dosage ou la mesure, être transformé :

- par extraction d'acides nucléiques, notamment d'ARN, plus particulièrement d'ARNm, et/ou par transcription inverse de ces ARN, notamment de ces ARNm (le plus généralement, par extraction des ARN et transcription inverse de ces ARN), ou
- par séparation et/ou extraction de la fraction sérique, ou par extraction ou purification des protéines et/ou polypeptides et/ou peptides sériques.

Ainsi, selon un mode de réalisation de l'invention, ledit échantillon obtenu à partir dudit sujet comprend (par exemple dans une solution), ou est, un échantillon de fluide biologique dudit sujet, tel qu'un échantillon de sang, de sérum, de plasma ou d'urine, et/ou est un échantillon qui comprend (par exemple dans une solution) :

- des ARN, notamment des ARNm, susceptibles d'avoir été extraits ou purifiés d'un fluide biologique, tel que du sang ou de l'urine, notamment du sang ; et/ou des ADNc susceptibles d'avoir été obtenus par transcription inverse de tels ARN ; et/ou
- des protéines et/ou polypeptides et/ou peptides susceptibles d'avoir été extraits ou purifiés d'un fluide biologique, tel que du sang ou de l'urine, notamment du sang, et/ou susceptibles d'avoir été codés par de tels ARN,

de préférence,

- des protéines et/ou polypeptides et/ou peptides susceptibles d'avoir été extraits ou purifiés d'un fluide biologique, tel que du sang ou de l'urine, notamment du sang, et/ou susceptibles d'avoir été codés par de tels ARN.

Lorsque ledit échantillon obtenu à partir dudit sujet comprend un échantillon biologique obtenu, ou susceptible d'être obtenu par prélèvement d'un fluide biologique, tel que du sang ou de l'urine, ou lorsque ledit échantillon obtenu à partir dudit sujet est issu, ou susceptible d'être issu d'un tel échantillon biologique par extraction et/ou purification de molécules contenues dans cet échantillon biologique, le dosage est de préférence un dosage de protéines et/ou polypeptides et/ou peptides, plutôt qu'un dosage d'acides nucléiques.

Lorsque l'échantillon biologique collecté ou prélevé est un échantillon comprenant une portion de tissu, notamment une portion de tissu hépatique, plus particulièrement une portion de parenchyme hépatique, tel que par exemple un échantillon biologique prélevé ou susceptible d'être prélevé par ponction biopsique hépatique (PBH), ou lorsque l'échantillon biologique collecté ou prélevé est un échantillon comprenant des cellules obtenues, ou susceptibles d'être obtenues à partir d'un tel tissu, tel que par exemple un échantillon collecté, ou susceptible d'être collecté, par cyto-ponction hépatique, cet échantillon biologique peut être transformé :

- par extraction d'acides nucléiques, notamment d'ARN, plus particulièrement d'ARNm, et/ou par transcription inverse de ces ARN, notamment de ces ARNm (le plus généralement, par extraction de ces ARN et transcription inverse de ces ARN), ou
- par séparation et/ou extraction des protéines et/ou polypeptides et/ou peptides.

Une étape de lyse des cellules, notamment de lyse des cellules hépatiques, contenues dans ledit échantillon biologique, peut être préalablement réalisée de sorte à rendre acides nucléiques, ou le cas échéant, protéines et/ou polypeptides et/ou peptides, directement accessibles à l'analyse.

Ainsi, selon un mode de réalisation de l'invention, ledit échantillon obtenu à partir dudit sujet est un échantillon de tissu dudit sujet, notamment de tissu hépatique, plus particulièrement de parenchyme hépatique, ou est un échantillon de cellules d'un tel tissu, et/ou est un échantillon qui comprend (par exemple dans une solution):

- des cellules hépatiques, plus particulièrement des cellules du parenchyme hépatique, par exemple des cellules obtenues, ou susceptibles d'être obtenues par dissociation des cellules d'une biopsie de tissu hépatique ou par cyto-ponction hépatique; et/ou
- des ARN, notamment des ARNm, susceptibles d'avoir été extraits ou purifiés de telles cellules ; et/ou
- des ADNc susceptibles d'avoir été obtenus par transcription inverse de tels ARN ; et/ou
- des protéines et/ou polypeptides et/ou peptides susceptibles d'avoir été extraits ou purifiés de telles cellules, et/ou susceptibles d'avoir été codés par de tels ARN.

Conformément à l'invention, ledit sujet est un humain ou un animal non-humain, notamment un humain ou un mammifère non-humain, plus particulièrement un humain.

Du fait de la sélection particulière de gènes proposée par l'invention, le score de fibrose hépatique dudit sujet peut être déduit, ou déterminé à partir, des valeurs de dosage ou de mesure obtenues pour ledit sujet, notamment par induction statistique et/ou classification statistique (*cf.* Figure 1), par exemple vis-à-vis de cohortes de référence (pré-)établies selon leur score de fibrose hépatique.

En sus du fait de doser (ou mesurer) le niveau auquel des gènes choisis s'expriment dans l'organisme dudit sujet, une méthode de l'invention peut donc en outre comprendre une étape de déduction ou détermination du score de fibrose hépatique dudit sujet à partir des valeurs de dosage obtenues pour ledit sujet. Cette étape de déduction ou détermination est une étape par laquelle les valeurs de dosage ou de mesure obtenues pour ledit sujet sont analysées pour en induire le score de fibrose hépatique dudit sujet.

La déduction ou détermination du score de fibrose hépatique dudit sujet peut être faite en comparant les valeurs de dosage obtenues pour ledit sujet à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance (c'est-à-dire pour attribuer audit sujet son score de fibrose hépatique).

Les dosages effectués sur ledit sujet et sur les individus des cohortes ou sous-populations de référence sont des dosages de niveaux d'expression (transcription ou traduction) de gènes.

Pour doser le niveau de transcription d'un gène, on dose son niveau de transcription en ARN. Un tel dosage peut par exemple comprendre la mesure de la concentration en ARN transcrits de chacun desdits gènes choisis, soit par mesure de la concentration de ces ARN, soit par mesure de la concentration des ADNc obtenus par transcription inverse de ces ARN. Le dosage d'acides nucléiques est bien connu de la personne du métier. Par exemple, le dosage d'ARN ou des ADNc correspondants peut se faire par amplification d'acide nucléique, notamment par PCR. Des réactifs sont décrits ci-dessous à cet effet (*cf.* exemple 1 ci-dessous). Des exemples d'amorces et sondes appropriés en sont également donnés (*cf.* par exemple tableau 17 ci-dessous). Les conditions d'amplification d'acides nucléiques peuvent être choisies par la personne du métier. Des exemples de conditions d'amplification sont donnés dans la partie « exemples » qui suit (*cf.* exemple 1 ci-dessous).

Pour doser le niveau de traduction d'un gène, on dose son niveau de traduction en protéines. Un tel dosage peut par exemple comprendre la mesure de la concentration en protéines traduites à partir de chacun desdits gènes choisis (par exemple, dosage des protéines dans la circulation générale, notamment dans le sérum). Le dosage de protéines est bien connu de la personne du métier. Par exemple, les protéines (et/ou polypeptides et/ou peptides) peuvent être dosées par ELISA ou par toute autre méthode immunométrique connue de la personne du métier ou par une méthode utilisant la spectrométrie de masse connue de la personne du métier.

De préférence, chaque dosage est réalisé au moins en duplicate.

Les valeurs de dosage sont des valeurs de concentration ou de proportion, ou des valeurs qui représentent une concentration ou une proportion. L'objectif est qu'au sein d'une même combinaison, les valeurs de dosage des niveaux d'expression de chacun desdits gènes choisis reflètent le plus précisément possible, à tout le moins l'une par rapport à l'autre, le degré auquel chacun de ces gènes est exprimé (degré de transcription ou degré de traduction), notamment en étant proportionnelles à ces degrés respectifs.

Par exemple, dans le cas du dosage du niveau d'expression d'un gène par dosage des ARN transcrits, c'est-à-dire dans le cas du dosage du niveau de transcription de ce gène, le dosage se fait généralement par amplification des ARN par transcription inverse et PCR (RT-PCR), et par mesure de valeurs de Ct (*Cycle threshold*).

Une valeur de Ct donne une mesure de la quantité initiale des ARN amplifiés (plus la valeur de Ct est faible, plus la quantité de ces acides nucléiques est élevée). Les valeurs de Ct mesurées pour un ARN cible ($Ct_{cible}$) sont généralement rapportées à la quantité d'ARN total initialement présente dans l'échantillon, par exemple en déduisant de ce $Ct_{cible}$ la valeur d'un Ct de référence ($Ct_{référence}$), telle que la valeur de Ct qui a été mesurée dans les mêmes conditions opératoires pour l'ARN d'un gène contrôle endogène dont le niveau d'expression est stable (par exemple un gène impliqué dans une cascade métabolique cellulaire, tel que RPLP0 ou TBP ; *cf.* exemple 1 ci-dessous).

Selon un mode de réalisation de l'invention, la différence ($Ct_{cible} - Ct_{référence}$), ou $\Delta Ct$, peut en outre être exploitée par la méthode connue sous le nom de méthode du $2^{-\Delta Ct}$ (Livak et Schmittgen 2001 ; Schmittgen et Livak 2008), sous

la forme :

$$2^{-\Delta Ct} = 2^{-(Ct\ cible\ -\ Ct\ référence)}.$$

Ainsi, selon un mode de réalisation de l'étape i), le dosage des niveaux auxquels chacun desdits gènes choisis sont transcrits est réalisée par :

- amplification, d'un fragment des ARN transcrits par chacun desdits gènes choisis, par exemple par transcription inverse et PCR de ces fragments d'ARN, pour obtenir les valeurs de Ct de chacun de ces ARN,
- optionnellement, normalisation de chacune de ces valeurs de Ct par rapport à la valeur de Ct obtenue pour l'ARN d'un gène contrôle endogène, tel que RPLP0 ou TBP, par exemple par la méthode du $2^{-\Delta Ct}$,
- optionnellement, transformation Box-Cox desdites valeurs normalisées de Ct.

Dans le cas du dosage du niveau d'expression d'un gène par dosage des protéines exprimées par ce gène, c'est-à-dire dans le cas du dosage du niveau de traduction de ce gène, le dosage se fait généralement par une méthode immuno-métrique à l'aide d'anticorps spécifiques, et par expression des mesures ainsi faites en quantités pondérales ou en unités internationales, à l'aide d'une gamme étalon. Des exemples d'anticorps spécifiques sont indiqués dans le tableau 14 ci-dessous. Une valeur de dosage du niveau de traduction d'un gène peut par exemple être exprimée en quantité de cette protéine par volume de fluide biologique, par exemple, par volume de sérum (par exemple, en mg/mL ou en μg/mL ou en ng/mL ou en pg/mL).

Si souhaité ou requis, la distribution des valeurs de dosage obtenues pour les individus d'une cohorte peut être lissée, de sorte à la rapprocher d'une loi gaussienne.

A cet effet, les valeurs de dosage obtenues sur les individus de cette cohorte, par exemple les valeurs obtenues par la méthode du $2^{-\Delta Ct}$, peuvent être transformées par une transformation de type Box-Cox (Box et Cox, 1964 ; *cf.* tableaux 8, 9, 11 et 13 ci-dessous ; *cf.* exemples 2 et 3 ci-dessous).

L'invention est donc notamment relative à une méthode *in vitro* pour déterminer le score de fibrose hépatique d'un sujet, plus particulièrement d'un sujet infecté par un ou des virus de l'hépatite, tels que par le VHC et/ou le VHB et/ou le VHD, notamment par au moins le VHC, caractérisée en ce qu'elle comprend les étapes suivantes :

i) dans un échantillon préalablement obtenu à partir dudit sujet, doser le niveau auquel des gènes choisis sont transcrits ou traduits, lesdits gènes choisis étant :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1,

et

ii) comparer les valeurs de dosage de chacun desdits gènes choisis obtenues pour ledit sujet, à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance.

La comparaison de l'étape ii) peut notamment être faite en combinant les valeurs de dosage (ou de mesure) obtenues pour ledit sujet dans un modèle de classification multivariée.

Un tel modèle de classification multivariée compare (de manière combinée) les valeurs de dosage obtenues pour ledit sujet à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance, par exemple en lui attribuant une valeur de sortie indicatrice du score de fibrose hépatique dudit sujet.

Un tel modèle de classification multivariée peut être construit, notamment préalablement construit, en faisant une comparaison inter-cohorte des valeurs de dosage obtenues pour lesdites cohortes de référence ou des distributions de ces valeurs de dosage.

Plus particulièrement, un tel modèle de classification multivariée peut être construit, notamment préalablement construit, en dosant ou mesurant les niveaux d'expression desdits gènes choisis dans des cohortes de référence pré-établies selon leur score de fibrose hépatique, et en analysant ces valeurs de dosage, ou leur distribution, par une méthode statistique multivariée pour construire un modèle de classification multivariée qui induit ou détermine un score de fibrose hépatique à partir des valeurs de niveaux d'expression desdits gènes choisis.

Si les valeurs dosées pour ledit sujet comprennent, en sus des valeurs de dosage des niveaux de transcription ou de traduction desdits gènes choisis, la ou les valeurs d'un ou plusieurs autres facteurs, tels que un ou plusieurs facteurs virologiques et/ou un ou plusieurs facteurs cliniques et/ou un ou plusieurs des autres facteurs biologiques (*cf.* ci-desssous et dans les exemples), le modèle de classification est bien sûr construit, notamment préalablement construit, en dosant ou mesurant les mêmes valeurs dans des cohortes de référence pré-établies selon leur score de fibrose hépatique, et en analysant ces valeurs, ou leur distribution, par une méthode statistique multivariée pour construire un modèle de classification multivariée qui induit ou détermine un score de fibrose hépatique à partir de ces valeurs.

Par exemple, un modèle peut être construit par une fonction mathématique, une technique non paramétrique, une procédure de classification heuristique ou encore une approche de prédiction probabiliste. Un exemple typique de classification fondée sur la quantification du niveau d'expression de biomarqueurs consiste en la discrimination de sujets "sains" versus "malades". La formalisation de ce problème consiste en *m* échantillons indépendants, décrits par *n* variables aléatoires. Chaque individu *i* (*i*=1,..., *m*) est caractérisé par un vecteur $x_i$ décrivant les *n* valeurs caractéristiques:

$$x_{ij}, \qquad i=1,\dots m \qquad j=1,\dots n$$

Ces valeurs caractéristiques peuvent par exemple représenter des valeurs d'expression génique et/ou des intensités de données protéiques et/ou des intensités de données métaboliques et/ou des données cliniques.

Chaque échantillon $x_i$ est associé à une valeur discrète $y_i$, représentant le statut clinique de l'individu *i*. A titre d'exemple, $y_i$ = 0 si le patient *i* a un score de fibrose hépatique F1, $y_i$ = 1 si le patient *i* a un score de fibrose hépatique F2.

Un modèle offre une règle décisionnelle (par exemple, une fonction mathématique, un algorithme ou une procédure), qui utilise l'information disponible depuis $x_i$ pour prédire $y_j$ dans chaque échantillon observé. L'objectif est d'utiliser ce modèle afin de prédire le statut clinique du patient *p,* à savoir $y_p$, à partir des valeurs biologiques et/ou cliniques disponibles, à savoir $x_p$.

Un processus de classification du patient *p* est schématisé sur la Figure 1.

Différents modèles de classification multivariée sont connus de la personne du métier (*cf.* Hastie, Tibishirani et Friedman, 2009 ; Falissard, 2005 ; Theodoridis et Koutroumbos 2009).

Ils sont généralement construits par traitement et interprétation des données selon, par exemple :

- une méthode d'analyse statistique multivariée, par exemple :

  ◦ une fonction mathématique linéaire ou non linéaire, notamment une fonction mathématique linéaire, tel qu'une fonction générée par la méthode mROC (méthode ROC multivariée), ou
  ◦ une méthode ROC (*Receiver Operating Characteristics*) ;
  ◦ une méthode de régression, linéaire ou non linéaire, comme par exemple la régression logistique ;
  ◦ une méthode PLS-DA (*Partial Least Squares - Discriminant Analysis*) ;
  ◦ une méthode LDA (*Linear Discriminant Analysis)* ;

- une méthode par apprentissage ou intelligence artificielle, par exemple, un algorithme d'apprentissage ou d'intelligence artificielle, une méthode de classification non paramétrique, ou heuristique, ou de prédiction probabiliste, telle que :

  ◦ un arbre décisionnel ; ou
  ◦ une méthode de type *boosting*, fondée sur des classifieurs binaires (ex : *Adaboost*) ou une méthode liée au *boosting* (*bagging*) ; ou
  ◦ une méthode des k plus proches voisins (*k-nearest neighbors* ou KNN), ou plus généralement la méthode des k plus proches voisins pondérés (*weighed k-nearest neighbors* ou WKNN), ou
  ◦ une méthode (par exemple, un algorithme) Machines à Vecteurs de support (*Support Vector Machine* ou SVM) ; ou
  ◦ une forêt aléatoire (*Random Forest* ou RF); ou
  ◦ un réseau bayésien ; ou
  ◦ un réseau de neurones (*Neural Network*) ; ou
  ◦ un treillis de Galois (ou *Formal Concept Analysis*).

Les règles décisionnelles des modèles de classification multivariée peuvent par exemple être fondées sur une formule mathématique du type $y = f(x_1, x_2, ...x_n)$ où *f* est une fonction mathématique linéaire ou non linéaire (régression logistique, mROC, par exemple), ou sur un algorithme d'apprentissage automatique ou d'intelligence artificielle dont les caractéristiques consistent en une série de paramètres de réglage identifiés comme étant les plus efficaces pour la discrimination des sujets (par exemple, KNN, WKNN, SVM, RF).

La méthode ROC multivariée (mROC) est une généralisation de la méthode ROC (*Receiver Operating Characteristic*) (*cf*. Reiser et Faraggi 1997 ; Su et Liu 1993, Shapiro, 1999). Elle calcule l'aire sous la courbe ROC (*Area Under the Curve* ou AUC) relative à une combinaison linéaire de biomarqueurs et/ou de transformations de biomarqueurs (dans le cas d'une normalisation), sous l'hypothèse d'une distribution normale multivariée. La méthode mROC a notamment été décrite dans Kramar *et al.* 1999 et Kramar *et al.* 2001. Référence est également faite aux exemples ci-dessous, notamment au point 2 de l'exemple 1 ci-dessous (modèle mROC).

Le logiciel mROC version 1.0, disponible commercialement auprès des concepteurs (A. Kramar, A. Fortune, D. Farragi et B. Reiser) peut par exemple être utilisé pour construire un modèle mROC.

Andrew Kramar et Antoine Fortune peuvent être contactés auprès de, ou via, l'Unité de Biostatistique du Centre Régional de Lutte contre le Cancer (CRLC) Val dAurelle - Paul Lamarque (208, rue des Apothicaires ; Parc Euromédecine ; 34298 Montpellier Cedex 5 ; France).

David Faraggi et Benjamin Reiser peuvent être contactés auprès du, ou via le, Département de Statistique de l'Université de Haifa (Mount Carmel ; Haifa 31905 ; Israël).

La famille des méthodes d'intelligence artificielle ou d'apprentissage automatique est une famille d'algorithmes qui, au lieu de procéder à une généralisation explicite, comparent les exemples d'un nouveau problème avec les exemples considérés en apprentissage et qui ont été stockés en mémoire. Ces algorithmes construisent directement les hypothèses à partir des exemples d'apprentissage eux-mêmes. Un exemple simple de ce type d'algorithme est l'algorithme des *k* plus proches voisins (*k-nearest neighbors* ou KNN), et une de ses extensions possibles connue sous le nom d'algorithme des *k* plus proches voisins pondérés (*weighted k nearest neighbors* ou WKNN) (Hechenbichler et Schliep, 2004).

Dans le contexte de classification d'une nouvelle observation x, l'idée fondatrice simple est de faire voter les plus proches voisins de cette observation. La classe (ou statut clinique) de *x* est déterminée en fonction de la classe majoritaire parmi les *k* plus proches voisins de l'observation *x*.

Des bibliothèques de fonctions spécifiques KKNN sont disponibles par exemple dans le logiciel R (http://www. R-project.org/). Le logiciel R a été initialement développé par John Chambers et les laboratoires Bell (*cf*. Chambers 2008). La version actuelle de cette suite logicielle est la version 2.11.1. Le code source est disponible librement sous les termes de la licence publique générale « Free Software Foundation's GNU », sur le site http://www. R-project.org/. Ce logiciel peut être utilisé pour construire un modèle WKNN. Référence est également faite aux exemples ci-dessous, notamment au point 2 de l'exemple 1 ci-dessous (modèle WKNN).

Une Forêt Aléatoire (*Random Forest* ou RF) est constituée d'un ensemble d'arbres simples de prévision, chacun étant capable de produire une réponse lorsqu'on lui présente un sous-ensemble de prédicteurs (Breiman 2001 ; Liaw et Wiener 2002). Les calculs sont réalisés avec le logiciel R. Ce logiciel peut être utilisé pour construire des modèles RF. Référence est également faite aux exemples ci-dessous, notamment au point 2 de l'exemple 1 ci-dessous (modèle RF).

Un réseau de neurones est constitué d'un graphe pondéré orienté dont les noeuds symbolisent les neurones. Le réseau est construit à partir d'exemples de chaque classe (par exemple, F2 versus F1), et est ensuite utilisé pour déterminer à quelle classe appartient un nouvel élément ; *cf*. Intrator et Intrator 1993, Riedmiller et Braun 1993, Riedmiller 1994, Anastasiadis *et. al.* 2005 ; *cf*. http://cran.r-project.org/web/packages/neuralnet/index.html. Le logiciel R librement disponible sur http://www.r-project.org/, (version 1.3 de *Neuralnet*, écrit par Stefan Fritsch et Frauke Guenther, suivant le travail de Marc Suling) peut par exemple être utilisé pour construire un réseau de neurones.

Référence est également faite aux exemples ci-dessous, notamment au point 2 de l'exemple 1 ci-dessous (modèle NN).

La comparaison de ladite étape ii) peut donc être notamment réalisée en suivant une méthode, et/ou en utilisant un algorithme ou un logiciel :

- mROC,
- KNN, WKNN, plus particulièrement WKNN,
- RF, ou
- NN,

plus particulièrement mROC.

Chacun de ces algorithmes, logiciels ou méthodes permet de construire un modèle de classification multivariée, à partir des valeurs de dosage de chacune desdites cohortes de référence, et de combiner les valeurs de dosage obtenues sur ledit sujet dans ce modèle pour en induire son score fibrotique.

Selon un mode de réalisation de l'invention, le modèle de classification multivariée mis en oeuvre dans la méthode de l'invention est exprimé par une fonction mathématique, linéaire ou non linéaire, plus particulièrement une fonction linéaire (par exemple, un modèle mROC). Le score de fibrose hépatique dudit sujet est alors induit par combinaison desdites valeurs de dosage obtenues pour ledit sujet dans cette fonction mathématique, notamment une fonction linéaire ou non linéaire, pour obtenir une valeur de sortie, plus particulièrement une valeur numérique de sortie, indicatrice du score de fibrose hépatique dudit sujet.

Selon un mode de réalisation de l'invention, le modèle de classification multivariée mis en oeuvre dans la méthode de l'invention est un modèle par apprentissage ou intelligence artificielle, un modèle de classification non paramétrique, ou heuristique, ou de prédiction probabiliste (par exemple, un modèle WKNN, RF ou NN). Le score de fibrose hépatique dudit sujet est alors induit par combinaison desdites valeurs de dosage obtenues pour ledit sujet dans un modèle de classification non paramétrique, ou heuristique, ou de prédiction probabiliste (par exemple, un modèle WKNN, RF ou NN), pour obtenir une valeur de sortie, plus particulièrement une étiquette de sortie, indicatrice du score de fibrose hépatique dudit sujet.

De manière alternative ou complémentaire, ladite comparaison de l'étape ii) peut comprendre le fait de comparer les valeurs des dosages du niveau d'expression desdits gènes choisis obtenues pour ledit sujet, à au moins une valeur de référence qui discrimine entre une fibrose hépatique dont le score fibrotique Metavir est d'au plus F1 et une fibrose hépatique dont le score fibrotique Metavir est d'au moins F2, pour classer le score de fibrose hépatique dudit sujet dans le groupe des scores fibrotiques d'au plus F1 selon le système de scores Metavir, ou dans le groupe des scores fibrotiques d'au moins F2 selon le système de scores Metavir.

Par exemple, les valeurs des dosages du niveau d'expression desdits gènes choisis peuvent être comparées à leurs valeurs de référence dans :

- une sous-population d'individus qui sont de la même espèce que ledit sujet, qui, de préférence, sont infectés par le ou les mêmes virus de l'hépatite que ledit sujet, et qui présentent un score de fibrose hépatique d'au plus F1 selon le système de scores Metavir, et/ou
- une sous-population d'individus de sujets qui sont de la même espèce que ledit sujet, qui, de préférence, sont infectés par le ou les mêmes virus de l'hépatite que ledit sujet, et qui présentent un score de fibrose hépatique d'au moins F2 selon le système de scores Metavir,

ou à une valeur de référence qui représente la combinaison de ces valeurs de référence.

Une valeur de référence peut par exemple être :

- la valeur du dosage du niveau d'expression de chacun desdits gènes choisis dans chacun des individus pour chacune des sous-populations ou cohortes de référence, ou
- un critère de position, par exemple la moyenne ou la médiane, ou un quartile, ou le minimum, ou le maximum de ces valeurs dans chacune de ces sous-populations ou cohortes de référence, ou
- une combinaison de ces valeurs ou moyennes. ou médiane, ou quartile, ou minimum, ou maximum.

La ou les valeurs de référence utilisées doivent permettre de discriminer les différents scores de fibrose hépatique.

Il peut par exemple s'agir d'un seuil de décision ou de prédiction, établi en fonction de la distribution des valeurs de dosage desdits gènes dans chacune desdites sous-populations ou cohortes, et en fonction des niveaux de sensibilité (Se) et de spécificité (Spe) fixés par l'utilisateur (*cf.* Figure 2 et ci-dessous ; $Se = VP / (VP + FN)$ et $Sp = VN / (VN + FP)$, avec VP = le nombre de vrais positifs, FN = le nombre de faux négatifs, VN = le nombre de vrais négatifs, FP = le nombre de faux positifs). Ce seuil de décision ou prédiction peut notamment être un seuil optimal qui attribue un poids équitable à la sensibilité (Se) et à la spécificité (Spe), tel que le seuil maximisant l'indice de Youden (J) défini par $J = Se + Spe -1$.

Alternativement ou complémentairement, il peut y avoir comparaison à plusieurs valeurs de référence. C'est notamment le cas lorsque les valeurs de dosage obtenues pour ledit sujet sont comparées à leurs valeurs dans chacune desdites sous-populations ou cohortes de référence, par exemple à l'aide d'une méthode de classification par apprentissage automatique ou par intelligence artificielle.

Ainsi, la comparaison de l'étape ii) peut par exemple être faite comme suit :

- choisir les niveaux de sensibilité (Se) et spécificité (Spe) que l'on souhaite donner à la méthode,
- établir une fonction mathématique, linéaire ou non linéaire, notamment une fonction mathématique linéaire (par exemple, par la méthode mROC), à partir des valeurs de dosage desdits gènes dans chacune desdites sous-populations ou cohortes, et calculer le seuil de décision ou prédiction associé à cette fonction du fait des choix de niveaux de sensibilité (Se) et spécificité (Spe) faits (par exemple, en calculant le seuil maximisant l'indice de Youden),
- combiner les valeurs de dosage obtenues pour ledit sujet dans cette fonction mathématique, pour obtenir une valeur de sortie, qui, comparée audit seuil de décision ou prédiction, permet d'attribuer un score de fibrose hépatique audit sujet, c'est-à-dire de classer ledit sujet dans celle de ces sous-populations ou cohortes de référence, vis-à-vis de laquelle il a la plus forte probabilité d'appartenance.

L'invention repose notamment sur la démonstration que, lorsqu'ils sont pris en combinaison, les niveaux d'expression de :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1,

sont des biomarqueurs qui donnent une « signature » du score de fibrose hépatique.

La personne du métier disposant d'une combinaison de gènes décrite par l'invention est en mesure de construire un modèle de classification multivariée, notamment un modèle d'analyse statistique multivariée (par exemple une fonction mathématique linéaire ou non linéaire) ou un modèle d'apprentissage ou d'intelligence artificielle (par exemple, un algorithme d'apprentissage ou d'intelligence artificielle), à l'aide de ses connaissances générales dans le domaine des techniques et moyens statistiques, notamment dans le domaine du traitement et de l'interprétation statistiques de données, plus particulièrement de données biologiques.

Un modèle de classification multivariée peut par exemple être construit, notamment préalablement construit, comme suit :

a) pour une population d'individus qui sont de la même espèce que ledit sujet, et qui, de préférence, sont infectés par le ou les mêmes virus de l'hépatite que ledit sujet, déterminer le score de fibrose hépatique de chacun desdits individus de la population, et les classer en sous-populations selon leur score de fibrose hépatique, constituant ainsi des cohortes de référence établies selon leur score de fibrose hépatique ;

b) dans au moins un échantillon préalablement obtenu à partir de chacun desdits individus (la nature de cet échantillon étant de préférence identique à celle de l'échantillon dudit sujet), doser le niveau de transcription ou de traduction de chacun desdits gènes choisis ;

c) faire une comparaison inter-cohorte des valeurs de dosage obtenues à l'étape b), ou de la distribution de ces valeurs de dosage, (par exemple par analyse statistique multivariée), pour construire un modèle de classification multivariée, qui induit une valeur de score de fibrose hépatique (ou une valeur représentative d'un tel score) à partir de la combinaison des niveaux de transcription, ou le cas échéant de traduction, desdits gènes choisis.

Si ledit ou lesdits sujets dont on cherche à déterminer le score de fibrose hépatique présentent cette fibrose du fait d'une affection chronique hépatique particulière connue, par exemple, du fait d'une infection par le virus de l'hépatite C (VHC), on utilise avantageusement des individus de situation clinique comparable. Par exemple, si la fibrose dudit ou desdits sujets dont le score de fibrose hépatique doit être déterminé est exclusivement due à une infection par le virus de l'hépatite C (VHC), on choisit de préférence des individus qui sont infectés par un VHC, et on exclut de préférence les individus dont la fibrose hépatique, ou son évolution, peut être ou avoir été influencée par des facteurs autres que le VHC, tels qu'une (co-)infection par un autre virus (par exemple, virus de la immunodéficience humaine (VIH), virus de l'hépatite B), une consommation d'alcool excessive, une hémochromatose, une hépatite auto-immune, la maladie de Wilson, une déficience en $\alpha$-1 antitrypsine, une angiocholite sclérosante primitive, une cirrhose biliaire primitive. On choisit de préférence des individus qui n'ont pas encore reçu de traitement destiné à traiter leur fibrose hépatique ou son origine. Les individus sont par ailleurs choisis de sorte à constituer une cohorte statistiquement acceptable, ne présentant pas de biais particulier, notamment pas de biais clinique particulier. L'objectif est de construire un modèle de classification multivariée qui soit le plus pertinent possible d'un point de vue statistique.

De préférence, les cohortes ou sous-populations d'individus qui sont utilisées pour mesurer des valeurs de dosage ou déterminer des distributions de valeurs de dosage auxquelles vont être comparées les valeurs de dosage obtenues sur ledit sujet, et/ou pour construire les modèles de classification multivariée, comprennent le plus d'individus possibles.

Si le nombre d'individus est trop faible, la comparaison ou le modèle construit peut ne pas être suffisamment fiable et généralisable en vue des applications médicales visées.

Notamment, on utilisera des cohortes ou sous-populations qui comprennent chacune au moins 30 individus, par exemple au moins 40 individus, de préférence au moins 50 individus, plus particulièrement au moins 70 individus, de manière encore plus particulière au moins 100 individus.

De préférence, un nombre comparable d'individus est présent dans chaque cohorte ou sous-population. Par exemple, le nombre d'individus d'une cohorte ou sous-population ne dépasse pas le seuil de 3 fois le nombre d'individus d'une autre cohorte, plus particulièrement le seuil de 2,5 fois le nombre d'individus d'une autre cohorte.

Lorsque l'analyse statistique menée utilise une fonction mathématique, telle que par exemple dans le cas d'une méthode mROC, le nombre d'individus requis par cohorte peut éventuellement être de l'ordre de 20 à 40 individus par cohorte de référence. Dans le cas d'une méthode d'analyse par apprentissage automatique, telle qu'une méthode KNN, WKNN, RF ou NN, il est préférable d'avoir au moins 30 individus par cohorte, de préférence au moins 70 individus, de manière encore plus particulière au moins 100 individus.

Dans les exemples qui suivent, le nombre total d'individus compris dans l'ensemble des cohortes (cohorte de score F1 et cohorte de score F2) est de plus de 150.

Pour déterminer le score de fibrose hépatique d'un individu, et par suite, affecter cet individu à une cohorte de référence, la personne du métier peut mettre en oeuvre tout moyen qu'elle juge approprié. Par exemple, une ponction biopsie hépatique (PBH) peut être pratiquée sur ledit individu, le tissu hépatique ainsi prélevé peut alors être analysé par examen anatomo-pathologique, pour déterminer le score de fibrose hépatique de cet individu (par exemple, au plus F1 ou au moins F2). Comme les scores de chaque individu sont utilisés comme base d'analyse statistique, et non comme diagnostic individuel de l'individu, les moyens de mesure de score utilisés peuvent éventuellement être les moyens de l'art antérieur, tels que le test Fibrotest®, Fibrometrer® ou Hepascore®. Il est néanmoins préféré d'utiliser l'examen anatomo-pathologique d'un échantillon de PBH, car, contrairement aux tests Fibrotest®, Fibrometrer® ou Hepascore®, cet examen est capable de discriminer un score de fibrose hépatique d'au plus F1 d'un score d'au moins F2.

Bien qu'il faille bien sûr limiter le nombre d'échantillons pris sur un même individu, notamment en cas de ponction biopsie hépatique, plusieurs échantillons peuvent être collectés sur un même individu. Dans ce cas, les résultats de dosage des différents échantillons d'un même individu sont considérés dans leur moyenne résultante ; il n'est pas considéré qu'ils puissent être équivalents à des valeurs de dosage obtenues sur des individus distincts.

La comparaison des valeurs de dosage des niveaux d'expression desdits gènes choisis dans chacune desdites cohortes peut se faire par tout moyen connu de la personne du métier. Elle se fait généralement par traitement et interprétation statistiques des valeurs de dosage des niveaux d'expression desdits gènes choisis, qui ont été mesurées pour chacune desdites cohortes. Cette comparaison statistique multivariée permet de construire un modèle de classification multivariée qui induit une valeur de score de fibrose hépatique à partir de la combinaison des niveaux d'expression desdits gènes choisis, plus particulièrement un modèle de classification multivariée qui utilise une combinaison des niveaux d'expression desdits gènes choisis pour discriminer selon le score de fibrose hépatique.

Une fois que ledit modèle de classification multivariée est construit, il peut être utilisé pour l'analyse des valeurs de dosage obtenues pour ledit sujet, et surtout être ré-utilisé pour l'analyse du dosage d'autres sujets ultérieurs. Ainsi, le ledit modèle de classification multivariée peut être établi de manière indépendante du dosage effectué pour ledit ou lesdits sujets, et peut être préalablement construit.

Si besoin en était, plutôt que de constituer des cohortes et réunir les données des individus qui les constituent, la personne du métier peut, pour construire des exemples de modèles de classification multivariée conformes à l'invention, utiliser, à titre d'individus de cohortes, les sujets qui sont décrits en partie exemples ci-dessous, et peut, à titre de données d'individus de cohortes (en l'occurrence, cohortes F1 et F2), utiliser les données qui sont présentées pour ces sujets dans les exemples ci-dessous, plus particulièrement :

- en tableau 22 et/ou en tableau 23, qui présentent les valeurs de dosage d'un groupe de 20 patients (10 patients F1 et 10 patients F2) pour les gènes A2M, CXCL10, IL8, SPP1, VIM ; et/ou
- en tableau 25 et/ou en tableau 26 et/ou en tableau 27 et/ou en tableau 28 ci-dessous, qui présentent les valeurs de dosage d'un groupe de 158 patients (102 patients F1 et 56 patients F2) pour chacun des gènes qui peuvent être choisis conformément à l'invention.

Il est préférable d'utiliser les données des tableaux 25 et/ou 26 et/ou 27 et/ou 28, qui portent sur un groupe de 158 patients, plutôt que de n'utiliser que celles des tableaux 22 et/ou 23, qui ne portent que sur 20 patients.

Pour les 158 patients dont les valeurs de dosage des niveaux d'expression de tous les gènes susceptibles d'être choisis conformément à l'invention sont présentées en tableaux 25, 26, 27 et 28 ci-dessous, les valeurs de facteurs cliniques, de facteurs virologiques et de facteurs biologiques autres que les niveaux d'expression desdits gènes choisis sont en outre présentées en tableau 24 ci-dessous.

De préférence, ledit modèle de classification multivariée est un système particulièrement discriminant. Avantageusement, ledit modèle de classification multivariée présente une aire sous la courbe ROC (ou AUC) et/ou une valeur d'erreur LOOCV particulière(s). L'acronyme « AUC » désigne *Area Under the Curve*, c'est-à-dire l'aire sous la courbe ROC (*Receiver Operating Characteristic*). L'acronyme « LOOCV » désigne Leave-One-Out-Cross-Validation, cf. Hastie, Tibishirani et Friedman, 2009.

La caractéristique d'AUC vient notamment s'appliquer aux modèles de classification multivariée, qui sont définis par une fonction mathématique, comme par exemple les modèles utilisant une méthode mROC de classification.

Les modèles d'intelligence artificielle ou d'apprentissage automatique multivariés ne sont quant à eux pas à proprement parler définis par une fonction mathématique. Néanmoins, dès lors qu'ils impliquent un seuil décisionnel (*threshold*), ils peuvent être appréhendés au moyen d'une courbe ROC, et donc d'un calcul d'AUC. C'est par exemple le cas des modèles utilisant une méthode RF (*Random Forest*). En effet, dans le cas de la méthode RF, une courbe ROC peut être calculée à partir des prédictions des échantillons OOB (*Out-Of-Bag*, ou « hors-du-sac » selon la traduction en langue française).

Ceux des modèles d'intelligence artificielle ou d'apprentissage automatique multivariés, qui ne pourraient être caractérisés par une valeur d'AUC, peuvent par contre, comme tous les autres modèles d'intelligence artificielle ou d'apprentissage automatique multivariés, être caractérisés par la valeur du paramètre « erreur de classification » qui leur est associée, telle que par exemple la valeur de l'erreur LOOCV.

Ladite valeur particulière d'AUC peut notamment être d'au moins 0,60, d'au moins 0,61, d'au moins 0,66, plus particulièrement d'au moins 0,69, d'au moins 0,70, d'au moins 0,71, d'au moins 0,72, d'au moins 0,73, d'au moins 0,74, encore plus particulièrement d'au moins 0,75, encore plus particulièrement d'au moins 0,76, encore plus particulièrement d'au moins 0,77, notamment d'au moins 0,78, d'au moins 0,79, d'au moins 0,80 (de préférence, avec un intervalle de confiance à 95% à au plus ± 11%, plus particulièrement à moins de ± 10,5%, encore plus particulièrement à moins de ± 9,5%, notamment à moins de ± 8,5%) ; *cf.* par exemple, tableaux 5, 7, 11, 13 ci-dessous.

Avantageusement, ladite valeur particulière d'erreur LOOCV est d'au plus 30%, au plus 29%, au plus 25%, au plus 20%, au plus 18%, au plus 15%, au plus 14%, au plus 13%, au plus 12%, au plus 11%, au plus 10%, au plus 9%, au plus 8%, au plus 7%, au plus 6%, au plus 5%, au plus 4%, au plus 3%, au plus 2%, au plus 1%.

Les performances diagnostiques d'un biomarqueur sont généralement caractérisées selon au moins un des deux indices suivants :

- la sensibilité (Se), qui représente sa capacité à détecter la population dite "pathologique" constituée d'individus dits "cas" (en l'occurrence, les patients dont le score de fibrose hépatique est F2 ou plus) ;
- la spécificité (Sp ou Spe), qui représente sa capacité à détecter la population dite "saine", constituée des patients dits "contrôles" (en l'occurrence, les patients dont le score de fibrose hépatique est F1 ou moins).

Lorsqu'un biomarqueur génère des valeurs continues (par exemple, des valeurs de concentration), différentes positions de seuil prédictif (*Prediction Threshold* ou PT) peuvent être définies afin d'assigner un échantillon à la classe positive (test positif : $y = 1$). La comparaison de la concentration du biomarqueur à la valeur PT permet de classer le sujet dans la cohorte à laquelle il a la plus forte probabilité d'appartenance.

Par exemple, si l'on considère une cohorte d'individus qui présentent un score fibrotique d'au moins F2 et une cohorte d'individus un score fibrotique d'au plus F1, et si l'on considère un sujet ou patient p, dont le stade clinique doit être déterminé, pour lequel la valeur de la combinaison des niveaux d'expression desdits gènes choisis est V (V étant égal à Z dans le cas des modèles mROC), la règle décisionnelle est la suivante :

- lorsque la valeur moyenne de la combinaison des niveaux d'expression desdits gènes choisis dans la cohorte des individus "F2 ou plus" est supérieure à celle de la cohorte des individus "F1 ou moins" (*cf.* graphe du haut de la Figure 2) :

  - si V ≥ PT : le test est positif, un score fibrotique "F2 ou plus" est assigné audit patient p,
  - si V < PT : le test est négatif, un score fibrotique "F1 ou moins" est assigné audit patient p,

  ou
- lorsque la valeur moyenne de la combinaison des niveaux d'expression desdits gènes choisis dans la cohorte des individus "F2 ou plus" est inférieure à celle de la cohorte des individus "F1 ou moins" :

  - si V ≤ PT : le test est positif, un score fibrotique "F2 ou plus" est assigné audit patient p,
  - si V > PT : le test est négatif, un score fibrotique "F1 ou moins" est assigné audit patient p.

Comme la combinaison de biomarqueurs de l'invention est effectivement discriminante, les distributions, supposées gaussiennes, de la combinaison de biomarqueurs dans chaque population d'intérêt (par exemple, dans la cohorte "F2 ou plus" et dans la cohorte "F1 ou moins") se différencient nettement. Il convient alors de définir la valeur seuil optimale qui conférera à cette combinaison de biomarqueurs les meilleures performances diagnostiques. En effet, pour un seuil donné PT, les valeurs suivantes peuvent être calculées (*cf.* Figure 2) :

- le nombre de vrais positifs : VP ;
- le nombre de faux négatifs : FN ;
- le nombre de faux positifs : FP ;

- le nombre de vrais négatifs : VN.

Les calculs des paramètres de sensibilité (Se) et spécificité (Sp) se déduisent des formules suivantes :

$$Se = VP / (VP + FN) ;$$

$$Sp = VN / (VN + FP).$$

La sensibilité peut donc être considérée comme étant la probabilité que le test soit positif sachant que le score F Metavir de sujet testé est au moins F2 ; et la spécificité peut être considérée comme étant la probabilité que le test soit négatif sachant que le score F Metavir de sujet testé est au plus F1.

Une courbe ROC peut être utilisée pour visualiser le pouvoir prédictif du biomarqueur (ou pour l'approche multivariée, le pouvoir prédictif de la combinaison de biomarqueurs intégrés dans le modèle) pour différentes valeurs PT (Swets 1988). Chaque point de la courbe représente la Sensibilité versus (1-Spécificité) pour une valeur spécifique PT.

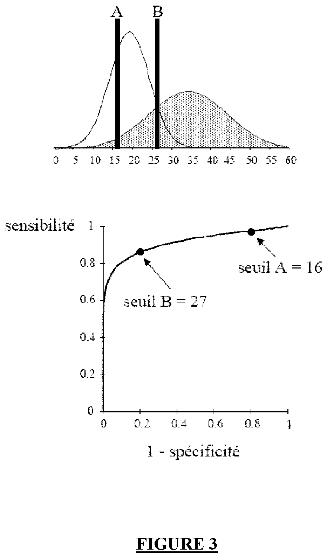

Par exemple, si les concentrations du biomarqueur d'intérêt varient de 0 à 35, différentes valeurs PT peuvent être successivement positionnées à 0,5 ; 1 ; 1,5 ; ... ; 35. Ainsi, pour chaque valeur PT, les échantillons testés sont classifiés, la sensibilité et la spécificité sont calculées et les points résultants sont reportés sur un graphique (*cf.* Figure 3).

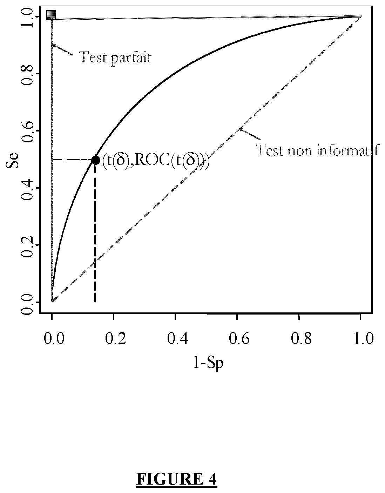

Plus la courbe ROC est proche de la première diagonale (droite reliant le coin bas gauche au coin haut droit), plus les performances discriminantes du modèle sont pauvres (*cf.* Figure 4). Un test à fort pouvoir discriminant occupera la partie supérieure gauche du graphique. Un test peu discriminant avoisinera la première diagonale du graphique. L'aire sous la courbe ROC (AUC) est un bon indicateur de performance diagnostique. Celle-ci varie de 0,5 (biomarqueur non discriminant) à 1 (biomarqueur parfaitement discriminant). La valeur 0,70 induit un biomarqueur discriminant.

Une courbe ROC peut être approximée par deux principales techniques : paramétrique et non paramétrique (Shapiro 1999). Dans le premier cas, les données sont supposées suivre une distribution statistique spécifique (par exemple, gaussienne), qui est alors ajustée aux données observées pour produire une courbe ROC lissée. Des approches non paramétriques considèrent l'estimation de Se et (1-Sp) à partir des données observées. La courbe ROC empirique résultante n'est pas une fonction mathématique lissée, mais une courbe constante par morceaux.

Le choix du seuil ou du seuil optimal, noté $\delta$ (delta), dépend des priorités de l'utilisateur en termes de sensibilité et spécificité. Dans le cas de poids équitables attribués aux sensibilité et spécificité, ce dernier peut être défini comme le seuil maximisant l'indice de Youden (J = Se + Sp - 1).

Avantageusement, les moyens de l'invention permettent d'atteindre :

- une sensibilité [Se = VP / (VP + FN)] d'au moins 67% (ou plus), et/ou
- une spécificité [Sp = VN / (VN + FP)] d'au moins 67% (ou plus).

Dans le cadre de l'invention, la sensibilité est une caractéristique particulièrement importante, dans la mesure où le besoin clinique prioritaire est l'identification des patients dont le score F Metavir est d'au moins F2.

Par suite, et de manière avantageuse, l'invention est plus particulièrement relative aux moyens de l'invention qui atteignent, ou permettent d'atteindre, une sensibilité de 67% ou plus.

Plus particulièrement, les moyens de l'invention atteignent, ou permettent d'atteindre, une sensibilité de 67% ou plus et une spécificité de 67% ou plus.

C'est la sélection particulière de gènes proposée par l'invention qui permet d'atteindre ces scores de sensibilité et/ou spécificité, plus particulièrement ces scores de sensibilité, de manière encore plus particulière ces scores de sensibilité et spécificité.

Ainsi, selon un mode de réalisation avantageux de l'invention, le score de fibrose hépatique dudit sujet est induit :

- avec une sensibilité d'au moins 67% (ou plus) et/ou une spécificité d'au moins 67% (ou plus),
- plus particulièrement avec une sensibilité d'au moins 67% (ou plus),
- de manière encore plus particulière avec une sensibilité d'au moins 67% (ou plus) et une spécificité d'au moins 67% (ou plus).

Conformément à l'invention, la sensibilité peut être d'au moins 67%, d'au moins 68%, d'au moins 69%, d'au moins 70%, d'au moins 71%, d'au moins 72%, d'au moins 73%, d'au moins 74%, d'au moins 75% (*cf.*, par exemple, gènes choisis des combinaisons n° 1 à 29 du tableau 3 ci-dessous, plus particulièrement les caractéristiques de sensibilité des combinaisons des niveaux de transcription ou de traduction de ces gènes présentées en tableaux 5, 7, 11 et 13 ci-dessous).

Alternativement ou complémentairement, la spécificité peut être d'au moins 67%, d'au moins 68%, d'au moins 69%, d'au moins 70%, d'au moins 71%, d'au moins 72%, d'au moins 73%, d'au moins 74%, d'au moins 75% (*cf.*, par exemple, gènes choisis des combinaisons n° 1 à 29 du tableau 3 ci-dessous, plus particulièrement les caractéristiques de spécificité des combinaisons des niveaux de transcription ou de traduction de ces gènes présentées en tableaux 5, 7, 11 et 13 ci-dessous).

Toutes les combinaisons de ces seuils de sensibilité et de ces seuils de spécificité sont explicitement incluses dans le contenu de la description de l'invention(*cf.*, par exemple, gènes choisis des combinaisons n° 1 à 29 du tableau 3 ci-dessous).

Par exemple, la sensibilité peut être d'au moins 71%, d'au moins 73%, ou d'au moins 75%, et la spécificité d'au moins 70% ou un seuil plus élevé (*cf.*, par exemple, gènes choisis des combinaisons n° 1, 4, 7, 9 à 11, 13, 14, 16, 18, 19, 20 à 24, 26, 27 et 29 du tableau 3 ci-dessous, plus particulièrement les caractéristiques de sensibilité et de spécificité des combinaisons n° 1, 4, 7, 9 à 11, 13, 14, 18 à 24, 26 à 27, 29 de niveaux de transcription présentées en tableau 5 ci-dessous, et les caractéristiques de sensibilité et de spécificité des combinaisons n° 4 et 16 de niveaux de transcription présentées en tableau 11 ci-dessous).

Plus particulièrement, toutes les combinaisons comprenant au moins la combinaison d'un seuil de sensibilité et d'un seuil de spécificité sont explicitement incluses dans le contenu de la description de l'invention.

Alternativement ou complémentairement à ces caractéristiques de sensibilité et/ou spécificité, les Valeurs de Prédiction Négative (VPN) atteintes, ou susceptibles d'être atteintes, par les moyens de l'invention sont particulièrement élevées.

La VPN est égale à VN / (VN+FN), avec VN = Vrais Négatifs et FN = Faux Négatifs, et représente donc la probabilité que le sujet testé soit au plus F1, sachant que le test de l'invention est négatif (le résultat donné par le test est : score de F1 ou moins). Conformément à l'invention, la VPN peut être d'au moins 80%, d'au moins 81%, d'au moins 82%, d'au moins 83%, d'au moins 84% (*cf.*, par exemple, gènes choisis des combinaisons n° 1 à 29 du tableau 3 ci-dessous, plus particulièrement les caractéristiques de VPN des combinaisons des niveaux de transcription ou de traduction de ces gènes présentées en tableaux 5, 7, 11, 13 ci-dessous).

Ici encore, c'est la sélection particulière de gènes proposée par l'invention qui permet d'atteindre ces niveaux de VPN.

Par exemple, les moyens de l'invention atteignent, ou permettent d'atteindre :

- une sensibilité d'au moins 71%, d'au moins 73%, ou d'au moins 75%, et
- une spécificité d'au moins 70% (ou un seuil plus élevé), et/ou une VPN d'au moins 81% ou d'au moins 82%.

(*cf.*, par exemple, gènes choisis des combinaisons n° 1, 4, 7, 9 à 11, 13, 14, 16, 18, 19, 20 à 24, 26, 27 et 29 du tableau 3 ci-dessous, plus particulièrement les caractéristiques de sensibilité, spécificité et de VPN des combinaisons n° 1, 4, 7, 9 à 11, 13, 14, 18, 19, 20 à 24, 26, 27 et 29 de niveaux de transcription présentées en tableau 5 ci-dessous, et les caractéristiques de sensibilité, de spécificité et de VPN des combinaisons n° 4 et 16 de niveaux de transcription présentées en tableau 11 ci-dessous).

Plus particulièrement, les moyens de l'invention atteignent, ou permettent d'atteindre :

- une sensibilité d'au moins 73%, et
- une spécificité d'au moins 70% (ou un seuil plus élevé), et/ou une VPN d'au moins 81% ou d'au moins 82%,

(*cf.*, par exemple, gènes choisis des combinaisons n° 1, 4, 7, 10, 13, 19, 21, 23 du tableau 3 ci-dessous, plus particulièrement les caractéristiques de sensibilité, spécificité et VPN de la combinaison des niveaux de transcription de ces gènes présentées en tableaux 5 et 11 ci-dessous).

Plus particulièrement, les moyens de l'invention atteignent, ou permettent d'atteindre :

- une sensibilité d'au moins 75%, et
- une spécificité d'au moins 70% (ou un seuil plus élevé), et/ou une VPN d'au moins 81% ou d'au moins 82%,

(*cf.*, par exemple, gènes choisis des combinaisons n° 1, 4, 7 et 13 du tableau 3 ci-dessous, plus particulièrement les caractéristiques de sensibilité, spécificité et VPN de la combinaison des niveaux de transcription de ces gènes présentées en tableaux 5 et 11 ci-dessous).

Toutes les combinaisons de seuils de VPN et/ou de seuils de sensibilité et/ou de seuils de spécificité sont explicitement incluses dans le contenu de la description de l'invention. Plus particulièrement, toutes les combinaisons comprenant au moins la combinaison d'un seuil de sensibilité et d'un seuil de VPN sont explicitement incluses dans le contenu de la description de l'invention.

Alternativement ou complémentairement à ces caractéristiques de sensibilité et/ou spécificité et/ou de VPN, les Valeurs de Prédiction Positive (VPP) atteintes, ou susceptibles d'être atteintes, par les moyens de l'invention sont particulièrement élevées. La VPP est égale à VP / (VP + FP) avec VP = les Vrais Positifs et FP = Faux Positifs, et représente donc la probabilité que le sujet testé soit au moins F2, sachant que le test de l'invention est positif (le résultat du test est : score de F2 ou plus).

Conformément à l'invention, la VPP peut être d'au moins 50%, ou d'au moins 55%, ou d'au moins 56%, ou d'au moins 57%, ou d'au moins 58%, ou d'au moins 59% ou d'au moins 60% (*cf.*, par exemple, gènes choisis des combinaisons n° 1 à 29 du tableau 3 ci-dessous, plus particulièrement les caractéristiques de VPP des combinaisons des niveaux de transcription ou de traduction de ces gènes présentées en tableaux 5, 7, 11, 13 ci-dessous).

Ici encore, c'est la sélection particulière de gènes proposée par l'invention qui permet d'atteindre ces niveaux de VPP.

Par exemple, les moyens de l'invention atteignent, ou permettent d'atteindre :

- une sensibilité d'au moins 71%, d'au moins 73%, ou d'au moins 75%, et
- une spécificité d'au moins 70% (ou un seuil plus élevé), et/ou une VPN d'au moins 81% ou d'au moins 82%, et/ou une VPP d'au moins 55%, ou d'au moins 57%,

(*cf.*, par exemple, gènes choisis des combinaisons n° 1, 4, 7, 9 à 11, 13, 14, 16, 18, 19, 20 à 24, 26, 27 et 29 du tableau 3 ci-dessous, plus particulièrement les caractéristiques de sensibilité, spécificité, VPN et VPP des combinaisons n° 1, 4, 7, 9 à 11, 13, 14, 18, 19, 20 à 24, 26, 27 et 29 de niveaux de transcription présentées en tableau 5 ci-dessous, et les caractéristiques de sensibilité, de spécificité, VPN et VPP des combinaisons n° 4 et 16 de niveaux de transcription présentées en tableau 11 ci-dessous).

Plus particulièrement, les moyens de l'invention atteignent, ou permettent d'atteindre :

- une sensibilité d'au moins 73%, et
- une spécificité d'au moins 70% (ou un seuil plus élevé), et/ou une VPN d'au moins 81% ou d'au moins 82%, et/ou une VPP d'au moins 57%,

(*cf.*, par exemple, gènes choisis des combinaisons n° 1, 4, 7, 10, 13, 19, 21, 23 du tableau 3 ci-dessous, plus particulièrement les caractéristiques de sensibilité, de spécificité, VPN et VPP, de la combinaison des niveaux de transcription de ces gènes présentées en tableaux 5 et 11 ci-dessous).

Plus particulièrement, les moyens de l'invention atteignent, ou permettent d'atteindre :

- une sensibilité d'au moins 75%, et
- une spécificité d'au moins 70% (ou un seuil plus élevé), et/ou une VPN d'au moins 81% ou d'au moins 82%, et/ou une VPP d'au moins 57%,

(*cf.*, par exemple, gènes choisis des combinaisons n° 1, 4, 7 et 13 du tableau 3 ci-dessous, plus particulièrement les caractéristiques de sensibilité, de spécificité, VPN et VPP de la combinaison des niveaux de transcription de ces gènes présentées en tableaux 5 et 11 ci-dessous).

Toutes les combinaisons de seuils de VPP et/ou VPN et/ou de seuils de sensibilité et/ou de seuils de spécificité sont explicitement incluses dans le contenu de la description de l'invention.

Plus particulièrement, toutes les combinaisons comprenant au moins la combinaison d'un seuil de sensibilité et d'un seuil de VPP sont explicitement incluses dans le contenu de la description de l'invention.

Plus particulièrement, toutes les combinaisons comprenant au moins un desdits seuils de VPN et/ou au moins un desdits seuils de sensibilité, plus particulièrement au moins un desdits seuils de VPN et un desdits seuils de sensibilité, plus particulièrement au moins un desdits seuils de VPN et un desdits seuils de sensibilité et un desdits seuils de spécificité, sont incluses dans la description de l'invention.

Les tableaux 5, 7, 11 et 13 présentés ci-dessous donnent des illustrations :

- de valeurs d'aire sous la courbe ROC (AUC),
- de valeurs de sensibilité et/ou spécificité, plus particulièrement de sensibilité, de manière encore plus particulière de sensibilité et spécificité,
- de valeurs de Valeur Prédictive Négative (VPN) et/ou Valeur Prédictive Positive (VPP), plus particulièrement de VPN, de manière encore plus particulière de VPN et VPP,

atteintes par des combinaisons de gènes conformes à l'invention (tableaux 5 et 11 : combinaisons de niveaux de transcription ; tableaux 7 et 13 : combinaisons de niveaux de traduction).

Les combinaisons prédictives de l'invention comprennent des combinaisons des niveaux d'expression de gè-

EP 2 673 375 B1

nes, choisis comme ci-dessus indiqué.

Comme indiqué plus en détail ci-dessous, et comme illustré dans les exemples ci-dessous (*cf.* exemples 2c, 2d, 3b) ci-dessous), il peut néanmoins être choisi de faire intervenir dans ces combinaisons un ou plusieurs facteurs autres que les niveaux d'expression de ces gènes, pour combiner cet ou ces autres facteurs et les niveaux d'expression des gènes choisis en une règle décisionnelle.

Cet ou ces autres facteurs sont préférablement choisis de sorte à construire un modèle de classification dont le pouvoir prédictif est encore amélioré par rapport au modèle qui ne comprendrait pas cet ou ces autres facteurs.

En sus du niveau d'expression desdits gènes choisis, on peut également mesurer ou doser un ou plusieurs autres facteurs, tels qu'un ou plusieurs facteurs cliniques et/ou un ou plusieurs facteurs virologiques et/ou un ou plusieurs facteurs biologiques autres que le niveau d'expression desdits gènes choisis.

La(les) valeur(s) de cet(ces) autre(s) facteur(s) peuvent alors être prises en compte pour construire le modèle de classification multivariée, et peuvent ainsi conduire à des performances de classification encore améliorées, plus particulièrement à des caractéristiques de sensibilité et/ou spécificité et/ou VPN et/ou VPP augmentées.

Par exemple, si l'on compare les valeurs présentées pour la combinaison n°16 ou n°4 en tableaux 5 et 11 ci-dessous, on constate que plusieurs des valeurs d'AUC, Se, Spe, VPN et VPP, plus particulièrement les valeurs d'AUC, Se, VPN, augmentent lorsqu'à la combinaison des niveaux de transcription desdits gènes choisis, sont outre combinés d'autres facteurs, notamment d'autres facteurs biologiques.

De même, si l'on compare les valeurs présentées pour la combinaison n°16 en tableaux 7 et 13 ci-dessous, on constate que plusieurs des valeurs d'AUC, Se, Spe, VPN et VPP, plus particulièrement les valeurs les valeurs d'AUC, Spe et VPN, augmentent lorsqu'à la combinaison des niveaux de traduction desdits gènes choisis, sont outre combinés d'autres facteurs, notamment d'autres facteurs biologiques.

Avantageusement, lorsque qu'un ou plusieurs autres facteurs sont combinés à une combinaison de gènes choisis parmi ladite liste de vingt-deux gènes de l'invention, au moins l'une des caractéristiques d'AUC (le cas échéant, d'erreur LOOCV), de sensibilité, de spécificité, de VPN et de VPP, s'en trouve améliorée.

Selon un mode de réalisation de l'invention, la valeur particulière d'AUC associée à une telle combinaison améliorée est d'au moins 0,70, d'au moins 0,71, d'au moins 0,72, d'au moins 0,73, plus particulièrement d'au moins 0,74, encore plus particulièrement d'au moins 0,75, encore plus particulièrement d'au moins 0,76, encore plus particulièrement d'au moins 0,77, notamment d'au moins 0,78, d'au moins 0,79, d'au moins 0,80 (de préférence, avec un intervalle de confiance à 95% à au plus $\pm$ 11%, plus particulièrement à moins de $\pm$ 10,5%, encore plus particulièrement à moins de $\pm$ 9,5%, notamment à moins de $\pm$ 8,5%) ; *cf.* par exemple, tableaux 5, 11 et 13 ci-dessous.

Selon un mode de réalisation de l'invention, la valeur de seuil de spécificité associée à telle combinaison améliorée est d'au moins 70%, d'au moins 71%, d'au moins 72%, d'au moins 73%, d'au moins 74%, d'au moins 75% (*cf.*, par exemple, gènes choisis des combinaisons n° 1 à 29 du tableau 3 ci-dessous, plus particulièrement les caractéristiques de spécificité des combinaisons des niveaux de transcription de ces gènes présentées en tableaux 11 et 13 ci-dessous).

Comme précédemment indiqué, et comme illustré ci-dessous, les moyens de l'invention font intervenir le dosage du niveau d'expression de :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

Conformément à l'invention, le nombre total de gènes ainsi choisis dont le niveau d'expression est dosé est donc d'au moins trois.

Selon un mode de réalisation de l'invention, ce nombre total de gènes ainsi choisis est de 3, 4, 5, 6, 7, 8, 9, 10, 11, 12, 13, 14, 15, 16, 17, 18, 19, 20, 21 ou 22, plus particulièrement de 3, 4, 5, 6, 7, 8, 9, 10, de manière plus particulière de 3, 4, 5, 6, 7, de manière encore plus particulière de 3, 4, 5 ou 6. Avantageusement, ce nombre de gènes choisis est de 3, 4 ou 5, notamment de 4 ou 5 (*cf.*, par exemple, gènes choisis des combinaisons n°1 à 29 du tableau 3 ci-dessous).

Selon un mode de réalisation, le nombre total de gènes choisis parmi ladite liste de vingt-deux gènes de l'invention est de 3, 4, 5 ou 6 gènes, plus particulièrement de 4 ou 5 gènes, avec:

- une sensibilité d'au moins 70%, d'au moins 71%, d'au moins 72%, d'au moins 73%, d'au moins 74%, d'au moins 75% ; et/ou avec
- une spécificité d'au moins 70%, d'au moins 71%, d'au moins 72%, d'au moins 73%, d'au moins 74%, d'au moins 75% ; et/ou avec

- une VPN d'au moins 80%, d'au moins 81%, d'au moins 82%, d'au moins 83%, d'au moins 84% ;

(*cf.*, par exemple, gènes choisis des combinaisons n° 1 à 29 du tableau 3 ci-dessous).

Par exemple, l'invention vise un nombre de 3, 4, 5 ou 6 gènes choisis parmi ladite liste de vingt-deux gènes de l'invention, plus particulièrement de 4 ou 5 gènes choisis parmi ladite liste de vingt-deux gènes de l'invention, avec:

- une sensibilité d'au moins 70%, d'au moins 71%, d'au moins 72%, d'au moins 73%, d'au moins 74%, d'au moins 75% ; et/ou avec
- une spécificité d'au moins 70%, d'au moins 71%, d'au moins 72%, d'au moins 73%, d'au moins 74%, d'au moins 75% ;

plus particulièrement, un nombre de 3, 4, 5 ou 6 gènes choisis parmi ladite liste de vingt-deux gènes de l'invention, plus particulièrement de 4 ou 5 gènes choisis parmi ladite liste de vingt-deux gènes de l'invention, avec une sensibilité d'au moins 70%, d'au moins 71%), d'au moins 72%, d'au moins 73%, d'au moins 74%, d'au moins 75% (*cf.*, par exemple, gènes choisis des combinaisons n° 1 à 29 du tableau 3 ci-dessous).

Toutes les combinaisons de nombre total de gènes choisis et/ou de seuil de sensibilité et/ou de seuil de spécificité et/ou de seuil de VPN et/ou de seuil de VPP ci-dessus indiquées sont explicitement incluses dans le contenu de l'invention.

Plus particulièrement, le nombre total de gènes choisis parmi ladite liste de vingt-deux gènes de l'invention est de 3, 4, 5 ou 6 gènes, plus particulièrement de 4 ou 5 gènes, avec:

- une sensibilité d'au moins 73% ; et/ou avec
- une spécificité d'au moins 70% ; et/ou avec
- une VPN d'au moins 83% ;

(*cf.*, par exemple, gènes choisis des combinaisons n° 1, 4, 7, 10, 13, 19, 21, 23 du tableau 3 ci-dessous).

Plus particulièrement, le nombre total de gènes choisis parmi ladite liste de vingt-deux gènes de l'invention est de 3, 4, 5 ou 6 gènes, plus particulièrement de 4 ou 5 gènes, avec:

- une sensibilité d'au moins 75% ; et/ou avec
- une spécificité d'au moins 70% ; et/ou avec
- une VPN d'au moins 83% ;

(*cf.*, par exemple, gènes choisis des combinaisons n° 1, 4, 7, 13 du tableau 3 ci-dessous).

Les gènes qui sont choisis conformément à l'invention sont :

- SPP1, et
- au moins un gène parmi A2M et VIM, et
- au moins un gène parmi IL8, CXCL10 et ENG, et
- optionnellement, au moins un gène parmi les seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

Le choix de gènes est fait selon les exigences ou souhaits de performances à atteindre, par exemple en fonction de la sensibilité et/ou spécificité et/ou VPN et/ou VPP que l'on souhaite ou désire atteindre. Bien entendu, plus le nombre de gènes choisis est faible, plus la mise en oeuvre des moyens de l'invention est simple.

Tous les choix possibles de gènes sont explicitement inclus dans l'invention.

De manière similaire à ce qui a été indiqué ci-dessus pour les seuils de sensibilité, les seuils de spécificité, les seuils de VPN, les seuils de VPP, et le nombre total de gènes choisis, toutes les combinaisons de gènes choisis dans chacune des listes de gènes et/ou de nombres totaux de gènes choisis et/ou de seuils de sensibilité et/ou de seuils de spécificité et/ou de seuils de VPN et/ou de seuils de VPP sont explicitement incluses dans le contenu de l'invention.

Comme les gènes choisis parmi ladite liste de vingt-deux gènes de l'invention sont :

- SPP1, et
- au moins un gène parmi une première liste de gènes formée par A2M et VIM, et
- au moins un gène parmi une deuxième liste de gènes formée par IL8, CXCL10 et ENG, et
- optionnellement, au moins un gène parmi une troisième liste de gènes formée par les seize gènes suivants : IL6ST, pl4ARF, MMP9, ANGPT2, CXCL11, MMP2, MMP7, S100A4, TIMP1, CHI3L1, COL1A1, CXCL1, CXCL6, IHH, IRF9, MMP1,

on peut donc, en sus de SPP1, choisir :

- un ou deux gènes parmi A2M et VIM (première liste de gènes), et
- un, deux ou trois gènes parmi IL8, CXCL10 et ENG (deuxième liste de gènes), et
- de zéro à seize gènes, par exemple, zéro, un, deux ou trois gènes, notamment zéro, un ou deux gènes parmi ladite troisième liste de seize gènes (liste optionnelle).

Alternativement ou complémentairement, on choisit :

- zéro, un, deux ou trois gènes, plus particulièrement zéro, un ou deux gènes, parmi la liste des seize gènes optionnels ; et/ou
- un nombre total de gènes choisis de quatre ou cinq gènes ;

*cf.* par exemple, les combinaisons n°1 à 29 du tableau 3 ci-dessous.

Avantageusement, on choisit :

- SPP1, et
- un ou deux gènes parmi A2M et VIM, plus particulièrement au moins A2M, et
- un, deux ou trois gènes parmi IL8, CXCL10 et ENG, plus particulièrement au moins IL8, et
- zéro, un, deux ou trois gènes parmi ladite liste optionnelle de seize gènes, plus particulièrement zéro, un ou deux gènes parmi cette liste.

Parmi les gènes de la première liste, on peut choisir A2M et/ou VIM. On peut donc choisir :

- A2M, ou
- VIM, ou
- A2M et VIM,

par exemple, au moins A2M, c'est-à-dire :

- A2M, ou
- A2M et VIM ;

*cf.* par exemple, les combinaisons n°1 à 28 du tableau 3 ci-dessous.

Alternativement ou complémentairement, parmi les gènes de la deuxième liste, on peut choisir IL8 et/ou CXCL10 et/ou ENG. Avantageusement, on choisit au moins IL8, c'est-à-dire :

- IL8, ou
- IL8 et CXCL10, ou
- IL8 et ENG, ou
- IL8 et CXCL10 et ENG ;

*cf.* par exemple, les combinaisons n°1 à 18 et 22 à 29 du tableau 3 ci-dessous.

Selon un mode de réalisation de l'invention, on choisit au moins A2M dans la première liste comme ci-dessus indiqué et/ou au moins IL8 dans la deuxième liste comme ci-dessus indiqué (*cf.* par exemple, les combinaisons n°1 à 29 du tableau 3 ci-dessous).

Alternativement ou complémentairement, parmi les gènes de la troisième liste, c'est-à-dire parmi la liste des seize gènes optionnels, on peut notamment choisir zéro, un, deux ou trois gènes, plus particulièrement zéro, un ou deux gènes.

Plus particulièrement, on peut choisir zéro, un, deux ou trois gènes, notamment zéro, un ou deux gènes parmi IL6ST, MMP9, S100A4, p14ARF, CHI3L1.

Selon un mode de réalisation de l'invention, on choisit :

- au moins A2M dans la première liste, et/ou au moins IL8 dans la deuxième liste, et
- zéro gène parmi la troisième liste, c'est-à-dire parmi la liste des seize gènes optionnels, ou un ou plusieurs gènes parmi cette liste des seize gènes optionnels, parmi lesquels au moins un ou deux gènes parmi IL6ST, MMP9, S100A4, p14ARF, CHI3L1 (par exemple, un ou deux de ces gènes), plus particulièrement au moins un ou deux gènes parmi IL6ST, MMP9, S100A4 (par exemple, un ou deux de ces gènes) ;

*cf.* par exemple, les combinaisons n°1 à 17, 19 à 23, 25, 27, plus particulièrement les combinaisons n°1 à 17, 19 à 23 du tableau 3 ci-dessous.

Selon un mode de réalisation de l'invention, on choisit :

- A2M ou au moins A2M dans la première liste de gènes, et
- zéro, un, deux ou trois gènes, plus particulièrement zéro, un ou deux gènes, parmi la liste des seize gènes optionnels, et
- un nombre total de gènes choisis de quatre ou cinq gènes ;

*cf.* par exemple, les combinaisons n°1 à 28 du tableau 3 ci-dessous.

Selon un mode de réalisation de l'invention, on choisit :

- IL8 ou au moins IL8 dans la deuxième liste de gènes, et
- zéro, un, deux ou trois gènes, plus particulièrement zéro, un ou deux gènes, parmi la liste des seize gènes optionnels, et
- un nombre total de gènes choisis pour l'ensemble de la combinaison de quatre ou cinq gènes ;

*cf.* par exemple, les combinaisons n°1 à 18 et 22 à 29 du tableau 3 ci-dessous.

Selon un mode de réalisation de l'invention, on choisit :

- A2M ou au moins A2M dans la première liste, et/ou IL8 ou au moins IL8 dans la deuxième liste, et
- zéro gène parmi la troisième liste, c'est-à-dire la liste des seize gènes optionnels, ou un ou plusieurs gènes parmi cette liste des seize gènes optionnels, parmi lesquels au moins un ou deux gènes parmi IL6ST, MMP9, S100A4, p14ARF, CHI3L1 (par exemple, un ou deux de ces gènes), plus particulièrement au moins un ou deux gènes parmi IL6ST, MMP9, S100A4 (par exemple, un ou deux de ces gènes), et
- un nombre total de gènes choisis pour l'ensemble de la combinaison de quatre ou cinq gènes ;

*cf.* par exemple, les combinaisons n°1 à 17, 19 à 23, 25, 27, plus particulièrement les combinaisons n°1 à 17, 19 à 23 du tableau 3 ci-dessous.

Selon un mode de réalisation de l'invention, on choisit :

- A2M ou au moins A2M dans la première liste de gènes, et
- IL8 ou au moins IL8 dans la deuxième liste de gènes, et/ou MMP9 ou au moins MMP9 parmi la liste des seize gènes optionnels,
- le nombre total de gènes choisis parmi la liste des seize gènes optionnels étant de zéro, un ou deux gènes (par exemple MMP9, ou MMP9 et p14ARF), et
- un nombre total de gènes choisis pour l'ensemble de la combinaison étant de quatre ou cinq gènes ;

*cf.* par exemple, les combinaisons n°1 à 18, 19, 21, et 22 à 29 du tableau 3 ci-dessous.

Selon un mode de réalisation de l'invention, lesdits gènes choisis sont :

- SPP1, et
- A2M, ou au moins A2M parmi A2M et VIM, et
- IL8, ou au moins IL8 parmi IL8, CXCL10 et ENG,
- optionnellement, au moins un gène parmi la liste de seize gènes ci-dessus mentionnée ;

*cf.* par exemple, les combinaisons de gènes n°1 à 18 et 22 à 28 présentées dans le tableau 3 ci-dessous.

Selon un mode de réalisation, lesdits gènes choisis parmi ladite liste de vingt-deux gènes de l'invention sont :

- SPP1, et
- A2M, ou au moins A2M parmi A2M et VIM, et
- CXCL10 et/ou ENG, ou au moins CXCL10 et/ou ENG parmi IL8, CXCL10 et ENG, et
- optionnellement, au moins un gène parmi la liste de seize gènes ci-dessus mentionnée ;

*cf.* par exemple, les combinaisons de gènes n°4, 7, 8, 13, 16, 18, 19, 20, 21, 25, 28 présentées dans le tableau 3 ci-dessous, plus particulièrement les combinaisons de gènes n°19 à 21.

Selon un mode de réalisation, lesdits gènes choisis parmi ladite liste de vingt-deux gènes de l'invention sont :

- SPP1, et
- A2M, ou au moins A2M parmi A2M et VIM, et
- CXCL10 et/ou ENG, ou au moins CXCL10 et/ou ENG parmi IL8, CXCL10 et ENG, et
- optionnellement, MMP9 ou au moins MMP9 parmi ladite liste de seize gènes (par exemple, MMP9 et p14ARF) ;

*cf.* par exemple, les combinaisons de gènes n°4, 19 et 21 présentées dans le tableau 3 ci-dessous.

Ainsi, selon un mode de réalisation de l'invention, lesdits gènes choisis parmi ladite liste de vingt-deux gènes de l'invention peuvent être définis comme étant :

- SPP1, et
- au moins un gène parmi A2M et VIM, et
- au moins un gène parmi IL8, CXCL10 et ENG, et
- optionnellement, au moins un gène parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1,

tout en comprenant au moins :

- A2M, et
- IL8 et/ou MMP9 ;

*cf.* par exemple, les combinaisons de gènes n°1 à 19 et 21 à 28 présentées dans le tableau 3 ci-dessous.

Selon un mode de réalisation, lesdits gènes choisis parmi ladite liste de vingt-deux gènes de l'invention peuvent être :

- SPP1, et
- au moins un gène parmi A2M et VIM, de préférence A2M ou au moins A2M parmi A2M et VIM, et
- au moins un gène parmi IL8, CXCL10 et ENG, et
- optionnellement, au moins un gène parmi IL6ST, MMP9, S100A4, p14ARF, CHI3L1.

Lorsque lesdits gènes choisis parmi ladite liste de vingt-deux gènes de l'invention comprennent au moins un gène parmi IL6ST, MMP9, S100A4, p14ARF, CHI3L1, ils peuvent en outre comprendre au moins un gène parmi ANGPT2, CXCL11, MMP2, MMP7, TIMP1, COL1A1, CXCL1, CXCL6, IHH, IRF9, MMP1.

Voir, par exemple, les combinaisons de gènes n°1 à 6, 8 à 9, 11 à 12, 14 à 17, 19, 21 à 23, 25 à 27 présentées dans le tableau 3 ci-dessous.

Selon un mode de réalisation, lesdits gènes choisis parmi ladite liste de vingt-deux gènes de l'invention peuvent être :

- SPP1, et
- au moins un gène parmi A2M et VIM, de préférence A2M ou au moins A2M parmi A2M et VIM, et
- au moins un gène parmi IL8, CXCL10 et ENG, et
- optionnellement, au moins un gène parmi IL6ST, MMP9, S100A4.

Lorsque lesdits gènes choisis parmi ladite liste de vingt-deux gènes de l'invention comprennent au moins un gène parmi IL6ST, MMP9, S100A4, ils peuvent en outre comprendre au moins un gène parmi p14ARF, CHI3L1, ANGPT2, CXCL11, MMP2, MMP7, TIMP1, COL1A1, CXCL1, CXCL6, IHH, IRF9, MMP1.

Voir, par exemple, les combinaisons de gènes n°1 à 6, 8 à 9, 11 à 12, 14 à 17, 19, 21 à 23 présentées dans le tableau 3 ci-dessous.

Par exemple, lesdits gènes choisis parmi ladite liste de vingt-deux gènes de l'invention comprennent, ou sont :

- SPP1, A2M, IL8, CHI3L1 et IL6ST (combinaison n°1) ; ou
- SPP1, A2M, IL8, ANGPT2 et IL6ST (combinaison n°2) ; ou
- SPP1, A2M, IL8, IL6ST et MMP2 (combinaison n°3) ; ou
- SPP1, A2M, IL8, VIM et CXCL10 (combinaison n°4) ; ou
- SPP1, A2M, IL8, IL6ST et MMP9 (combinaison n°5) ; ou
- SPP1, A2M, IL8, IL6ST et MMP1 (combinaison n°6) ; ou
- SPP1, A2M, IL8, VIM, et ENG (combinaison n°7) ; ou
- SPP1, A2M, IL8, CXCL10 et IL6ST, (combinaison n°8) ; ou
- SPP1, A2M, IL8, CXCL1 et IL6ST (combinaison n°9) ; ou

- SPP1, A2M, IL8 et VIM (combinaison n°10) ; ou
- SPP1, A2M, IL8, COL1A1 et IL6ST (combinaison n°11) ; ou
- SPP1, A2M, IL8, CXCL11 et IL6ST (combinaison n°12) ; ou
- SPP1, A2M, IL8, CXCL10 et ENG (combinaison n°13) ; ou
- SPP1, A2M, IL8, IL6ST et TIMP1 (combinaison n°14) ; ou
- SPP1, A2M, IL8, IHH et IL6ST (combinaison n°15) ; ou
- SPP1, A2M, IL8, CXCL10 et S100A4 (combinaison n°16) ; ou
- SPP1, A2M, IL8, IL6ST et MMP7 (combinaison n°17) ; ou
- SPP1, A2M, IL8, ENG et CXCL11 (combinaison n°18) ; ou
- SPP1, A2M, ENG et MMP9 (combinaison n°19) ; ou
- SPP1, A2M, CXCL10 et ENG (combinaison n°20) ; ou
- SPP1, A2M, CXCL10, pl4ARF et MMP9 (combinaison n°21) ; ou
- SPP1, A2M, IL8, CXCL6 et IL6ST (combinaison n°22) ; ou
- SPP1, A2M, IL8 et S100A4 (combinaison n°23) ; ou
- SPP1, A2M, IL8, ANGPT2 et MMP7 (combinaison n°24) ; ou
- SPP1, A2M, IL8, CXCL10 et pl4ARF (combinaison n°25) ; ou
- SPP1, A2M, IL8 et TIMP1 (combinaison n°26) ; ou
- SPP1, A2M, IL8 et pl4ARF (combinaison n°27) ; ou
- SPP1, A2M, IL8, CXCL10 et IRF9 (combinaison n°28) ; ou
- SPP1, IL8, VIM et MMP2 (combinaison n°29).

Plus particulièrement, lesdits gènes choisis parmi ladite liste de vingt-deux gènes de l'invention comprennent, ou sont :

- SPP1, A2M, IL8, CHI3L1 et IL6ST, (combinaison n°1) ; ou
- SPP1, A2M, IL8, VIM et CXCL10 (combinaison n°4) ; ou
- SPP1, A2M, IL8, VIM, et ENG (combinaison n°7) ; ou
- SPP1, A2M, IL8 et VIM (combinaison n°10) ; ou
- SPP1, A2M, IL8, CXCL10 et ENG (combinaison n°13) ; ou
- SPP1, A2M, ENG et MMP9 (combinaison n°19) ; ou
- SPP1, A2M, CXCL10, pl4ARF et MMP9 (combinaison n°21) ; ou
- SPP1, A2M, IL8 et S100A4 (combinaison n°23).

De manière similaire à ce qui a été indiqué ci-dessus pour les seuils de sensibilité, les seuils de spécificité, les seuils de VPN, le nombre total de gènes choisis, le nombre de gènes choisis dans chacune des listes de gènes, toutes les combinaisons de choix de gènes et/ou de nombres de gènes choisis dans chacune des listes de gènes et/ou de nombres totaux de gènes choisis et/ou de seuils de sensibilité et/ou de seuils de spécificité et/ou de seuils de VPN sont explicitement incluses dans le contenu de l'invention.

Vingt-neuf exemples de combinaisons de gènes conformes à l'invention sont présentés dans le tableau 3 ci-dessous.

Des exemples de modèles de classification multivariée ont été construits pour chacune de ces combinaisons de gènes.

Les tableaux 4, 6, 10 et 12 ci-dessous en présentent des exemples (en l'occurrence, modèles mROC avec fonction linéaire Z) :

- tableaux 4 et 10 : combinaison des niveaux de transcription des gènes (dosage des ARN, en l'occurrence des ARN contenus dans un échantillon contenant des acides nucléiques susceptibles d'être obtenus à partir d'un prélèvement contenant une portion de parenchyme hépatique ou des cellules d'un tel tissu);
- tableaux 6 et 12 : combinaison des niveaux de traduction des gènes (dosage des protéines, en l'occurrence des protéines sériques).

Pour chacune des fonctions Z des tableaux 4, 6, 10 et 12 :

- la valeur de l'aire sous la courbe ROC (AUC),
- un exemple de seuil décisionnel PT (en l'occurrence, seuil maximisant l'indice de Youden $\delta$), et les valeurs de sensibilité (Se, en %), spécificité (Spe, en %), valeurs prédictives négatives (VPN, en %) et valeurs prédictives positives (VPP, en %) associées,

sont présentés en tableaux 5, 7, 11 et 13 respectivement.

VPN = VN / (VN+ FN) avec VN = Vrais Négatifs et FN = Faux Négatifs ;

VPP = VP / (VP + FP) avec VP = les Vrais Positifs et FP = Faux Positifs.

Par exemple, dans le cadre d'une détection F2 versus F1, la VPN représente la probabilité pour le sujet testé soit F1 sachant que le test est négatif (résultat donné par le test = score F1) ; et la VPP représente la probabilité pour que le sujet testé soit F2 sachant que le test est positif (résultat donné par le test = score F2).

**Tableau 3 : vingt-neuf exemples de combinaisons de niveaux d'expression de gènes**

| n° de la combinaison | Gènes choisis | | | |
|---|---|---|---|---|
| 1 | **A2M** | CHI3L1 | IL6ST | IL8 | **SPP1** |
| 2 | **A2M** | ANGPT2 | IL6ST | IL8 | **SPP1** |
| 3 | **A2M** | IL6ST | IL8 | MMP2 | **SPP1** |
| 4 | **A2M** | CXCL10 | IL8 | **SPP1** | **VIM** |
| 5 | **A2M** | IL6ST | IL8 | MMP9 | **SPP1** |
| 6 | **A2M** | IL6ST | IL8 | MMP1 | **SPP1** |
| 7 | **A2M** | ENG | IL8 | **SPP1** | **VIM** |
| 8 | **A2M** | CXCL10 | IL6ST | IL8 | **SPP1** |
| 9 | **A2M** | CXCL1 | IL6ST | IL8 | **SPP1** |
| 10 | **A2M** | IL8 | **SPP1** | **VIM** | |
| 11 | **A2M** | COL1A1 | IL6ST | IL8 | **SPP1** |
| 12 | **A2M** | CXCL11 | IL6ST | IL8 | **SPP1** |
| 13 | **A2M** | CXCL10 | ENG | IL8 | **SPP1** |
| 14 | **A2M** | IL6ST | IL8 | **SPP1** | TIMP1 |
| 15 | **A2M** | IHH | IL6ST | IL8 | **SPP1** |
| 16 | **A2M** | CXCL10 | IL8 | S100A4 | **SPP1** |
| 17 | **A2M** | IL6ST | IL8 | MMP7 | **SPP1** |
| 18 | **A2M** | CXCL11 | ENG | IL8 | **SPP1** |
| 19 | **A2M** | ENG | MMP9 | **SPP1** | |
| 20 | **A2M** | CXCL10 | ENG | SPP1 | |
| 21 | **A2M** | p14ARF | CXCL10 | MMP9 | **SPP1** |
| 22 | **A2M** | CXCL6 | IL6ST | IL8 | **SPP1** |
| 23 | **A2M** | IL8 | S100A4 | **SPP1** | |
| 24 | **A2M** | ANGPT2 | IL8 | MMP7 | **SPP1** |
| 25 | **A2M** | p14ARF | CXCL10 | IL8 | **SPP1** |
| 26 | **A2M** | IL8 | **SPP1** | TIMP1 | |
| 27 | **A2M** | p14ARF | IL8 | **SPP1** | |
| 28 | **A2M** | CXCL10 | IL8 | IRF9 | **SPP1** |
| 29 | IL8 | MMP2 | **SPP1** | **VIM** | |

**Tableau 4 : exemples de modèles de classification (en l'occurrence, modèles mROC) combinant des niveaux de transcription (transcrits ARN, en l'occurrence ARN contenus dans un prélèvement de tissu ou cellules hépatiques)**

| n° de combinaison de gènes (*cf.* tableau 3 ci-dessus) | Fonction Z combinant les niveaux de transcription (ARN) des gènes choisis | nom de la fonction |
|---|---|---|
| 1 | $Z = 0,400 \times A2M^t + 0,003\,(-CHI3L1) + 0,363 \times (-IL6ST)^t + 0,015 \times IL8 + 0,438 \times SPP1^t$ | **Z1ARN** |
| 2 | $Z = 0,404 \times A2M^t + 0,062 \times ANGPT2 + 0,414 \times (-IL6ST)^t + 0,015 \times IL8 + 0,316 \times SPP1^t$ | **Z2ARN** |
| 3 | $Z = 0,392 \times A2M^t + 0,396 \times (-IL6ST)^t + 0,021 \times IL8 + 0,104 \times MMP2^t + 0,271 \times SPP1^t$ | **Z3ARN** |
| 4 | $Z = 0,297 \times A2M^t - 0,046 \times CXCL10 + 0,020 \times IL8 + 0,274 \times SPP1^t + 0,253 \times VIM^t$ | **Z4ARN** |
| 5 | $Z = 0,407 \times A2M^t + 0,406 \times (-IL6ST)^t + 0,013 \times IL8 + 0,038 \times MMP9 + 0,309 \times SPP1^t$ | **Z5ARN** |
| 6 | $Z = 0,406 \times A2M^t + 0,389 \times (-IL6ST)^t + 0,021 \times IL8 + 0,195 \times (-MMP1) + 0,332 \times SPP1^t$ | **Z6ARN** |
| 7 | $Z = 0,230 \times A2M^t + 0,204 \times ENG^t + 0,012 \times IL8 + 0,262 \times SPP1^t + 0,177 \times VIM^t$ | **Z7ARN** |
| 8 | $Z = 0,414 \times A2M^t - 0,013 \times CXCL10 + 0,373 \times (-IL6ST)^t + 0,023 \times IL8 + 0,335 \times SPP1^t$ | **Z8ARN** |
| 9 | $Z = 0,401 \times A2M^t + 0,062 \times CXCL1 + 0,392 \times (-IL6ST)^t + 0,019 \times IL8 + 0,305 \times SPP1^t$ | **Z9ARN** |
| 10 | $Z = 0,259 \times A2M^t + 0,012 \times IL8 + 0,267 \times SPP1^t + 0,227 \times VIM^t$ | **Z10ARN** |
| 11 | $Z = 0,427 \times A2M^t - 0,137 \times COL1A1^t + 0,369 \times (-IL6ST)^t + 0,020 \times IL8 + 0,397 \times SPP1^t$ | **Z11ARN** |
| 12 | $Z = 0,397 \times A2M^t + 0,033 \times CXCL1\,1^t + 401 \times (-IL6ST)^t + 0,020 \times IL8 + 0,321 \times SPP1^t$ | **Z12ARN** |
| 13 | $Z = 0,238 \times A2M^t - 0,050 \times CXCL10 + 0,531 \times ENG^t + 0,023 \times IL8 + 0,320 \times SPP1^t$ | **Z13ARN** |
| 14 | $Z = 0,373 \times A2M^t + 0,389 \times (-IL6ST)^t + 0,020 \times IL8 + 0,309 \times SPP1^t + 0,105 \times TIMP1^t$ | **Z14ARN** |
| 15 | $Z = 0,412 \times A2M^t + 0,001 \times IHH + 0,413 \times (-IL6ST)^t + 0,027 \times IL8 + 0,327 \times SPP1^t$ | **Z15ARN** |

(suite)

| n° de combinaison de gènes (*cf.* tableau 3 ci-dessus) | Fonction Z combinant les niveaux de transcription (ARN) des gènes choisis | nom de la fonction |
|---|---|---|
| 16 | $Z = 0{,}360 \times A2M^t - 0{,}047 \times CXCL10 + 0{,}025 \times IL8 + 0{,}332 \times S100A4 + 0{,}272 \times SPP1^t$ | **Z16ARN** |
| 17 | $Z = 0{,}399 \times A2M^t + 0{,}406 \times (-IL6ST)^t + 0{,}017 \times IL8 + 0{,}540 \times MMP7 + 0{,}328 \times SPP1^t$ | **Z17ARN** |
| 18 | $Z = 0{,}242 \times A2M^t - 0{,}094 \times CXCL1\ 1^t + 0{,}477 \times ENG^t + 0{,}016 \times IL8 + 0{,}323 \times SPP1^t$ | **Z18ARN** |
| 19 | $Z = 0{,}221 \times A2M^t + 0{,}371 \times ENG^t + 0{,}028 \times MMP9 + 0{,}316 \times SPP1^t$ | **Z19ARN** |
| 20 | $Z = 0{,}239 \times A2M^t - 0{,}029 \times CXCL10 + 0{,}489 \times ENG^t + 0{,}333 \times SPP1^t$ | **Z20ARN** |
| 21 | $Z = 0{,}303 \times A2M^t + 2{,}807 \times p14ARF^t - 0{,}033 \times CXCL10 + 0{,}040 \times MMP9 + 0{,}359 \times SPP1^t$ | **Z21ARN** |
| 22 | $Z = 0{,}406 \times A2M^t - 0{,}001 \times CXCL6 + 0{,}384 \times (-IL6ST)^t + 0{,}021 \times IL8 + 0{,}298 \times SPP1^t$ | **Z22ARN** |
| 23 | $Z = 0{,}313 \times A2M^t + 0{,}016 \times IL8 + 0{,}280 \times S100A4 + 0{,}272 \times SPP1^t$ | **Z23ARN** |
| 24 | $Z = 0{,}291 \times A2M^t + 0{,}072 \times ANGPT2 + 0{,}014 \times IL8 - 0{,}822 \times MMP7 + 0{,}361 \times SPP1^t$ | **Z24ARN** |
| 25 | $Z = 0{,}305 \times A2M^t + 3{,}153 \times p14ARF - 0{,}038 \times CXCL10 + 0{,}020 \times IL8 + 0{,}368 \times SPP1^t$ | **Z25ARN** |
| 26 | $Z = 0{,}256 \times A2M^t + 0{,}013 \times IL8 + 0{,}360 \times SPP1^t + 0{,}084 \times TIMP1^t$ | **Z26ARN** |
| 27 | $Z = 0{,}274 \times A2M^t + 2{,}545 \times p14ARF + 0{,}013 \times IL8 + 0{,}357 \times SPP1^t$ | **Z27ARN** |
| 28 | $Z = 0{,}298 \times A2M^t - 0{,}035 \times CXCL10 + 0{,}020 \times IL8 + 0{,}079 \times IRF9^t + 0{,}375 \times SPP1^t$ | **Z28ARN** |
| 29 | $Z = 0{,}015 \times IL8 - 0{,}047 \times MMP2^t + 0{,}340 \times SPP1^t + 0{,}297 \times VIM^t$ | **Z29ARN** |

**Tableau 5 : AUC des fonctions Z du tableau 4, exemple de seuil PT (en l'occurrence, seuil maximisant l'indice de Youden δ) pour ces fonctions, et valeurs de Se, Spe, VPN, VPP associées**

| n° de combinaison de gènes (*cf.* tableau 3 ci-dessus) | Nom de la fonction (*cf.* tableau 4 ci-dessus) | AUC | AUC limite inférieure | AUC limite supérieure | Seuil choisi (δ) | Se | Spe | VPN | VPP |
|---|---|---|---|---|---|---|---|---|---|
| 1 | **Z1ARN** | 0,8 | 0,72 | 0,862 | 0,561 | **75** | 72 | 84 | 60 |

(suite)

| n° de combinaison de gènes (*cf.* tableau 3 ci-dessus) | Nom de la fonction (*cf.* tableau 4 ci-dessus) | AUC | AUC limite inférieure | AUC limite supérieure | Seuil choisi ($\delta$) | Se | Spe | VPN | VPP |
|---|---|---|---|---|---|---|---|---|---|
| 2 | Z2ARN | 0,79 | 0,708 | 0,854 | 0,999 | 70 | 73 | 81 | 59 |
| 3 | Z3ARN | 0,788 | 0,706 | 0,853 | 0,68 | 70 | 74 | 81 | 60 |
| 4 | Z4ARN | 0,787 | 0,705 | 0,852 | -0,764 | **75** | 70 | 83 | 58 |
| 5 | Z5ARN | 0,787 | 0,704 | 0,851 | 0,941 | 70 | 72 | 81 | 58 |
| 6 | Z6ARN | 0,787 | 0,705 | 0,851 | 0,79 | 70 | 70 | 80 | 56 |
| 7 | Z7ARN | 0,786 | 0,703 | 0,851 | -0,605 | **75** | 70 | 83 | 57 |
| 8 | Z8ARN | 0,786 | 0,73 | 0,85 | 0,753 | 70 | 70 | 80 | 55 |
| 9 | Z9ARN | 0,786 | 0,703 | 0,85 | 0,874 | 71 | 70 | 81 | 56 |
| 10 | Z10ARN | 0,785 | 0,701 | 0,851 | -0,699 | **73** | 70 | 83 | 57 |
| 11 | Z11ARN | 0,785 | 0,701 | 0,85 | 0,82 | 71 | 70 | 81 | 56 |
| 12 | Z12ARN | 0,785 | 0,702 | 0,85 | 0,797 | 70 | 73 | 81 | 59 |
| 13 | Z13ARN | 0,784 | 0,702 | 0,848 | -0,301 | **75** | 70 | 83 | 57 |
| 14 | Z14ARN | 0,784 | 0,701 | 0,849 | 0,649 | 71 | 72 | 82 | 58 |
| 15 | Z15ARN | 0,784 | 0,702 | 0,849 | 0,88 | 70 | 70 | 80 | 55 |
| 16 | Z16ARN | 0,783 | 0,7 | 0,848 | 0,321 | 70 | 75 | 81 | 60 |
| 17 | Z17ARN | 0,783 | 0,701 | 0,848 | 0,838 | 70 | 70 | 80 | 56 |
| 18 | Z18ARN | 0,781 | 0,698 | 0,846 | -0,066 | 71 | 70 | 81 | 56 |
| 19 | Z19ARN | 0,779 | 0,694 | 0,845 | -0,222 | **73** | 70 | 83 | 57 |
| 20 | Z20ARN | 0,779 | 0,695 | 0,845 | -0,327 | 71 | 70 | 81 | 57 |
| 21 | Z21ARN | 0,778 | 0,692 | 0,845 | -0,162 | **73** | 70 | 83 | 57 |
| 22 | Z22ARN | 0,786 | 0,703 | 0,851 | 0,778 | 71 | 70 | 81 | 56 |
| 23 | Z23ARN | 0,777 | 0,692 | 0,844 | 0,236 | **73** | 70 | 83 | 57 |
| 24 | Z24ARN | 0,775 | 0,689 | 0,842 | -0,133 | 71 | 70 | 81 | 56 |
| 25 | Z25ARN | 0,775 | 0,691 | 0,842 | -0,181 | 70 | 70 | 80 | 55 |
| 26 | Z26ARN | 0,773 | 0,688 | 0,841 | -0,354 | 71 | 70 | 81 | 57 |
| 27 | Z27ARN | 0,771 | 0,685 | 0,839 | -0,132 | 71 | 71 | 82 | 58 |
| 28 | Z28ARN | 0,771 | 0,685 | 0,838 | -0,39 | 70 | 70 | 81 | 56 |
| 29 | Z29ARN | 0,759 | 0,671 | 0,829 | -1,202 | 71 | 70 | 81 | 56 |

**Tableau 6 : exemples de modèles de classification (en l'occurrence, modèles mROC) combinant des niveaux de traduction (protéines, en l'occurrence protéines sériques)**

| n° de combinaison de gènes (*cf.* tableau 3 ci-dessus) | Fonction Z combinant les niveaux de traduction (protéine sérique) des gènes choisis | Nom de la fonction |
|---|---|---|
| 16 | $Z = 0{,}241 \times A2M^t + 0{,}137 \times CXCL10^t + 0{,}001 \times IL8^t + 0{,}062 \times SPP1^t + 0{,}226 \times S100A4^t$ | Z16prot |

**Tableau 7 : AUC de la fonction Z du tableau 6, exemple de seuil PT (en l'occurrence, seuil maximisant l'indice de Youden δ) pour cette fonction, et valeurs de Se, Spe, VPN, VPP associées (*cf.* exemple 3a) ci-dessous)**

| n° de combinaison de gènes (*cf.* tableau 3 ci-dessus) | Nom de la fonction (*cf.* tableau 6 ci-dessus) | AUC | AUC limite inférieure | AUC limite supérieure | Seuil choisi (δ) | Se | Spe | VPN | VPP |
|---|---|---|---|---|---|---|---|---|---|
| 16 | Z16PROT | 0,694 | 0,612 | 0,765 | 2,905 | 68 | 67 | 81 | 50 |

Dans le tableau 4 ci-dessus, les échantillons des individus ayant permis de construire le modèle de classification (fonction Z) étaient des prélèvements de tissu ou cellules hépatiques, et c'est le niveau de transcription ARN des gènes choisis qui a été mesuré. Les valeurs de dosage sont donc celles obtenues sur des échantillons contenant des ARN d'un échantillon biologique susceptible d'être obtenu par PBH ou par cyto-ponction hépatique (par exemple, par extraction des ARN de cet échantillon biologique).

Dans le tableau 4 ci-dessus, le nom de chacun des gènes indiqués à titre de variables dans une fonction Z (par exemple, pour la fonction Z de la combinaison n°1 : A2M, CHI3L1, IL6ST, IL8 et SPP1) symbolise la valeur de dosage d'un produit de transcription (ARN) de ce gène, c'est-à-dire la quantité d'ARN du gène concerné rapportée à la quantité totale d'ARN initialement contenue dans l'échantillon, plus particulièrement la valeur de Ct qui a été mesurée pour les transcrits de ce gène et qui a été normalisée par la méthode du $2^{-\Delta Ct}$. Si l'on utilise le symbole BMQ (biomarqueur) pour désigner de manière générique chacune de ces variables, on peut alors considérer que BMQ = la valeur obtenue pour l'ARN de ce gène par la méthode du $2^{-\Delta Ct}$ (*cf.* exemple 1 ci-dessous).

Dans le tableau 6 ci-dessus, les échantillons des individus ayant permis de construire le modèle de classification (fonction Z) étaient des prélèvements sanguins, et c'est le niveau de traduction (protéine) des gènes choisis qui a été mesuré. Les valeurs de dosage sont donc celles obtenues sur des échantillons contenant des protéines d'un échantillon biologique susceptible d'être obtenu par prélèvement sanguin (par exemple, par séparation et récolte de la fraction sérique de ce prélèvement sanguin).

Dans le tableau 6 ci-dessus, le nom de chacun des gènes indiqués à titre de variables dans une fonction Z (pour la fonction Z de la combinaison n°16 : A2M, CXCL10, IL8, SPP1 et S100A4) symbolise la valeur de dosage du produit de traduction de ce gène (produit protéique), c'est-à-dire la concentration en ce produit de traduction, plus particulièrement, la concentration de la protéine codée par ce gène mesurée dans un fluide biologique du patient, tel que le sérum. Si l'on utilise le symbole BMQ (biomarqueur) pour désigner de manière générique chacune de ces variables, on peut alors considérer que BMQ = la concentration obtenue pour le produit de transcription de ce gène (*cf.* exemple 3 ci-dessous).

Dans les tableaux 4 et 6 ci-dessus, l'exposant t associé à une valeur BMQ (« $BMQ^t$ ») indique une transformation Box-Cox ($BMQ^t = (BMQ^\lambda-1)/\lambda$) ; *cf.* Box et Cox, 1964.

Le tableau 8 ci-dessous indique une liste des gènes, pour lesquels il est conseillé de normaliser les valeurs de dosage de niveaux de transcription (ARN) mesurées (en l'occurrence, A2M, ENG, SPP1, VIM, IRF9, CXCL11, TIMP1, MMP2, IL6ST, TIMP1, COL1A1, MMP1), par exemple par une normalisation Box-Cox, et présente un exemple de valeur de paramètre Box-Cox (λ) utilisable dans les fonctions Z indiquées au tableau 4 ci-dessus.

**Tableau 8 :** liste des gènes, pour lesquels il est conseillé de normaliser les valeurs de dosage mesurées (notamment, les dosages des niveaux de transcription ARN, plus particulièrement lorsque ces ARN sont susceptibles d'être issus d'un prélèvement de tissu ou cellules hépatiques), par exemple par une normalisation Box-Cox, et exemple de valeurs de paramètre Box-Cox (λ) utilisables dans les fonctions Z indiquées au tableau 4 ci-dessus.

| Gènes, pour lesquels il est conseillé de normaliser la valeur du niveau de transcription (ARN) | Exemple de valeur de paramètre Box-Cox (λ) utilisable pour les fonctions Z du tableau 4 ci-dessus |
|---|---|
| A2M | 0,33 |
| ENG | 0,08 |
| SPP1 | 0,12 |
| VIM | -0,23 |
| IRF9 | 0,17 |
| CXCL11 | 0,06 |

(suite)

| Gènes, pour lesquels il est conseillé de normaliser la valeur du niveau de transcription (ARN) | Exemple de valeur de paramètre Box-Cox ($\lambda$) utilisable pour les fonctions Z du tableau 4 ci-dessus |
|---|---|
| TIMP1 | 0,02 |
| MMP2 | -0,03 |
| IL6ST | 0 |
| TIMP1 | 0,02 |
| COL1A1 | 0,24 |
| MMP1 | 0,02 |

Le tableau 9 ci-dessous indique une liste des gènes, pour lesquels il est conseillé de normaliser les valeurs de dosage de niveaux de traduction (protéine) mesurées (en l'occurrence, A2M, CXCL10, IL8, SPP1, S100A4), par exemple par une normalisation Box-Cox, et présente un exemple de valeur de paramètre Box-Cox ($\lambda$) utilisable dans les fonctions Z indiquées au tableau 6 ci-dessus.

Tableau 9 : liste des gènes, pour lesquels il est conseillé de normaliser les valeurs de dosage mesurées (notamment, les dosages des niveaux de traduction protéique, plus particulièrement lorsque ces protéines sont susceptibles d'être issus d'un prélèvement de sang, sérum ou plasma), par exemple par une normalisation Box-Cox, et exemple de valeurs de paramètre Box-Cox ($\lambda$) utilisables dans les fonctions Z indiquées au tableau 6 ci-dessus.

| Gènes, pour lesquels il est conseillé de normaliser la valeur du niveau de traduction (protéine) | Exemple de valeur de paramètre Box-Cox ($\lambda$) utilisables pour les fonctions Z du tableau 6 ci-dessus |
|---|---|
| A2M | 0,46 |
| CXCL10 | 0,08 |
| IL8 | 0,05 |
| SPP1 | 0,43 |
| S100A4 | -0,15 |

En sus des niveaux d'expression desdits gènes choisis, les moyens de l'invention peuvent en outre comprendre la combinaison d'un ou plusieurs facteurs autres que les niveaux d'expression desdits gènes choisis, tels que :

- un ou plusieurs facteurs cliniques, tels que :

  ◦ sexe (féminin F ou masculin M),
  ◦ âge à la date du prélèvement (Age), par exemple, âge à la date de la PBH, âge à la date de la cytoponction hépatique, âge à la date du prélèvement de sang, de sérum, de plasma ou d'urine),
  ◦ âge du patient à la date de la contamination,
  ◦ indice de masse corporelle (IMC),
  ◦ indice de sensibilité à l'insuline (HOMA),
  ◦ diabète,
  ◦ consommation d'alcool,
  ◦ degré de stéatose,
  ◦ mode de contamination,
  ◦ activité Metavir ;

  et/ou
- un ou plusieurs facteurs virologiques, tels que :

  ◦ génotype viral,
  ◦ durée de l'infection,
  ◦ charge virale mesurée chez le patient à la date du début du traitement (charge virale à J0),

◦ charge virale mesurée chez le patient à la date du prélèvement ;

et/ou

- un ou plusieurs facteurs biologiques autres que les niveaux d'expression desdits gènes choisis, qui peuvent notamment être choisis parmi des concentrations, teneurs ou taux en protéines intracorporelles, des concentrations, teneurs ou taux en métabolites intracorporels, des concentrations, teneurs ou taux en éléments figurés du sang, des mesures représentatives de la quantité de fer circulant, de tels que :

    ◦ concentration en haptoglobine (Hapto),
    ◦ concentration en apolipoprotéine A1 (ApoA1),
    ◦ teneur en bilirubine totale (BLT),
    ◦ concentration en gamma glutamyl transpeptidase (GGT),
    ◦ concentration en aspartate aminotransférase (AST),
    ◦ concentration en alanine aminotransférase (ALT),
    ◦ teneur en plaquettes (PLQ),
    ◦ taux de prothrombine (TP),
    ◦ teneur en cholestérol HDL (Chol-HDL),
    ◦ teneur en cholestérol total,
    ◦ concentration en ferritine (Ferritine),
    ◦ teneur glycémique (glycémie),
    ◦ concentration en peptide C,
    ◦ teneur en insuline (insulinémie),
    ◦ concentration en triglycérides (TG),
    ◦ teneur en albumine,
    ◦ coefficient de saturation en fer de la transferrine (CS),
    ◦ concentration en phosphatase alcaline (PAL).

Cet ou ces autres facteurs peuvent être mesurés sur un échantillon de nature différente de celui utilisé pour mesurer les niveaux d'expression desdits gènes choisis. Par exemple, l'échantillon biologique pour la mesure des niveaux d'expression desdits gènes choisis parmi ladite liste de vingt-deux gènes de l'invention peut être un échantillon de PBH ou de cyto-ponction hépatique, et l'échantillon biologique pour la mesure des valeurs desdits autres facteurs peut être un échantillon de fluide biologique, tel que sang, plasma ou sérum ou urine. De même, la nature du niveau d'expression mesuré peut être différente ; par exemple, pour la mesure du niveau d'expression desdits gènes choisis, on peut mesurer les niveaux de leur transcription en ARN, alors que, pour ceux desdits autres facteurs qui sont des facteurs biologiques, le niveau d'expression mesuré sera généralement une concentration protéique.

Avantageusement, cet ou ces autres facteurs sont ou comprennent un ou plusieurs facteurs parmi :

- les facteurs cliniques suivants :

    ◦ sexe (féminin F ou masculin M),
    ◦ âge à la date du prélèvement,
    ◦ indice de masse corporelle (IMC),
    ◦ indice de sensibilité à l'insuline (HOMA),
    ◦ diabète,
    ◦ consommation d'alcool,
    ◦ degré de stéatose ;

et/ou
- les facteurs virologiques suivants :

    ◦ génotype viral,
    ◦ durée de l'infection ;

et/ou
- les facteurs biologiques suivants :

    ◦ concentration en haptoglobine (Hapto),
    ◦ concentration en apolipoprotéine A1 (ApoA1),

  ◦ teneur en bilirubine totale (BLT),
  ◦ concentration en gamma glutamyl transpeptidase (GGT),
  ◦ concentration en aspartate aminotransférase (AST),
  ◦ concentration en alanine aminotransférase (ALT),
  ◦ teneur en plaquettes (PLQ),
  ◦ taux de prothrombine (TP),
  ◦ teneur en cholestérol total,
  ◦ teneur en cholestérol HDL (Chol-HDL),
  ◦ concentration en ferritine (Ferritine),
  ◦ teneur glycémique (glycémie),
  ◦ concentration en peptide C,
  ◦ concentration en triglycérides (TG).

Le dosage de certains de ces facteurs pourrait parfois être considéré comme le dosage du niveau de traduction (mesure de la concentration protéique) d'un gène autre qu'un gène choisi conformément à l'invention (par exemple ALT).

Le nombre de gènes dont le niveau d'expression est dosé, et qui ne sont pas des gènes choisis conformément à l'invention(par exemple gène codant ALT), est de préférence au maximum de 18, plus particulièrement de 14 ou moins, plus particulièrement de 11 ou moins, plus particulièrement de 6 ou moins, plus particulièrement de 4 ou 3 ou 2, plus particulièrement de 1 ou 0.

Avantageusement, cet ou ces autres facteurs sont ou comprennent un ou plusieurs facteurs biologiques, notamment un ou plusieurs facteurs parmi les facteurs biologiques suivants :

  ◦ concentration en gamma glutamyl transpeptidase (GGT),
  ◦ concentration en alanine aminotransférase (ALT),
  ◦ concentration en ferritine (Ferritine),
  ◦ concentration en triglycérides (TG),

plus particulièrement, un ou plusieurs facteurs parmi les facteurs biologiques suivants :

  ◦ concentration en alanine aminotransférase (ALT),
  ◦ concentration en triglycérides (TG).

Alternativement ou complémentairement, ce ou ces facteurs peuvent plus particulièrement être ou comprendre le facteur clinique âge à la date du prélèvement (Age).

Les exemples 2c), 2d) et 3b) ci-dessus donnent une illustration de telles combinaisons.

Les exemples 2c), 2d) et 3b) ci-dessus donnent également des exemples de modèle de classification multivariée (en l'occurrence, des modèles mROC) pour des combinaisons faisant intervenir :

- les niveaux d'expression (dosage des ARN ou des protéines) de gènes choisis parmi ladite liste de vingt-deux gènes de l'invention, ainsi que
- des facteurs biologiques autres que le niveau d'expression de gènes choisis parmi ladite liste de vingt-deux gènes de l'invention (en l'occurrence, plusieurs facteurs parmi concentration en triglycérides (TG), concentration en alanine aminotransférase (ALT), concentration en gamma glutamyl transpeptidase (GGT), concentration en ferritine), et,
- éventuellement, un facteur clinique (en l'occurrence, le facteur clinique âge à la date du prélèvement).

Selon un mode de réalisation de l'invention, lesdits gènes choisis parmi ladite liste de vingt-deux gènes de l'invention sont, ou comprennent A2M, CXCL10, IL8, SPP1 et S100A4 (combinaison n°16 dans le tableau 3 ci-dessus), et la combinaison de la valeur de leurs niveaux respectifs d'expression (dosage des ARN ou des protéines, notamment des ARN hépatiques ou des protéines sériques) est en outre combinée à au moins un ou plusieurs facteurs biologiques autres que les niveaux d'expression de gènes choisis parmi ladite liste de vingt-deux gènes de l'invention, notamment à au moins un ou plusieurs facteurs biologiques parmi :

  ◦ concentration en gamma glutamyl transpeptidase (GGT),
  ◦ concentration en alanine aminotransférase (ALT),
  ◦ concentration en triglycérides (TG),
  ◦ optionnellement, concentration en ferritine (Ferritine),

plus particulièrement à au moins un ou plusieurs de ces facteurs biologiques et en outre un ou plusieurs facteurs cliniques (tel que âge à la date du prélèvement).

Les exemples 2c) et 3b) ci-dessous donnent une illustration de telles combinaisons.

Selon un mode de réalisation de l'invention, lesdits gènes choisis parmi ladite liste de vingt-deux gènes de l'invention sont, ou comprennent A2M, CXCL10, IL8, SPP1 et VIM (combinaison n°4 dans le tableau 3 ci-dessus), et la combinaison de la valeur de leurs niveaux respectifs d'expression (plus particulièrement, dosage des ARN, notamment des ARN hépatiques) est en outre combinée à au moins un ou plusieurs facteurs biologiques autres que le niveaux d'expression de gènes choisis parmi ladite liste de vingt-deux gènes de l'invention, notamment à au moins un ou plusieurs facteurs biologiques parmi :

- ◦ concentration en gamma glutamyl transpeptidase (GGT),
- ◦ concentration en alanine aminotransférase (ALT),
- ◦ concentration en triglycérides (TG),
- ◦ concentration en ferritine (Ferritine).

L'exemple 2d) ci-dessous donne une illustration de telles combinaisons.

Des exemples de modèles de classification multivariée pour de telles combinaisons comprennent les fonctions linéaires Z (modèles mROC) présentées dans les tableaux 10 et 12 suivants (*cf.* également, exemples 2c), 3b) et 2d) ci-dessous).

Dans le tableau 10 ci-dessous, les échantillons des individus ayant permis de construire le modèle de classification (fonction Z) étaient des prélèvements de tissu ou cellules hépatiques, et c'est le niveau de transcription ARN des gènes choisis qui a été mesuré. Comme pour le tableau 4 ci-dessus, le nom de chacun des gènes indiqués à titre de variables dans une fonction Z (par exemple, pour la fonction Z de la combinaison n°16 : A2M, CXCL10, IL8, SPP1 et S100A4) symbolise la valeur de dosage d'un produit de transcription (ARN) de ce gène, c'est-à-dire la quantité d'ARN du gène concerné rapportée à la quantité totale d'ARN initialement contenue dans l'échantillon, plus particulièrement la valeur de Ct qui a été mesurée pour les transcrits de ce gène et qui a été normalisée par la méthode du $2^{-\Delta Ct}$.

Dans le tableau 12 ci-dessous, les échantillons des individus ayant permis de construire le modèle de classification (fonction Z) étaient des prélèvements sanguins, et c'est le niveau de traduction (protéine) des gènes choisis qui a été mesuré. Comme pour le tableau 6 ci-dessus, le nom de chacun des gènes indiqués à titre de variables dans une fonction Z (pour la fonction Z de la combinaison n°16 : A2M, CXCL10, IL8, SPP1 et S100A4) symbolise la valeur de dosage du produit de traduction de ce gène (produit protéique), c'est-à-dire la concentration en ce produit de traduction, plus particulièrement, la concentration de la protéine codée par ce gène mesurée dans un fluide biologique du patient, tel que le sérum. Les tableaux 11 et 13 suivants présentent des exemples de valeurs de paramètres lambda des transformées Box-Cox utilisables pour les fonctions Z des tableaux 10 et 12, donnent l'AUC de ces fonctions Z, et indiquent un exemple de valeur seuil PT (en l'occurrence, seuil maximisant l'indice de Youden, δ), ainsi que les valeurs de Se, Sp, VPN et VPP associées.

Ainsi, selon un mode de réalisation de l'invention,

- les gènes choisis à l'étape i) sont les gènes de l'une desdites combinaisons n°1 à n°29, par exemple les gènes de la combinaison n°16 ou n°4,
- à l'étape i), on dose le niveau auquel chacun de ces gènes choisis sont transcrits ou traduits,
- on détermine la valeur de plusieurs autres facteurs autres que le niveaux d'expression de gènes choisis parmi ladite liste de vingt-deux gènes de l'invention, parmi lesquels au moins les facteurs suivants :

  - concentration en alanine aminotransférase (ALT),
  - concentration en triglycérides (TG),
  - optionnellement, concentration en gamma glutamyl transpeptidase (GGT) et/ou concentration en ferritine (Ferritine) et/ou âge à la date du prélèvement,

- les valeurs des autres facteurs ainsi déterminées et les valeurs de dosage obtenues à l'étape i) étant alors combinées ensemble à l'étape ii) pour être comparées à leurs valeurs, ou à la distribution de leurs valeurs, dans lesdites cohortes de référence.

**Tableau 10 : combinaison de gènes choisis conforme à l'invention (dosage de leurs niveaux de transcription en ARN), combinée en outre à d'autres facteurs**

| Gènes choisis et nature du niveau d'expression mesuré pour ces gènes | | Autres facteurs | Exemple de modèle de classification multivariée (modèle mROC) | Nom de la fonction |
|---|---|---|---|---|
| **combinaison n°16** du tableau 3 ci-dessus | **ARN (&)** | **Age** **GGT** (protéine) **ALT** (protéine) **TG** (protéine) **Ferritine** (protéine) | $Z = 0{,}272 \times A2M^t - 0{,}032 \times CXCL10 + 0{,}058 \times IL8 + 0{,}419 \times SPP1^t + 0{,}012 \times S100A4^t + 0{,}025 \times Age^t + 0{,}566 \times TG^t + 3{,}874 \times ALT^t - 0{,}039 \times Ferritine^t$ | **Z16ARNsupp** |
| **combinaison n°4** du tableau 3 ci-dessus | **ARN (&)** | **GGT** (protéine) **ALT** (protéine) **TG** (protéine) **Ferritine** (protéine) | $Z = 0{,}315 \times A2M^t - 0{,}043 \times CXCL10 + 0{,}058 \times IL8 + 0{,}383 \times SPP1^t + 0{,}064 \times VIM^t + 0{,}56 \times TG^t + 3{,}657 \times ALT^t + 0{,}188 \times GGT^t - 0{,}05 \times Ferritine^t$ | **Z4ARNsupp** |

(&) : plus particulièrement, ARN contenus dans un prélèvement de tissu ou cellules hépatiques Age = âge à la date du prélèvement ;

GGT = concentration en gamma glutamyl transpeptidase dans le sérum ;

ALT = concentration en alanine aminotransférase dans le sérum ;

TG = concentration en triglycérides (TG) dans le sérum;

Ferritine = concentration en ferritine dans le sérum.

**Tableau 11 : pour les fonctions du tableau 10, exemple de valeur de paramètre lambda, valeurs d'AUC, exemples de seuils PT (en l'occurrence, seuil maximisant l'indice de Youden $\delta$) pour ces fonctions, et valeurs de Se, Spe, VPN, VPP associées**

| Nom de la fonction | Exemple de valeur du paramètre lambda (*) | AUC | AUC limite inférieure | AUC limite supérieure | Seuil ($\delta$) | Se | Spe | VPN | VPP |
|---|---|---|---|---|---|---|---|---|---|
| **Z16ARNsupp** (*cf*. tableau 10) | 0,21 pour A2M 0,04 pour SPP1 0,48 pour S100A4 0,79 pour Age -0,22 pour TG -0,41 pour ALT 0,15 pour Ferritine | 0,840 | 0,760 | 0,897 | 8,014 | 72 | 82 | 85 | 67 |
| **Z4ARNsupp** (*cf*. tableau 10) | 0,21 pour A2M 0,04 pour SPP1 -0,26 pour VIM -0,22 pour TG -0,41 pour ALT -0,12 pour GGT 0,15 pour Ferritine | 0,841 | 0,764 | 0,896 | 7,016 | 80 | 71 | 88 | 59 |

(*) paramètre lambda des transformations Box-Cox [$BMQ^t = (BMQ^\lambda - 1)/\lambda$]

**Tableau 12 : combinaison de gènes choisis conforme à l'invention (dosage de leurs niveaux de traduction en protéines), combinée en outre à d'autres facteurs**

| Gènes choisis et nature du niveau d'expression mesuré pour ces gènes | | Autres facteurs | Exemple de modèle de classification multivariée (modèle mROC) | Nom de la fonction |
|---|---|---|---|---|
| **combinaison n°16** du tableau 3 ci-dessus | **Protéines** (§) | **Age** **GGT** (protéine) **ALT** (protéine) **TG** (protéine) | $Z = 0{,}2 \times A2M^t + 0{,}05 \times CXCL10^t - 0{,}026 \times IL8^t + 0{,}051 \times SPP1^t + 0{,}204 \times S100A4^t + 0{,}020 \times Age^t + 0{,}266 \times TG^t + 3{,}354 \times ALT^t + 0{,}141 \times GGT^t$ | **Z16PROTsupp** |
| (§) : plus particulièrement, protéines contenues dans un prélèvement sanguin, en l'occurrence dans la partie sérique de ce prélèvement Age = âge à la date du prélèvement ; GGT = concentration en gamma glutamyl transpeptidase dans le sérum ; ALT = concentration en alanine aminotransférase dans le sérum ; TG = concentration en triglycérides (TG) dans le sérum ; Ferritine = concentration en ferritine dans le sérum. | | | | |

**Tableau 13 : pour la fonction du tableau 12, exemple de paramètres lambda, valeur d'AUC, exemple de seuil PT (en l'occurrence, seuil maximisant l'indice de Youden $\delta$) pour cette fonction, et valeurs de Se, Spe, VPN, VPP associées**

| Nom de la fonction | Exemple de valeur du paramètre lambda (*) | AUC | AUC limite inférieure | AUC limite supérieure | Seuil ($\delta$) | Se | Spe | VPN | VPP |
|---|---|---|---|---|---|---|---|---|---|
| **Z16PROTsupp** (*cf.* tableau 12) | 0,46 pour A2M 0,08 pour CXCL10 0,05 pour IL8 0,43 pour SPP1 -0,15 pour S100A4 0,9 pour Age -0,27 pour TG -0,13 pour GGT -0,47 pour ALT | 0,743 | 0,666 | 0,809 | 8,792 | 67 | 72 | 83 | 52 |
| (*) paramètre lambda des transformations Box-Cox $[BMQ^t = (BMQ^\lambda - 1)/\lambda]$ | | | | | | | | | |

Le facteur « activité Metavir » est une évaluation semi-quantitative de l'activité de l'hépatite tenant compte de la nécrose parcellaire et de la nécrose lobulaire, par exemple suivant la méthode décrite dans Bedossa *et al.* 1996, dont le résultat est un score de 0 à 3 :

A0 : sans activité,
A1 : activité minime,
A2 : activité modérée,
A3 : activité sévère.

Le facteur « stéatose » est une évaluation semi-quantitative du pourcentage d'hépatocytes contenant des vacuoles de stéatose lors de la lecture de la biopsie en anatomo-pathologie, par exemple selon le système de scores suivants :

Grade 0 : <1% des hépatocytes atteints,
Grade 1 : 1-33% des hépatocytes atteints,

Grade 2 : 33-66% des hépatocytes atteints,
Grade 3 : > 66% des hépatocytes atteints.

Cet ou ces autres facteurs peuvent être associés, à titre de co-variables, à la combinaison de niveaux d'expression desdits gènes choisis. Les valeurs de ces facteurs et niveaux d'expression peuvent par exemple être combinées dans un modèle de classification multivariée combinant à la fois les paramètres relatifs aux niveaux d'expression desdits gènes choisis et les paramètres relatifs à ce ou ces facteurs (*cf*. exemples 2c) ; 2d) et 3b) ci-dessous).

Selon un aspect complémentaire de l'invention, l'invention est relative à des produits ou réactifs pour la détection et/ou la détermination et/ou le dosage des niveaux d'expression desdits gènes choisis, et à des articles manufacturés, des compositions, des compositions pharmaceutiques, des kits, des tubes, des supports solides comprenant de tels réactifs, ainsi qu'à des systèmes informatiques (notamment, produit programme d'ordinateur et dispositif informatique), qui sont spécialement adaptés à la mise en oeuvre d'une méthode de l'invention.

L'invention peut être mise en oeuvre via notamment un **réactif** qui détecte de manière spécifique un produit de transcription (ARN) d'un desdits gènes choisis parmi ladite liste de vingt-deux gènes de l'invention, ou un produit de traduction d'un desdits gènes choisis parmi ladite liste de vingt-deux gènes de l'invention (protéine, ou forme posttraductionnelle de cette protéine, telle que fragment spécifique de cette protéine).

L'invention peut être mise en oeuvre via des réactifs qui détectent de manière spécifique chacun des produits de transcription (ARN) desdits gènes choisis parmi ladite liste de vingt-deux gènes de l'invention, ou chacun des produits de traduction desdits gènes choisis parmi ladite liste de vingt-deux gènes de l'invention (protéine, ou forme post-traductionnelle de cette protéine, telle que fragment spécifique de cette protéine).

Avantageusement, un ensemble de tels réactifs est formé, qui détecte chacun desdits produits de transcription desdits gènes choisis et/ou qui détecte chacun desdits produits de traduction desdits gènes choisis parmi ladite liste de vingt-deux gènes de l'invention, c'est-à-dire un ensemble de réactifs qui détecte de manière spécifique au moins un produit d'expression pour chacun de ces gènes.

De préférence, lesdits réactifs non seulement détectent spécifiquement un produit de transcription ou traduction, mais permettent également de le quantifier.

L'invention est notamment relative à un **article manufacturé** comprenant lesdits réactifs comme produit de combinaison (ou sous forme combinée, ou en préparation combinée), notamment pour leur utilisation simultanée, séparée ou étalée dans le temps. Cet article manufacturé peut par exemple se présenter sous forme d'un ensemble de réactifs, ou d'un kit.

Bien entendu les caractéristiques de combinaisons de gènes choisies décrites ci-dessus, ainsi que celles illustrées ci-dessous, s'appliquent aux réactifs de l'invention *mutatis mutandis*.

Lesdits réactifs peuvent par exemple s'hybrider de manière spécifique à l'ARN desdits gènes choisis et/ou à l'ADNc correspondant à ces ARN (en conditions au moins stringentes d'hybridation), ou se lier de manière spécifique aux protéines codées par lesdits gènes choisis (ou à des fragments spécifiques de ces protéines), par exemple selon une réaction de type antigène-anticorps.

Des conditions au moins stringentes d'hybridation sont connues de la personne du métier. Il peut par exemple s'agir des conditions suivantes :

- pour une hybridation sur filtre : dans 5xSSC, 2% dodecyl sulfate de sodium (SDS), 100 microgrammes/mL d'ADN simple-brin à 55-65°C pendant 8 heures, et lavage dans 0,2xSSC et 0,2% SDS à 60-65°C pendant trente minutes ;
- pour une hybridation par PCR : les conditions de PCR indiquées en exemple 1 ci-dessous.

Lesdits réactifs de l'invention peuvent notamment être :

- des acides nucléiques (ADN, ARN, ARNm, ADNc), y compris des aptamères oligonucléotidiques, optionnellement marqués pour permettre leur détection, notamment avec des marqueurs fluorescents bien connus de la personne du métier, ou
- des ligands de protéines, tels que des protéines, polypeptides ou peptides, par exemple des aptamères, et/ou des anticorps ou fragments d'anticorps.

Les acides nucléiques de l'invention peuvent par exemple être des amorces et/ou des sondes (*cf.* SEQ ID NO : 1 à 44 dans le tableau 17 ci-dessous), notamment des paires d'amorces (*cf.* les paires d'amorces indiquées dans le tableau 17 ci-dessous). Pour chacun desdits gènes choisis parmi ladite liste de vingt-deux gènes de l'invention, la personne du métier peut construire une paire d'amorces et/ou une sonde qui s'hybrident spécifiquement à ce gène. Un article manufacturé de l'invention peut donc comprendre le nombre d'amorces et/ou sondes nécessaire à la détection des ARN ou ADNc de chacun desdits gènes choisis.

La séquence des acides nucléiques de l'invention peut par exemple être constituée de 9 à 40 nucléotides, plus particulièrement de 10 à 30 nucléotides, plus particulièrement de 14 à 29 nucléotides, plus particulièrement de 19 à 24 nucléotides.

Les séquences des amorces d'une même paire peuvent par exemple être les séquences d'un fragment de la séquence d'un desdits gènes choisis et d'un fragment de sa séquence complémentaire (*cf.* tableau 2 indiquant les numéros d'accession de séquences de ces gènes). L'une et/ou l'autre de ces deux séquences d'amorces peuvent ne pas être strictement identiques à la séquence d'un fragment du gène ou de sa séquence complémentaire ; l'une et/ou l'autre de ces deux séquences d'amorces peuvent :

- en dériver par une ou plusieurs substitutions et/ou additions et/ou délétions nucléotidiques, plus particulièrement par une ou plusieurs substitutions nucléotidiques, et/ou présenter une identité de séquence d'au moins 80%, ou d'au moins 85%, ou d'au moins 90%, ou d'au moins 95% avec la séquence de ce fragment ou de sa séquence complémentaire (identité calculée sur la plus longue des deux séquences alignées - alignement optimal),
- dans la mesure où la paire d'amorces en résultant a conservé la capacité à s'hybrider spécifiquement à un desdits gènes choisis.

Une paire d'amorces de l'invention présente avantageusement un delta Tm de 1°C environ ou moins. Selon un mode de réalisation de l'invention, une paire d'amorces de l'invention cible un amplicon de 70 à 120 pb environ (c'est-à-dire que l'amorce sens et l'amorce anti-sens s'hybrident à des positions telles sur l'acide nucléique cible, que l'amplicon produit par élongation de ces amorces hybridées a une longueur de 70 à 120 pb environ).

Des exemples de telles amorces et paires d'amorces sont présentées dans le tableau 17 ci-dessous (SEQ ID NO: 1 à 44, formant 22 paires d'amorces).

La séquence d'une sonde de l'invention, peut par exemple être :

- la séquence d'un fragment de la séquence d'un desdits gènes choisis (*cf.* tableau 2 indiquant les numéros d'accession de séquences de ces gènes), ledit fragment s'hybridant de manière spécifique à la séquence de ce gène ;
- une séquence :

  o qui dérive de la séquence d'un tel fragment par une ou plusieurs substitutions et/ou additions et/ou délétions nucléotidiques, plus particulièrement par une ou plusieurs substitutions nucléotidiques, et/ou une séquence qui présente une identité de séquence d'au moins 80%, ou d'au moins 85%, ou d'au moins 90%, ou d'au moins 95% avec la séquence de ce fragment ou de sa séquence complémentaire (identité calculée sur la plus longue des deux séquences alignées -alignement optimal-), mais
  ○ qui a conservé la capacité à s'hybrider spécifiquement à un desdits gènes choisis ;

et/ou
- une séquence complémentaire de telles séquences.

Une sonde de l'invention peut notamment être une sonde pour amplification en temps réel, destinée à être utilisée avec une paire d'amorces de l'invention. Alternativement, la détection en PCR en temps réel peut utiliser des molécules dites intercalentes (par exemple ; SYB green) qui ont la propriété de s'intercaler dans les structures doubles brins.

Les ligands de l'invention qui se lient de manière spécifique aux protéines codées par les gènes choisis parmi ladite liste de vingt-deux gènes de l'invention (ou à des fragments spécifiques de ces protéines) peuvent être par exemple des protéines, polypeptides ou peptides, par exemple des aptamères, ou des anticorps ou fragments d'anticorps.

Pour chacun desdits gènes choisis, la personne du métier peut produire un tel ligand.

Des anticorps peuvent par exemple être produits par immunisation d'un mammifère non-humain (tel qu'un lapin) par une protéine codée par ledit gène choisi, ou par un fragment antigénique d'une telle protéine, éventuellement associée ou couplée à un adjuvant d'immunisation (tel qu'un adjuvant de Freund ou tel que KLH -*keyhole limpet hemocyanin*-), par exemple par injection intrapéritonéale ou sous-cutanée, et par collecte des anticorps ainsi obtenus dans le sérum dudit mammifère.

Des anticorps monoclonaux peuvent être produits selon une technique d'hybridation lymphocytaire (hybridomes) telle que la technique de Köhler and Milstein 1975 (*cf.* également US 4 376 110), la technique d'hybridomes à cellules B humaines (Kosbor *et* al. 1983; Cole *et al.* 1983), ou la technique d'immortalisation des lymphocytes à l'aide du virus d'Epstein-Barr -EBV- (Cole *et al.* 1985). De tels anticorps peuvent par exemple être des IgG, IgM, IgE, IgA, IgD ou toute sous-classe de ces immunoglobulines.

Des anticorps modifiés par génie génétique peuvent être produits, tels que des anticorps recombinant, chimères, humanisés par greffage d'un ou plusieurs CDR -*Complementary Determining Region*-.

Les anticorps mis en oeuvre dans l'invention peuvent être des fragments d'anticorps ou des dérivés artificiels de tels fragments, dans la mesure où ces fragments ou dérivés présentent ladite propriété de liaison spécifique. De tels fragments peuvent par exemple être des fragments Fab, F(ab')2, Fv, Fab/c, scFv (*single chain Fragment variable*).

Des exemples d'anticorps sont donnés dans le tableau 14 suivant.

**Tableau 14 : exemples d'anticorps spécifiques**

| Gène codant | Anticorps | Exemple de fournisseur | Référence catalogue du produit |
|---|---|---|---|
| A2M | Anticorps polyclonal de chèvre anti-alpha 2 macroglobuline humaine, 100 $\mu$g | **R&D systems** 77, boulevard Vauban 59041 Lille Cedex France | AF1938 |
| SPP1 | Anticorps polyclonal de chèvre anti-ostéopontine humaine, 100 $\mu$g | **R&D systems** | AF1433 |
| VIM | Anticorps polyclonal de lapin anti-vimentine, 1mL (0,2 mg/mL) | **Abcam** 24, rue Louis Blanc 75010 Paris ; France | ab 15248-1 |
| p14ARF transcrit n°4 du gène CDKN2A | Anticorps polyclonal de lapin anti-CDKN2A/p14ARF, 100 $\mu$L (1 mg/mL) | **Abcam** | ab53031-100 |
| CXCL10 | Anticorps polyclonal anti-CXCL10/IP-10 humain (IgG de chèvre) | **R&D systems** | AB-266-PB |
| CXCL11 | Anticorps monoclonal (clone 87328) anti-CXCL11 /I-TAC humaine (IgG2A de souris) | **R&D systems** | MAB672 |
| ENG | Anticorps monoclonal anti-ENG produit chez la souris (clone 4C11) | **Sigma-Aldrich** | WH0002022M1 |
| IL8 | Anticorps monoclonal anti-IL8 produit chez la souris (clone 6G4) | **Sigma-Aldrich** | WH0003576M5 |
| IRF9 | Anticorps anti-facteur de transcription IRF9 | **Abcam** | ab56677 |
| MMP2 | Anticorps anti-MMP2 [EP1329Y] | **Abcam** | ab51127 |
| MMP9 | Anticorps anti-MMP9 | **Abcam** | ab7299 |
| S100A4 | Anticorps polyclonal de lapin anti-S100A4, 250 $\mu$L (0,72 mg/mL) | **Abcam** | ab27957-250 |
| TIMP1 | Anticorps anti-TIMP1 | **Abcam** | ab77847 |
| ANGPT2 | Anticorps monoclonal anti-angiopoïétine 2 humaine (clone 180102), (IgG2B de souris) | **R&D systems** | MAB0983 |
| IL6ST | Anticorps monoclonal anti-gp130 humaine (clone 29104), (IgG1 de souris) | **R&D systems** | MAB2281 |
| CXCL1 | Anticorps monoclonalanti-CXCL1/GRO alpha humaine (IgG2B de souris) | **R&D systems** | MAB275 |
| COL1A1 | Anticorps polyclonal anti-COL1A1 produit chez le lapin | **Sigma-Aldrich** | HPA011795 |
| IHH | Anticorps polyclonal de lapin anti-IHH | **Abcam** | ab39634 |
| CHI3L1 | Anticorps polyclonal anti-CHI3L1 produit chez le lapin | **Sigma-Aldrich** | AV51929 |

(suite)

| Gène codant | Anticorps | Exemple de fournisseur | Référence catalogue du produit |
|---|---|---|---|
| MMP1 | Anticorps monoclonal anti-Pro-MMP1 humaine (clone 36660), (IgG1 de souris) | R&D systems | MAB900 |
| MMP7 | Anticorps monoclonal anti-Pro-MMP7 humaine (clone 6A4), (IgG1 de souris) | R&D systems | MAB907 |
| CXCL6 | Anticorps monoclonal anti-CXCL6/GCP2 humaine (clone 60910), (IgG1 de souris) | R&D systems | MAB333 |

D'autres exemples de moyens de dosage des niveaux de transcription de gènes choisis (A2M, CXCL10, CXCL8, SPP1, S100A4) sont par ailleurs présentés dans le tableau 29 ci-dessous (kits d'immuno-essai).

Lesdits réactifs peuvent en outre comprendre un marqueur pour leur détection (par exemple, un fluorophore).

Lesdits réactifs peuvent être sous la forme de composition(s), de composition(s) pharmaceutique(s), par exemple dans un(des) tube(s) ou dans un(des) puit(s) d'une plaque d'amplification d'acide nucléique

Lesdits réactifs peuvent être en mélange, ou sous formes distinctes ou physiquement séparées l'une de l'autre.

Lesdits réactifs peuvent être fixés à un support solide, par exemple un support en polymère, en plastique, notamment en polystyrène, en verre ou en silicium.

Lesdits réactifs peuvent être attachés directement ou indirectement audit support solide, par exemple *via* un agent de liaison ou de capture qui est attaché au support solide. Cet agent de liaison ou de capture peut comprendre une partie fixée audit support solide et une partie qui comprend un ligand qui se lie de manière spécifique à un desdits gènes choisis. Un tel ligand peut par exemple être un anticorps, un anticorps monoclonal, notamment un anticorps humain tel qu'une IgG, IgM ou IgA, ou un fragment d'un tel anticorps ayant conservé la spécificité de liaison.

Ledit support solide peut par exemple être une plaque en plastique, notamment en polystyrène, comprenant plusieurs puits d'analyse, telle qu'une plaque de titrage ou microtitrage protéique, par exemple, une plaque ELISA.

Ledit support solide peut encore être des microbilles magnétiques ou non, pour micro-titrages, par exemple selon la technique décrite par Luminex.

Ledit support solide peut par exemple être une puce à acide nucléique, à protéine ou à peptide, par exemple une puce en plastique, verre ou silicium.

Lesdits réactifs peuvent ne pas être fixés à un support solide et peuvent par exemple être contenus dans une solution, telle qu'un tampon, par exemple pour leur conservation jusqu'à utilisation. Plus particulièrement, les réactifs peuvent être des acides nucléiques non liés à un support solide, dont la séquence nucléotidique est adaptée à l'amplification spécifique (cas d'amorces ou de paires d'amorces) et/ou à l'hybridation spécifique (cas de sondes) du produit de transcription (ARN) d'un desdits gènes choisis parmi ladite liste de vingt-deux gènes de l'invention.

En sus de réactifs qui détectent les produits de transcription ou de traduction de gènes de mammifères, plus particulièrement de gènes humains, et notamment de gènes choisis parmi ladite liste de vingt-deux gènes de l'invention, un article manufacturé conforme à l'invention peut optionnellement comprendre d'autres réactifs, par exemple des réactifs permettent de doser ou déterminer un ou plusieurs facteurs virologiques et/ou un ou plusieurs facteurs cliniques.

Par exemple, un article manufacturé conforme à l'invention peut comprendre des réactifs qui détectent de manière spécifique un ou des virus de l'hépatite, et/ou son ou leur génotype.

Selon un mode de réalisation, l'invention est relative à un article manufacturé comprenant des réactifs en préparation combinée pour leur utilisation simultanée, séparée ou étalée dans le temps, lesdits réactifs étant constitués :

- de réactifs qui détectent de manière spécifique (de préférence, qui détectent de manière spécifique et permettent de quantifier) chacun des produits de transcription ou de traduction de 3 à 22 gènes de mammifères, plus particulièrement de 3 à 22 gènes humains, (par exemple, en s'hybridant de manière spécifique à l'ARN de ces gènes et/ou à l'ADNc obtenu par transcription inverse de ces ARN, ou en se liant de manière spécifique aux protéines codées par ces gènes), lesdits 3 à 22 gènes de mammifères, ou, le cas échéant, lesdits 3 à 22 gènes humains, comprenant lesdits gènes choisis parmi ladite liste de vingt-deux gènes de l'invention,
Et
- optionnellement, de réactifs qui permettent de détecter, doser ou déterminer un ou plusieurs facteur(s) virologique(s) tel(s) que défini(s) dans la description de l'invention ; et/ou un ou plusieurs facteur(s) biologique(s) autre(s) tel(s) que défini(s) dans la description de l'invention ;

- optionnellement, de réactifs qui détectent de manière spécifique (de préférence qui détectent de manière spécifique et permettent de quantifier) un virus de l'hépatite et/ou le génotype d'un virus de l'hépatite.

Dans cet article manufacturé, le nombre de gènes de mammifères, plus particulièrement de gènes humains dont les produits de transcription ou de traduction peuvent être détectés est de 3 à 40, plus particulièrement de 3 à 36, plus particulièrement de 3 à 33, plus particulièrement de 3 à 28, plus particulièrement de 3 à 26, plus particulièrement de 3 à 25, plus particulièrement de 3 à 24, plus particulièrement de 3 à 23, plus particulièrement de 3 à 22, plus particulièrement de 3 à 20, plus particulièrement de 3 à 21, plus particulièrement de 3 à 20, plus particulièrement de 3 à 19, plus particulièrement de 3 à 18, plus particulièrement de 3 à 17, plus particulièrement de 3 à 16, plus particulièrement de 3 à 15, plus particulièrement de 3 à 14, plus particulièrement de 3 à 13, plus particulièrement de 3 à 12, plus particulièrement de 3 à 11, plus particulièrement de 3 à 10, plus particulièrement de 3 à 9, plus particulièrement de 3 à 8, plus particulièrement de 3 à 7, plus particulièrement de 3 à 6, plus particulièrement de 3 à 5, par exemple de 3, 4 ou 5, notamment de 4 ou 5.

Les gènes de mammifères, plus particulièrement les gènes humains, dont les produits de transcription ou de traduction peuvent être détectés par les réactifs contenus dans l'article manufacturé de l'inventioncomprennent lesdits gènes choisis parmi ladite liste de vingt-deux gènes de l'invention, et optionnellement d'autres gènes, qui ne sont pas des gènes choisis parmi ladite liste de vingt-deux gènes de l'invention, mais dont le produit d'expression, plus particulièrement, de traduction peut être d'intérêt, tels que les gènes ici listés en tant que « autres facteurs biologiques » (par exemple, le gène codant l'alanine-amino-transférase).

Le nombre de gènes choisis parmi ladite liste de vingt-deux gènes de l'invention est au maximum de 22 gènes (SPP1, et au moins un gène parmi A2M et VIM, et au moins un gène parmi IL8, CXCL10 et ENG, et optionnellement, au moins un gène parmi la liste de seize gènes). Avantageusement, ce nombre peut être inférieur à 22 : ce nombre peut plus particulièrement être de 3 à 10, plus particulièrement de 3 à 9, plus particulièrement de 3 à 8, plus particulièrement de 3 à 7, plus particulièrement de 3 à 6, plus particulièrement de 3 à 5, par exemple de 3, 4 ou 5, notamment de 4 ou 5.

Dans l'article manufacturé de l'invention, le nombre de réactifs qui détectent de manière spécifique le produit d'expression de gènes de mammifère (plus particulièrement de gènes humains) qui ne sont pas des gènes choisis parmi ladite liste de vingt-deux gènes de l'invention (par exemple, un réactif détectant de manière spécifique ALT), est de préférence au maximum de 18, plus particulièrement de 14 ou moins, plus particulièrement de 11 ou moins, plus particulièrement de 6 ou moins, plus particulièrement de 4 ou 3 ou 2, plus particulièrement de 1 ou 0.

Ledit article manufacturé peut donc par exemple être :

- un ou des tubes,
- un kit, notamment un kit comprenant un ou des tubes,
- un support solide, par exemple, en plastique, polystyrène, verre, silicium ou polymère, ou comprenant un matériau magnétique tel que de l'oxyde de fer, tel que :

    ◦ une plaque en plastique comprenant plusieurs puits d'analyse telle que :

        ▪ une plaque d'amplification d'acide nucléique comportant des puits destinés à recevoir un échantillon biologique et un mélange réactionnel d'amplification d'acide nucléique,
        ▪ une plaque de titrage ou microtitrage protéique, plus particulièrement une plaque ELISA,

    ◦ des microbilles magnétiques (par exemple, des microbilles faites à partir d'oxyde de fer, et recouvertes d'un polymère auquel les protéines ou polypeptides peuvent adhérer ou être attachées par couplage chimique) ;
    ◦ une puce à acide nucléique, à protéine, à polypeptide ou à peptide.

Optionnellement, l'article manufacturé de l'invention comprend en outre des instructions (par exemple, une feuille d'instructions) pour doser le niveau d'expression desdits gènes choisis sur un échantillon biologique collecté ou obtenu dudit sujet, plus particulièrement pour mettre en oeuvre une méthode de l'invention.

Ledit article manufacturé peut en outre comprendre un ou plusieurs des éléments suivants :

- un instrument pour le prélèvement dudit échantillon, notamment :

    ◦ une aiguille et/ou une seringue, plus particulièrement une aiguille et/ou une seringue pour le prélèvement d'un liquide intracorporel, tel que du sang, et/ou
    ◦ une aiguille adaptés à la cyto-ponction hépatique, par exemple une aiguille de diamètre 18 à 22G), et/ou

◦ une aiguille et/ou un cathéter et/ou un pistolet à biopsie adaptés à la PBH ;

- un produit programme d'ordinateur ou logiciel, notamment un produit programme d'ordinateur ou logiciel d'analyse statistique, par exemple un produit programme d'ordinateur de l'invention tel que ci-dessous décrit ;
- des réactifs d'extraction d'ARN ;
- une transcriptase inverse ;
- une polymérase, par exemple une Taq polymérase ;
- des nucléotides (dNTP).

L'invention est notamment relative audit article manufacturé, ou auxdits réactifs, pour leur utilisation dans une méthode pour détecter ou diagnostiquer une hépatopathie qui comporte une atteinte tissulaire du foie, plus particulièrement une fibrose hépatique, plus particulièrement pour déterminer le score de fibrose hépatique d'un sujet, avantageusement pour déterminer si la fibrose hépatique d'un sujet a un score de fibrose Metavir d'au plus F1 ou bien d'au moins F2.

L'invention est notamment relative audit article manufacturé, ou auxdits réactifs, pour leur utilisation dans une méthode de l'invention.

Cette utilisation peut notamment comprendre :

- le prélèvement d'un échantillon biologique dudit sujet, notamment par insertion d'une aiguille ou cathéter dans le corps dudit sujet, et
- la mise en oeuvre desdits réactifs dans ladite méthode sur cet échantillon biologique, ou sur un échantillon comprenant des acides nucléiques et/ou protéines et/ou polypeptides et/ou peptides extraits ou purifiés dudit échantillon biologique, ou sur un échantillon comprenant des ADNc susceptibles d'avoir été obtenus par transcription inverse desdits acides nucléiques.

Cette utilisation peut par exemple comprendre :

- le prélèvement d'un échantillon biologique dudit sujet, optionnellement transformé par :

  ◦ extraction ou purification des ARN dudit échantillon prélevé et, optionnellement par transcription inverse des ARN extraits, ou par
  ◦ extraction ou purification de ses protéines dudit échantillon prélevé, et

- la mise en oeuvre desdits réactifs de l'invention sur cet échantillon biologique optionnellement transformé.

Ledit prélèvement d'échantillon biologique peut être fait par insertion d'un instrument de prélèvement, notamment par insertion d'une aiguille ou d'un cathéter, dans le corps dudit sujet.

L'insertion de l'instrument de prélèvement est notamment faite pour prélever du fluide intracorporel dudit sujet (tel que par exemple du sang) et/ou une portion de tissu hépatique dudit sujet (par exemple par PBH) et/ou des cellules hépatiques dudit sujet (par exemple par cyto-ponction hépatique).

Cet instrument peut donc par exemple être inséré :

- dans une veine, une artère ou un vaisseau sanguin dudit sujet pour prélever du sang dudit sujet ; et/ou
- dans le foie dudit sujet, pour prélever un échantillon de parenchyme hépatique, c'est-à-dire pour faire une ponction biopsie hépatique (PBH), par exemple par voie transjugulaire ou par voie transpariétale ; et/ou
- à travers la peau jusqu'au foie dudit sujet, de sorte à faire une cyto-ponction hépatique.

L'invention est notamment relative audit article manufacturé, ou auxdits réactifs, pour leur utilisation dans une méthode pour le traitement d'une hépatopathie qui comporte une atteinte tissulaire du foie, plus particulièrement une fibrose hépatique.

Cette utilisation peut notamment comprendre :

- la mise en oeuvre desdits réactifs dans une méthode de l'invention pour déterminer le score de fibrose hépatique dudit sujet, et
- le fait d'administrer audit sujet un traitement visant à bloquer la progression de la fibrose hépatique, tel que interféron standard ou pégylé, en monothérapie, ou en plurithérapie associant ribavirine, si le sujet présente un score de fibrose hépatique qui, lorsqu'il est exprimé selon le système Metavir, est d'au moins F2.

Cette utilisation peut par exemple comprendre :

- la mise en oeuvre desdits réactifs de l'invention sur échantillon biologique qui a été prélevé dudit sujet, et qui optionnellement a été transformé, par exemple :

  ◦ par extraction et/ou purification des ARN dudit échantillon prélevé et, optionnellement par transcription inverse des ARN extraits, ou
  ◦ par extraction et/ou purification des protéines et/ou polypeptides et/ou peptides dudit échantillon prélevé,

  pour détecter ou diagnostiquer une hépatopathie qui comporte une fibrose hépatique, plus particulièrement pour déterminer le score de fibrose hépatique dudit sujet, avantageusement pour déterminer si la fibrose hépatique dudit sujet a un score de fibrose Metavir :

  ◦ d'au plus F1 (c'est-à-dire une fibrose hépatique sans septa), ou bien
  ◦ d'au moins F2 (c'est-à-dire une fibrose hépatique avec septas),

- optionnellement, dans le cas d'une hépatopathie impliquant un virus de l'hépatite, notamment un VHC et/ou un VHB et/ou un VHD, notamment au moins un VHC, déterminer le génotype de ce virus, et
- le fait d'administrer un traitement visant à bloquer ou ralentir la progression de la fibrose hépatique, si le sujet présente un score de fibrose Metavir d'au moins F2.

Cette méthode peut en outre comprendre le fait de ne pas administrer ce traitement si, ou tant que, ce score est d'au plus F1.

Ledit traitement peut par exemple être un traitement par interféron standard ou interféron pégylé, en monothérapie, ou en plurithérapie associant un ou plusieurs autres principes actifs, notamment de la ribavirine et/ou un inhibiteur de protéase virale et/ ou inhibiteur de polymérase virale (par exemple, en combinaison thérapeutique, notamment en bithérapie ou trithérapie).

Ce traitement peut par exemple être :

- interféron alpha-2b pégylé (tel que PEG-INTRON®, Schering Plough Corporation, Kenilworth, NJ) à une dose d'environ 1,5 g/kg/semaine, et ribavirine (REBETOL® ; Schering Plough Corporation, Kenilworth, NJ) à une dose de 800 à 1200 mg/kg/jour (si l'hépatopathie implique un VHC de génotype 2 ou 3, une dose d'environ 800 mg/kg et par jour est généralement conseillée), ou
- interféron alpha-2a pégylé (tel que PEGASYS® ; Roche Corp., F. Hoffmann-La Roche Ltd. ; Basel, Suisse) à une concentration de 180 g/kg/semaine et ribavirine (COPEGUS®, Roche Corp. ; F. Hoffmann-La Roche Ltd. ; Basel, Suisse) à raison de 1000 à 1200 mg/kg/jour.

La durée du traitement peut par exemple être d'au moins 24 semaines, par exemple de 24 semaines pour une hépatopathie à VHC de génotype 2 ou 3, ou de 48 semaines pour une hépatopathie à VHC de génotype 1, 4 ou 5, ou pour un patient non-répondeur au traitement au bout de 24 semaines.

L'invention est également relative à un médicament ou association médicamenteuse destiné au traitement d'une hépatopathie comportant une atteinte tissulaire du foie, plus particulièrement une fibrose hépatique (tel que interféron standard ou interféron pégylé, en monothérapie, ou en plurithérapie associant un ou plusieurs autres principes actifs, notamment de la ribavirine), pour son utilisation dans la méthode de traitement de l'invention.

Dans la description, le terme hépatopathie est entendu dans son sens ordinaire, à savoir une atteinte du foie, plus particulièrement une atteinte tissulaire du foie, plus particulièrement des lésions du foie, notamment une fibrose hépatique.

L'invention s'adresse plus particulièrement aux hépatopathies chroniques (agressions chroniques du foie de 6 mois ou plus).

Différentes maladies causent et /ou conduisent à des lésions du foie, telles qu'une fibrose hépatique. Peuvent notamment être citées :

- les hépatites chroniques virales (notamment l'hépatite chronique B, l'hépatite chronique C, l'hépatite chronique D ;
- les stéatoses et stéato-hépatites (associées au syndrome métabolique, à l'obésité, au diabète).
- les hépatites alcooliques ;
- l'hématochromatose génétique et les surcharges en fer secondaire
- Les maladies auto-immunes ;
- les maladies biliaires (cirrhose biliaire primitive et cholangie sclérosante primitive) ;

- les intoxications par médicament ou substance toxique ;
- des maladies métaboliques.

L'invention est plus particulièrement adaptée aux hépatites virales, notamment aux hépatites à virus C (VHC) et/ou à virus B (VHB) et/ou à virus D (VHD), notamment aux hépatites virales à au moins VHC (et optionnellement à VHB et/ou VHD).

L'invention peut être mise en oeuvre par un **produit programme d'ordinateur**, destiné à être stocké dans une mémoire d'une unité de traitement, ou sur un support mémoire amovible destiné à coopérer avec un lecteur de ladite unité de traitement. Le produit programme d'ordinateur comprend des instructions pour la mise en oeuvre d'une méthode de l'invention, notamment pour la mise en oeuvre d'une analyse statistique adaptée à la mise en oeuvre d'une méthode de l'invention (notamment adaptée à l'analyse statistique multivariée des niveaux d'expression desdits gènes choisis) et/ou pour la construction d'un modèle de classification multivariée adapté à la mise en oeuvre d'une méthode de l'invention.

L'invention peut également être mise en oeuvre via une **installation informatique**, un dispositif informatique, ou ordinateur, comprenant une unité de traitement dans la mémoire de laquelle sont stockés ou enregistrés :

- un produit programme d'ordinateur de l'invention, et, optionnellement,
- des dosages, ou des valeurs de dosage, des niveaux d'expression (transcription et/ou traduction) desdits gènes choisis.

Le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé. Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où cet(ces) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et " essentiellement constitué".

Dans le but de faciliter la lecture de l'invention, la description a été séparée en divers paragraphes, sections et modes de réalisation. Il ne doit pas être considéré que ces séparations déconnectent la substance d'un paragraphe, section ou mode de réalisation, de celle d'un autre paragraphe, section ou mode réalisation. Au contraire, la description englobe toutes les combinaisons possibles des différents paragraphes, sections, phrases et modes de réalisations qu'elle contient.

Les exemples qui suivent sont donnés à titre purement illustratif. Ils ne limitent en aucune façon l'invention.

## EXEMPLES

### EXEMPLE 1 : CONSTRUCTION DE MODELES DE CLASSIFICATION

#### 1. Populations et patients, détermination du score de fibrose hépatique, dosage du niveau d'expression de gènes :

Les biopsies de foie ont été réalisées à partir d'une cohorte de patients adultes suivis à l'Hôpital Beaujon (Clichy, France), présentant une hépatite chronique suite à une infection par le virus de l'Hépatite C (VHC). Les biopsies ont été immédiatement stockées à -80°C en vue de l'extraction des ARN totaux, et traitées avec de la paraffine pour des études histologiques.

L'étude réalisée a été approuvée par le Comité Ethique local, conformément à la Déclaration d'Helsinki et tous les patients ont donné leur consentement éclairé par écrit. Les ponctions biopsies hépatiques ont été réalisées en respectant les bonnes pratiques cliniques, et l'histologie a été interprétée par un anatomo-pathologiste avec le score d'activité et fibrose (score Metavir).

Présentation des patients

Le diagnostic clinique d'infection par le virus de l'hépatite C des patients sélectionnés a été établi sur la base de la détection d'anticorps dirigés contre des protéines du VHC et de la détection d'ARN circulant du VHC.

La sérologie du VHC de dépistage a été réalisée par le test de quantification de l'ARN de VHC VERSANT® HCV-RNA 3.0 (bDNA) ASSAY de la société Siemens Healthcare Diagnostics (limite de quantification = 615 - 7 690 000 UI/mL).

Les patients sont des patients infectés par le virus de l'hépatite C. Afin d'établir une cohorte homogène et parfaitement représentative de la pathologie exemplifiée, les patients susceptibles de présenter des affections chroniques hépatiques d'origines autres que le virus de l'hépatite C (telles qu'une affection chronique hépatique due à une infection par le virus de l'hépatite B) ont été exclus de l'étude.

D'autres critères d'exclusion ont en outre été appliqués, à savoir consommation d'alcool excessive, hémochromatose, hépatite auto-immune, maladie de Wilson, déficience en $\alpha$-1 antitrypsine, angiocholite sclérosante primitive, cirrhose biliaire primitive, la prise antérieure d'un traitement anti-VHC. Les patients ayant déjà bénéficié d'un traitement antiviral dans le cadre du suivi de leur hépatite chronique C ont également été exclus de l'étude.

Le stade de fibrose hépatique a été déterminé par l'examen anatomo-pathologique d'un prélèvement de tissus hépatiques (ponction biopsique hépatique ou PBH). Cet examen a été réalisé par deux lectures indépendantes par un anatomo-pathologiste confirmé. Le stade de fibrose hépatique a été défini selon la classification METAVIR ainsi que selon la classification Ishak (*cf.* tableau 1 ci-dessus, pour la correspondance entre les deux systèmes de score).

Un échantillon de sérum a été prélevé sur chacun des patients inclus dans l'étude dans un délai de +/- 6 mois par rapport à la date de la biopsie.

Deux cent quarante-quatre patients ont été sélectionnés sur la base de leur stade de fibrose hépatique déterminée selon les classifications METAVIR et Ishak. Les deux cent quarante-quatre patients sélectionnés présentent un score de fibrose Métavir F1 ou F2, et/ou un score de fibrose Ishak F1/F2 ou F3.

Le tableau 15 ci-dessous présente les données cliniques, biologiques et virologiques des patients ainsi sélectionnés.

**Tableau 15 :**

| Données cliniques, biologique et virologiques | Patients | Patients F1 | Patients F2 |
|---|---|---|---|
| Cohorte (n) | 244 | 162 | 82 |
| homme (%)/ femme (%) | 114 (47)/130(53) | 69(43)/93(57) | 45(55)/37(45) |
| Age | | | |
| moyenne $\pm$ SD | 50,2 $\pm$ 11.1 | 50 $\pm$ 10,5 | 50,5 $\pm$ 12,1 |
| Min-Max | 18-71 | 21-71 | 18-70 |
| Source d'infection (n(%)) | | | |
| - Transfusion sanguine | 62 (25) | 41(25) | 21(26) |
| - Toxicomanie | 49 (20) | 34 (21) | 15 (18) |
| - Inconnue | 133 (55) | 87 (54) | 46 (56) |
| Alanine aminotranférase (ALT) UI/L : | | | |
| moyenne $\pm$ SD | 92 $\pm$ 78 | 87 $\pm$ 89 | 101 $\pm$ 55 |
| Min-Max | 22-647 | 22-510 | 32-647 |
| Génotypes VHC, n (%) | | | |
| - 1 | 135 (55,3) | 81 (50) | 54(65,9) |
| - 2 | 27 (11,1) | 23 (14,2) | 4 (4,9) |
| - 3 | 26 (10,7) | 15 (9,3) | 11 (13,4) |
| - 4 | 49 (20,1) | 38 (23,4) | 11 (13,4) |
| - 5 | 4 (1,6) | 3 (1,9) | 1 (1,2) |
| - 6 | 2 (0,8) | 1 (0,6) | 1 (1,2) |
| - Inconnu | 1 (0,4) | 1 (0,6) | 0(0) |
| Nombre de copies virales par mL de sérum : Moyenne (Min-Max) | $2,28.10^6$ ($3,2.10^3$-$1,9.10^8$) | $1,83.10^6$ ($3,1.10^4$-$1,9.10^8$) | $3,28.10^6$ ($3,2.10^3$-$5,9.10^7$) |
| UI = Unité Internationale | | | |

Les niveaux d'expression des gènes ont été dosés sur chacune des 244 biopsies (1 biopsie par patient).

Traitement des échantillons

Les biopsies hépatiques ont été broyées dans de l'azote en utilisant un pilon et un mortier en céramique (broyage 100% manuel).
La poudre a été récupérée à l'aide d'un scalpel (Swann Morton 22, Référence 0208).

a) Extraction des ARN

La poudre ainsi obtenue a été solubilisée dans 1 mL de RNAble® Réf. GEXEXT00, Laboratoires Eurobio, France, auquel ont été ajoutés 100 $\mu$L de chloroforme.
Le mélange obtenu a été placé dans la glace ou à 4°C pendant 5 minutes puis a été centrifugé à 13 000 g pendant 15 minutes.
La phase supérieure aqueuse contenant les ARN a été récupérée dans un tube neuf et 1 volume d'isopropanol y a été ajouté.
Le tube a été agité par retournement et a été placé à 4°C toute la nuit, puis a été centrifugé à 13 000 g pendant 15 minutes. Le surnageant a été éliminé, et le culot contenant les ARN a été repris dans un volume d'éthanol à 70% (préparé extemporanément) et à nouveau centrifugé.
Le culot de précipité d'ARN obtenu a été séché à l'air libre pendant environ 1 heure, puis dissout dans 15 $\mu$L d'eau puis stocké à -80°C.

b) Dosage des ARN

L'évaluation de la concentration des ARN extraits a été réalisée par la mesure de la densité optique par un spectromètre (Nanodrop), et a été vérifiée après un cycle de congélation/ décongélation.
Les ARN extraits ont ensuite été dilués pour obtenir une solution à 50 ng/$\mu$L.
Les contrôles qualités de l'ARN ont été réalisés par PCR temps réel (*cf.* ci-dessous), par ciblage d'un gène contrôle (dit endogène), à expression ubiquitaire, pour vérifier que l'ARN n'a pas été dégradé (en l'occurrence, ciblage de RPLPO).

Etape de transcription inverse (*Reverse Transcription* ou RT) :

La transcription inverse a été réalisée sur 200 ng d'ARN dans un mélange réactionnel réalisé dans un volume de 20 $\mu$L comprenant les réactifs suivants :

**Tableau 16 :**

| Réactif et référence produit | Solution de départ | Volume |
|---|---|---|
| Transcriptase inverse SUPERSCRIPT II RNase H, Invitrogen, réf: 18064014 | 200 U/$\mu$L | 0,5 $\mu$L |
| Tampon SUPER SCRIPT 5X Invitrogen, réf : 18064014 | - | 4,0 $\mu$L |
| RNAsin Promega, réf: N2111 | 40 U/$\mu$L | 0,5 $\mu$L |
| DTT | 100 mM | 2,0 $\mu$L |
| Les 4 dNTP GE Healthcare, réf: 28406552 | 10 mM | 1$\mu$L |
| Amorces Pd(N) RANDOMS HEXAMERS<br>50 (A260) units,<br>51 Perbio, réf : MB216601 | 0,5 $\mu$g/$\mu$L | 6,0 $\mu$L |
| ARN | 50 ng/$\mu$L | 4,0 $\mu$L |
| H$_2$0 | | QSP 20 $\mu$L |

Les réactions de transcription inverse ont été effectuées aux températures suivantes :

- à 20°C pendant 10 minutes, puis

- à 42°C pendant 30 minutes, et

- à 99°C pendant 5 minutes.

A ce stade, les mélanges réactionnels ont été congelés, ou aliquotés, ou directement utilisés pour une amplification par PCR temps réel.

Etape de PCR en temps réel quantitative (PCRq) :

L'amplification a été réalisée en utilisant un light Cycler® 480 (Roche Diagnostics, Mannheim, Allemagne). Les résultats sont générés par le logiciel light Cycler® Software 4.05/4.1.

La technologie Light Cycler® permet le suivi en continu de l'apparition des produits d'amplification grâce à l'émission d'une quantité de fluorescence proportionnelle à la quantité de produit amplifié, elle-même dépendante de la quantité de cibles initialement présentes dans l'échantillon à analyser. La quantification (en valeurs relatives) de l'expression génique est réalisée par la méthode connue sous le nom de $2^{-\Delta Ct}$ ($2^{-\Delta Ct} = 2^{-(Ct\text{cible}-Ct\text{ référence})}$) ; *cf.* Livak et Schmittgen 2001 ; Schmitten et Livak 2008), exploitant les valeurs de « Cycle Threshold » ou Ct déterminées par l'appareil de PCR quantitative en temps réel. Plus la valeur de Ct est faible, plus la quantité initiale d'ARN transcrit est élevée.

Les mélanges réactionnels et le protocole utilisés sont décrits dans la notice de la trousse light Cycler® 480 SYBR Green I Master mix (Roche Diagnostics, Mannheim, Allemagne ; US 4,683,202 ; US 4,683,195 ; US 4,965,188 ; US 6,569,627).

Après l'étape de transcription inverse, les mélanges réactionnels (cDNAs) ont été dilués au 1/40ème (pour la vérification de la qualité) ou au 1/100ème (pour les gènes cibles) avant leur utilisation en PCRq.

Pour chaque gène, les PCRq ont été réalisées dans un volume réactionnel de 10 μL sur une plaque à 384 puits :

- 5 μL de la réaction de transcription inverse diluée au 1/40ème (ou au 1/100ème) ;
- 4,8 μL de mélange réactionnel de la trousse light Cycler® 480 SYBR Green I Master mix
- 0,1 μL d'une solution à 50μM pour chacune des deux amorces, soit un volume final de 0,5 μM pour chaque amorce.

Les mélanges réactionnels sont généralement préparés pour des plaques de 384 puits.
Les amorces suivantes ont été utilisées :

**Tableau 17 :**

| Symbole | Amorce sens | SEQ ID NO : | Amorce antisens | SEQ ID NO : |
|---------|-------------|-------------|-----------------|-------------|
| A2M | GCAAGTAAAAACCAAGGTCTTCCA | 1 | TCCAGTCAATTCCACCACTGTTC | 2 |
| SPP1 | TCGCAGACCTGACATCCAGTACC | 3 | CCATTCAACTCCTCGCTTTCCAT | 4 |
| VIM | CTCCCTCTGGTTGATACCCACTC | 5 | AGAAGTTTCGTTGATAACCTGTCCA | 6 |
| CXCL10 | CTGACTCTAAGTGGCATTCAAGGAG | 7 | GGTTGATTACTAATGCTGATGCAGG | 8 |
| IL8 | CACCGGAAGGAACCATCTCACTGT | 9 | TCCTTGGCAAAACTGCACCTTCA | 10 |
| ENG | CACAACATGCAGATCTGGACCACT | 11 | TGGGAGCTTGAAGCCACGAA | 12 |
| ANGPT2 | ACGTGAGGATGGCAGCGTT | 13 | GAAGGGTTACCAAATCCCACTTTAT | 14 |
| p14ARF | GGTTTTCGTGGTTCACATCCC | 15 | CCCATCATCATGACCTGGTCTT | 16 |
| CHI3L1 | GACCACAGGCCATCACAGTCC | 17 | TGTACCCCACAGCATAGTCAGTGTT | 18 |
| COL1A1 | CCTCCGGCTCCTGCTCCTCTT | 19 | GGCAGTTCTTGGTCTCGTCACA | 20 |
| CXCL1 | TCGAAAAGATGCTGAACAGTGACA | 21 | CTTCAGGAACAGCCACCAGTGA | 22 |
| CXCL6 | GTTTACGCGTTACGCTGAGAGTAAA | 23 | CGTTCTTCAGGGAGGCTACCA | 24 |
| CXCL11 | GTGTGCTACAGTTGTTCAAGGCTT | 25 | CTCAATATCTGCCACTTTCACTGCT | 26 |
| IHH | AGGCCGGCTTTGACTGGGTGTATT | 27 | GCGGCCGAGTGCTCGGACTT | 28 |
| IL6ST | CCTGCCTGTGACTTTCAAGCTACT | 29 | CATTCCACCCAAAGCATGTTATCT | 30 |

(suite)

| Symbole | Amorce sens | SEQ ID NO : | Amorce antisens | SEQ ID NO : |
|---|---|---|---|---|
| IRF9 | GGCCGCATGGATGTTGCTGAG | 31 | TCTGAGTCCCTGGCTGGCCAGA | 32 |
| MMP1 | GGCTTGAAGCTGCTTACGAATTT | 33 | ACAGCCCAGTACTTATTCCCTTTGA | 34 |
| MMP2 | ACTGCGGTTTTCTCGAATCCA | 35 | GGTATCCATCGCCATGCTCC | 36 |
| MMP7 | AGTGGGAACAGGCTCAGGACTATC | 37 | GGTAGGCCAAAGAATTTTTGCATC | 38 |
| MMP9 | CGGCTTGCCCTGGTGCAGT | 39 | CGTCCCGGGTGTAGAGTCTCTG | 40 |
| S100A4 | CTCGGGCAAAGAGGGTGACAA | 41 | GCTTCATCTGTCCTTTTCCCCAA | 42 |
| TIMP1 | GAGCCCCTGGCTTCTGGCA | 43 | GCCCTGATGACGAGGTCGGAA | 44 |
| RPLP0 | GGCGACCTGGAAGTCCAACT | 45 | CCATCAGCACCACAGCCTT | 46 |

Les PCRq ont été réalisées suivant les conditions de température suivantes :

- une étape d'initiation de la dénaturation à 95°C pendant 10 minutes ;
- 50 cycles de :

  - dénaturation à 95°C pendant 15 secondes ;
  - hybridation / élongation à 65°C pendant 30 secondes.

Chaque échantillon cible est amplifié en duplicate.

Afin de s'affranchir des variations des quantités initiales d'ARN total d'un échantillon à l'autre, on réalise en parallèle sur le même échantillon l'amplification en duplicate des ARN d'un gène utilisé comme contrôle endogène, tel qu'un gène impliqué dans des cascades métabolique cellulaires, par exemple RPLP0 (aussi connu sous le nom 36B4 ; Genbank accession NM_001002) ou TBP (Genbank accession NM_003194). En l'occurrence, c'est le gène RPLP0 qui a été ici utilisé comme contrôle endogène.

La qualité de l'extraction d'ARN faite à partir des 244 biopsies a été évaluée sur la base de la valeur de Ct du gène de référence RPLP0. La classification a été réalisée de la façon suivante :

- Ct RPL0 inférieur à 22 : qualité ARN très bonne ;
- Ct RPLP0 de 22 à 24 : qualité ARN bonne ;
- Ct RPLP0 supérieur à 24 et inférieur à 26 : qualité ARN moyenne ;
- Ct RPLP0 supérieur ou égal à 26 : qualité ARN mauvaise.

Afin d'augmenter la fiabilité des analyses bio-stastistiques, seules les données provenant d'extraction d'ARN de très bonne qualité (Ct RPLP0 < 22) ont été retenues, soit 158 biopsies (64,8% des 244 échantillons).

La quantité de transcrits d'un gène cible a été déduite du Ct (« *Cycle threshold* ») qui correspond au nombre de cycles de PCR nécessaire pour obtenir un signal de fluorescence significatif. Les échantillons cibles ont été normalisés sur la base de leur contenu en RPLP0 (ou, le cas échéant, en TBP), conformément à la méthode du $2^{-\Delta Ct}$.

Les valeurs de dosage des biomarqueurs ou BMQ (concentration en ARN, en l'occurrence valeur de Ct normalisée par la méthode du $2^{-\Delta Ct}$) obtenues pour chacun des 158 patients sont présentées dans les tableaux 24 à 27 ci-dessous.

**Tableau 24 : données cliniques, biologiques et virologiques**

| Données cliniques, biologiques et virologiques | Patients | Patients F1 | Patients F2 |
|---|---|---|---|
| n | 158 | 102 | 56 |
| Sexe : mâle (%) / femelle (%) | 71 (45) / 87 (55) | 41 (40) / 61 (60) | 30 (54) / 26 (46) |
| Age [moyenne $\pm$ déviation standard (Min-Max)] | 48,3 $\pm$ 11,0 (19 - 71) | 51,2 $\pm$ 11,2 (19 - 71) | 46,7 $\pm$ 11,0 (24 - 70) |
| Source d'infection [n(%)] | | | |

(suite)

| Données cliniques, biologiques et virologiques | Patients | Patients F1 | Patients F2 |
|---|---|---|---|
| transfusion sanguine | 37 (23) | 23 (23) | 14 (25) |
| toxicomanie | 33 (21) | 23 (23) | 10 (18) |
| inconnue | 88 (56) | 56 (55) | 32 (57) |
| Alanine aminotransférase (ALT) UI/L [moyenne ± déviation standard (Min-Max)] | 85 ± 60 (22 - 458) | 75 ± 48 (22 - 299) | 104 ± 73 (36 - 458) |
| Génotypes VHC [n(%)] | | | |
| 1 | 86 (54) | 49 (48) | 37(66) |
| 2 | 21 (13) | 18 (18) | 3 (5) |
| 3 | 15 (9) | 9 (9) | 6 (11) |
| 4 | 31 (20) | 23 (23) | 8 (14) |
| 5 | 2 (1) | 1 (1) | 1(2) |
| 6 | 2 (1) | 1 (1) | 1 (2) |
| inconnu | 1 (1) | 1 (1) | 0 (0) |
| Nombre de copies virales par mL de sérum : moyenne (Min-Max) | $6{,}2.10^6$ ($3{,}2.10^3$- $1{,}9.10^8$) | $6{,}5.10^6$ ($4{,}8.10^4$- $1{,}9.10^8$) | $5{,}8.10^6$ ($3{,}2.10^3$- $5{,}9.10^7$) |

**Tableau 25 : valeurs BMQ des patients pour les gènes SPP1, A2M, VIM, IL8, CXCL10, ENG (Ct normalisé conformément à la méthode du $2^{-\Delta Ct}$)**

| Patient | Statut (F1 ou F2) | SPP1 | A2M | VIM | IL8 | CXCL10 | ENG |
|---|---|---|---|---|---|---|---|
| 1 | F1 | 0,228 | 3,387 | 0,346 | 4,332 | 0,574 | 1,141 |
| 8 | F1 | 0,050 | 4,141 | 0,170 | 0,000 | 1,297 | 1,371 |
| 9 | F1 | 0,071 | 1,495 | 0,117 | 0,625 | 2,078 | 0,997 |
| 11 | F1 | 0,242 | 4,014 | 0,360 | 2,971 | 1,860 | 1,454 |
| 16 | F1 | 0,105 | 1,352 | 0,120 | 1,237 | 0,000 | 0,388 |
| 22 | F1 | 0,133 | 1,676 | 0,176 | 6,791 | 0,346 | 0,674 |
| 25 | F1 | 0,054 | 6,543 | 0,167 | 1,546 | 0,357 | 1,597 |
| 26 | F1 | 0,755 | 8,754 | 0,380 | 0,923 | 2,370 | 1,899 |
| 32 | F1 | 0,167 | 3,399 | 0,125 | 4,515 | 0,549 | 1,157 |
| 33 | F1 | 0,551 | 3,643 | 0,256 | 69,878 | 17,692 | 0,946 |
| 38 | F1 | 0,143 | 1,347 | 0,177 | 0,967 | 2,780 | 1,000 |
| 40 | F1 | 0,117 | 2,151 | 0,142 | 0,423 | 4,857 | 1,014 |
| 41 | F1 | 0,277 | 1,653 | 0,129 | 1,264 | 0,333 | 0,163 |
| 46 | F1 | 0,200 | 5,081 | 0,221 | 3,362 | 4,141 | 1,094 |
| 48 | F1 | 0,100 | 0,509 | 0,119 | 1,805 | 2,567 | 0,880 |
| 56 | F1 | 0,129 | 4,790 | 0,232 | 4,088 | 3,193 | 0,901 |
| 65 | F1 | 0,196 | 5,205 | 0,124 | 2,218 | 1,003 | 1,010 |
| 66 | F1 | 0,779 | 2,289 | 0,161 | 1,847 | 0,700 | 1,177 |
| 69 | F1 | 0,223 | 1,664 | 0,068 | 2,936 | 0,509 | 0,901 |
| 74 | F1 | 0,067 | 2,063 | 0,119 | 5,772 | 1,765 | 0,674 |

(suite)

| Patient | Statut (F1 ou F2) | SPP1 | A2M | VIM | IL8 | CXCL10 | ENG |
|---------|-------------------|------|-----|-----|-----|--------|-----|
| 83 | F1 | 0,156 | 2,959 | 0,166 | 4,918 | 6,255 | 1,400 |
| 86 | F1 | 0,124 | 3,446 | 0,179 | 3,789 | 2,858 | 1,266 |
| 88 | F1 | 0,157 | 1,597 | 0,087 | 0,766 | 0,735 | 0,667 |
| 91 | F1 | 0,224 | 3,543 | 0,171 | 0,595 | 1,641 | 1,079 |
| 95 | F1 | 0,069 | 3,931 | 0,116 | 3,118 | 0,264 | 0,727 |
| 98 | F1 | 0,163 | 1,173 | 0,089 | 3,717 | 0,292 | 0,847 |
| 105 | F1 | 0,176 | 0,383 | 0,079 | 2,017 | 0,149 | 0,782 |
| 107 | F1 | 0,324 | 1,414 | 0,173 | 0,443 | 0,727 | 1,613 |
| 109 | F1 | 0,092 | 0,644 | 0,082 | 1,610 | 1,275 | 0,722 |
| 113 | F1 | 0,144 | 2,558 | 0,133 | 1,725 | 0,434 | 0,798 |
| 116 | F1 | 0,094 | 1,537 | 0,228 | 0,792 | 18,316 | 1,343 |
| 125 | F1 | 0,010 | 0,382 | 0,063 | 1,421 | 0,234 | 0,241 |
| 126 | F1 | 0,158 | 2,204 | 0,074 | 0,450 | 0,460 | 1,039 |
| 134 | F1 | 0,390 | 1,270 | 0,108 | 3,028 | 0,345 | 0,633 |
| 135 | F1 | 0,048 | 0,690 | 0,115 | 8,625 | 1,032 | 0,425 |
| 139 | F1 | 0,067 | 1,079 | 0,087 | 0,410 | 1,197 | 0,664 |
| 141 | F1 | 0,066 | 1,664 | 0,059 | 2,615 | 1,352 | 0,362 |
| 143 | F1 | 0,089 | 1,873 | 0,343 | 1,184 | 0,301 | 0,732 |
| 144 | F1 | 0,269 | 1,899 | 0,113 | 1,206 | 1,676 | 0,655 |
| 145 | F1 | 0,438 | 5,315 | 0,235 | 5,681 | 0,626 | 1,347 |
| 146 | F1 | 0,029 | 0,153 | 0,331 | 0,509 | 0,027 | 0,465 |
| 151 | F1 | 0,034 | 1,597 | 0,075 | 0,297 | 1,288 | 0,406 |
| 152 | F1 | 0,074 | 1,892 | 0,074 | 0,887 | 1,068 | 0,818 |
| 153 | F1 | 0,128 | 2,297 | 0,064 | 1,004 | 0,200 | 0,509 |
| 154 | F1 | 0,168 | 3,204 | 0,089 | 2,011 | 0,639 | 0,616 |
| 155 | F1 | 0,199 | 2,918 | 0,072 | 0,694 | 1,324 | 1,007 |
| 157 | F1 | 0,044 | 1,939 | 0,076 | 1,257 | 3,182 | 0,465 |
| 159 | F1 | 0,449 | 5,897 | 0,117 | 4,345 | 1,853 | 0,599 |
| 161 | F1 | 0,187 | 2,949 | 0,082 | 0,707 | 0,256 | 0,622 |
| 163 | F1 | 0,700 | 5,959 | 0,076 | 3,998 | 2,258 | 0,570 |
| 164 | F1 | 0,378 | 1,395 | 0,084 | 2,616 | 0,745 | 0,766 |
| 165 | F1 | 0,176 | 0,901 | 0,043 | 0,757 | 0,033 | 0,593 |
| 167 | F1 | 0,147 | 5,483 | 0,111 | 4,209 | 2,354 | 1,474 |
| 169 | F1 | 0,009 | 0,914 | 0,042 | 0,415 | 0,574 | 0,637 |
| 170 | F1 | 0,066 | 2,639 | 0,060 | 1,308 | 0,083 | 0,509 |
| 171 | F1 | 0,530 | 4,213 | 0,089 | 6,820 | 0,664 | 0,505 |
| 172 | F1 | 0,066 | 1,873 | 0,086 | 0,000 | 0,774 | 0,838 |

(suite)

| Patient | Statut (F1 ou F2) | SPP1 | A2M | VIM | IL8 | CXCL10 | ENG |
|---------|-------------------|------|-----|-----|-----|--------|-----|
| 175 | F1 | 0,231 | 1,966 | 0,134 | 10,676 | 0,257 | 0,697 |
| 178 | F1 | 0,211 | 3,824 | 0,089 | 0,671 | 1,057 | 0,886 |
| 182 | F1 | 0,055 | 1,821 | 0,059 | 0,544 | 0,236 | 0,793 |
| 189 | F1 | 0,182 | 3,031 | 0,073 | 0,828 | 3,771 | 0,963 |
| 210 | F1 | 0,043 | 2,338 | 0,073 | 0,325 | 0,355 | 0,448 |
| 214 | F1 | 0,031 | 3,694 | 0,071 | 1,456 | 5,637 | 0,766 |
| 216 | F1 | 0,030 | 1,404 | 0,042 | 0,844 | 0,333 | 0,349 |
| 217 | F1 | 0,026 | 1,815 | 0,085 | 1,033 | 1,068 | 0,674 |
| 219 | F1 | 0,101 | 2,990 | 0,088 | 0,577 | 1,688 | 0,707 |
| 223 | F1 | 0,052 | 2,042 | 0,043 | 2,416 | 1,429 | 0,437 |
| 224 | F1 | 0,241 | 2,250 | 0,090 | 2,211 | 1,025 | 1,007 |
| 226 | F1 | 0,060 | 1,017 | 0,095 | 0,000 | 0,735 | 0,737 |
| 227 | F1 | 0,079 | 2,181 | 0,052 | 1,629 | 0,336 | 0,695 |
| 229 | F1 | 0,136 | 6,521 | 0,093 | 2,709 | 14,074 | 0,835 |
| 235 | F1 | 0,078 | 3,294 | 0,047 | 0,787 | 0,597 | 0,603 |
| 240 | F1 | 0,150 | 5,011 | 0,110 | 2,478 | 7,336 | 0,865 |
| 241 | F1 | 0,052 | 0,624 | 0,036 | 0,865 | 0,750 | 0,457 |
| 243 | F1 | 0,105 | 4,199 | 0,120 | 4,407 | 1,449 | 1,028 |
| 244 | F1 | 0,299 | 9,318 | 0,093 | 1,121 | 2,828 | 0,956 |
| 245 | F1 | 0,049 | 0,388 | 0,336 | 0,517 | 0,211 | 0,586 |
| 246 | F1 | 0,125 | 4,708 | 0,069 | 1,103 | 5,598 | 1,007 |
| 247 | F1 | 0,174 | 2,000 | 0,076 | 1,916 | 1,682 | 0,603 |
| 248 | F1 | 0,080 | 4,127 | 0,056 | 1,969 | 1,185 | 0,337 |
| 249 | F1 | 0,106 | 0,669 | 0,026 | 0,402 | 0,105 | 0,236 |
| 250 | F1 | 0,068 | 2,858 | 0,038 | 0,848 | 0,367 | 0,434 |
| 257 | F1 | 0,065 | 3,160 | 0,094 | 0,000 | 0,476 | 0,853 |
| 263 | F1 | 0,068 | 3,375 | 0,262 | 1,506 | 0,607 | 0,868 |
| 264 | F1 | 0,066 | 2,437 | 0,133 | 1,856 | 3,106 | 0,678 |
| 265 | F1 | 0,012 | 0,771 | 0,037 | 0,000 | 0,454 | 0,722 |
| 269 | F1 | 0,041 | 1,469 | 0,098 | 1,135 | 1,602 | 0,671 |
| 273 | F1 | 0,014 | 1,169 | 0,061 | 0,973 | 0,519 | 0,732 |
| 275 | F1 | 0,075 | 2,129 | 0,069 | 3,144 | 0,416 | 0,674 |
| 276 | F1 | 0,116 | 0,395 | 0,087 | 5,860 | 0,284 | 0,633 |
| 277 | F1 | 0,033 | 1,072 | 0,060 | 4,183 | 0,195 | 0,413 |
| 285 | F1 | 0,158 | 2,107 | 0,132 | 3,584 | 0,611 | 0,993 |
| 286 | F1 | 0,179 | 1,297 | 0,209 | 7,950 | 1,275 | 0,892 |
| 297 | F1 | 0,016 | 2,313 | 0,110 | 7,908 | 3,422 | 0,983 |

(suite)

| Patient | Statut (F1 ou F2) | SPP1 | A2M | VIM | IL8 | CXCL10 | ENG |
|---|---|---|---|---|---|---|---|
| 305 | F1 | 0,026 | 0,850 | 0,072 | 6,873 | 0,241 | 0,519 |
| 337 | F1 | 0,074 | 0,801 | 0,039 | 4,015 | 0,174 | 0,395 |
| 338 | F1 | 0,036 | 3,352 | 0,153 | 2,986 | 0,441 | 1,218 |
| 339 | F1 | 0,074 | 4,056 | 0,094 | 3,178 | 0,722 | 1,449 |
| 340 | F1 | 0,043 | 6,431 | 0,246 | 5,234 | 4,547 | 2,648 |
| 351 | F1 | 0,029 | 1,181 | 0,081 | 4,623 | 0,180 | 0,818 |
| 357 | F1 | 0,099 | 2,858 | 0,139 | 4,456 | 0,853 | 1,253 |
| 361 | F1 | 0,077 | 5,560 | 0,135 | 10,793 | 0,787 | 0,807 |
| 2 | F2 | 2,979 | 7,235 | 0,572 | 4,613 | 1,939 | 2,428 |
| 6 | F2 | 0,210 | 6,821 | 0,149 | 34,819 | 8,225 | 1,653 |
| 7 | F2 | 0,366 | 1,693 | 0,190 | 4,161 | 1,548 | 0,880 |
| 10 | F2 | 0,366 | 4,272 | 0,280 | 1,632 | 3,519 | 1,270 |
| 12 | F2 | 2,219 | 4,377 | 0,516 | 9,754 | 6,821 | 2,189 |
| 13 | F2 | 0,168 | 1,000 | 0,131 | 5,083 | 1,905 | 0,678 |
| 14 | F2 | 0,239 | 1,664 | 0,149 | 1,802 | 0,457 | 1,169 |
| 17 | F2 | 0,209 | 11,672 | 0,214 | 2,986 | 3,272 | 1,815 |
| 20 | F2 | 0,203 | 7,438 | 0,186 | 2,584 | 0,061 | 1,424 |
| 23 | F2 | 0,821 | 7,013 | 0,344 | 7,993 | 2,151 | 1,361 |
| 27 | F2 | 0,653 | 3,283 | 0,443 | 8,377 | 1,032 | 0,990 |
| 28 | F2 | 0,678 | 4,112 | 0,247 | 7,404 | 9,781 | 1,129 |
| 31 | F2 | 0,168 | 1,682 | 1,490 | 6,551 | 1,324 | 1,979 |
| 34 | F2 | 0,067 | 3,329 | 0,085 | 0,806 | 0,184 | 1,007 |
| 39 | F2 | 0,936 | 3,972 | 0,177 | 0,000 | 1,257 | 1,133 |
| 42 | F2 | 0,142 | 3,294 | 0,075 | 2,525 | 1,361 | 0,815 |
| 43 | F2 | 0,235 | 1,840 | 0,195 | 4,416 | 1,424 | 0,930 |
| 44 | F2 | 0,202 | 4,547 | 0,242 | 0,000 | 2,878 | 1,218 |
| 45 | F2 | 0,599 | 3,931 | 0,470 | 3,963 | 2,219 | 3,042 |
| 50 | F2 | 0,081 | 5,408 | 0,250 | 2,339 | 0,451 | 1,526 |
| 55 | F2 | 0,747 | 3,193 | 0,109 | 8,586 | 0,809 | 0,553 |
| 58 | F2 | 0,275 | 2,676 | 0,191 | 5,677 | 1,283 | 1,248 |
| 60 | F2 | 0,154 | 1,121 | 0,118 | 1,219 | 1,711 | 1,636 |
| 63 | F2 | 0,110 | 7,413 | 0,254 | 1,073 | 6,940 | 1,000 |
| 67 | F2 | 0,299 | 3,986 | 0,133 | 5,520 | 0,563 | 0,956 |
| 72 | F2 | 0,232 | 0,880 | 0,115 | 2,042 | 0,257 | 0,917 |
| 75 | F2 | 0,702 | 16,000 | 0,410 | 5,710 | 8,846 | 2,136 |
| 76 | F2 | 0,148 | 5,205 | 0,227 | 0,506 | 4,392 | 0,997 |
| 80 | F2 | 0,462 | 1,939 | 0,147 | 6,084 | 2,751 | 0,914 |

(suite)

| Patient | Statut (F1 ou F2) | SPP1 | A2M | VIM | IL8 | CXCL10 | ENG |
|---|---|---|---|---|---|---|---|
| 87 | F2 | 1,575 | 7,413 | 0,271 | 12,800 | 9,815 | 1,972 |
| 90 | F2 | 0,150 | 7,781 | 0,263 | 1,357 | 2,196 | 1,586 |
| 92 | F2 | 0,033 | 0,693 | 0,096 | 1,853 | 0,618 | 0,568 |
| 97 | F2 | 0,144 | 1,087 | 0,134 | 1,234 | 0,459 | 0,722 |
| 114 | F2 | 0,662 | 4,925 | 0,408 | 3,995 | 15,889 | 2,979 |
| 148 | F2 | 0,115 | 4,908 | 0,151 | 6,687 | 5,046 | 1,464 |
| 160 | F2 | 0,340 | 3,745 | 0,138 | 6,966 | 1,548 | 0,502 |
| 166 | F2 | 0,973 | 0,313 | 0,545 | 0,895 | 0,003 | 0,835 |
| 168 | F2 | 0,068 | 2,612 | 0,172 | 5,740 | 0,538 | 0,742 |
| 174 | F2 | 0,505 | 4,228 | 0,112 | 12,409 | 3,106 | 0,997 |
| 176 | F2 | 0,232 | 4,959 | 0,072 | 1,981 | 1,039 | 0,714 |
| 177 | F2 | 0,146 | 5,389 | 0,102 | 0,825 | 2,378 | 0,959 |
| 179 | F2 | 0,262 | 6,476 | 0,087 | 3,246 | 2,799 | 0,724 |
| 185 | F2 | 0,269 | 1,409 | 0,052 | 0,963 | 0,388 | 0,593 |
| 194 | F2 | 0,088 | 6,892 | 0,123 | 2,131 | 0,920 | 0,678 |
| 213 | F2 | 0,241 | 6,105 | 0,089 | 14,082 | 3,758 | 0,986 |
| 218 | F2 | 0,406 | 6,298 | 0,134 | 2,276 | 1,390 | 0,969 |
| 225 | F2 | 0,129 | 3,127 | 0,057 | 5,396 | 2,166 | 0,620 |
| 232 | F2 | 0,599 | 8,969 | 0,177 | 8,744 | 5,187 | 1,014 |
| 237 | F2 | 0,201 | 7,701 | 0,053 | 1,224 | 0,605 | 1,106 |
| 239 | F2 | 0,296 | 4,423 | 0,113 | 2,604 | 3,084 | 1,057 |
| 279 | F2 | 0,002 | 2,136 | 0,058 | 11,209 | 0,204 | 0,557 |
| 294 | F2 | 0,056 | 1,919 | 0,092 | 7,272 | 0,753 | 0,700 |
| 298 | F2 | 0,063 | 2,395 | 0,076 | 10,422 | 0,369 | 0,530 |
| 325 | F2 | 0,056 | 3,668 | 0,108 | 4,756 | 1,306 | 1,113 |
| 333 | F2 | 0,014 | 8,282 | 0,168 | 7,291 | 6,589 | 1,102 |
| 343 | F2 | 0,238 | 4,213 | 0,189 | 7,357 | 1,873 | 1,157 |

**Tableau 26 : valeurs BMQ des patients pour les gènes IL6ST, p14ARF, MMP9, ANGPT2, CXCL11, MMP2 (Ct normalisé conformément à la méthode du $2^{-\Delta Ct}$)**

| Patient | Statut (F1 ou F2) | IL6ST | p14ARF | MMP9 | ANGPT2 | CXCL11 | MMP2 |
|---|---|---|---|---|---|---|---|
| 1 | F1 | 0,101 | 0,000 | 7,336 | 1,772 | 0,168 | 0,204 |
| 8 | F1 | 0,488 | 0,000 | 0,568 | 2,107 | 1,079 | 0,109 |
| 9 | F1 | 0,213 | 0,026 | 0,853 | 4,228 | 0,082 | 0,077 |
| 11 | F1 | 0,190 | 0,036 | 3,138 | 2,078 | 0,892 | 0,159 |
| 16 | F1 | 0,049 | 0,003 | 1,091 | 1,061 | 0,420 | 0,064 |

(suite)

| Patient | Statut (F1 ou F2) | IL6ST | p14ARF | MMP9 | ANGPT2 | CXCL11 | MMP2 |
|---|---|---|---|---|---|---|---|
| 22 | F1 | 0,069 | 0,344 | 5,897 | 2,021 | 0,149 | 0,111 |
| 25 | F1 | 0,155 | 0,009 | 0,244 | 1,952 | 0,031 | 0,095 |
| 26 | F1 | 0,175 | 0,031 | 1,288 | 4,807 | 0,307 | 0,332 |
| 32 | F1 | 0,143 | 0,027 | 3,053 | 5,081 | 0,175 | 0,180 |
| 33 | F1 | 0,376 | 0,000 | 1,218 | 1,853 | 2,648 | 0,267 |
| 38 | F1 | 0,236 | 0,013 | 0,595 | 4,611 | 0,441 | 0,083 |
| 40 | F1 | 0,316 | 0,029 | 0,611 | 3,643 | 0,850 | 0,069 |
| 41 | F1 | 0,060 | 0,024 | 1,834 | 0,997 | 0,094 | 0,141 |
| 46 | F1 | 0,174 | 0,032 | 10,411 | 1,469 | 0,202 | 0,210 |
| 48 | F1 | 0,115 | 0,034 | 1,197 | 3,681 | 0,572 | 0,060 |
| 56 | F1 | 0,167 | 0,000 | 0,236 | 1,860 | 0,396 | 0,085 |
| 65 | F1 | 0,093 | 0,038 | 2,612 | 3,531 | 0,345 | 0,096 |
| 66 | F1 | 0,085 | 0,041 | 1,419 | 1,149 | 0,153 | 0,229 |
| 69 | F1 | 0,124 | 0,000 | 0,933 | 1,828 | 0,183 | 0,109 |
| 74 | F1 | 0,203 | 0,037 | 3,021 | 1,759 | 0,419 | 0,197 |
| 83 | F1 | 0,307 | 0,020 | 2,888 | 2,685 | 0,662 | 0,111 |
| 86 | F1 | 0,127 | 0,034 | 4,469 | 3,824 | 0,405 | 0,226 |
| 88 | F1 | 0,144 | 0,000 | 2,158 | 2,454 | 0,144 | 0,040 |
| 91 | F1 | 0,068 | 0,019 | 2,908 | 0,540 | 0,261 | 0,058 |
| 95 | F1 | 0,129 | 0,037 | 2,594 | 2,378 | 0,066 | 0,104 |
| 98 | F1 | 0,102 | 0,020 | 0,222 | 1,625 | 0,099 | 0,093 |
| 105 | F1 | 0,042 | 0,023 | 0,717 | 1,613 | 0,023 | 0,005 |
| 107 | F1 | 0,154 | 0,005 | 1,283 | 2,751 | 0,060 | 0,247 |
| 109 | F1 | 0,070 | 0,011 | 1,235 | 1,602 | 0,126 | 0,100 |
| 113 | F1 | 0,146 | 0,047 | 0,344 | 2,129 | 0,100 | 0,156 |
| 116 | F1 | 0,177 | 0,132 | 0,000 | 8,056 | 1,735 | 0,128 |
| 125 | F1 | 0,074 | 0,010 | 0,161 | 0,826 | 0,042 | 0,013 |
| 126 | F1 | 0,076 | 0,025 | 0,798 | 1,464 | 0,074 | 0,061 |
| 134 | F1 | 0,015 | 0,007 | 1,091 | 0,276 | 0,036 | 0,030 |
| 135 | F1 | 0,067 | 0,011 | 1,121 | 1,809 | 0,195 | 0,027 |
| 139 | F1 | 0,074 | 0,018 | 0,143 | 0,103 | 0,116 | 0,099 |
| 141 | F1 | 0,067 | 0,025 | 3,238 | 0,180 | 0,766 | 0,024 |
| 143 | F1 | 0,117 | 0,009 | 1,297 | 0,176 | 0,444 | 0,078 |
| 144 | F1 | 0,128 | 0,012 | 0,653 | 0,366 | 0,597 | 0,142 |
| 145 | F1 | 0,253 | 0,038 | 3,317 | 0,498 | 0,131 | 0,245 |
| 146 | F1 | 0,032 | 0,028 | 0,534 | 0,143 | 0,007 | 1,352 |

(suite)

| Patient | Statut (F1 ou F2) | IL6ST | p14ARF | MMP9 | ANGPT2 | CXCL11 | MMP2 |
|---------|-------------------|-------|--------|------|--------|--------|------|
| 151 | F1 | 0,120 | 0,008 | 0,292 | 0,181 | 0,123 | 0,043 |
| 152 | F1 | 0,188 | 0,012 | 0,444 | 0,403 | 0,165 | 0,029 |
| 153 | F1 | 0,076 | 0,006 | 1,361 | 0,124 | 0,065 | 0,014 |
| 154 | F1 | 0,263 | 0,017 | 0,093 | 0,312 | 0,182 | 0,018 |
| 155 | F1 | 0,106 | 0,023 | 1,094 | 0,104 | 0,235 | 0,025 |
| 157 | F1 | 0,156 | 0,019 | 0,473 | 0,534 | 0,898 | 0,029 |
| 159 | F1 | 0,253 | 0,026 | 4,469 | 0,073 | 0,186 | 0,049 |
| 161 | F1 | 0,171 | 0,013 | 0,853 | 0,097 | 0,061 | 0,038 |
| 163 | F1 | 0,282 | 0,022 | 0,841 | 0,033 | 0,188 | 0,012 |
| 164 | F1 | 0,188 | 0,007 | 0,758 | 0,097 | 0,166 | 0,041 |
| 165 | F1 | 0,107 | 0,013 | 0,249 | 0,044 | 0,045 | 0,007 |
| 167 | F1 | 0,160 | 0,035 | 3,618 | 0,041 | 0,390 | 0,057 |
| 169 | F1 | 0,200 | 0,015 | 0,529 | 0,029 | 0,033 | 0,029 |
| 170 | F1 | 0,162 | 0,010 | 0,507 | 0,042 | 0,007 | 0,046 |
| 171 | F1 | 0,098 | 0,016 | 1,197 | 0,047 | 0,246 | 0,051 |
| 172 | F1 | 1,741 | 0,000 | 8,664 | 0,000 | 0,147 | 0,030 |
| 175 | F1 | 0,116 | 0,013 | 0,821 | 0,120 | 0,124 | 0,022 |
| 178 | F1 | 0,202 | 0,016 | 0,345 | 0,088 | 0,087 | 0,024 |
| 182 | F1 | 0,063 | 0,005 | 1,046 | 0,054 | 0,080 | 0,049 |
| 189 | F1 | 0,202 | 0,015 | 0,127 | 0,076 | 0,300 | 0,052 |
| 210 | F1 | 0,134 | 0,010 | 0,361 | 0,031 | 0,124 | 0,009 |
| 214 | F1 | 0,157 | 0,005 | 0,247 | 0,132 | 0,379 | 0,039 |
| 216 | F1 | 0,039 | 0,014 | 0,324 | 0,173 | 0,069 | 0,006 |
| 217 | F1 | 0,454 | 0,006 | 0,053 | 1,521 | 0,092 | 0,053 |
| 219 | F1 | 0,114 | 0,010 | 0,155 | 0,676 | 0,231 | 0,023 |
| 223 | F1 | 0,112 | 0,009 | 0,053 | 0,702 | 0,277 | 0,031 |
| 224 | F1 | 0,207 | 0,015 | 1,137 | 1,558 | 0,256 | 0,053 |
| 226 | F1 | 0,314 | 0,013 | 1,711 | 1,315 | 0,208 | 0,036 |
| 227 | F1 | 0,134 | 0,012 | 0,438 | 2,676 | 0,059 | 0,052 |
| 229 | F1 | 0,257 | 0,022 | 1,061 | 1,490 | 2,454 | 0,027 |
| 235 | F1 | 0,151 | 0,012 | 0,183 | 4,155 | 0,324 | 0,040 |
| 240 | F1 | 0,355 | 0,023 | 0,940 | 0,862 | 1,608 | 0,051 |
| 241 | F1 | 0,169 | 0,009 | 0,582 | 0,940 | 0,115 | 0,018 |
| 243 | F1 | 0,240 | 0,039 | 0,766 | 1,580 | 0,338 | 0,075 |
| 244 | F1 | 0,631 | 0,037 | 0,722 | 2,549 | 0,574 | 0,064 |
| 245 | F1 | 0,219 | 0,005 | 0,212 | 1,670 | 0,050 | 0,637 |

(suite)

| Patient | Statut (F1 ou F2) | IL6ST | p14ARF | MMP9 | ANGPT2 | CXCL11 | MMP2 |
|---------|-------------------|-------|--------|------|--------|--------|------|
| 246 | F1 | 0,215 | 0,012 | 0,182 | 0,593 | 0,315 | 0,053 |
| 247 | F1 | 0,202 | 0,005 | 1,064 | 0,717 | 0,626 | 0,028 |
| 248 | F1 | 0,151 | 0,007 | 3,605 | 0,247 | 0,184 | 0,033 |
| 249 | F1 | 0,177 | 0,009 | 0,135 | 0,547 | 0,061 | 0,021 |
| 250 | F1 | 0,041 | 0,000 | 0,350 | 1,209 | 0,079 | 0,022 |
| 257 | F1 | 0,194 | 0,009 | 0,177 | 1,145 | 0,275 | 0,021 |
| 263 | F1 | 0,354 | 0,003 | 0,351 | 2,395 | 0,277 | 0,030 |
| 264 | F1 | 0,133 | 0,017 | 0,509 | 0,712 | 0,380 | 0,053 |
| 265 | F1 | 0,047 | 0,009 | 0,291 | 0,923 | 0,024 | 0,008 |
| 269 | F1 | 0,240 | 0,014 | 0,207 | 0,555 | 0,280 | 0,019 |
| 273 | F1 | 0,238 | 0,004 | 0,091 | 0,584 | 0,135 | 0,015 |
| 275 | F1 | 0,122 | 0,006 | 0,371 | 0,790 | 0,053 | 0,022 |
| 276 | F1 | 0,115 | 0,007 | 1,165 | 0,267 | 0,084 | 0,054 |
| 277 | F1 | 0,277 | 0,011 | 0,076 | 0,576 | 0,062 | 0,008 |
| 285 | F1 | 0,122 | 0,011 | 0,774 | 0,859 | 0,101 | 0,053 |
| 286 | F1 | 0,138 | 0,008 | 0,480 | 2,858 | 0,354 | 0,071 |
| 297 | F1 | 0,205 | 0,012 | 0,590 | 1,202 | 0,521 | 0,035 |
| 305 | F1 | 0,092 | 0,008 | 0,337 | 0,712 | 0,149 | 0,024 |
| 337 | F1 | 0,116 | 0,000 | 0,163 | 0,463 | 0,059 | 0,013 |
| 338 | F1 | 0,151 | 0,005 | 2,346 | 0,904 | 0,076 | 0,067 |
| 339 | F1 | 0,313 | 0,006 | 0,804 | 1,952 | 0,182 | 0,093 |
| 340 | F1 | 0,342 | 0,008 | 0,396 | 2,462 | 0,357 | 0,272 |
| 351 | F1 | 0,206 | 0,000 | 0,332 | 2,204 | 0,111 | 0,014 |
| 357 | F1 | 0,241 | 0,004 | 0,113 | 1,735 | 0,203 | 0,058 |
| 361 | F1 | 0,144 | 0,004 | 1,014 | 0,653 | 0,148 | 0,094 |
| 2 | F2 | 0,214 | 0,000 | 2,908 | 3,494 | 0,620 | 0,584 |
| 6 | F2 | 0,601 | 0,000 | 36,002 | 20,749 | 0,490 | 0,152 |
| 7 | F2 | 0,087 | 0,000 | 2,266 | 2,007 | 0,360 | 0,059 |
| 10 | F2 | 0,049 | 0,012 | 1,772 | 12,553 | 0,712 | 0,136 |
| 12 | F2 | 0,148 | 0,034 | 1,873 | 1,670 | 0,695 | 0,495 |
| 13 | F2 | 0,050 | 0,037 | 0,460 | 2,049 | 0,225 | 0,014 |
| 14 | F2 | 0,048 | 0,029 | 6,364 | 1,602 | 0,075 | 0,169 |
| 17 | F2 | 0,261 | 0,039 | 4,332 | 2,000 | 0,349 | 0,171 |
| 20 | F2 | 0,142 | 0,028 | 3,127 | 2,959 | 0,184 | 0,052 |
| 23 | F2 | 0,207 | 0,148 | 1,320 | 2,144 | 0,440 | 0,296 |
| 27 | F2 | 0,090 | 0,077 | 1,439 | 1,035 | 0,198 | 0,246 |

(suite)

| Patient | Statut (F1 ou F2) | IL6ST | p14ARF | MMP9 | ANGPT2 | CXCL11 | MMP2 |
|---------|-------------------|-------|--------|------|--------|--------|------|
| 28 | F2 | 0,080 | 0,137 | 12,168 | 1,053 | 1,106 | 0,166 |
| 31 | F2 | 0,131 | 0,044 | 1,279 | 2,732 | 0,644 | 0,124 |
| 34 | F2 | 0,067 | 0,016 | 0,826 | 2,266 | 0,025 | 0,125 |
| 39 | F2 | 0,159 | 0,000 | 1,784 | 3,329 | 0,144 | 0,290 |
| 42 | F2 | 0,075 | 0,000 | 0,563 | 0,644 | 0,174 | 0,045 |
| 43 | F2 | 0,108 | 0,038 | 2,676 | 2,049 | 0,378 | 0,264 |
| 44 | F2 | 0,282 | 0,020 | 1,464 | 3,375 | 1,361 | 0,137 |
| 45 | F2 | 0,286 | 0,068 | 8,515 | 5,081 | 0,927 | 0,326 |
| 50 | F2 | 0,044 | 0,039 | 8,369 | 0,662 | 0,050 | 0,232 |
| 55 | F2 | 0,086 | 0,065 | 0,986 | 1,647 | 0,164 | 0,132 |
| 58 | F2 | 0,120 | 0,062 | 3,630 | 1,602 | 0,234 | 0,200 |
| 60 | F2 | 0,191 | 0,000 | 2,594 | 10,339 | 0,222 | 0,076 |
| 63 | F2 | 0,200 | 0,015 | 0,177 | 1,439 | 0,486 | 0,068 |
| 67 | F2 | 0,098 | 0,068 | 1,682 | 3,010 | 0,146 | 0,129 |
| 72 | F2 | 0,073 | 0,012 | 1,079 | 3,329 | 0,047 | 0,064 |
| 75 | F2 | 0,175 | 0,121 | 1,091 | 3,732 | 1,790 | 0,491 |
| 76 | F2 | 0,360 | 0,004 | 0,829 | 2,078 | 0,476 | 0,273 |
| 80 | F2 | 0,116 | 0,041 | 0,969 | 0,648 | 0,425 | 0,046 |
| 87 | F2 | 0,184 | 0,035 | 3,972 | 1,986 | 0,611 | 0,202 |
| 90 | F2 | 0,176 | 0,017 | 4,611 | 3,918 | 0,301 | 0,198 |
| 92 | F2 | 0,063 | 0,014 | 0,616 | 0,543 | 0,146 | 0,036 |
| 97 | F2 | 0,077 | 0,017 | 1,227 | 4,000 | 0,089 | 0,064 |
| 114 | F2 | 0,253 | 0,070 | 12,817 | 4,773 | 5,205 | 0,642 |
| 148 | F2 | 0,363 | 0,072 | 0,551 | 0,525 | 0,351 | 0,125 |
| 160 | F2 | 0,189 | 0,040 | 7,490 | 0,021 | 0,543 | 0,045 |
| 166 | F2 | 0,054 | 0,000 | 8,515 | 0,000 | 0,007 | 0,278 |
| 168 | F2 | 0,136 | 0,053 | 4,807 | 0,146 | 0,103 | 0,094 |
| 174 | F2 | 0,100 | 0,047 | 1,137 | 0,000 | 0,688 | 0,034 |
| 176 | F2 | 0,137 | 0,013 | 0,532 | 0,040 | 0,182 | 0,049 |
| 177 | F2 | 0,158 | 0,032 | 0,502 | 0,064 | 0,387 | 0,033 |
| 179 | F2 | 0,268 | 0,023 | 0,428 | 0,054 | 0,685 | 0,026 |
| 185 | F2 | 0,132 | 0,005 | 0,432 | 0,023 | 0,100 | 0,036 |
| 194 | F2 | 0,138 | 0,023 | 1,257 | 0,061 | 0,191 | 0,050 |
| 213 | F2 | 0,254 | 0,030 | 0,304 | 0,043 | 0,432 | 0,018 |
| 218 | F2 | 0,108 | 0,017 | 0,057 | 4,405 | 0,257 | 0,113 |
| 225 | F2 | 0,059 | 0,025 | 0,568 | 0,409 | 0,361 | 0,011 |

(suite)

| Patient | Statut (F1 ou F2) | IL6ST | p14ARF | MMP9 | ANGPT2 | CXCL11 | MMP2 |
|---|---|---|---|---|---|---|---|
| 232 | F2 | 0,419 | 0,036 | 1,809 | 1,699 | 0,886 | 0,138 |
| 237 | F2 | 0,140 | 0,000 | 0,378 | 6,080 | 0,236 | 0,084 |
| 239 | F2 | 0,290 | 0,034 | 0,620 | 1,094 | 0,502 | 0,057 |
| 279 | F2 | 0,132 | 0,007 | 0,273 | 1,206 | 0,071 | 0,014 |
| 294 | F2 | 0,211 | 0,010 | 0,260 | 1,404 | 0,087 | 0,020 |
| 298 | F2 | 0,182 | 0,007 | 0,218 | 0,590 | 0,093 | 0,020 |
| 325 | F2 | 0,162 | 0,011 | 1,449 | 1,039 | 0,228 | 0,090 |
| 333 | F2 | 0,662 | 0,050 | 1,329 | 0,911 | 1,257 | 0,075 |
| 343 | F2 | 0,173 | 0,006 | 2,445 | 1,490 | 0,186 | 0,126 |

**Tableau 27 : valeurs BMQ des patients pour les gènes MMP7, S100A41, TIMP1, CHI3L1, COL1A1, CXCL1 (Ct normalisé conformément à la méthode du $2^{-\Delta Ct}$)**

| Patient | Statut (F1 ou F2) | MMP7 | S100A41 | TIMP1 | CHI3L1 | COL1A1 | CXCL1 |
|---|---|---|---|---|---|---|---|
| 1 | F1 | 0,150 | 1,338 | 0,252 | 0,547 | 0,727 | 2,329 |
| 8 | F1 | 0,023 | 0,821 | 0,347 | 1,912 | 0,295 | 1,474 |
| 9 | F1 | 0,018 | 0,979 | 0,198 | 0,009 | 0,226 | 0,151 |
| 11 | F1 | 0,129 | 2,505 | 0,626 | 1,145 | 0,465 | 0,871 |
| 16 | F1 | 0,007 | 1,083 | 0,121 | 0,011 | 0,151 | 0,200 |
| 22 | F1 | 0,010 | 0,451 | 0,168 | 1,231 | 0,204 | 0,549 |
| 25 | F1 | 0,005 | 0,717 | 0,288 | 1,459 | 0,637 | 0,448 |
| 26 | F1 | 0,002 | 1,459 | 0,807 | 1,227 | 1,821 | 2,505 |
| 32 | F1 | 0,026 | 1,000 | 0,383 | 1,366 | 0,446 | 0,688 |
| 33 | F1 | 0,000 | 0,669 | 0,540 | 0,495 | 0,553 | 4,257 |
| 38 | F1 | 0,023 | 0,877 | 0,195 | 0,856 | 0,184 | 0,409 |
| 40 | F1 | 0,019 | 0,478 | 0,136 | 0,201 | 0,151 | 0,154 |
| 41 | F1 | 0,009 | 0,880 | 0,263 | 0,017 | 0,369 | 0,555 |
| 46 | F1 | 0,073 | 1,185 | 0,551 | 0,338 | 2,078 | 0,815 |
| 48 | F1 | 0,006 | 0,821 | 0,241 | 0,313 | 0,227 | 0,296 |
| 56 | F1 | 0,034 | 0,683 | 0,204 | 0,350 | 0,354 | 0,000 |
| 65 | F1 | 0,033 | 1,161 | 0,296 | 0,051 | 0,344 | 0,429 |
| 66 | F1 | 0,073 | 1,125 | 0,285 | 0,151 | 0,901 | 0,760 |
| 69 | F1 | 0,020 | 0,471 | 0,176 | 0,191 | 0,325 | 0,536 |
| 74 | F1 | 0,058 | 0,507 | 0,207 | 0,379 | 0,246 | 1,490 |
| 83 | F1 | 0,020 | 0,933 | 0,244 | 0,801 | 0,790 | 0,431 |
| 86 | F1 | 0,049 | 2,078 | 0,514 | 0,973 | 0,512 | 0,753 |

(suite)

| Patient | Statut (F1 ou F2) | MMP7 | S100A41 | TIMP1 | CHI3L1 | COL1A1 | CXCL1 |
|---|---|---|---|---|---|---|---|
| 88 | F1 | 0,065 | 0,572 | 0,158 | 0,065 | 0,293 | 0,252 |
| 91 | F1 | 0,100 | 1,061 | 0,298 | 1,137 | 0,563 | 0,265 |
| 95 | F1 | 0,029 | 0,683 | 0,245 | 0,688 | 0,470 | 0,285 |
| 98 | F1 | 0,014 | 0,541 | 0,173 | 0,009 | 0,277 | 0,362 |
| 105 | F1 | 0,010 | 0,580 | 0,166 | 0,012 | 0,387 | 0,187 |
| 107 | F1 | 0,020 | 0,437 | 0,242 | 0,085 | 0,753 | 0,280 |
| 109 | F1 | 0,012 | 0,940 | 0,182 | 0,350 | 0,378 | 0,247 |
| 113 | F1 | 0,011 | 0,613 | 0,174 | 0,074 | 0,320 | 0,568 |
| 116 | F1 | 0,007 | 1,014 | 0,388 | 0,712 | 0,514 | 0,396 |
| 125 | F1 | 0,009 | 0,370 | 0,078 | 0,000 | 0,121 | 0,130 |
| 126 | F1 | 0,017 | 0,467 | 0,276 | 0,178 | 0,177 | 0,503 |
| 134 | F1 | 0,012 | 0,529 | 0,137 | 0,091 | 0,220 | 0,290 |
| 135 | F1 | 0,021 | 1,032 | 0,133 | 0,247 | 0,149 | 0,669 |
| 139 | F1 | 0,009 | 0,493 | 0,186 | 0,370 | 0,210 | 0,184 |
| 141 | F1 | 0,036 | 1,042 | 0,142 | 0,275 | 0,139 | 0,148 |
| 143 | F1 | 0,021 | 0,607 | 0,228 | 0,184 | 0,246 | 0,252 |
| 144 | F1 | 0,013 | 0,807 | 0,111 | 0,136 | 0,328 | 0,295 |
| 145 | F1 | 0,237 | 1,380 | 0,232 | 0,225 | 1,079 | 1,454 |
| 146 | F1 | 0,000 | 1,231 | 0,033 | 0,011 | 0,376 | 0,191 |
| 151 | F1 | 0,013 | 0,607 | 0,076 | 0,226 | 0,260 | 0,071 |
| 152 | F1 | 0,029 | 0,132 | 0,032 | 0,074 | 0,255 | 0,331 |
| 153 | F1 | 0,003 | 0,226 | 0,140 | 0,106 | 0,278 | 0,213 |
| 154 | F1 | 0,074 | 0,966 | 0,125 | 0,378 | 0,313 | 0,142 |
| 155 | F1 | 0,008 | 1,181 | 0,284 | 0,277 | 0,382 | 0,251 |
| 157 | F1 | 0,016 | 0,269 | 0,091 | 0,010 | 0,252 | 0,212 |
| 159 | F1 | 0,124 | 0,669 | 0,135 | 0,164 | 0,382 | 0,267 |
| 161 | F1 | 0,063 | 0,267 | 0,098 | 0,054 | 0,176 | 0,523 |
| 163 | F1 | 0,051 | 0,000 | 0,184 | 1,380 | 0,578 | 0,519 |
| 164 | F1 | 0,073 | 0,676 | 0,143 | 0,012 | 0,426 | 0,428 |
| 165 | F1 | 0,013 | 0,362 | 0,088 | 0,035 | 0,061 | 0,104 |
| 167 | F1 | 0,092 | 0,787 | 0,979 | 6,320 | 1,079 | 0,904 |
| 169 | F1 | 0,029 | 0,295 | 0,136 | 0,004 | 0,168 | 0,062 |
| 170 | F1 | 0,022 | 0,419 | 0,094 | 0,135 | 0,168 | 0,191 |
| 171 | F1 | 0,093 | 0,904 | 0,184 | 0,232 | 0,440 | 0,838 |
| 172 | F1 | 0,285 | 0,742 | 0,206 | 0,387 | 0,221 | 0,338 |
| 175 | F1 | 0,292 | 0,678 | 0,222 | 1,145 | 0,258 | 0,787 |

(suite)

| Patient | Statut (F1 ou F2) | MMP7 | S100A41 | TIMP1 | CHI3L1 | COL1A1 | CXCL1 |
|---------|-------------------|------|---------|-------|--------|--------|-------|
| 178 | F1 | 0,082 | 0,398 | 0,164 | 0,028 | 0,275 | 0,329 |
| 182 | F1 | 0,019 | 0,481 | 0,170 | 0,022 | 0,325 | 0,207 |
| 189 | F1 | 0,023 | 0,798 | 0,475 | 1,469 | 0,253 | 0,683 |
| 210 | F1 | 0,035 | 0,434 | 0,088 | 0,111 | 0,090 | 0,055 |
| 214 | F1 | 0,008 | 0,490 | 0,198 | 0,171 | 0,362 | 0,457 |
| 216 | F1 | 0,013 | 0,454 | 0,089 | 0,082 | 0,102 | 0,110 |
| 217 | F1 | 0,017 | 0,374 | 0,151 | 0,008 | 0,122 | 0,529 |
| 219 | F1 | 0,022 | 0,534 | 0,158 | 0,023 | 0,164 | 0,221 |
| 223 | F1 | 0,004 | 0,476 | 0,189 | 0,467 | 0,153 | 0,121 |
| 224 | F1 | 0,042 | 0,856 | 0,177 | 0,277 | 0,865 | 0,829 |
| 226 | F1 | 0,011 | 0,305 | 0,082 | 0,033 | 0,128 | 0,566 |
| 227 | F1 | 0,021 | 0,169 | 0,045 | 0,012 | 0,070 | 0,136 |
| 229 | F1 | 0,029 | 1,098 | 0,221 | 0,090 | 0,367 | 0,671 |
| 235 | F1 | 0,019 | 0,229 | 0,108 | 0,943 | 0,134 | 0,225 |
| 240 | F1 | 0,026 | 0,710 | 0,310 | 0,252 | 0,418 | 0,818 |
| 241 | F1 | 0,035 | 0,283 | 0,071 | 0,044 | 0,156 | 0,516 |
| 243 | F1 | 0,076 | 0,599 | 0,306 | 1,986 | 0,618 | 1,320 |
| 244 | F1 | 0,167 | 0,378 | 0,350 | 0,315 | 0,616 | 0,543 |
| 245 | F1 | 0,002 | 5,152 | 0,785 | 0,030 | 4,014 | 0,719 |
| 246 | F1 | 0,046 | 0,578 | 0,380 | 2,732 | 0,662 | 0,714 |
| 247 | F1 | 0,067 | 0,434 | 0,188 | 0,033 | 0,340 | 0,337 |
| 248 | F1 | 0,055 | 0,369 | 0,101 | 0,055 | 0,242 | 0,541 |
| 249 | F1 | 0,006 | 0,135 | 0,268 | 1,699 | 0,121 | 0,072 |
| 250 | F1 | 0,219 | 0,234 | 0,095 | 0,559 | 0,176 | 1,371 |
| 257 | F1 | 0,015 | 0,237 | 0,167 | 0,275 | 0,195 | 0,444 |
| 263 | F1 | 0,046 | 0,344 | 0,131 | 0,001 | 0,062 | 0,398 |
| 264 | F1 | 0,023 | 0,518 | 0,250 | 0,146 | 0,473 | 0,099 |
| 265 | F1 | 0,000 | 0,130 | 0,060 | 0,004 | 0,004 | 0,000 |
| 269 | F1 | 0,013 | 0,559 | 0,116 | 4,925 | 0,150 | 0,176 |
| 273 | F1 | 0,008 | 0,339 | 0,140 | 0,020 | 0,180 | 0,146 |
| 275 | F1 | 0,022 | 0,366 | 0,159 | 0,576 | 0,092 | 0,117 |
| 276 | F1 | 0,011 | 0,470 | 0,112 | 0,745 | 0,139 | 0,275 |
| 277 | F1 | 0,014 | 0,418 | 0,055 | 0,102 | 0,087 | 0,077 |
| 285 | F1 | 0,029 | 0,478 | 0,249 | 0,049 | 0,145 | 0,557 |
| 286 | F1 | 0,037 | 0,572 | 0,228 | 0,639 | 0,252 | 0,127 |
| 297 | F1 | 0,005 | 0,002 | 0,140 | 0,162 | 0,115 | 0,651 |

(suite)

| Patient | Statut (F1 ou F2) | MMP7 | S100A41 | TIMP1 | CHI3L1 | COL1A1 | CXCL1 |
|---|---|---|---|---|---|---|---|
| 305 | F1 | 0,037 | 0,241 | 0,121 | 0,245 | 0,107 | 0,286 |
| 337 | F1 | 0,009 | 0,135 | 0,090 | 0,129 | 0,035 | 0,076 |
| 338 | F1 | 0,026 | 0,370 | 0,277 | 0,116 | 0,483 | 0,338 |
| 339 | F1 | 0,031 | 0,457 | 0,215 | 0,013 | 0,419 | 0,193 |
| 340 | F1 | 0,099 | 0,812 | 0,465 | 0,239 | 0,847 | 0,336 |
| 351 | F1 | 0,063 | 0,419 | 0,176 | 0,024 | 0,142 | 0,272 |
| 357 | F1 | 0,017 | 0,478 | 0,255 | 0,057 | 0,669 | 0,204 |
| 361 | F1 | 0,052 | 0,549 | 0,233 | 0,144 | 0,976 | 0,874 |
| 2 | F2 | 0,151 | 1,664 | 0,631 | 0,460 | 1,828 | 4,891 |
| 6 | F2 | 1,197 | 0,532 | 0,415 | 0,798 | 0,521 | 0,346 |
| 7 | F2 | 0,072 | 1,189 | 0,245 | 0,082 | 0,408 | 1,181 |
| 10 | F2 | 0,000 | 1,248 | 0,333 | 0,208 | 0,553 | 1,521 |
| 12 | F2 | 0,119 | 2,868 | 0,787 | 0,914 | 2,412 | 1,429 |
| 13 | F2 | 0,000 | 1,177 | 0,118 | 0,402 | 0,075 | 1,490 |
| 14 | F2 | 0,015 | 1,253 | 0,293 | 0,000 | 0,000 | 0,416 |
| 17 | F2 | 0,006 | 0,883 | 0,616 | 0,401 | 0,288 | 0,467 |
| 20 | F2 | 0,047 | 0,664 | 0,418 | 0,058 | 0,012 | 0,319 |
| 23 | F2 | 0,020 | 0,862 | 0,856 | 0,092 | 0,657 | 1,500 |
| 27 | F2 | 0,017 | 1,886 | 0,305 | 1,210 | 0,908 | 1,210 |
| 28 | F2 | 0,042 | 1,753 | 0,710 | 3,719 | 0,188 | 0,375 |
| 31 | F2 | 0,016 | 1,240 | 0,402 | 0,106 | 0,193 | 1,007 |
| 34 | F2 | 0,003 | 0,398 | 0,197 | 0,124 | 0,334 | 0,094 |
| 39 | F2 | 0,024 | 0,923 | 0,275 | 0,350 | 0,693 | 0,676 |
| 42 | F2 | 0,012 | 0,416 | 0,199 | 0,429 | 0,174 | 0,226 |
| 43 | F2 | 0,032 | 1,521 | 0,361 | 0,246 | 0,491 | 0,956 |
| 44 | F2 | 0,035 | 1,400 | 0,305 | 0,235 | 0,258 | 0,540 |
| 45 | F2 | 0,027 | 2,395 | 0,758 | 0,125 | 0,793 | 2,289 |
| 50 | F2 | 0,009 | 1,210 | 0,396 | 0,166 | 1,892 | 0,747 |
| 55 | F2 | 0,024 | 0,576 | 0,232 | 2,558 | 0,432 | 0,953 |
| 58 | F2 | 0,024 | 0,664 | 0,211 | 0,386 | 0,807 | 0,413 |
| 60 | F2 | 0,036 | 0,719 | 0,171 | 0,086 | 0,543 | 0,651 |
| 63 | F2 | 0,023 | 0,880 | 0,310 | 3,084 | 0,175 | 0,420 |
| 67 | F2 | 0,065 | 0,983 | 0,228 | 0,188 | 0,529 | 0,601 |
| 72 | F2 | 0,025 | 0,620 | 0,207 | 0,244 | 0,374 | 0,067 |
| 75 | F2 | 0,094 | 1,729 | 1,682 | 2,990 | 2,732 | 1,257 |
| 76 | F2 | 0,050 | 0,859 | 0,415 | 5,579 | 0,671 | 0,844 |

(suite)

| Patient | Statut (F1 ou F2) | MMP7 | S100A41 | TIMP1 | CHI3L1 | COL1A1 | CXCL1 |
|---|---|---|---|---|---|---|---|
| 80 | F2 | 0,044 | 0,824 | 0,192 | 0,369 | 0,646 | 0,678 |
| 87 | F2 | 0,093 | 1,575 | 0,639 | 1,564 | 1,288 | 1,735 |
| 90 | F2 | 0,104 | 1,113 | 0,449 | 0,570 | 0,868 | 0,776 |
| 92 | F2 | 0,017 | 0,874 | 0,185 | 0,025 | 0,113 | 0,157 |
| 97 | F2 | 0,053 | 0,648 | 0,081 | 0,249 | 0,378 | 0,388 |
| 114 | F2 | 0,165 | 2,211 | 0,609 | 0,807 | 1,439 | 3,494 |
| 148 | F2 | 0,069 | 0,410 | 0,333 | 0,405 | 1,028 | 1,459 |
| 160 | F2 | 0,110 | 1,083 | 0,228 | 0,370 | 0,536 | 1,165 |
| 166 | F2 | 0,021 | 3,797 | 0,041 | 0,237 | 1,257 | 0,175 |
| 168 | F2 | 0,102 | 1,253 | 0,212 | 0,432 | 0,883 | 1,366 |
| 174 | F2 | 0,074 | 1,400 | 0,372 | 4,332 | 0,976 | 1,729 |
| 176 | F2 | 0,051 | 0,467 | 0,173 | 0,437 | 0,454 | 0,732 |
| 177 | F2 | 0,039 | 0,877 | 0,164 | 0,026 | 0,296 | 0,263 |
| 179 | F2 | 0,036 | 0,563 | 0,255 | 0,114 | 0,523 | 0,366 |
| 185 | F2 | 0,059 | 0,357 | 0,097 | 0,032 | 0,270 | 0,514 |
| 194 | F2 | 0,030 | 0,422 | 0,209 | 0,346 | 0,309 | 1,072 |
| 213 | F2 | 0,105 | 0,660 | 0,475 | 5,028 | 0,444 | 0,886 |
| 218 | F2 | 0,144 | 0,719 | 0,326 | 0,431 | 0,859 | 0,853 |
| 225 | F2 | 0,168 | 1,025 | 0,386 | 2,639 | 0,434 | 0,766 |
| 232 | F2 | 0,139 | 0,920 | 0,521 | 2,313 | 0,760 | 1,952 |
| 237 | F2 | 0,188 | 0,605 | 0,232 | 0,275 | 0,534 | 0,438 |
| 239 | F2 | 0,042 | 0,238 | 0,195 | 0,184 | 0,570 | 1,310 |
| 279 | F2 | 0,012 | 0,192 | 0,125 | 0,000 | 0,004 | 0,105 |
| 294 | F2 | 0,019 | 0,312 | 0,133 | 0,296 | 0,068 | 0,311 |
| 298 | F2 | 0,000 | 0,006 | 0,133 | 0,006 | 0,060 | 0,152 |
| 325 | F2 | 0,010 | 0,660 | 0,237 | 0,089 | 0,221 | 0,366 |
| 333 | F2 | 0,249 | 0,646 | 0,403 | 0,262 | 0,361 | 0,555 |
| 343 | F2 | 0,022 | 0,470 | 0,152 | 0,015 | 0,574 | 0,292 |

**Tableau 28 : valeurs BMQ des patients pour les gènes CXCL6, IHH, IRF9, MMP1 (Ct normalisé conformément à la méthode du $2^{-\Delta Ct}$)**

| Patient | Statut (F1 ou F2) | CXCL6 | IHH | IRF9 | MMP1 |
|---|---|---|---|---|---|
| 1 | F1 | 7,863 | 0,259 | 0,120 | 0,000 |
| 8 | F1 | 0,760 | 0,727 | 0,227 | 0,243 |
| 9 | F1 | 3,084 | 0,212 | 0,176 | 0,044 |
| 11 | F1 | 3,986 | 0,339 | 0,213 | 0,000 |

(suite)

| Patient | Statut (F1 ou F2) | CXCL6 | IHH | IRF9 | MMP1 |
|---|---|---|---|---|---|
| 16 | F1 | 1,608 | 0,044 | 0,109 | 2,828 |
| 22 | F1 | 13,454 | 1,129 | 0,182 | 0,409 |
| 25 | F1 | 0,737 | 0,155 | 0,102 | 0,000 |
| 26 | F1 | 2,549 | 0,173 | 0,362 | 0,020 |
| 32 | F1 | 20,821 | 0,847 | 0,177 | 0,509 |
| 33 | F1 | 22,706 | 0,000 | 0,308 | 0,000 |
| 38 | F1 | 1,057 | 0,166 | 0,254 | 0,042 |
| 40 | F1 | 2,107 | 0,737 | 0,191 | 0,000 |
| 41 | F1 | 4,807 | 0,162 | 0,082 | 0,000 |
| 46 | F1 | 9,254 | 0,105 | 0,354 | 0,129 |
| 48 | F1 | 1,091 | 0,164 | 0,192 | 0,061 |
| 56 | F1 | 3,668 | 0,071 | 0,182 | 0,019 |
| 65 | F1 | 1,772 | 0,292 | 0,334 | 0,073 |
| 66 | F1 | 4,993 | 0,142 | 0,184 | 0,107 |
| 69 | F1 | 3,719 | 0,742 | 0,142 | 0,000 |
| 74 | F1 | 2,403 | 0,142 | 0,333 | 0,133 |
| 83 | F1 | 10,411 | 0,124 | 0,302 | 0,000 |
| 86 | F1 | 15,137 | 0,211 | 0,248 | 0,080 |
| 88 | F1 | 3,117 | 0,271 | 0,159 | 0,000 |
| 91 | F1 | 9,190 | 0,157 | 0,120 | 0,195 |
| 95 | F1 | 4,925 | 0,221 | 0,092 | 0,082 |
| 98 | F1 | 4,141 | 0,236 | 0,144 | 0,000 |
| 105 | F1 | 2,858 | 0,307 | 0,121 | 0,000 |
| 107 | F1 | 6,845 | 0,697 | 0,225 | 0,000 |
| 109 | F1 | 3,411 | 0,352 | 0,149 | 0,000 |
| 113 | F1 | 4,362 | 0,269 | 0,122 | 0,076 |
| 116 | F1 | 2,523 | 0,176 | 0,525 | 0,000 |
| 125 | F1 | 0,000 | 0,000 | 0,037 | 0,000 |
| 126 | F1 | 47,177 | 0,153 | 0,115 | 0,027 |
| 134 | F1 | 1,548 | 0,112 | 0,056 | 0,000 |
| 135 | F1 | 6,409 | 0,000 | 0,074 | 0,133 |
| 139 | F1 | 1,075 | 0,072 | 0,130 | 0,000 |
| 141 | F1 | 0,486 | 0,230 | 0,163 | 0,140 |
| 143 | F1 | 2,114 | 0,235 | 0,093 | 0,036 |
| 144 | F1 | 0,717 | 0,266 | 0,093 | 0,040 |
| 145 | F1 | 26,723 | 0,356 | 0,273 | 0,000 |
| 146 | F1 | 18,831 | 0,027 | 0,028 | 0,000 |
| 151 | F1 | 0,454 | 0,000 | 0,132 | 0,035 |

(suite)

| Patient | Statut (F1 ou F2) | CXCL6 | IHH | IRF9 | MMP1 |
|---|---|---|---|---|---|
| 152 | F1 | 4,112 | 0,121 | 0,084 | 0,022 |
| 153 | F1 | 0,045 | 0,100 | 0,092 | 0,000 |
| 154 | F1 | 2,042 | 0,286 | 0,091 | 0,021 |
| 155 | F1 | 0,776 | 0,203 | 0,148 | 0,063 |
| 157 | F1 | 0,032 | 0,249 | 0,135 | 0,157 |
| 159 | F1 | 3,249 | 0,166 | 0,246 | 0,055 |
| 161 | F1 | 8,785 | 0,529 | 0,062 | 0,055 |
| 163 | F1 | 6,892 | 0,184 | 0,128 | 0,025 |
| 164 | F1 | 5,502 | 0,155 | 0,126 | 0,000 |
| 165 | F1 | 4,377 | 0,050 | 0,059 | 0,074 |
| 167 | F1 | 2,121 | 0,233 | 0,187 | 0,086 |
| 169 | F1 | 1,357 | 0,271 | 0,053 | 0,109 |
| 170 | F1 | 1,659 | 0,444 | 0,079 | 0,027 |
| 171 | F1 | 23,344 | 0,109 | 0,136 | 0,057 |
| 172 | F1 | 30,169 | 9,747 | 0,106 | 0,037 |
| 175 | F1 | 1,279 | 0,126 | 0,136 | 0,055 |
| 178 | F1 | 20,252 | 0,336 | 0,160 | 0,037 |
| 182 | F1 | 2,235 | 0,173 | 0,067 | 0,057 |
| 189 | F1 | 4,098 | 0,117 | 0,247 | 0,000 |
| 210 | F1 | 1,537 | 0,119 | 0,111 | 0,032 |
| 214 | F1 | 1,129 | 0,117 | 0,214 | 0,036 |
| 216 | F1 | 0,060 | 0,108 | 0,047 | 0,093 |
| 217 | F1 | 0,664 | 0,256 | 0,091 | 0,089 |
| 219 | F1 | 4,317 | 0,050 | 0,140 | 0,057 |
| 223 | F1 | 0,892 | 0,072 | 0,166 | 0,107 |
| 224 | F1 | 1,329 | 0,131 | 0,163 | 0,027 |
| 226 | F1 | 0,380 | 0,232 | 0,180 | 0,146 |
| 227 | F1 | 0,369 | 0,415 | 0,100 | 0,000 |
| 229 | F1 | 1,297 | 0,057 | 0,221 | 0,000 |
| 235 | F1 | 1,061 | 0,141 | 0,119 | 0,066 |
| 240 | F1 | 1,619 | 0,324 | 0,186 | 0,000 |
| 241 | F1 | 1,553 | 0,310 | 0,064 | 0,016 |
| 243 | F1 | 10,520 | 0,114 | 0,098 | 0,093 |
| 244 | F1 | 4,056 | 0,134 | 0,252 | 0,069 |
| 245 | F1 | 0,785 | 0,018 | 0,058 | 0,064 |
| 246 | F1 | 5,979 | 0,211 | 0,187 | 0,071 |
| 247 | F1 | 1,945 | 0,107 | 0,094 | 0,109 |
| 248 | F1 | 18,063 | 0,238 | 0,039 | 0,000 |

(suite)

| Patient | Statut (F1 ou F2) | CXCL6 | IHH | IRF9 | MMP1 |
|---|---|---|---|---|---|
| 249 | F1 | 1,125 | 0,242 | 0,091 | 0,000 |
| 250 | F1 | 0,572 | 0,288 | 0,076 | 0,187 |
| 257 | F1 | 0,000 | 0,215 | 0,091 | 0,000 |
| 263 | F1 | 1,479 | 0,058 | 0,052 | 0,034 |
| 264 | F1 | 8,456 | 0,164 | 0,211 | 0,071 |
| 265 | F1 | 1,333 | 0,312 | 0,039 | 0,000 |
| 269 | F1 | 0,000 | 0,099 | 0,079 | 0,058 |
| 273 | F1 | 0,588 | 0,405 | 0,063 | 0,084 |
| 275 | F1 | 0,182 | 0,061 | 0,029 | 0,000 |
| 276 | F1 | 0,760 | 0,198 | 0,049 | 0,000 |
| 277 | F1 | 0,000 | 0,093 | 0,082 | 0,000 |
| 285 | F1 | 0,280 | 0,218 | 0,096 | 0,099 |
| 286 | F1 | 0,653 | 0,316 | 0,111 | 0,107 |
| 297 | F1 | 0,681 | 0,255 | 0,085 | 0,236 |
| 305 | F1 | 0,298 | 0,279 | 0,033 | 0,064 |
| 337 | F1 | 0,133 | 0,301 | 0,031 | 0,067 |
| 338 | F1 | 0,467 | 0,165 | 0,038 | 0,036 |
| 339 | F1 | 0,405 | 0,349 | 0,089 | 0,125 |
| 340 | F1 | 0,771 | 0,367 | 0,201 | 0,041 |
| 351 | F1 | 0,237 | 0,075 | 0,089 | 0,000 |
| 357 | F1 | 2,042 | 0,419 | 0,060 | 0,049 |
| 361 | F1 | 0,332 | 0,242 | 0,049 | 0,000 |
| 2 | F2 | 6,409 | 0,309 | 0,486 | 0,265 |
| 6 | F2 | 68,832 | 2,918 | 0,343 | 0,114 |
| 7 | F2 | 5,579 | 0,182 | 0,132 | 0,000 |
| 10 | F2 | 4,790 | 0,000 | 0,374 | 0,000 |
| 12 | F2 | 16,622 | 0,405 | 0,285 | 0,862 |
| 13 | F2 | 1,035 | 0,057 | 0,131 | 0,000 |
| 14 | F2 | 2,181 | 0,207 | 0,092 | 0,000 |
| 17 | F2 | 1,474 | 0,257 | 0,399 | 0,000 |
| 20 | F2 | 3,745 | 0,137 | 0,168 | 0,096 |
| 23 | F2 | 3,784 | 0,278 | 0,294 | 0,150 |
| 27 | F2 | 6,727 | 0,049 | 0,125 | 0,115 |
| 28 | F2 | 8,664 | 0,323 | 0,219 | 0,000 |
| 31 | F2 | 13,642 | 0,719 | 0,310 | 0,104 |
| 34 | F2 | 3,668 | 0,261 | 0,086 | 0,000 |
| 39 | F2 | 4,028 | 0,616 | 0,206 | 0,000 |
| 42 | F2 | 1,003 | 0,345 | 0,129 | 0,000 |

(suite)

| Patient | Statut (F1 ou F2) | CXCL6 | IHH | IRF9 | MMP1 |
|---|---|---|---|---|---|
| 43 | F2 | 6,892 | 0,150 | 0,134 | 0,000 |
| 44 | F2 | 2,266 | 0,264 | 0,326 | 0,122 |
| 45 | F2 | 6,298 | 1,028 | 0,339 | 0,463 |
| 50 | F2 | 2,979 | 0,491 | 0,219 | 0,103 |
| 55 | F2 | 5,426 | 0,075 | 0,149 | 0,000 |
| 58 | F2 | 6,233 | 0,422 | 0,358 | 0,000 |
| 60 | F2 | 11,713 | 0,667 | 0,269 | 0,000 |
| 63 | F2 | 3,053 | 0,144 | 0,283 | 0,236 |
| 67 | F2 | 3,618 | 0,000 | 0,115 | 0,126 |
| 72 | F2 | 5,187 | 0,514 | 0,064 | 0,225 |
| 75 | F2 | 9,221 | 0,174 | 0,497 | 0,000 |
| 76 | F2 | 13,177 | 0,648 | 0,276 | 0,074 |
| 80 | F2 | 3,681 | 0,120 | 0,119 | 0,193 |
| 87 | F2 | 12,906 | 0,216 | 0,388 | 0,053 |
| 90 | F2 | 7,781 | 0,478 | 0,247 | 0,000 |
| 92 | F2 | 0,624 | 0,200 | 0,083 | 0,000 |
| 97 | F2 | 1,469 | 0,199 | 0,136 | 0,024 |
| 114 | F2 | 11,081 | 0,416 | 0,963 | 0,000 |
| 148 | F2 | 7,621 | 0,140 | 0,132 | 0,072 |
| 160 | F2 | 10,483 | 0,171 | 0,167 | 0,055 |
| 166 | F2 | 0,511 | 0,000 | 0,020 | 0,000 |
| 168 | F2 | 2,313 | 0,209 | 0,093 | 0,113 |
| 174 | F2 | 9,918 | 0,418 | 0,241 | 0,081 |
| 176 | F2 | 10,375 | 0,347 | 0,127 | 0,049 |
| 177 | F2 | 2,219 | 0,127 | 0,228 | 0,112 |
| 179 | F2 | 5,483 | 0,158 | 0,214 | 0,028 |
| 185 | F2 | 1,753 | 0,148 | 0,091 | 0,021 |
| 194 | F2 | 5,696 | 0,184 | 0,198 | 0,079 |
| 213 | F2 | 2,878 | 0,081 | 0,272 | 0,080 |
| 218 | F2 | 3,506 | 0,107 | 0,149 | 0,031 |
| 225 | F2 | 3,352 | 0,056 | 0,095 | 0,109 |
| 232 | F2 | 47,340 | 0,074 | 0,208 | 0,000 |
| 237 | F2 | 41,499 | 0,197 | 0,190 | 0,000 |
| 239 | F2 | 6,190 | 0,247 | 0,219 | 0,000 |
| 279 | F2 | 0,412 | 0,088 | 0,047 | 0,051 |
| 294 | F2 | 0,150 | 0,262 | 0,084 | 0,090 |
| 298 | F2 | 0,000 | 0,253 | 0,023 | 0,107 |
| 325 | F2 | 0,622 | 0,768 | 0,136 | 0,000 |

(suite)

| Patient | Statut (F1 ou F2) | CXCL6 | IHH | IRF9 | MMP1 |
|---|---|---|---|---|---|
| 333 | F2 | 1,185 | 0,250 | 0,115 | 0,250 |
| 343 | F2 | 0,712 | 0,220 | 0,081 | 0,000 |

**2. Comparaison des valeurs de dosage des sous-populations F1 et F2 pour établir un modèle de classification**

Les valeurs de dosage obtenues au §1. ci-dessus pour les sous-populations F1 et F2 ont été comparées entre elles pour construire un modèle de classification qui, à partir de la combinaison de ces valeurs, induit un score de fibrose hépatique.

Un modèle de classification peut par exemple être obtenu en suivant une méthode d'analyse statistique multivariée, ou une méthode d'analyse mathématique multivariée.

Modèles mROC :

Une méthode d'analyse mathématique multivariée appropriée est la méthode mROC (méthode *Receiver Operating Characteristic* multivariée).

En utilisant les valeurs de dosage obtenues au §1. ci-dessus pour les sous-populations F1 et F2, des modèles mROC ont été construits comme décrit dans Kramar *et al.* 1999 et Kramar *et al.* 2001. A cet effet, le logiciel mROC version 1.0, disponible commercialement auprès des concepteurs (Andrew Kramar, Antoine Fortune, David Farragi, Benjamin Reiser) a été utilisé.

Andrew Kramar et Antoine Fortune peuvent être contactés auprès de, ou via, l'Unité de Biostatistique du Centre Régional de Lutte contre le Cancer (CRLC) Val d'Aurelle - Paul Lamarque (208, rue des Apothicaires ; Parc Euromédecine ; 34298 Montpellier Cedex 5 ; France).

David Faraggi et Benjamin Reiser peuvent être contactés auprès du, ou via le, Département de Statistique de l'Université de Haifa (Mount Carmel ; Haifa 31905 ; Israël).

A partir des données de dosage entrées, la méthode mROC génère une règle décisionnelle sous la forme d'une fonction linéaire [Z = f(BMQ$_1$, BMQ$_2$, BMQ$_3$,...)] du type Z = $\alpha$.BMQ$_1$ + $\beta$.BMQ$_2$ + $\gamma$.BMQ$_3$..., où BMQ$_1$, BMQ$_2$, BMQ$_3$... sont les valeurs de dosage des niveaux d'expression de chacun des gènes choisis, et

l'utilisateur identifie la valeur de référence ou seuil ($\delta$) qui confère les meilleures performances à cette combinaison.

Cette fonction et ce seuil constituent un modèle de classification multivariée.

La fonction f calculée par la méthode mROC a ensuite été appliquée aux valeurs de dosage du niveau d'expression des gènes BMQ$_1$, BMQ$_2$, BMQ$_3$... mesurées pour un sujet test p. La valeur Z calculée pour un sujet test p a ensuite été comparée au seuil $\delta$.

Par exemple, lorsque la valeur moyenne de la combinaison des niveaux d'expression desdits gènes choisis dans la cohorte « F2 » est supérieure à celle de la cohorte des individus « F1 » (*cf.* graphe du haut de la Figure 2) :

- si Z $\geq \delta$, le test est positif (sujet "pathologique") : il est déclaré que le sujet p présente un score de fibrose hépatique F2 ;
- si Z < $\delta$, le test est négatif (sujet "sain") : il est déclaré que le sujet p présente un score de fibrose hépatique F1.

Inversement, lorsque la valeur moyenne de la combinaison des niveaux d'expression desdits gènes choisis dans la cohorte « F2 » est inférieure à celle de la cohorte des individus « F1 »

- si Z $\geq \delta$, le test est négatif (sujet "sain") : il est déclaré que le sujet p présente un score de fibrose hépatique F1 ;
- si Z < $\delta$, le test est positif (sujet "pathologique") : il est déclaré que le sujet p présente un score de fibrose hépatique F2.

Modèles WKNN :

Une méthode d'analyse statistique multivariée appropriée est la méthode WKNN (*Weighed k Nearest Neighbors*).

En utilisant les valeurs de dosage obtenues au §1. ci-dessus pour les sous-populations F1 et F2, des modèles WKNN ont été construits comme décrit dans Hechenbichler et Schliep 2004.

Schématiquement, une méthode WKNN attribue chaque nouveau cas (*y,x*) à la classe *l* de poids maximal dans un voisinage à *k* voisins selon la formule :

$$l = \max_r \left( \sum_{i=1}^{k} K\big(D\big(x, x_{(i)}\big)\big) I\big(y_{(i)} = r\big) \right)$$

où *r* représente l'indice des classes cliniques d'intérêt (en l'occurrence score de fibrose hépatique F1 ou F2), et est égal à 0 ou 1.

Pour construire les modèles WKNN, le logiciel R (librairie WKNN), librement disponible sur http://www.r-project.org/, a été utilisé. Les paramètres de réglage suivants ont été utilisés :

- Kernel (K) : epanechnikov,
- Distance (D) : 2,
- Nombre de voisins (k) : 3,

ou

- Kernel (K) : triangulaire,
- Distance (D) : 2,
- Nombre de voisins (k) : 6.

Les modèles WKNN ainsi construits ont ensuite été utilisés pour déterminer le score de fibrose hépatique des sujets, en entrant les valeurs de dosage de ces sujets dans les modèles WKNN ainsi construits.

Les valeurs de dosage des niveaux d'expression des gènes choisis d'un sujet test p ont été comparées à celles de ces voisins (k). Le modèle WKNN calcule le poids devant être attribué à la classe « score F1 » et celui devant être attribué à la classe « score F2 » pour ce sujet p. Le sujet p est alors classé par le modèle WKNN dans la classe majoritaire (par exemple, dans la classe « score F2 » si les poids des classes F1 et F2 calculés par la méthode WKNN étaient de 0,3 et 0,7, respectivement).

Modèles à forêt aléatoire :

En utilisant les valeurs de dosage obtenues au §1. ci-dessus pour les sous-populations F1 et F2, des modèles à forêt aléatoires (*Random Forest* ou RF) ont été construits comme décrit dans Breiman 2001, Liaw et Wiener 2002.

A cet effet, le logiciel R, librement disponible sur http://www.r-project.org/, a été utilisé. Les paramètres de réglage suivants ont été utilisés :

- NumberOfTrees = 500 ;
- NumberOfDescriptors = Sqrt(D).

Les données numériques listées dans le fichier sortie de R permettent d'évaluer les signatures avec le calcul des paramètres suivants : Vrai Positif (VP), Faux Positif (FP), Vrai Négatif (VN) et Faux Négatif (FN) (*cf.* ci-dessous).

Les données extraites du fichier sortie des modèles RF ainsi construits ont présenté la forme suivante :
"*OOB estimate of error rate: 34.18%*
*Confusion matrix:*

|    | F1 | F2 | Class.error |
|----|----|----|-------------|
| F1 | 71 | 31 | 0.3039216   |
| F2 | 23 | 33 | 0.4107143   |

*ROC score (out-of-bag data): 0.673"*

OOB est l'acronyme de *Out-Of-Bag*, et représente une évaluation de l'erreur.

Ces données sorties indiquent directement les valeurs des paramètres VP (nombre de patients F2 qui ont été classés F2), FP (nombre de patients F1 qui ont été classés F2), VN (nombre de patients F1 qui ont été classés F1) et FN (nombre de patients F2 qui ont été classés F1).

Pour l'exemple présenté ci-dessus, on peut lire que :
VP = 33 ; FP = 31 ; VN = 71 et FN = 23.

Les valeurs de sensibilité (Se), de spécificité (Spe), de Valeur Prédictive Positive (VPP), et de Valeur Prédictive Négative (VPN) sont calculées par les formules suivantes :

$$Se = VP/ (VP + FN) ;$$

$$Sp = VN / (VN + FP) ;$$

$$VPP = VP / (VP + FP) ;$$

$$VPN = VN / (VN + FN).$$

Les données sorties indiquent également de manière directe le taux d'erreur et le score ROC du modèle construit.

Les modèles RF ainsi construits ont ensuite été utilisés pour déterminer le score de fibrose hépatique de sujets tests. Les valeurs de dosage des niveaux d'expression des gènes de ces sujets tests ont été entrées dans un modèle RF, lequel génère des données sorties comme ci-dessus présentées, et classe le sujet testé dans la classe « score F1 » ou « score F2 ».

Modèles à réseaux de neurones :

Une autre méthode d'analyse statistique multivariée appropriée est une méthode à réseaux de neurones. Schématiquement, un réseau de neurones comprend un graphe pondéré orienté dont les noeuds symbolisent les neurones. Le réseau est construit à partir des valeurs de dosage des sous-populations (en l'occurrence, F2 versus F1), et est ensuite utilisé pour déterminer à quelle classe (en l'occurrence, F1 ou F2) appartient un nouvel élément (en l'occurrence, un patient test p).

En utilisant les valeurs de dosage obtenues au §1. ci-dessus pour les sous-populations F1 et F2, des modèles à réseaux de neurones ont été construits comme décrit dans Intrator et Intrator 1993, Riedmiller et Braun 1993, Riedmiller 1994, Anastasiadis *et. al*. 2005 ; *cf*. http://cran.r-project.org/web/packages/neuralnet/index.html.

A cet effet, le logiciel R, librement disponible sur http://www.r-project.org/, a été utilisé (version 1.3 de *Neuralnet*, écrit par Stefan Fritsch et Frauke Guenther, suivant le travail de Marc Suling).

Les options de calcul suivantes ont été utilisées :

« *NumberOfHiddenNodes = 1 et 2*

*WeightDecayfactor = 0.001*

*CrossValidate = True*

*CrossValidationFolds = 5*

*MaxNumberIterations = 2000*

*MaxNumberWeights=2000* ».

Pour chacune des combinaisons, la matrice de confusion est extraite sous une forme du type suivant :
« *Cross-validation results (5-fold):*

| Nodes | | Decay | ROC | Score Best |
|---|---|---|---|---|
| *1* | *1* | *0.001* | *0.7033* | |
| *2* | *2* | *0.001* | *0.7305* | \*\*\* |

*Contingency Table (best CV model):*

| | Predicted | |
|---|---|---|
| *Actual* | *F2* | *F1* |
| *F2* | *25* | *19* |

(suite)

| Actual | Predicted | |
| --- | --- | --- |
| | F2 | F1 |
| F1 | 12 | 51 |

Dans cet exemple, on observe que le meilleur modèle est le modèle 2, signalé par « \*\*\* » dans la colonne « ScoreBest ».

Ces données sorties indiquent directement les valeurs des paramètres VP (nombre de patients F2 qui ont été classés F2), FP (nombre de patients F1 qui ont été classés F2), VN (nombre de patients F1 qui ont été classés F1) et FN (nombre de patients F2 qui ont été classés F1). Pour l'exemple présenté ci-dessus, on peut lire que :
VP = 25, FP = 12, VN = 51, FN = 19.

Les paramètres d'évaluation, tels que la sensibilité (Se), la spécificité (Spe), la Valeur Prédictive Positive (VPP) et la Valeur Prédictive Négative (VPN), sont calculés (*cf.* formules de Se, Spe, VPP et VPN ci-dessus).

Le score ROC est extrait directement du fichier de sortie sur la ligne identifiée par « \*\*\* » qui correspond au meilleur modèle. Le taux d'erreur est calculé par la formule suivante :

$$\text{Class\_err} = (FP + FN) / (FP + VP + FN + VN).$$

Les modèles à réseau de neurones ainsi construits ont ensuite été utilisés pour déterminer le score de fibrose hépatique de sujets tests. Les valeurs de dosage des niveaux d'expression des gènes de ces sujets tests ont été entrées dans un modèle à réseau de neurones, lequel génère des données sorties comme ci-dessus présentées, et classe le sujet testé dans la classe « score F1 » ou « score F2 ».

### 3. Exemples de modèles de classification obtenus :

Les inventeurs ont ainsi identifié des gènes dont les niveaux d'expression constituent des biomarqueurs qui, lorsqu'ils sont pris en combinaison, sont pertinents pour déterminer le degré de fibrose hépatique d'un sujet.

Ces gènes sont les vingt-deux gènes suivants : SPP1, A2M, VIM, IL8, CXCL10, ENG, IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

De manière particulièrement avantageuse, il peut être observé que ces vingt-deux gènes sont tous des gènes non-membranaires, c'est-à-dire des gènes qui codent pour une protéine dont la localisation est intracellulaire et/ou extracellulaire, et qui est donc susceptible d'être détectée dans un fluide biologique du sujet, tel que le sang.

Les inventeurs ont en outre identifié que les combinaisons les plus pertinentes comprennent toutes plusieurs gènes choisis parmi un sous-groupe de six gènes, à savoir SPP1, A2M, VIM, IL8, CXCL10 et ENG, plus particulièrement :

- au moins deux gènes parmi SPP1, A2M et VIM, et
- au moins un gène parmi IL8, CXCL10 et ENG.

Les inventeurs ont ainsi identifié que des combinaisons particulièrement pertinentes comprennent les combinaisons répondant aux caractéristiques suivantes :

- au moins deux gènes parmi SPP1, A2M et VIM,
- au moins un gène parmi IL8, CXCL10 et ENG,
- optionnellement au moins un gène parmi les seize gènes suivants :
  IL6ST, p14ARF, MMP9, ANGPT2, CXCL11, MMP2, MMP7, S100A4, TIMP1, CHI3L1, COL1A1, CXCL1, CXCL6, IHH, IRF9, MMP1.

A titre illustratif, des exemples de combinaisons de biomarqueurs appropriées comprennent notamment les 29 combinaisons de biomarqueurs (combinaisons des niveaux d'expression des gènes) présentées dans le tableau 3 ci-dessus, en partie descriptive.

Des exemples de modèles de classification qui peuvent être mis en oeuvre avec ces combinaisons de marqueurs sont présentés dans :

- les tableaux 4, 5, 8 ci-dessus,
- les tableaux 6, 7, 9 ci-dessus,

- les tableaux 10, 11 ci-dessus,
- les tableaux 12, 13 ci-dessus,

(en l'occurrence, exemples de modèles mROC).

D'autres exemples de modèles de classification peuvent être construits à partir de la méthode mROC, ou d'une autre méthode de classification (par exemple, méthode WKNN ou RF ou à réseau de neurones ; *cf.* paragraphe 2 ci-dessus).

Les combinaisons prédictives de l'invention sont des combinaisons des niveaux d'expression de gènes, choisis comme ci-dessus indiqué.

Il peut néanmoins être choisi de faire intervenir dans ces combinaisons un ou plusieurs facteurs autres que les niveaux d'expression de ces gènes, pour combiner cet ou ces autres facteurs et les niveaux d'expression des gènes choisis en une règle décisionnelle.

Cet ou ces autres facteurs sont préférablement choisis de sorte à construire un modèle de classification dont le pouvoir prédictif est encore amélioré par rapport au modèle qui ne comprendrait pas cet ou ces autres facteurs.

Cet ou ces autres facteurs peuvent par exemple être des facteurs cliniques, biologiques, virologiques, par exemple :

- un ou plusieurs facteurs cliniques, tels que sexe (féminin F ou masculin M), âge à la date du prélèvement (âge à PBH), indice de masse corporelle (IMC), indice de sensibilité à l'insuline (HOMA), diabète, consommation d'alcool, degré de stéatose, mode de contamination, activité Metavir ;
  et/ou
- un ou plusieurs facteurs biologiques, tels que concentration en haptoglobine (Hapto), concentration en apolipoprotéine A1 (ApoA1), teneur en bilirubine totale (BLT), concentration en gamma glutamyl transpeptidase (GGT), concentration en aspartate aminotransférase (AST), concentration en alanine aminotransférase (ALT), teneur en plaquettes (PLQ), taux de prothrombine (TP), teneur en cholestérol HDL (Chol-HDL), teneur en cholestérol total, concentration en ferritine, teneur glycémique (glycémie), concentration en peptide C, teneur en insuline, concentration en triglycérides (TG), teneur en albumine, coefficient de saturation en fer de la transferrine (CS), concentration en phosphatase alcaline (PAL) ;
  et/ou
- un ou plusieurs facteurs virologiques, tels que génotype viral, durée de l'infection, charge virale mesurée chez le patient à la date du début du traitement (charge virale à J0), charge virale mesurée chez le patient à la date du prélèvement (charge virale à PBH).

**EXEMPLE 2 : ARN de ponction biopsie hépatique (PBH) / applications des modèles construits à des patients tests**

**a) exemple d'application de la combinaison des niveaux d'expression (ARN) des gènes A2M, SPP1, CXCL10, IL8, S100A4** (combinaison n°16 dans le tableau 3 ci-dessus) :

L'AUC relative à la combinaison des niveaux d'expression des gènes A2M, SPP1, CXCL10, IL8, S100A4 calculée sur la population d'étude complète de l'exemple 1 (n = 158 patients) est de 0,783 (*cf.* tableau 5 ci-dessus).

Par la méthode mROC (*cf.* exemple 1 ci-dessus), le seuil maximisant l'indice de Youden ($\delta$) est de 0,321 (*cf.* tableau 5 ci-dessus). Pour le choix de ce seuil, les performances de la combinaison sont les suivantes : sensibilité (Se) = 70%; spécificité (Sp) = 76% (*cf.* tableau 5 ci-dessus).

Un exemple de règle décisionnelle est la règle suivante :

$$Z = 0{,}360 \text{ x } A2M^t - 0{,}047 \text{ x } CXCL10 + 0{,}025 \text{ x } IL8 + 0{,}332 \text{ x } S100A4 + 0{,}272 \text{ x } SPP1^t$$

(fonction Z16ARN ; *cf.* tableau 4 ci-dessus), où :

- A2M, CXCL10, IL8, S100A4 et SPP1 sont les valeurs de dosage des biomarqueurs BMQ, c'est-à-dire les valeurs de dosage des niveaux d'expression des gènes indiqués (en l'occurrence valeur de Ct normalisée par la méthode du $2^{-\Delta Ct}$), et où
- l'exposant t (ici porté par A2M et SPP1) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont de 0,33 pour A2M et de 0,12 pour SPP1 (*cf.* tableau 8 ci-dessus).
Si $Z \geq 0,321$ : le test diagnostic est positif (prédiction mROC = 1), le sujet est déclaré 'F2'.
Si $Z < 0,321$ : le test est négatif (prédiction mROC = 0), le sujet est déclaré 'F1'.

Un exemple de prédiction sur 20 sujets (patients humains) est donné dans le tableau 18 ci-dessous, qui présente les valeurs de dosage des niveaux d'expression des gènes choisis (valeurs BMQ obtenues par la méthode du $2^{-\Delta Ct}$; *cf.* exemple 1 ci-dessus).

Un ou plusieurs facteurs cliniques, biologiques et virologiques peuvent être combinés aux cinq biomarqueurs indiqués ci-dessus (niveaux d'expression de cinq gènes), et conduire à une règle décisionnelle dont le pouvoir prédictif est encore supérieur à celui de la règle ci-dessus présentée.

Les tableaux 19 à 21 suivants présentent des exemples de tels facteurs cliniques, biologiques et virologiques, ainsi que leurs valeurs pour les sujets tests du tableau 18. ND = non déterminé

**Tableau 18 : exemple d'application d'un modèle de classification basé sur la combinaison des niveaux d'expression des gènes A2M, SPP1, CXCL10, IL8, S100A4 (combinaison n°16 du tableau 3 ci-dessus)**

| n° du sujet test | score de fibrose hépatique établi par PBH | modèle mROC (seuil = 0,321) | | | | | | |
|---|---|---|---|---|---|---|---|---|
| | | A2M | CXCL10 | IL8 | S100A4 | SPP1 | Z | prédiction mROC |
| 8 | F1 | 4,141 | 1,297 | 0,000 | 0,821 | 0,050 | 0,179 | **0** |
| 9 | F1 | 1,495 | 2,078 | 0,625 | 0,979 | 0,071 | -0,220 | **0** |
| 16 | F1 | 1,352 | 0,000 | 1,237 | 1,083 | 0,105 | -0,032 | **0** |
| 22 | F1 | 1,676 | 0,346 | 6,791 | 0,451 | 0,133 | 0,019 | **0** |
| 38 | F1 | 1,347 | 2,780 | 0,967 | 0,877 | 0,143 | -0,174 | **0** |
| 40 | F1 | 2,151 | 4,857 | 0,423 | 0,478 | 0,117 | -0,260 | **0** |
| 41 | F1 | 1,653 | 0,333 | 1,264 | 0,880 | 0,277 | 0,181 | **0** |
| 48 | F1 | 0,509 | 2,567 | 1,805 | 0,821 | 0,100 | -0,569 | **0** |
| 69 | F1 | 1,664 | 0,509 | 2,936 | 0,471 | 0,223 | 0,032 | **0** |
| 74 | F1 | 2,063 | 1,765 | 5,772 | 0,507 | 0,067 | -0,104 | **0** |
| 177 | F2 | 5,389 | 2,378 | 0,825 | 0,877 | 0,146 | 0,543 | **1** |
| 179 | F2 | 6,476 | 2,799 | 3,246 | 0,563 | 0,262 | 0,730 | **1** |
| 194 | F2 | 6,892 | 0,920 | 2,131 | 0,422 | 0,088 | 0,548 | **1** |
| 213 | F2 | 6,105 | 3,758 | 14,082 | 0,660 | 0,241 | 0,929 | **1** |
| 218 | F2 | 6,298 | 1,390 | 2,276 | 0,719 | 0,406 | 0,910 | **1** |
| 225 | F2 | 3,127 | 2,166 | 5,396 | 1,025 | 0,129 | 0,378 | **1** |
| 232 | F2 | 8,969 | 5,187 | 8,744 | 0,920 | 0,599 | 1,304 | **1** |
| 237 | F2 | 7,701 | 0,605 | 1,224 | 0,605 | 0,201 | 0,855 | **1** |
| 239 | F2 | 4,423 | 3,084 | 2,604 | 0,238 | 0,296 | 0,382 | **1** |
| 343 | F2 | 4,213 | 1,873 | 7,357 | 0,470 | 0,238 | 0,556 | **1** |

**Tableau 19 (données cliniques) :**

| n° sujet | sexe | âge à PBH | IMC (kg/m2) | indice de sensibilité à l'insuline (HOMA) | diabète | consommation d'alcool (g/jour) | degré de stéatose | mode de contamination | activité Metavir |
|---|---|---|---|---|---|---|---|---|---|
| 8 | F | 55 | 23 | 1,6 | Non | 0 | 0 | Transfusion | 1 |
| 9 | M | 31 | 25 | 1,3 | Non | 0 | 0 | ND | 1 |
| 16 | M | 34 | 23 | 0,8 | Non | 0 | 0 | Nosocomiale | 1 |
| 22 | F | 56 | 26 | 3,9 | Non | 0 | 1 | Nosocomiale | 1 |
| 38 | F | 45 | 27 | 1,5 | Non | 30 | 0 | Toxicomanie | 0 |
| 40 | F | 42 | 24 | 1,4 | Non | 0 | 1 | Toxicomanie | 1 |
| 41 | M | 49 | 26 | 2,2 | Non | 0 | 1 | ND | 1 |
| 48 | F | 52 | 40 | 3,6 | Oui | 0 | 1 | ND | 1 |
| 69 | M | 48 | 23 | 1,3 | Non | 0 | 0 | Toxicomanie | 1 |
| 74 | F | 51 | 27 | 1,4 | Non | 0 | 0 | ND | 1 |
| 83 | F | 51 | 26 | ND | Non | ND | 2 | ND | 1 |
| 177 | M | 51 | 26 | 1,6 | Non | 10 | 1 | Toxicomanie | 1 |
| 179 | M | 47 | 21 | 1,2 | Non | 0 | 0 | Toxicomanie | 1 |
| 194 | F | 62 | 19 | 1,3 | Non | 0 | 0 | Transfusion | 1 |
| 213 | F | 68 | 40 | 1,5 | Non | 0 | 1 | Nosocomiale | 1 |
| 218 | M | 54 | 20 | 1,7 | Non | 0 | 1 | Transfusion | 1 |
| 225 | M | 71 | 27 | 18,0 | Oui | 0 | 1 | ND | 2 |
| 232 | F | 57 | 35 | 4,1 | Non | 0 | 2 | Transfusion | 1 |
| 237 | M | 68 | 24 | 0,8 | Oui | 0 | 0 | Nosocomiale | 1 |
| 239 | F | 46 | 24 | 1,2 | Non | 0 | 0 | Transfusion | 1 |
| 333 | F | 60 | 19 | ND | Non | 0 | 1 | Transfusion | 1 |
| 343 | F | 19 | 22 | ND | Non | 0 | 1 | Néo-natale | 1 |

**Tableau 20 (données virologiques) :**

| n° patient | génotype | durée de l'infection (années) | charge virale à PBH (copies/mL .10³) |
|---|---|---|---|
| 8 | 1 | 24 | 3975 |
| 9 | 4 | ND | 1509 |
| 16 | 4 | ND | 179 |
| 22 | 1 | 33 | 1116 |
| 38 | 1 | 17 | 5641 |
| 40 | 3 | 21 | 1823 |
| 41 | 4 | ND | 8286 |
| 48 | 2 | ND | 4911 |
| 69 | 1 | 25 | 13267 |
| 74 | 4 | ND | 2101 |
| 83 | 1 | ND | 1579 |
| 177 | 1 | 23 | 4743 |
| 179 | 4 | 25 | 5986 |
| 194 | 1 | 24 | 5051 |
| 213 | 1 | 41 | 2677 |
| 218 | 1 | 24 | 3706 |
| 225 | 3 | ND | 5406 |
| 232 | 1 | 18 | 2117 |
| 237 | 1 | ND | 1408 |
| 239 | 1 | 22 | 2476 |
| 333 | 1 | 40 | 2383 |
| 343 | 1 | ND | 305 |

**Tableau 21 (données biologiques) :**

| n° sujet | A2M (g/L) | Hapto (g/L) | Apo A1 (g/L) | BLT (μmol/L ) | (U/L) | (U/L) | ALT (U/L) | PLQ (x 10³/mm³) | TP (%) | Chol-HDL (mmol/L) |
|---|---|---|---|---|---|---|---|---|---|---|
| 8 | 3,13 | 1,29 | 1,63 | 7 | 28 | 60 | 98 | 293 | 100 | 1,27 |
| 9 | 1,84 | 0,92 | 1,58 | 12 | 82 | 40 | 63 | 209 | 100 | 1,28 |
| 16 | 2,57 | 0,82 | 1,42 | 11 | 34 | 78 | 157 | 186 | 100 | 1,08 |
| 22 | 3,52 | 0,93 | 2 | 14 | 50 | 74 | 119 | 175 | 95 | 0,66 |
| 38 | 1,78 | 0,93 | 2,24 | 10 | 70 | 33 | 42 | 265 | 100 | 1,89 |
| 40 | 1,69 | 0,86 | 1,99 | 6 | 22 | 43 | 79 | 288 | 100 | 0,58 |
| 41 | 2,83 | 1,8 | 1,37 | 20 | 23 | 35 | 64 | 207 | 93 | 1,1 |
| 48 | 1,78 | 1,83 | 2,01 | 11 | 53 | 32 | 48 | 270 | 100 | 1,95 |
| 69 | 2,41 | 1,81 | 1,66 | 9 | 30 | 40 | 64 | 224 | 100 | 1,27 |
| 74 | 1,72 | 1,08 | 1,51 | 16 | 25 | 43 | 40 | 211 | 91 | 1,49 |
| 83 | ND | ND | ND | 13 | 82 | 78 | 95 | 232 | 101 | ND |

(suite)

| n° sujet | A2M (g/L) | Hapto (g/L) | Apo A1 (g/L) | BLT (μmol/L ) | (U/L) | (U/L) | ALT (U/L) | PLQ (x $10^3$/mm$^3$) | TP (%) | Chol-HDL (mmol/L) |
|---|---|---|---|---|---|---|---|---|---|---|
| 177 | 4,14 | 0,97 | 1,49 | 14 | 153 | 41 | 76 | 210 | 102 | 1,06 |
| 179 | 3,26 | 0,36 | 1,4 | 13 | 105 | 34 | 36 | 217 | 100 | 1,27 |
| 194 | 3,5 | 0,82 | 1,75 | 10 | 24 | 72 | 94 | 157 | 92 | 1,55 |
| 213 | 3,7 | 0,23 | 1,82 | 11 | 102 | 71 | 89 | 246 | 96 | 1,35 |
| 218 | 2,99 | 0,45 | 1,67 | 18 | 47 | 25 | 41 | 194 | 90 | 1,46 |
| 225 | 3,6 | 0,47 | 1,64 | 13 | 64 | 52 | 49 | 268 | 96 | 1,17 |
| 232 | 3,23 | 0,95 | 2,09 | 8 | 90 | 115 | 212 | 196 | 97 | 1,86 |
| 237 | 2,63 | 1,21 | 1,66 | 14 | 24 | 44 | 49 | 226 | 98 | 1,44 |
| 239 | 2,56 | 0,98 | 2,62 | 9 | 47 | 103 | 164 | 291 | 100 | 2,49 |
| 333 | 3,38 | 0,63 | 1,44 | 16 | 51 | 119 | 191 | 159 | 100 | ND |
| 343 | 2,69 | 0,65 | 1,44 | 17 | 22 | 26 | 38 | 332 | 92 | ND |
| 8 | ND | 5 | 1,94 | 7,1 | 0,99 | 47 | ND | 5,4 | | 69 |
| 9 | 99 | 4,5 | 1,9 | 6,7 | 1,04 | 48 | 59 | 4,4 | | 59 |
| 16 | 101 | 5 | 1,2 | 3,5 | 1,49 | 59 | 19 | 4,8 | | 61 |
| 22 | 390 | 5,2 | 3,2 | 16,7 | 0,8 | 44 | 27 | 3,7 | | 93 |
| 38 | 39 | 5,4 | 1,9 | 6,1 | 0,76 | 43 | 30 | 5,4 | | 58 |
| 40 | 24 | 4,4 | 1,7 | 7,1 | 1,19 | 45 | 17 | 4,7 | | 49 |
| 41 | 178 | 4,7 | 2,6 | 10,3 | 1 | 42 | 37 | 5,0 | | 56 |
| 48 | 92 | 6,7 | 2,33 | 12,0 | 0,66 | 42 | 19 | 6,5 | | 173 |
| 69 | 178 | 4,2 | 1,99 | 6,8 | 1,74 | 57 | 35 | 7,2 | | 61 |
| 74 | 14 | 3 | 1,89 | 10,2 | 0,86 | 45 | 17 | 5,3 | | 55 |
| 83 | 71 | 5,6 | ND | ND | 0,76 | 44 | 37 | 3,68 | | 97 |
| 177 | 339 | 4,8 | 2,29 | 7,6 | 0,76 | 46 | 46 | 4,7 | | 36 |
| 179 | 41 | 4,8 | 1,77 | 5,7 | 0,39 | 48 | 15 | 3,3 | | 57 |
| 194 | 129 | 4,3 | 1,66 | 6,7 | 0,91 | 48 | 38 | 5,2 | | 33 |
| 213 | 172 | 4,6 | 1,93 | 7,5 | 0,73 | 42 | 30 | 3,3 | | 83 |
| 218 | 156 | 5,6 | 2,35 | 6,7 | 1,03 | 40 | 48 | 4,3 | | 44 |
| 225 | 26 | 14,5 | 4,39 | 27,9 | 1,01 | 48 | 16 | 4,6 | | 93 |
| 232 | 399 | 5 | 4,73 | 18,6 | 0,98 | 42 | 28 | 4,9 | | 64 |
| 237 | 20 | 8,3 | 0,73 | 2,3 | 0,85 | 41 | 29 | 5,0 | | 58 |
| 239 | 170 | 4,6 | 1,81 | 5,6 | 0,93 | 50 | 29 | 6,6 | | 97 |
| 333 | 259 | 4,5 | ND | ND | 1,26 | 51 | 42 | 4,5 | | 96 |
| 343 | 305 | 4,3 | ND | ND | ND | 44 | 29 | ND | | 41 |

**b) exemple d'application de la combinaison des niveaux d'expression (ARN) des gènes A2M, CXCL10, IL8, SPP1, VIM** (combinaison n°4 dans le tableau 3 ci-dessus) :

L'AUC relative à la combinaison des niveaux d'expression des gènes **A2M, CXCL10, IL8, SPP1, VIM** calculée sur la population d'étude complète de l'exemple 1 (n = 158 patients) est de 0,787 (*cf.* tableau 5 ci-dessus). Par la méthode mROC (*cf.* exemple 1), le seuil maximisant l'indice de Youden (δ) pour cette combinaison est de -0,764 (*cf.* tableau 5 ci-dessus). Pour le choix de ce seuil, les performances de la combinaison sont les suivantes : Sensibilité (Se) = 75% ; spécificité (Spe) = 70% (*cf.* tableau 5 ci-dessus).

Un exemple de règle décisionnelle est la règle suivante :

$$Z = 0,297 \text{ x } A2M^t - 0,046 \text{ x } CXCL10 + 0,020 \text{ x } IL8 + 0,274 \text{ x } SPP1^t + 0,253 \text{ x } VIM^t$$

(fonction Z4ARN ; *cf.* tableau 4 ci-dessus), où :

- A2M, CXCL10, IL8, SPP1 et VIM sont les valeurs de dosage des biomarqueurs BMQ, c'est-à-dire les valeurs de dosage des niveaux d'expression des gènes indiqués (en l'occurrence valeur de Ct normalisée par la méthode du $2^{-\Delta Ct}$), et
- l'exposant t (ici porté par A2M, SPP1 et VIM) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante : $BMQ^t = (BMQ^\lambda - 1)/\lambda$.

Dans l'exemple de règle décisionnelle ci-dessus indiquée, les paramètres $\lambda$ sont de 0,33 pour A2M, 0,12 pour SPP1 et -0,23 pour VIM (*cf.* Tableau 8 ci-dessus).
Si $Z \geq$ -0,764 :le test diagnostic est positif (prédiction mROC = 1), le sujet est déclaré 'F2'.
Si Z < -0,764 :le test est négatif (prédiction mROC = 0), le sujet est déclaré 'F1'.
Un exemple de prédiction sur 20 sujets (patients humains) est donné dans le tableau 22 ci-dessous, qui présente les valeurs de dosage des niveaux d'expression des gènes choisis (valeurs BMQ obtenues par la méthode du $2^{-\Delta Ct}$ ; *cf.* exemple 1 ci-dessus).
Un ou plusieurs facteurs cliniques, biologiques et virologiques peuvent être combinés aux cinq marqueurs indiqués ci-dessus (niveaux d'expression de cinq gènes), et conduire à une règle décisionnelle dont le pouvoir prédictif est encore supérieur à celui de la règle ci-dessus présentée.
Les tableaux 19 à 21 ci-dessus présentent des exemples de tels facteurs cliniques, biologiques et virologiques, ainsi que leurs valeurs pour les patients tests du tableau 22.

**Tableau 22 : exemple d'application d'un modèle de classification basé sur la combinaison des niveaux d'expression des gènes A2M, CXCL10, IL8, SPP1 et VIM (combinaison n°4 du tableau 3 ci-dessus)**

| n° du sujet test | score de fibrose hépatique établi par PBH | modèle mROC (seuil = -0,764) | | | | | | |
|---|---|---|---|---|---|---|---|---|
| | | A2M | CXCL10 | IL8 | SPP1 | VIM | Z | prédiction mROC |
| 8 | **F1** | 4,141 | 1,297 | 0,000 | 0,050 | 0,170 | -0,764 | **0** |
| 9 | **F1** | 1,495 | 2,078 | 0,625 | 0,071 | 0,117 | -1,281 | **0** |
| 16 | **F1** | 1,352 | 0,000 | 1,237 | 0,105 | 0,120 | -1,112 | **0** |
| 38 | **F1** | 1,347 | 2,780 | 0,967 | 0,143 | 0,177 | -1,029 | **0** |
| 40 | **F1** | 2,151 | 4,857 | 0,423 | 0,117 | 0,142 | -1,100 | **0** |
| 41 | **F1** | 1,653 | 0,333 | 1,264 | 0,277 | 0,129 | -0,815 | **0** |
| 48 | **F1** | 0,509 | 2,567 | 1,805 | 0,100 | 0,119 | -1,510 | **0** |
| 69 | **F1** | 1,664 | 0,509 | 2,936 | 0,223 | 0,068 | -1,116 | **0** |
| 74 | **F1** | 2,063 | 1,765 | 5,772 | 0,067 | 0,119 | -1,049 | **0** |
| 83 | **F1** | 2,959 | 6,255 | 4,918 | 0,156 | 0,166 | -0,821 | **0** |
| 177 | **F2** | 5,389 | 2,378 | 0,825 | 0,146 | 0,102 | -0,654 | **1** |
| 179 | **F2** | 6,476 | 2,799 | 3,246 | 0,262 | 0,087 | -0,462 | **1** |
| 194 | **F2** | 6,892 | 0,920 | 2,131 | 0,088 | 0,123 | -0,456 | **1** |
| 213 | **F2** | 6,105 | 3,758 | 14,082 | 0,241 | 0,089 | -0,335 | **1** |

(suite)

| n° du sujet test | score de fibrose hépatique établi par PBH | modèle mROC (seuil = -0,764) | | | | | | |
|---|---|---|---|---|---|---|---|---|
| | | A2M | CXCL10 | IL8 | SPP1 | VIM | Z | prédiction mROC |
| 218 | **F2** | 6,298 | 1,390 | 2,276 | 0,406 | 0,134 | -0,148 | **1** |
| 232 | **F2** | 8,969 | 5,187 | 8,744 | 0,599 | 0,177 | 0,218 | **1** |
| 237 | **F2** | 7,701 | 0,605 | 1,224 | 0,201 | 0,053 | -0,599 | **1** |
| 239 | **F2** | 4,423 | 3,084 | 2,604 | 0,296 | 0,113 | -0,547 | **1** |
| 333 | **F2** | 8,282 | 6,589 | 7,291 | 0,014 | 0,168 | -0,716 | **1** |
| 343 | **F2** | 4,213 | 1,873 | 7,357 | 0,238 | 0,189 | -0,267 | **1** |

**c) combinaison des niveaux d'expression (ARN) des gènes A2M, CXCL10, IL8, SPP1, S100A4** (combinaison n°16 dans le tableau 3 ci-dessus), **combinée en outre à un facteur clinique et à des facteurs biologiques :**

Un ou plusieurs facteurs cliniques et/ou un ou plusieurs facteurs biologiques et/ou un ou plusieurs facteurs virologiques peuvent être combinés aux niveaux d'expression de gènes sélectionnés conformément à l'invention (en l'occurrence, niveaux de transcription ARN mesurés dans un échantillon de PBH), et ainsi conduire à une règle décisionnelle dont le pouvoir prédictif est encore supérieur à celui de la seule combinaison desdits niveaux d'expression.
Par exemple, la combinaison :

- des niveaux d'expression (ARN) des gènes A2M, CXCL10, IL8, SPP1, S100A4 (combinaison n°16 dans le tableau 3 ci-dessus; cf. exemple 2a ci-dessus) mesurés sur les ARN d'un échantillon de PBH,
- de la valeur du facteur clinique « âge à la date du prélèvement » (Age), en l'occurrence âge à la date de la PBH, et
- des valeurs des (autres) facteurs biologiques suivants :

  ◦ concentration en triglycérides (TG ; concentration protéique dans le sérum),
  ◦ concentration en alanine aminotransférase (ALT ; concentration protéique dans le sérum),
  ◦ concentration en ferritine (Ferritine ; concentration protéique dans le sérum),

conduit à une règle décisionnelle dont l'aire sous la courbe ROC (AUC), calculée sur la population d'étude complète de l'exemple 1 (n = 158 patients), est de 0,840 (alors qu'elle est de 0,783, lorsque la combinaison des niveaux d'expression des gènes A2M, CXCL10, IL8, SPP1, S100A4 est utilisée seule, sans être combinée au facteur clinique et aux autres facteurs biologiques ci-dessus indiqués).
Par la méthode mROC (*cf.* exemple 1), le seuil maximisant l'indice de Youden ($\delta$) pour cette combinaison est de 8,014 (*cf.* tableau 11 ci-dessus).
Pour le choix de ce seuil, les performances de la combinaison sont les suivantes : Sensibilité (Se) = 72% ; spécificité (Spe) = 82% (*cf.* tableau 11 ci-dessus).
Un exemple de règle décisionnelle est la règle suivante :

$$Z = 0{,}272 \times A2M^t - 0{,}032 \times CXCL10 + 0{,}058 \times IL8 + 0{,}419 \times SPP1^t + 0{,}012 \times S100A4^t$$
$$+ 0{,}025 \times Age^t + 0{,}566 \times TG^t + 3{,}874 \times ALT^t - 0{,}039 \times Ferritine^t$$

(fonction Z16ARNsupp ; cf. tableau 10 ci-dessus), où :

- A2M, CXCL10, IL8, SPP1 et S100A4 sont les valeurs de dosage BMQ des biomarqueurs, c'est-à-dire les valeurs de dosage des niveaux d'expression des gènes indiqués (en l'occurrence valeur de Ct normalisée par la méthode du $2^{-\Delta Ct}$) mesurés sur les ARN d'un échantillon de PBH,
- Age est l'âge du patient à la date du prélèvement,
- TG, ALT, et Ferritine sont les valeurs des facteurs biologiques indiqués (concentrations protéiques dans le sérum), et
- l'exposant t (ici porté par A2M, SPP1, S100A4, Age, TG, ALT et Ferritine) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres λ sont de 0,21 pour A2M, 0,04 pour SPP1, 0,48 pour S100A4, 0,79 pour Age, -0,22 pour TG, - 0,41 pour ALT et 0,15 pour Ferritine (*cf.* tableau 11 ci-dessus).
Si Z ≥ 8,014 : le test diagnostic est positif (prédiction mROC = 1), le sujet est déclaré 'F2'.
Si Z < 8,014 : le test est négatif (prédiction mROC = 0), le sujet est déclaré 'F1'.

**d) combinaison des niveaux d'expression (ARN) des gènes A2M,CXCL10, IL8, SPP1, VIM** (combinaison n°4 dans le tableau n°3 ci-dessus), **combinée en outre à des facteurs biologiques** :

Un ou plusieurs facteurs cliniques et/ou un ou plusieurs facteurs biologiques et/ou un ou plusieurs facteurs virologiques peuvent être combinés aux niveaux d'expression de gènes sélectionnés conformément à l'invention (en l'occurrence, niveaux de transcription ARN mesurés dans un échantillon de PBH), et ainsi conduire à une règle décisionnelle dont le pouvoir prédictif est encore supérieur à celui de la seule combinaison desdits niveaux d'expression.
Par exemple, la combinaison :

- des niveaux d'expression (ARN) des gènes A2M, CXCL10, IL8, SPP1, VIM combinaison n°4 dans le tableau 3 ci-dessus ; *cf.* exemple 2b ci-dessus) (en l'occurrence valeur de Ct normalisée par la méthode du $2^{-\Delta Ct}$) mesurés sur les ARN d'un échantillon de PBH, et
- des valeurs des (autres) facteurs biologiques suivants :

  ◦ concentration en triglycérides (TG ; concentration protéique dans le sérum),
  ◦ concentration en alanine aminotransférase (ALT ; concentration protéique dans le sérum),
  ◦ concentration en gamma glutamyl transpeptidase (GGT ; concentration protéique dans le sérum),
  ◦ concentration en ferritine (Ferritine ; concentration protéique dans le sérum),

conduit à une règle décisionnelle dont l'aire sous la courbe ROC (AUC), calculée sur la population d'étude complète de l'exemple 1 (n = 158 patients), est de 0,841 (contre 0,787 lorsque la combinaison des niveaux d'expression des gènes A2M, CXCL10, IL8, SPP1, VIM est utilisée seule, sans être combinée aux autres facteurs biologiques ci-dessus indiqués).
Par la méthode mROC (*cf.* exemple 1), le seuil maximisant l'indice de Youden pour cette combinaison est de 7,016 (*cf.* tableau 11 ci-dessus).
Pour le choix de ce seuil, les performances de la combinaison sont les suivantes : Sensibilité (Se) = 80% ; spécificité (Spe) = 71% (*cf.* tableau 11 ci-dessus).
Un exemple de règle décisionnelle est la règle suivante :

$$Z = 0{,}315 \times A2M^t - 0{,}043 \times CXCL10 + 0{,}058 \times IL8 + 0{,}383 \times SPP1^t + 0{,}064 \times VIM^t +$$
$$0{,}56 \times TG^t + 3{,}657 \times ALT^t + 0{,}188 \times GGT^t - 0{,}05 \times Ferritine^t$$

(fonction Z4ARNsupp ; cf. tableau 10 ci-dessus), où :

- A2M, CXCL10, IL8, SPP1 et VIM sont les valeurs de dosage BMQ des biomarqueurs, c'est-à-dire les valeurs de dosage des niveaux d'expression des gènes indiqués (en l'occurrence valeur de Ct normalisée par la méthode du $2^{-\Delta Ct}$) mesurés sur les ARN d'un échantillon de PBH,
- TG, ALT, GGT et Ferritine sont les valeurs des facteurs biologiques indiqués (concentrations protéiques dans le sérum), et
- l'exposant t (ici porté par A2M, SPP1, VIM, TG, ALT, GGT, Ferritine) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres λ sont de 0,21 pour A2M, 0,04 pour SPP1, -0,26 pour VIM, -0,22 pour TG, -0,41 pour ALT, - 0,12 pour GGT et 0,15 pour Ferritine (*cf*. tableau 11 ci-dessus).
Si Z ≥ 7,016 : le test diagnostic est positif (prédiction mROC = 1), le sujet est déclaré 'F2'.
Si Z < 7,016 : le test est négatif (prédiction mROC = 0), le sujet est déclaré 'F1'.

**EXEMPLE 3 : Protéines sériques / constructions de modèles et applications à des patients tests**

**a) exemple de construction d'un modèle de classification multivariée à partir de la combinaison des niveaux d'expression sérique des protéines A2M, SPP1, CXCL10, IL8 et S100A4** (combinaison n°16 dans le tableau 3 ci-dessus) :

Les niveaux d'expression des protéines A2M, SPP1, CXCL10, IL8 et S100A4 ont été mesurés dans le sérum de 228 patients qui, selon l'analyse d'une PBH prélevée sur chacun de ces patients, présentent :

- pour 149 d'entre eux : une fibrose de score F2 selon le système de score fibrotique Metavir (cohorte F2),
- pour 79 d'entre eux : une fibrose de score F1 selon le système de score fibrotique Metavir (cohorte F1).

Les dosages protéiques ont été réalisés à l'aide des kits indiqués dans le tableau 29 ci-dessus, en suivant les recommandations du fabricant.

**Tableau 29 : kits pour les dosages protéiques**

| Marqueurs | A2M (Alpha-2-Macroglobuline) | CXCL10/IP10 | CXCL8/IL-8 | SPP1 (Ostéopontine) | S100A4 |
|---|---|---|---|---|---|
| **Kit EIA** | Human alpha2-Macroglobulin ELISA Quantitation Kit | Quantikine Human CXCL10/IP10 Immunoassay | Quantikine Human CXCL8/IL-8 Immunoassay | Quantikine Human Osteopontin (OPN) Immunoassay | S100A4 ELISA Kit Circulex |
| **Fournisseur** | Gen Way | R&D Systems | R&D Systems | R&D Systems | MBL International |
| **Référence** | 40-288-20008F | DIP100 | D8000C | DOST00 | CY-8059 |
| **Type d'ELISA** | Sandwich | Sandwich | Sandwich | Sandwich | Sandwich |
| **Types d'échantillons** | Sérum ou autres liquides biologiques | Sérum, plasma, salive, milieu de culture cellulaire | Sérum, plasma, milieu de culture cellulaire | Surnageant de culture cellulaire, lait maternel, urine et plasma | Extrait cellulaire, milieu de culture tissulaire et autres milieux biologiques |
| **Prise d'essai** | 100µL (dilution 1/10000) | 75µL | 50µL | 50µL (dilution 1/25) | 100µL (dilution -> 1/6) |
| **Phase solide** | pAc de lapin anti-A2M | mAc anti-IP10 | mAc anti-IL8 | mAc anti-SPP1 | pAc anti-S100A4 |
| **Conjugué** | pAc de lapin-HRP anti-A2M | pAc-HRP anti-IP10 | pAc-HRP anti-IL8 | pAc-HRP anti-SPP1 | pAc-HRP anti-S100A4 |
| **Sensibilité** | 2,7 ng/mL | 1,67 pg/mL | 3,5 pg/mL | 0,011 ng/mL | 0,24 ng/mL |
| **Gamme de détection** | 2,7-2000 ng/mL | 7,8-500 pg/mL | 31,2-2000 pg/mL | 0,312-20 ng/mL | 0,78-50 ng/mL |

(suite)

| Marqueurs | A2M (Alpha-2-Macroglobuline) | CXCL10/IP10 | CXCL8/IL-8 | SPP1 (Ostéopontine) | S100A4 |
|---|---|---|---|---|---|
| **Spécificité** | A2M humaine | IP10 native et recombinante, pas de réaction croisée avec BLC/BCA-1, ENA-78, GCP-2, GROa, GROg, IFN-g, IL-8, IL-8 (*endothelial cell-derived*), I-TAC, MIG, NAP-2, SDF-1a, SDF-1b humain recombinant, BLC/BCA-1, CRG-2 (IP-10), GCP-2, KC, MIG, SDF-1a souris recombinant et I1-8 porc recombinante | IL8 humaine et recombinante, pas de réaction croisée avec ANG, AR, CNTF, b-ECGF, EGF, Epo, FGF acide, FGF basique, FGF-4, FGF-5, FGF-6, G-CSF, GM-CSF, GROa , GROb , GROg , sgp130, HB-EGF, HGF, I-309, IFN-g , IGF-I, IGF-II, IL-1a , IL-1b, IL-1ra, IL-1 sRI | Ostéopontine native et recombinante, pas de réaction croisée avec l'entérokinase humaine, MMP-3, MMP-7, la thrombine et avec l'ostéopontine de souris et de boeuf | S100A4, pas de réaction croisée avec S100P, S100A12 |

pAc = anticorps polyclonal
mAc = anticorps monoclonal

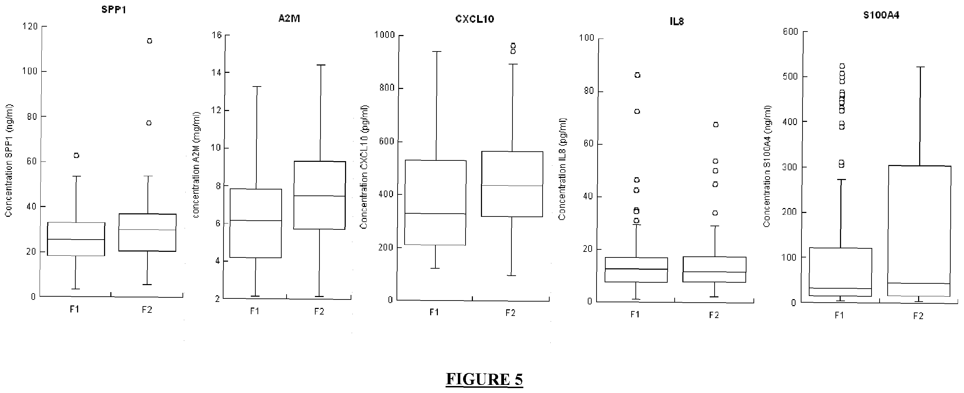

La distribution des concentrations sériques des protéines A2M, SPP1, CXCL10, IL8 et S100A4 selon le score de fibrose hépatique est présentée en Figure 5.

L'AUC relative à la combinaison des niveaux d'expression des protéines A2M, SPP1, CXCL10, IL8 et S100A4 calculée sur la population d'étude de 228 patients est de 0,694 (*cf*. tableau 7 ci-dessus).

Par la méthode mROC (*cf*. exemple 1), le seuil maximisant l'indice de Youden pour cette combinaison est de 2,905 (*cf*. tableau 7 ci-dessus).

Pour le choix de ce seuil, les performances de la combinaison sont les suivantes : Sensibilité (Se) = 68% ; spécificité (Spe) = 67% (*cf*. tableau 7 ci-dessus).

Un exemple de règle décisionnelle est la règle suivante :

$$Z = 0{,}241 \times A2M^t + 0{,}137 \times CXCL10^t + 0{,}001 \times IL8^t + 0{,}062 \times SPP1^t + 0{,}226 \times S100A4^t$$

(fonction Z16PROT ; cf. tableau 6 ci-dessus), où :

- A2M, CXCL10, IL8, SPP1 et S100A4 sont les valeurs de dosage BMQ des biomarqueurs, c'est-à-dire les valeurs de dosage des niveaux d'expression des gènes indiqués (en l'occurrence, concentration des protéines dans le sérum), et
- l'exposant t (ici porté par A2M, CXCL10, IL8, SPP1 et S100A4) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont de 0,46 pour A2M, 0,08 pour CXCL10, 0,05 pour IL8, 0,43 pour SPP1 et -0,15 pour S100A4 (*cf*. tableau 9 ci-dessus).

Si $Z \geq 2{,}905$, le test diagnostic est positif (prédiction mROC = 1), le sujet est déclaré 'F2'.

Si $Z < 2{,}905$, le test est négatif (prédiction mROC = 0), le sujet est déclaré 'F1'.

Un exemple de prédiction sur 20 sujets (patients humains) est donné dans le tableau 19 ci-dessous, qui présente les valeurs de dosage (BMQ) des niveaux d'expression sérique des gènes choisis.

Un ou plusieurs facteurs cliniques et/ou un ou plusieurs facteurs biologiques et/ou un ou plusieurs facteurs virologiques peuvent être combinés aux niveaux d'expression sérique de protéines sélectionnées conformément à l'invention, et conduire à une règle décisionnelle dont le pouvoir prédictif peut être encore supérieur à celui de la règle ci-dessus présentée.

Les tableaux 19 à 21 ci-dessus présentent des exemples de tels facteurs cliniques, biologiques et virologiques, ainsi que leurs valeurs pour les patients tests du tableau 23.

**Tableau 23 : exemple d'application d'un modèle de classification basé sur la combinaison des niveaux d'expression sérique des gènes A2M, CXCL10, IL8, SPP1 et S100A4 (combinaison n°16 du tableau 3 ci-dessus)**

| n° du sujet test | Score de fibrose hépatique établi par PBH | modèle mROC (seuil = 2,905) | | | | | | |
|---|---|---|---|---|---|---|---|---|
| | | A2M (mg/mL) | CXCL10 (pg/mL) | SPP1 (ng/mL) | IL8 (pg/mL) | S100A4 (ng/mL) | Z | Prédiction mROC |
| 9 | F1 | 4,21 | 317,93 | 28,8 | 5,34 | 14,0420 | 2,4553 | 0 |
| 11 | F1 | 4,02 | 461,77 | 51 | 23,72 | 53,3067 | 2,8731 | 0 |
| 18 | F1 | 4 | 846,53 | 26,1 | 8,16 | 10,9538 | 2,5901 | 0 |
| 21 | F1 | 4,09 | 306,3 | 45,3 | 6,18 | 105,0504 | 2,8299 | 0 |
| 22 | F1 | 4,04 | 529,67 | 46,1 | 9,29 | 54,8824 | 2,8752 | 0 |
| 26 | F1 | 7,25 | 150,84 | 27,9 | 10,13 | 9,0630 | 2,5111 | 0 |
| 32 | F1 | 3,2 | 149,56 | 10,6 | 6,46 | 500,0294 | 2,3834 | 0 |
| 35 | F1 | 3,05 | 241,65 | 29,2 | 34,83 | 19,5252 | 2,3126 | 0 |
| 38 | F1 | 2,93 | 419,56 | 31,3 | 3,65 | 265,5756 | 2,7441 | 0 |
| 40 | F1 | 2,16 | 660,26 | 26,7 | 3,93 | 70,2605 | 2,5494 | 0 |
| 2 | F2 | 9,19 | 268,86 | 29,9 | 7,87 | 127,2983 | 3,1541 | 1 |
| 6 | F2 | 14,45 | 564,09 | 53,4 | 7,87 | 8,4328 | 3,4643 | 1 |
| 10 | F2 | 5,02 | 692,35 | 34,4 | 5,9 | 41,7101 | 2,9172 | 1 |
| 17 | F2 | 7,16 | 430,95 | 39 | 7,87 | 44,3571 | 3,0504 | 1 |
| 23 | F2 | 13,8 | 338,51 | 38,3 | 16,07 | 93,5798 | 3,5387 | 1 |
| 27 | F2 | 6,79 | 522 | 45,6 | 18,62 | 85,1345 | 3,1905 | 1 |
| 28 | F2 | 7,14 | 643,05 | 27,7 | 10,13 | 392,9496 | 3,2816 | 1 |
| 43 | F2 | 6,19 | 557,47 | 51,9 | 16,92 | 492,5924 | 3,3739 | 1 |
| 44 | F2 | 8,21 | 496,77 | 45,4 | 20,89 | 19,3361 | 3,1003 | 1 |
| 50 | F2 | 9,15 | 403,28 | 22,7 | 8,44 | 303,4538 | 3,2592 | 1 |

**b) combinaison des niveaux d'expression dans le sérum des protéines A2M, CXCL10, IL8, SPP1, S100A4** (combinaison n°16 dans le tableau 3 ci-dessus), **combinée en outre à un facteur clinique et à des facteurs biologiques** :

Un ou plusieurs facteurs cliniques et/ou un ou plusieurs facteurs biologiques et/ou un ou plusieurs facteurs virologiques peuvent être combinés aux niveaux d'expression sériques de gènes sélectionnés conformément à l'invention (protéines sériques), et ainsi conduire à une règle décisionnelle dont le pouvoir prédictif est encore supérieur à celui de la seule combinaison desdits niveaux d'expression sérique.

Par exemple, la combinaison :

- des niveaux sériques de traduction des gènes A2M, CXCL10, IL8, SPP1, S100A4 (*cf.* exemple 3a ; combinaison n°16 dans le tableau 3 ci-dessus),
- de la valeur du facteur clinique « âge à la date du prélèvement », en l'occurrence âge à la date de du prélèvement de sérum (Age), et

- des valeurs des (autres) facteurs biologiques suivants :

  ◦ concentration en triglycérides (TG ; concentration protéique dans le sérum),
  ◦ concentration en alanine aminotransférase (ALT ; concentration protéique dans le sérum),
  ◦ concentration en gamma glutamyl transpeptidase (GGT ; concentration protéique dans le sérum),

conduit à une règle décisionnelle dont l'aire sous la courbe ROC (AUC), calculée sur la population d'étude complète de l'exemple 3a (n = 228 patients), est de 0,743 (alors qu'elle est de 0,694, lorsque la combinaison des niveaux sériques de traduction des gènes A2M, CXCL10, IL8, SPP1, S100A4 est utilisée seule, sans être combinée au facteur clinique et aux autres facteurs biologiques ci-dessus indiqués; *cf.* exemple 3a) ci-dessus).

Par la méthode mROC (*cf.* exemple 1), le seuil maximisant l'indice de Youden pour cette combinaison est de 8,792 (*cf.* tableau 13 ci-dessus).

Pour le choix de ce seuil, les performances de la combinaison sont les suivantes : Sensibilité (Se) = 67% ; spécificité (Spe) = 72% (*cf.* tableau 13 ci-dessus).

Un exemple de règle décisionnelle est la règle suivante :

$$Z = 0{,}2 \times A2M^t + 0{,}05 \times CXCL10^t - 0{,}026 \times IL8^t + 0{,}051 \times SPP1^t + 0{,}204 \times S100A4^t + 0{,}020 \times Age^t + 0{,}266 \times TG^t + 3{,}354 \times ALT^t + 0{,}141 \times GGT^t$$

(fonction Z16PROTsupp ; cf. tableau 12 ci-dessus), où :

- A2M, CXCL10, IL8, SPP1 et VIM sont les valeurs de dosage BMQ des biomarqueurs, c'est-à-dire les valeurs de dosage des niveaux sériques de traduction des gènes indiqués (concentration des protéines dans le sérum),
- Age est l'âge du patient à la date du prélèvement,
- TG, ALT et GGT sont les valeurs des facteurs biologiques indiqués (concentrations protéiques dans le sérum), et
- l'exposant t (ici porté par A2M, CXCL10, IL8, SPP1, S100A4, Age, TG, ALT, GGT) indique que la valeur à appliquer dans la règle décisionnelle est la transformée Box-Cox (Box et Cox, 1964) de la valeur de dosage du niveau d'expression (BMQ) du gène considéré, afin de la normaliser selon la formule suivante :

$$BMQ^t = (BMQ^\lambda - 1)/\lambda.$$

Dans l'exemple de règle décisionnelle ci-dessus indiquée, les paramètres $\lambda$ sont de 0,46 pour A2M, 0,08 pour CXCL10, 0,05 pour IL8, 0,43 pour SPP1 et -0,15 pour S100A4, 0,9 pour Age, -0,27 pour TG, -0,13 pour GGT et -0,47 pour ALT (*cf.* tableau 13 ci-dessus).

Si $Z \geq 8{,}792$, le test diagnostic est positif (prédiction mROC = 1), le sujet est déclaré 'F2'.

Si $Z < 8{,}792$, le test est négatif (prédiction mROC = 0), le sujet est déclaré 'F1'.

**RÉFÉRENCES BIBLIOGRAPHIQUES**

Anastasiadis et al. 2005 ; New globally convergent training scheme based on the resilient propagation algorithm. Neurocomputing 64: 253-270.

Bedossa, Poynard, pour le groupe Metavir français, 1996. An algorythm for the grading of activity in chronic hepatitis C. Hepatology 24: 289-93.

Box et Cox 1964 ; An analysis of transformations. Journal of the Royal Statistical Society, Series B 26: 211-243.

Breiman 2001 ; Random Forests. Machine Learning 45 : 5-32.

Castera et al. 2005 ; Prospective comparison of transient elastography, Fibrotest, APRI, and liver biopsy for the assessment of fibrosis in chronic hepatitis C; Gastroenterology 128:343-50.

Chambers 2008 ; Software for data analysis : programming with R. Springer, New York, ISBN 978-0-387-75935-7.

Cole et al. 1983 ; Proc. Natl. Acad. Sci. USA 80: 2026-2030.

Cole et al. 1985 ; Monoclonal Antibodies And Cancer Therapy, Alan R. Liss, Inc., pp. 77-96.

Dalgaard 2008 ; Introductory statistics with R, 2nd edition, Springer, ISBN 978-0-387-79053.

Falissard 2005 ; Comprendre et utiliser les statistiques dans les sciences de la vie, Masson.

Goodman 2007 ; Grading and staging systems for inflammation and fibrosis in chronic liver diseases. Journal of Hepatology 47: 598-607.

Hechenbichler et Schliep 2004 ; weighed k-nearest-neighbor techniques and ordinal classification. Sonderforschungsbereich 386, paper 399.

Hastie, Tibishirani et Friedman, 2009 ; "The Elements of Statistical Learning: Data Mining, Inference and Prediction", 2nd Edition, Springer.

Ikata et Gentleman 1996 ; R : a language for data analysis and graphics. Journal of computational and graphical statistics 5: 299-314.

Intrator et Intrator 1993 ; Using Neural Nets for Interpretation of Nonlinear Models. Proceedings of the Statistical Computing Section, San Francisco: American Statistical Society (eds), pages 244-249.

Köhler and Milstein 1975 ; Nature 256: 495-497.

Kosbor et al. 1983 ; Immunology Today 4: 72.

Kramar et al. 1999 ; Critères ROC généralisés pour l'évaluation de plusieurs marqueurs tumoraux. Revue d'Epidémiologie et Santé Publique 47 :376-383.

Kramar et al. 2001 ; mROC: a computer program for combining tumour markers in predicting disease states. Computer methods and programs in biomedicine 66: 199-207.

Liaw et Wiener 2002 ; Classification and regression by Random Forest. R. News 2.3: 18-22.

Livak et Schmittgen 2001 ; Analysis of relative gene expression data using real-time quantitative PCR and the 2(-Delta Delta C(T)) Method. Methods 25: 402-408.

Riedmiller 1994 ; Rprop - Description and Implementation Details. Technical Report. University of Karlsruhe.

Riedmiller et Braun 1993 ; A direct adaptive method for faster backpropagation learning: the RPROP algorithm. Proceedings of the IEEE International Conference on Neural Networks (ICNN), San Francisco, pages 586-591.

Reiser et Faraggi 1997 ; Confidence intervals for the generalized ROC criterion. Biometrics 53: 644-652.

Schmitten et Livak 2008 ; Analyzing real-time PCR data by the comparative Ct method. Nature Protocols 3(6) : 1101-1108.

Shaheen et al. 2007 ; FibroTest and FibroScan for the prediction of hepatitis C-related fibrosis : a systematic review of diagnostic test accuracy; Am. J. Gastroenterol. 102(11) : 2589-2600.

Shapiro 1999 ; The interprétation of diagnostic tests. Statistical Methods in Medical Research, 8: 113-134.

Su et Liu 1993 ; Linear combinations of multiple diagnostic markers. Journal of the American Statistical Association 88: 1350-1355.

Swets 1988 ; Measuring the accuracy of diagnostic systems. Science 240, 1285-1293.

Theodoridis et Koutroumbos 2009 ; Pattern Recognition. Academic Press, Elsevier. US4 376 110 (au nom de Hybritech Inc.).

WO 02/16949 A1 (au nom de Epigene).

WO 2006/103570 A2 (au nom de Assistance Publique - Hôpitaux de Paris).

WO 2006/082522 A1 (au nom de Assistance Publique - Hôpitaux de Paris).

## Revendications

**1.** **Méthode *in vitro*** pour déterminer si le score de fibrose hépatique d'un sujet infecté par un ou des virus de l'hépatite a un score fibrotique Metavir d'au plus F1 ou bien d'au moins F2, **caractérisée en ce qu'**elle comprend les étapes suivantes :

   i) dans un échantillon préalablement obtenu à partir dudit sujet, doser les niveaux auxquels des gènes choisis sont transcrits ou traduits, lesdits gènes choisis dont on dose les niveaux de transcription ou de traduction étant la combinaison de gènes suivante :

   - SPP1, et
   - au moins un gène parmi A2M et VIM, et
   - au moins un gène parmi IL8, CXCL10 et ENG, et
   - de zéro à seize gènes parmi la liste des seize gènes suivants : IL6ST, p14ARF, MMP9, ANGPT2, CXCL11, MMP2, MMP7, S100A4, TIMP1, CHI3L1, COL1A1, CXCL1, CXCL6, IHH, IRF9, MMP1,

   ii) comparer les valeurs de dosage de chacun desdits gènes choisis obtenues pour ledit sujet, à leurs valeurs, ou à la distribution de leurs valeurs, dans des cohortes de référence pré-établies selon leur score de fibrose hépatique, lesdites cohortes étant :

   - une cohorte d'individus dont la fibrose hépatique a un score fibrotique Metavir d'au plus F1, et
   - une cohorte d'individus dont la fibrose hépatique a un score fibrotique Metavir d'au moins F2 ;

   pour classer ledit sujet dans celle de ces cohortes de référence vis-à-vis de laquelle il a la plus forte probabilité d'appartenance.

**2.** La méthode de la revendication 1, dans laquelle la comparaison de l'étape ii) est faite en combinant les valeurs de dosage obtenues pour ledit sujet dans un modèle de classification multivariée, qui compare ces valeurs à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance,
et/ou
dans laquelle la comparaison de l'étape ii) est faite en combinant les valeurs de dosage obtenues pour ledit sujet dans un modèle de classification multivariée préalablement construit comme suit :

   a) pour une population d'individus qui sont de la même espèce que ledit sujet, et qui sont infectés par le ou les mêmes virus de l'hépatite que ledit sujet, déterminer le score de fibrose hépatique de chacun desdits individus de la population, et les classer en sous-populations selon leur score de fibrose hépatique, constituant ainsi des cohortes de référence établies selon leur score de fibrose hépatique ;

b) dans au moins un échantillon qui a été préalablement obtenu à partir de chacun desdits individus, dont la nature est identique à celle de l'échantillon dudit sujet, doser le niveau de transcription ou de traduction de chacun desdits gènes choisis ;

c) faire une comparaison inter-cohorte des valeurs de dosage obtenues à l'étape b), ou de la distribution de ces valeurs de dosage, pour construire un modèle de classification multivariée, qui induit une valeur de score de fibrose hépatique à partir de la combinaison des niveaux de transcription, ou le cas échéant de traduction, desdits gènes choisis.

3. La méthode selon l'une quelconque des revendications 1 à 2, dans laquelle la comparaison de l'étape ii) est réalisée par combinaison desdites valeurs de dosage obtenues à l'étape i) dans une fonction mathématique, notamment une fonction linéaire ou non linéaire, plus particulièrement une fonction linéaire, pour obtenir une valeur de sortie indicatrice du score de fibrose hépatique dudit sujet,

ou

dans laquelle la comparaison de l'étape ii) est réalisée par combinaison desdites valeurs de dosage obtenues à l'étape i) dans un modèle d'apprentissage automatique multivarié, par exemple un modèle de classification non paramétrique multivariée, un modèle heuristique multivarié, ou un modèle de prédiction probabiliste multivariée, pour obtenir une valeur de sortie indicatrice du score de fibrose hépatique dudit sujet.

4. La méthode selon l'une quelconque des revendications 1 à 3, dans laquelle le classement dudit sujet dans celle de ces cohortes de référence vis-à-vis de laquelle il a la plus forte probabilité d'appartenance est fait avec :

- une sensibilité (Se) d'au moins 67%, d'au moins 68%, d'au moins 69%, d'au moins 70%, ou d'au moins 71%, ou d'au moins 72%, ou d'au moins 73%, ou d'au moins 74%, ou d'au moins 75% ; et/ou avec une
- spécificité (Sp) d'au moins 67%, d'au moins 68%, d'au moins 69%, d'au moins 70%, ou d'au moins 71%, ou d'au moins 72%, ou d'au moins 73%, ou d'au moins 74%, ou d'au moins 75% ; et/ou avec
- une Valeur Prédictive Négative (VPN) d'au moins 80%, ou d'au moins 81%, ou d'au moins 82%, ou d'au moins 83% ou d'au moins 84% ; et/ou avec
- une Valeur Prédictive Positive (VPP) d'au moins 50%, ou d'au moins 55%, ou d'au moins 56%, ou d'au moins 57%, ou d'au moins 58%, ou d'au moins 59% ou d'au moins 60% ;

de préférence avec au moins avec ladite VPN et/ou ladite sensibilité, plus particulièrement avec au moins ladite VPN et ladite sensibilité et ladite spécificité,

et/ou

dans laquelle ledit modèle de classification multivariée présente une aire sous la courbe ROC (AUC) d'au moins 0,60, d'au moins 0,61, d'au moins 0,66, plus particulièrement d'au moins 0,69, encore plus particulièrement d'au moins 0,70, d'au moins 0,71, d'au moins 0,72, d'au moins 0,73, d'au moins 0,74, plus particulièrement d'au moins 0,75, encore plus particulièrement d'au moins 0,76, encore plus particulièrement d'au moins 0,77, notamment d'au moins 0,78, d'au moins 0,79, d'au moins 0,80.

5. La méthode selon l'une quelconque des revendications 1 à 4, dans laquelle lesdits gènes choisis à l'étape i) comprennent A2M et/ou IL8,

et/ou

dans laquelle :

- le nombre total desdits gènes choisis à l'étape i) est de quatre ou cinq ; et/ou dans laquelle
- lesdits gènes choisis à l'étape i) ne comprennent qu'un ou deux gène(s) parmi ladite liste de seize gènes, ou n'en comprennent pas.

6. La méthode selon l'une quelconque des revendications 1 à 5, dans laquelle lesdits gènes choisis à l'étape i) comprennent, ou sont :

- SPP1, A2M, IL8, CHI3L1 et IL6ST (combinaison n°1) ; ou
- SPP1, A2M, IL8, ANGPT2 et IL6ST (combinaison n°2) ; ou
- SPP1, A2M, IL8, IL6ST et MMP2 (combinaison n°3) ; ou
- SPP1, A2M, IL8, VIM et CXCL10 (combinaison n°4) ; ou
- SPP1, A2M, IL8, IL6ST et MMP9 (combinaison n°5) ; ou
- SPP1, A2M, IL8, IL6ST et MMP1 (combinaison n°6) ; ou

- SPP1, A2M, IL8, VIM, et ENG (combinaison n°7) ; ou
- SPP1, A2M, IL8, CXCL10 et IL6ST, (combinaison n°8) ; ou
- SPP1, A2M, IL8, CXCL1 et IL6ST (combinaison n°9) ; ou
- SPP1, A2M, IL8 et VIM (combinaison n°10) ; ou
- SPP1, A2M, IL8, COL1A1 et IL6ST (combinaison n°11) ; ou
- SPP1, A2M, IL8, CXCL11 et IL6ST (combinaison n°12) ; ou
- SPP1, A2M, IL8, CXCL10 et ENG (combinaison n°13) ; ou
- SPP1, A2M, IL8, IL6ST et TIMP1 (combinaison n°14) ; ou
- SPP1, A2M, IL8, IHH et IL6ST (combinaison n°15) ; ou
- SPP1, A2M, IL8, CXCL10 et S100A4 (combinaison n°16) ; ou
- SPP1, A2M, IL8, IL6ST et MMP7 (combinaison n°17) ; ou
- SPP1, A2M, IL8, ENG et CXCL11 (combinaison n°18) ; ou
- SPP1, A2M, ENG et MMP9 (combinaison n°19) ; ou
- SPP1, A2M, CXCL10 et ENG (combinaison n°20) ; ou
- SPP1, A2M, CXCL10, p14ARF et MMP9 (combinaison n°21) ; ou
- SPP1, A2M, IL8, CXCL6 et IL6ST (combinaison n°22) ; ou
- SPP1, A2M, IL8 et S100A4 (combinaison n°23) ; ou
- SPP1, A2M, IL8, ANGPT2 et MMP7 (combinaison n°24) ; ou
- SPP1, A2M, IL8, CXCL10 et p14ARF (combinaison n°25) ; ou
- SPP1, A2M, IL8 et TIMP1 (combinaison n°26) ; ou
- SPP1, A2M, IL8 et p14ARF (combinaison n°27) ; ou
- SPP1, A2M, IL8, CXCL10 et IRF9 (combinaison n°28) ; ou
- SPP1, IL8, VIM et MMP2 (combinaison n°29).

7. La méthode selon l'une quelconque des revendications 1 à 6, dans laquelle, en sus de SSP1, lesdits gènes choisis à l'étape i) comprennent :

- A2M, et
- IL8 et/ou MMP9, de préférence IL8,

ou

- A2M et/ou IL8, et
- zéro gène parmi ladite liste de seize gènes, ou un ou plusieurs gènes parmi ladite liste de seize gènes, parmi lesquels au moins un ou deux gènes parmi IL6ST, MMP9, S100A4, p14ARF, CHI3L1, plus particulièrement zéro gène parmi ladite liste de seize gènes ou un ou plusieurs gènes parmi ladite liste de seize gènes, parmi lesquels au moins un ou deux gènes parmi IL6ST, MMP9, S100A4.

8. La méthode selon l'une quelconque des revendications 1 à 7, dans laquelle lesdits gènes choisis à l'étape i) comprennent, ou sont :

- SPP1, A2M, IL8, CHI3L1 et IL6ST (combinaison n°1) ; ou
- SPP1, A2M, IL8, VIM et CXCL10 (combinaison n°4) ; ou
- SPP1, A2M, IL8, VIM, et ENG (combinaison n°7) ; ou
- SPP1, A2M, IL8 et VIM (combinaison n°10) ; ou
- SPP1, A2M, IL8, CXCL10 et ENG (combinaison n°13) ; ou
- SPP1, A2M, ENG et MMP9 (combinaison n°19) ; ou
- SPP1, A2M, CXCL10, p14ARF et MMP9 (combinaison n°21) ; ou
- SPP1, A2M, IL8 et S100A4 (combinaison n°23).

9. La méthode selon l'une quelconque des revendication 6 à 8, dans laquelle :

- les gènes choisis à l'étape i) sont les gènes de l'une desdites combinaisons n°1 à n°29,
- à l'étape i), on dose les niveaux auxquels chacun de ces gènes choisis sont transcrits, et
- la comparaison de l'étape ii) est réalisée en combinant lesdites valeurs de dosage obtenues à l'étape i) dans la fonction linéaire Z qui est indiquée pour cette combinaison de gènes dans le tableau 4, et, optionnellement, par comparaison de la valeur de sortie ainsi obtenue au seuil $\delta$ indiqué pour cette fonction Z dans le tableau 5,

ou
dans laquelle :

- les gènes choisis à l'étape i) sont SPP1, A2M, IL8, CXCL10 et S100A4 (combinaison n°16),
- à l'étape i), on dose les niveaux auxquels ces gènes choisis sont traduits, et
- la comparaison de l'étape ii) est réalisée en combinant lesdites valeurs de dosage obtenues à l'étape i) dans la fonction linéaire Z qui est indiquée pour cette combinaison de gènes dans le tableau 6, et, optionnellement, par comparaison de la valeur de sortie ainsi obtenue au seuil δ indiqué pour cette fonction Z dans le tableau 7.

**10.** La méthode selon l'une quelconque des revendications 1 à 9, dans laquelle :

- en sus de doser le niveau auquel les gènes choisis à l'étape i) sont transcrits ou traduits, on dose, mesure ou détermine, pour ledit sujet, la valeur de :

- un ou plusieurs facteur(s) clinique(s), tels qu'un ou plusieurs facteur(s) clinique(s) choisis(s) parmi : sexe, âge à la date du prélèvement, indice de masse corporelle, indice de sensibilité à l'insuline, diabète, consommation d'alcool, degré de stéatose, mode de contamination, activité Metavir ; et/ou
- un ou plusieurs facteur(s) virologique(s), tels qu'un ou plusieurs facteur(s) virologique(s) choisis(s) parmi : génotype viral, durée de l'infection, charge virale mesurée chez le patient à la date du début du traitement, charge virale mesurée chez le patient à la date du prélèvement ; et/ou
- un ou plusieurs facteur(s) biologique(s) autre(s) que les niveaux de transcription ou de traduction desdits gènes choisis, tels qu'un ou plusieurs facteur(s) biologique(s) choisis(s) parmi : concentration en haptoglobine, concentration en apolipoprotéine A1, teneur en bilirubine totale, concentration en gamma glutamyl transpeptidase, concentration en aspartate aminotransférase, concentration en alanine aminotransférase (ALT), teneur en plaquettes, taux de prothrombine, teneur en cholestérol HDL, teneur en cholestérol total, concentration en ferritine, teneur glycémique, concentration en peptide C, teneur en insuline, concentration en triglycérides, teneur en albumine, coefficient de saturation en fer de la transferrine, concentration en phosphatase alcaline,

et dans laquelle
- à l'étape ii), on compare la(les) valeur(s) de cet(ces) facteur(s) et les valeurs de dosage du niveau de transcription ou traduction desdits gènes choisis à l'étape i), à leurs valeurs, ou à la distribution de leurs valeurs, dans des cohortes de référence pré-établies selon leur score de fibrose hépatique, pour classer ledit sujet dans celle de ces cohortes de référence vis-à-vis de laquelle il a la plus forte probabilité d'appartenance,

et/ou
dans laquelle ledit échantillon préalablement obtenu à partir dudit sujet est :

- un échantillon biologique préalablement prélevé dudit sujet, ou
- un échantillon comprenant des acides nucléiques et/ou protéines et/ou polypeptides et/ou peptides extraits ou purifiés dudit échantillon biologique, ou
- un échantillon comprenant des ADNc susceptibles d'avoir été obtenus par transcription inverse desdits acides nucléiques,

lesdits acides nucléiques étant de préférence des ARN, notamment des ARNm,
ledit échantillon biologique étant de préférence :

- un échantillon biologique de tissu ou de cellules préalablement prélevé ou collecté du foie dudit sujet, par exemple par préalable ponction biopsique hépatique ou par préalable cyto-ponction hépatique, ou
- un échantillon de fluide biologique dudit sujet, tel qu'un échantillon de sang, de sérum, de plasma ou d'urine.

**11. Article manufacturé** comprenant des réactifs en préparation combinée pour leur utilisation simultanée, séparée ou étalée dans le temps, lesdits réactifs étant constitués :

- de réactifs qui détectent de manière spécifique chacun des produits de transcription ou de traduction de 3 à 22 gènes humains, lesdits 3 à 22 gènes humains comprenant lesdits gènes choisis conformément à l'étape i) de l'une quelconque des revendications 1 à 10,

dans lequel lesdits réactifs sont des acides nucléiques qui s'hybrident de manière spécifique à l'ARN desdits gènes choisis conformément à l'une quelconque des revendications 1 à 10, et/ou à l'ADNc obtenu par transcription inverse de ces ARN, ou sont des protéines, polypeptides ou peptides qui se lient de manière spécifique aux protéines codées par lesdits gènes choisis, et en particulier dans lequel lesdits réactifs sont des amorces d'amplification et/ou des sondes acides nucléiques, ou sont des anticorps ou fragments d'anticorps ou des aptamères protéiques, polypeptidiques ou peptides,

et

dans lequel lesdits réactifs sont contenus dans un ou des tubes, ou dans les puits d'une plaque d'amplification d'acide nucléique destinée à recevoir un échantillon contenant des acides nucléiques et un mélange réactionnel d'amplification d'acide nucléique, ou dans les puits d'une plaque de titrage protéique, plus particulièrement une plaque de microtitrage protéique, par exemple une plaque ELISA, ou sur des microbilles ou sur une puce pour la détection et la quantification d'acides nucléiques, protéines, polypeptides ou peptides.

12. **Article manufacturé** selon la revendication 11 pour mettre en oeuvre la méthode de la revendication 10, comprenant en outre d'autres réactifs en préparation combinée pour leur utilisation simultanée, séparée ou étalée dans le temps, lesdits autres réactifs permettant de détecter, doser ou déterminer :

- un ou plusieurs facteur(s) virologique(s), tels qu'un ou plusieurs facteur(s) virologique(s) choisis(s) parmi : génotype viral, durée de l'infection, charge virale mesurée chez le patient à la date du début du traitement, charge virale mesurée chez le patient à la date du prélèvement ; et/ou
- un ou plusieurs facteur(s) biologique(s) autre(s) que les niveaux de transcription ou de traduction desdits gènes choisis, tels qu'un ou plusieurs facteur(s) biologique(s) choisis(s) parmi : concentration en haptoglobine, concentration en apolipoprotéine A1, teneur en bilirubine totale, concentration en gamma glutamyl transpeptidase, concentration en aspartate aminotransférase, concentration en alanine aminotransférase (ALT), teneur en plaquettes, taux de prothrombine, teneur en cholestérol HDL, teneur en cholestérol total, concentration en ferritine, teneur glycémique, concentration en peptide C, teneur en insuline, concentration en triglycérides, teneur en albumine, coefficient de saturation en fer de la transferrine, concentration en phosphatase alcaline,

dans lequel lesdits réactifs sont des acides nucléiques qui s'hybrident de manière spécifique à l'ARN dudit ou desdits facteur(s) virologique (s) et/ou dudit ou desdits facteur(s) biologique(s) autre(s) choisi(s) conformément à la revendication 10, et/ou à l'ADNc obtenu par transcription inverse de ces ARN, ou sont des protéines, polypeptides ou peptides qui se lient de manière spécifique aux protéines codées par les gènes dudit ou desdits facteur(s) biologique(s) autre(s) choisi(s), en particulier dans lequel lesdits réactifs sont des amorces d'amplification et/ou des sondes acides nucléiques, ou sont des anticorps ou fragments d'anticorps ou des aptamères protéiques, polypeptidiques ou peptides,

et

dans lequel lesdits réactifs sont contenus dans un ou des tubes, ou dans les puits d'une plaque d'amplification d'acide nucléique destinée à recevoir un échantillon contenant des acides nucléiques et un mélange réactionnel d'amplification d'acide nucléique, ou dans les puits d'une plaque de titrage protéique, plus particulièrement une plaque de microtitrage protéique, par exemple une plaque ELISA, ou sur des microbilles ou sur une puce pour la détection et la quantification d'acides nucléiques, protéines, polypeptides ou peptides

13. **Utilisation d'un article manufacturé** selon l'une quelconque des revendications 11 ou 12, pour la mise en oeuvre d'une méthode selon l'une quelconque des revendications 1 à 10.

**Patentansprüche**

1. In vitro-Verfahren zur Bestimmung, ob der Leberfibrose-Score eines Patienten, der mit einem oder mehreren Hepatitis-Virus (Viren) infiziert ist, der einen Metavir-Fibrotisch-Score von höchstens als F1 oder wenigstens F2 aufweise, **dadurch gekennzeichnet, dass** es die folgenden Schritte umfasst:

i) in einer Probe, die von dem genannten Patienten vorhab erhalten wurde, Dosierung der Niveaus, zu denen die ausgewählten Gene transkribiert oder translatiert wurden, wobei die genannten ausgewählten Gene, deren Transkriptions- oder Translationsdosisniveaus dosiert wurden, die folgenden Genkombinationen aufweisen:

- SPP1 und
- wenigstens ein Gen aus A2M und V1M und

- wenigstens ein Gen aus IL8, CXCL10 und ENG und
- null bis sechzehn Gene aus der Liste der folgenden sechzehn Gene: IL6ST, p14ARF, MMP9, ANGPT2, CXCL11, MMP2, MMP7, S100A4, TIMP1, CHI3L1, COL1A1, CXCL1 CXCL6, IHH, IRF9, MMP1,

ii) Vergleichen der Dosierungswerte jedes der genannten ausgewählten Gene, die für den genannten Patienten erhalten werden, mit deren Werten oder mit der Verteilung ihrer Werte in Referenzkohorten, die als eine Funktion ihres Leberfibrose-Scores vorab etabliert wurden,

wobei die genannten Kohorten sind:

- eine Kohorte von Personen mit einer Leberfibrose, die einen Metavir-Fibrotisch-Score von höchstens F1 aufweist und
- eine Kohorte von Personen mit einer Leberfibrose, die einen Metavir-Fibrotisch-Score von wenigstens F2 aufweist.

um den genannten Patienten in die derjenigen Referenzkohorten einzuordnen, bei der die Wahrscheinlichkeit der Zugehörigkeit am höchsten ist.

2. Verfahren gemäß Anspruch 1, bei dem der Vergleich von Schritt ii) durch Kombination der Dosierungswerte, die für den genannten Patienten erhalten wurden, in einem multivariaten Klassifizierungsmodell, das diejenigen Werte mit deren Werten oder die Verteilung von deren Werten vergleicht, in Referenzkohorten, die als Funktion ihres Leberfibrose-Score vorab etabliert wurden, um den genannten Patienten in diejenige der Referenzkohorten einzuordnen, bei der die Wahrscheinlichkeit der Zugehörigkeit am höchsten ist, durchgeführt wird,
und /oder
bei der der Vergleich des Schritts ii) per Kombination der Dosierungswerte erfolgt, die für den genannten Patienten in einem multivariaten Klassifizierungsmodell erhalten werden, das zuvor wie folgt konstruiert wurde:

a) für eine Population von Personen, die zu derselben Spezies gehören wie der genannte Patient und die mit demselben Hepatitisvirus oder -viren infiziert sind wie der genannte Patient, Bestimmung des Leberfibrose-Scores jeder der genannten Personen der Population und deren Einordnung in Unter-Populationen in Abhängigkeit von ihrem Leberfibrose-Score, wodurch Referenzkohorten gebildet werden, die von ihrem Leberfibrose-Score etabliert sind;
b) in wenigstens einer Probe, die bereits von jeder der genannten Personen erhalten wurde, deren Typ mit dem der Probe von dem genannten Patienten identisch ist, Dosieren des Transkriptions- oder Translationsniveaus jedes der genannten ausgewählten Gene;
c) Durchführung eines Inter-Kohortenvergleichs der Dosierungswerte, die in Schritt b) erhalten wurden, oder die Verteilung dieser Dosierungswerte, um ein multivariates Klassifizierungsmodell zu konstruieren, das einen Leberfibrose-Score von der Kombination der Transkriptionsniveaus ableitet oder, falls angemessen, Translation der genannten ausgewählten Gene.

3. Verfahren gemäß Anspruch 1 bis Anspruch 2, bei dem der Vergleich von Schritt ii) durch Kombination der genannten Dosierungswerte realisiert wird, die in einer mathematischen Funktion, namentlich einer linearen oder nicht-linearen Funktion, insbesondere in einer linearen Funktion, erfolgt, um einen Ausgabewert zu erhalten, der für den Leberfibrose-Score des genannten Patienten indikativ ist
oder
bei dem der Vergleich des Schritts ii) per Kombination der genannten Dosierungswerte realisiert ist, die in Schritt i) in einem multivariaten automatisches-Lernmodell, z. B. einem multivariaten, nicht parametrischen Klassifizierungsmodell, einem multivariaten heuristischten Modell oder einem multivariaten probabilistischen Prognosemodell erhalten wurden, um einen Ausgabswert des Leberfibrose-Scores des genannten Patienten zu erhalten.

4. Verfahren gemäß irgendeinem der Ansprüche 1 bis 3, bei dem die Einordnung der genannten Patienten in diejenige der genannten Referenzkohorten, bei der die Wahrscheinlichkeit einer Zugehörigkeit am größten ist, erfolgt mit:

- einer Sensibilität (Se) von wenigstens 67 %, wenigstens 68 %, wenigstens 69 %, wenigstens 70 % oder wenigstens 71 % oder wenigstens 72 % oder wenigstens 73 % oder wenigstens 74 % oder wenigstens 75 %; und / oder mit einer
- Spezifizität (Sp) von wenigstens 67 %, wenigstens 68 %, wenigstens 69 %, wenigstens 70 % oder wenigstens 71 % oder wenigstens 72 oder wenigstens 73 % oder wenigstens 74 % oder wenigstens 75 %; und / oder mit

- einem negativen Prognosewert (VPN) von wenigstens 80 % oder wenigstens 81 % oder wenigstens 82 % oder wenigstens 83 % oder wenigstens 84 %; und / oder mit
- einem positiven Prognosewert (VPP) von wenigstens 50 % oder wenigstens 55 % oder wenigstens 56 % oder wenigstens 57 % oder wenigstens 58 % oder wenigstens 59 % oder wenigstens 60 %;

bevorzugt mit wenigstens dem genannten VPN und / oder der genannten Sensibilität, weiter bevorzugt mit wenigstens dem genannten VPN und der genannten Sensibilität und der genannten Spezifizität,
und / oder
bei dem das genannte multivariate Klassifizierungsmodell einen Bereich unter der ROC-Kurve (AUC) von wenigstens 0,60, wenigstens 0,61, insbesondere von wenigstens 0,66, weiter bevorzugt von wenigstens 0,69, noch weiter bevorzugt von wenigstens 0,70, wenigstens 0,71, wenigstens 0,72, wenigstens 0,73, wenigstens 0,74, weiter bevorzugt von 0,75, noch weiter bevorzugt von wenigstens 0,76, noch weiter bevorzugt von wenigstens 0,77, insbesondere von wenigstens 0,78, wenigstens 0,79 oder wenigstens 0,80 aufweist.

5.  Verfahren gemäß irgendeinem der Ansprüche 1 bis 4, bei dem bei dem die genannten, in Schritt 1) ausgewählten Gene A2M und / oder IL8 umfassen,
und / oder

- die Gesamtzahl der genannten, in Schritt i) ausgewählten Gene vier oder fünf beträgt; und / oder bei dem
- die genannten in Schritt i) ausgewählten Gene nur ein oder zwei Gen(e) aus der genannten Liste von sechzehn Genen umfasst oder überhaupt keines umfasst.

6.  Verfahren gemäß irgendeinem der Ansprüche 1 bis 5, bei dem die genannten, in Schritt i) ausgewählten Gene umfassen oder sind:

- SPP1, A2M, IL8, CHI3L1 und IL6ST (Kombination Nr. 1) oder
- SPP1, A2M, IL8, ANGPT2 und IL6ST (Kombination Nr. 2) oder
- SPP1, A2M, IL8, IL6ST und MMP2 (Kombination Nr. 3) oder
- SPP1, A2M, IL8, VIM und CXCL10 (Kombination Nr. 4) oder
- SPP1, A2M, IL8, IL6ST und MMP9 (Kombination Nr. 5) oder
- SPP1, A2M, IL8, IL6ST und MMP1 (Kombination Nr. 6) oder
- SSP1, A2M, IL8, VIM und ENG (Kombination Nr. 7) oder
- SSP1, A2M, IL8, CXCL10 und IL6ST, (Kombination Nr. 8) oder
- SSP1, A2M, IL8, CXCL1 und IL6ST (Kombination Nr. 9) oder
- SSP1, A2M, IL8 und VIM (Kombination Nr. 10); oder
- SSP1, A2M, IL8, COL1A1 und IL6ST (Kombination Nr. 11) oder
- SSP1, A2M, IL8, CXCL11 und IL6ST (Kombination Nr. 12) oder
- SSP1, A2M, IL8, CXCL10 und ENG (Kombination Nr. 13) oder
- SSP1, A2M, IL8, IL6ST und TIMP1 (Kombination Nr. 14) oder
- SSP1, A2M, IL8, IHH und IL6ST (Kombination Nr. 15) oder
- SSP1, A2M, IL8, CXCL10 und S100A4 (Kombination Nr. 16) oder
- SSP1, A2M, IL8, IL6ST und MMP7 (Kombination Nr. 17) oder
- SPP1, A2M, IL8, ENG und CXCL11 (Kombination Nr. 18), oder
- SSP1, A2M, ENG und MMP9 (Kombination Nr. 19) oder
- SSP1, A2M, CXCL10 und ENG (Kombination Nr. 20) oder
- SSP1, A2M, CXCL10, p14ARF und MMP9 (Kombination Nr. 21) oder
- SSP1, A2M, IL8, CXCL6 und IL6ST (Kombination Nr. 22) oder
- SSP1, A2M, IL8 und S100A4 (Kombination Nr. 23) oder
- SSP1, A2M, IL8, ANGPT2 und MMP7 (Kombination Nr. 24) oder
- SSP1, A2M, IL8, CXCL10 und p14ARF (Kombination Nr. 25) oder
- SPP1, A2M, IL8 und TIMP1 (Kombination Nr. 26) oder
- SSP1, A2M, IL8 und p14ARF (Kombination Nr. 27) oder
- SPP1, A2M, IL8, CXCL10 und IRF9 (Kombination Nr. 28) oder
- SSP1, IL8, VIM und MMP2 (Kombination Nr. 29).

7.  Verfahren gemäß irgendeinem der Ansprüche 1 bis 6, bei dem die in Schritt i) ausgewählten Gene zusätzlich zu SSP1 umfassen:

- A2M und
- IL8 und / oder MMP9, bevorzugt IL8,
oder
- A2M und / oder IL8 und
- null Gene von der genannten Liste von sechzehn Genen oder ein oder mehr Gene von der genannten Liste von sechzehn Genen, wobei wenigstens ein oder zwei Gene aus IL6St, MMP9, S100A4, p14ARF und CHI3L1, insbesondere null Gene von der genannten Liste von sechsten Genen oder ein oder mehr Gene aus der genannten Liste von sechzehn Genen, wobei wenigstens ein oder zwei Gene, aus IL6ST, MMP9 und S100A4 sind.

8. Verfahren gemäß irgendeinem der Ansprüche 1 bis 7, bei dem die genannten, in Schritt i) ausgewählten Gene umfassen oder sind:

- SPP1, A2M, IL8, CHI3L1 und IL6ST (Kombination Nr. 1) oder
- SPP1, A2M, IL8, VIM und CXCL10 (Kombination Nr. 4) oder
- SSP1, A2M, IL8, VIM und ENG (Kombination Nr. 7) oder
- SSP1 A2M, IL8 und VIM (Kombination Nr. 10); oder
- SSP1, A2M, IL8, CXCL10 und ENG (Kombination Nr. 13) oder
- SSP1, A2M, ENG und MMP9 (Kombination Nr. 19) oder
- SSP1, A2M, CXCL10, p14ARF und MMP9 (Kombination Nr. 21) oder
- SSP1, A2M, IL8 und S100A4 (Kombination Nr. 23).

9. Verfahren gemäß irgendeinem der Ansprüche 6 bis 8, bei dem:

- die genannten, in Schritt 1) ausgewählten Gene die Gene einer der genannten Kombinationen Nr. 1 bis Nr. 29 sind,
- in Schritt i) die Niveaus dosiert werden, in die jedes dieser gewählten Gene transkribiert werden und
- der Vergleich des Schritts ii) per Kombination der genannten, in Schritt i) erhaltenen Dosierungswerte ii) durch Kombination der in Schritt i) erhaltenen Dosierungswerte in der linearen Funktion Z, die für diese Genkombination in Tabelle 4 angegeben wird und optional per Vergleich des dadurch erhaltenen Ausgabewertes mit dem Schwellenwert $\delta$ erfolgt, der für diese Funktion Z in Tabelle 5 angegeben wird,

oder
bei der:

die in Schritt i) ausgewählten Gene SPP1, A2M, IL8, CXCL10 und S100A4 (Kombination Nr. 16) sind
- in Schritt i) die Niveaus dosiert werden, in die diese gewählten Gene translatiert werden und
- der Vergleich des Schritts ii) durch Kombinieren der genannten, in Schritt i) erhaltenen Dosierungswerte in der linearen Funktion Z erfolgt, die hinsichtlich ihrer Genkombination in Tabelle 6 angegeben wird und optional per Vergleich des dadurch erhaltenen Ausgabewertes mit dem Schwellen wert $\delta$, der in dieser Funktion Z in Tabelle 7 angegeben wird

10. Verfahren gemäß irgendeinem der Ansprüche 1 bis 9, bei dem:

- zusätzlich zur Dosierung des Niveaus, auf dem die in Schritt i) ausgewählten Gene für den genannten Patienten transkribiert oder translatiert werden, der Wert für Folgendes dosiert, gemessen oder bestimmt wird:
- ein oder mehr klinische (r) Faktor (en), wie z. B. ein oder mehr klinische Faktor(en), der die ausgewählt ist (sind) aus: Geschlecht, Alter am Tag der Probennahme, Body-Maß-Index, Insulinsensitivitätsindex, Diabetes, Alkoholkonsum, Steatosisgrad, Kontaminationsmodus oder Metaviraktivität; und / oder
- ein oder mehr virologische(r) Faktor (en), wie z. B. ein oder mehr virologische(r) Faktor (en), der / die ausgewählt ist (sind) aus: viraler Genotyp, Dauer der Infektion, virale Belastung, die für den Patienten am Tag des Beginns der Behandlung festgestellt wurde, virale Charge, die bei dem Patienten am Tag der Probennahme festgestellt wird; und / oder
- ein oder mehr biologische(r) Faktor(en), die (der) nicht die Transkriptions- oder Translationsniveaus der genannten ausgewählten Gene ist (sind), wie z. B. ein oder mehr biologische(r) Faktor(en), der (die) ausgewählt ist (sind) aus: Haptoglobinkonzentration, Apolipoproteinkonzentration A1, Bilirubin-Gesamtgehalt, Gamma-Glutamyltransferase-Konzentration, Aspartat-Aminotransferase-Konzentration, Alanin-Aminotransferase-Konzentration (ALT), Thrombozytenzahl, Prothrombinzahl, Menge des Cholesterins HDL, Gesamtcholesterin, Ferritinkonzentration, Glykämiespiegel, Peptid-C-Konzentration, Insulinspiegel, Trigylceridkonzentration, Albuminmenge, Transferrinsättigung und Alkalin-Phosphatasekonzentration,

und bei dem

- in Schritt ii) der (die) Wert(e) für diese(n) Faktor(en) und die Dosierungswerte für das Transkriptions- oder Translationsniveau der genannten, in Schritt i) ausgewählten Gene mit ihren Werten oder mit der Verteilung ihrer Werte in Referenzkohorten verglichen wird (werden), die als eine Funktion ihres Leberfibrose-Scores vorab etabliert wurden, um den genannten Patienten in diejenige der Referenzkohorten einzuordnen, bei der die Wahrscheinlichkeit der Zugehörigkeit am höchsten ist,

und / oder

- eine biologische Probe, die bereits von dem genannten Patienten entnommen wurde oder
- eine Probe, umfassend Nukleinsäuren und / oder Proteine und / oder Polypeptide und / oder Peptide, die aus der genannten biologischen Probe extrahiert oder davon gereinigt wurden, oder
- eine Probe, umfassend cDNA, die geeignet sind, per Umkehr-Transkription der genannten Nukleinsäuren erhalten worden zu sein,

wobei die genannten Nukleinsäuren bevorzugt RNAs, insbesondere mRNAs sind,
wobei die genannte biologische Probe ist

- eine biologische Gewebe- oder eine biologische Zellprobe, die bereits von der Leber des genannten Patienten entfernt oder entnommen worden ist, z. B. vor der hepatischen Biopsiepunktion oder per vorheriger hepatischer Zytopunktion oder
- eine biologische Flüssigkeit von dem genannten Patienten, wie z. B. eine Blutprobe, Serum, Plasma oder Urin.

11. Hergestellter Artikel, umfassend Reagenzien in einer kombinierten Zubereitung für ihre gleichzeitige, separate oder sequenzielle Verwendung, wobei die genannten Reagenzien gebildet sind durch:

- Reagenzien, die spezifisch jedes der Transkriptions- oder Translationsprodukte aus 3 bis 22 menschlichen Genen detektieren, wobei die genannten 3 bis 22 menschlichen Gene die genannten, gemäß Schritt i) ausgewählten Gene gemäß irgendeinem der Ansprüche 1 bis 10 umfassen,

bei dem die genannte Reagenzie Nukleinsäuren sind, die spezifisch mit der RNA der genannten ausgewählten Gene gemäß irgendeinem der Ansprüche 1 bis 10 und / oder der cDNA hybridisieren, die per Umkehr-Transkription dieser RNA erhalten wird, oder Proteine, Polypeptide oder Peptide sind, die sich spezifisch an die Proteine binden, die durch die genannten ausgewählten Gene kodiert sind und in dem insbesondere die genannten Reagenzien Amplifikations-Primer und / oder Nukleinsäure-Gensonden sind oder Antikörper oder Fragmente von Antikörpern oder Protein-, Polypeptid- oder Peptid-Aptamere sind
und
bei dem die genannten Reagenzien in einer oder mehr Röhre(n) oder in den Vertiefungen eines Nukleinsäure-Amplifikationsschälchens zur Aufnahme einer Probe, die Nukleinsäuren und eine Reaktionsmischung zur Nukleinsäure-Amplifikation enthält, oder in den Schälchen eines Protein-Titrierungsschälchens, weiter bevorzugt ein Protein-Mikrotitrierungsschälchen, z. B. ein ELISA-Schälchen, oder auf Microbeads oder auf einem Chip zur Detektion und Quantifizierung von Nukleinsäuren, Proteinen, Polypeptiden oder Peptiden enthalten sind.

12. Hergestellter Artikel gemäß Anspruch 11 zur Umsetzung des Anspruchs 10, umfassend darüber hinaus Reagenzien in einer kombinierten Zubereitung für ihre gleichzeitige, separate oder sequenzielle Verwendung, wobei die genannten anderen Reagenzien die Detektion, Dosierung oder Bestimmung erlauben:

- eines oder mehr virologische(r) Faktor(en), wie z. B. ein oder mehr virologische(r) Faktor (en), der / die ausgewählt ist (sind) aus: viraler Genotyp, Dauer der Infektion, virale Belastung, die für den Patienten am Tag des Beginns der Behandlung festgestellt wurde, virale Belastung, die bei dem Patienten am Tag der Probennahme festgestellt wird; und / oder
- eines oder mehr biologische(r) Faktor(en), die (der) nicht die Transkriptions- oder Translationsniveaus der genannten ausgewählten Gene ist (sind), wie z. B. ein oder mehr biologische(r) Faktor(en), der (die) ausgewählt ist (sind) aus: Haptoglobinkonzentration, Apolipoproteinkonzentration A1, Bilirubin-Gesamtgehalt, Gamma-Glutamyltransferase-Konzentration, Aspartat-Aminotransferase-Konzentration, Alanin-Aminotransferase-Konzentration (ALT), Thrombozytenzahl, Prothrombinzahl, Menge des Cholesterins HDL, Gesamtcholesterin, Ferritinkonzentration, Glykämiespiegel, Peptid-C-Konzentration, Insulinspiegel, Trigylceridkonzentration, Albuminmenge, Transferrinsättigung und Alkalin-Phosphatasekonzentration,

und bei dem die genannten Reagenzien Nukleinsäuren sind, die spezifisch mit der RNA der genannten ausgewählten virologischen Faktoren und / oder den anderen ausgewählten biologischen Faktoren gemäß Anspruch 10 und / oder der cDNA hybridisieren, die per Umkehr-Transkription dieser RNA erhalten wird, oder Proteine, Polypeptide oder Peptide sind, die sich spezifisch an die Proteine binden, die durch die genannten ausgewählten Gene kodiert sind und in dem insbesondere die genannten Reagenzien Amplifikations-Primer und / oder Nukleinsäure-Gensonden sind oder Antikörper oder Fragmente von Antikörpern oder Protein-, Polypeptid- oder Peptid-Aptamere sind und

bei dem die genannten Reagenzien in einer oder mehr Röhre(n) oder in den Vertiefungen eines Nukleinsäure-Amplifikationsschälchens zur Aufnahme einer Probe, die Nukleinsäuren und eine Reaktionsmischung zur Nukleinsäure-Amplifikation enthält, oder in den Schälchen eines Protein-Titrierungsschälchens, weiter bevorzugt ein Protein-Mikrotitrierungsschälchen, z. B. ein ELISA-Schälchen, oder auf Microbeads oder auf einem Chip zur Detektion und Quantifizierung von Nukleinsäuren, Proteinen, Polypeptiden oder Peptiden enthalten sind.

13. Verwendung eines hergestellten Artikels gemäß irgendeinem der Ansprüche 11 oder 12 zur Umsetzung eines Verfahrens gemäß irgendeinem der Ansprüche 1 bis 10.

## Claims

1. An *in vitro* method for determining whether the hepatic fibrosis score of a subject infected with one or more hepatitis viruses has a Metavir fibrotic score of no more than F1 or at least F2, **characterized in that** it comprises the following steps:

1) in a sample which has been obtained from said subject, measuring the levels to which selected genes are transcribed or translated, said selected genes the levels of transcription or translation of which are measured being the following combination of genes:

- SPP1, and
- at least one gene from among A2M and VIM, and
- at least one gene from among IL8, CXCL10 and ENG, and
- zero to sixteen genes from among the list of the following sixteen genes: IL6ST, p14ARF, MMP9, ANGPT2, CXCL11, MMP2, MMP7, S100A4, TIMP1, CHI3L1, COL1A1, CXCL1, CXCL6, IHH, IRF9 and MMP1,

ii) comparing the measuring values of each of said selected genes obtained for said subject with their values, or with the distribution of their values, in reference cohorts which have been pre-established according to their hepatic fibrosis score, said cohorts being:

- a cohort of individuals with a hepatic fibrosis which has a Metavir fibrotic score of no more than F1, and
- a cohort of individuals with a hepatic fibrosis which has a Metavir fibrotic score of at least F2;

in order to classify said subject in whichever of these reference cohorts to which it has the greatest probability of belonging.

2. The method according to claim 1, in which the comparison of step ii) is carried out by combining the measurement values obtained for said subject into a multivariate classification model which compares those values with their values, or the distribution of their values, in reference cohorts which have been pre-established according to their hepatic fibrosis score, in order to classify said subject in whichever of those reference cohorts to which it has the highest probability of belonging,
and/or
in which the comparison of step ii) is made by combining the measurement values obtained for said subject in a multivariate classification model previously constructed as follows:

a) for a population of individuals who are of the same species as said subject and who are infected with the same hepatitis virus or viruses as said subject, determining the hepatic fibrosis score of each of said individuals of the population, and classifying them in sub-populations according to their hepatic fibrosis score, thereby constituting reference cohorts established according to their hepatic fibrosis score;

b) in at least one sample which has already been obtained from each of said individuals the nature of which is identical to that of the sample from said subject, measuring the level of transcription or translation of each of said selected genes;

c) making an inter-cohort comparison of the measurement values obtained in step b) or the distribution of these measurement values, in order to construct a multivariate classification model which infers a hepatic fibrosis score from the combination of the levels of transcription or, if appropriate, translation of said selected genes.

3. The method according to claim 1 or 2, in which the comparison of step ii) is made by combining said measurement values obtained in step i) into a mathematical function, in particular a linear or non-linear function, more particularly a linear function, in order to obtain an output value which is indicative of the hepatic fibrosis score of said subject, or

in which the comparison of step ii) is made by combining said measurement values obtained in step i) into a multivariate machine learning model, for example a multivariate non-parametric classification model, a multivariate heuristic model, or a multivariate probabilistic prediction model, in order to obtain an output value which is indicative of the hepatic fibrosis score of said subject.

4. The method according to any one of claims 1 to 3, in which the classification of said subject in whichever of said reference cohorts to which it has the highest probability of belonging is made with:

- a sensitivity (Sc) of at least 67%, at least 68%, at least 69%, at least 70%, or at least 71%, or at least 72%, or at least 73%, or at least 74%, or at least 75%; and/or with a
- specificity (Sp) of at least 67%, at least 68%, at least 69%, at least 70%, or at least 71%, or at least 72%, or at least 73%, or at least 74%, or at least 75%; and/or with
- a negative predictive value (NPV) of at least 80%, or at least 81%, or at least 82%, or at least 83%, or at least 84%; and/or with
- a positive predictive value (PPV) of at least 50%, or at least 55%, or at least 56%, or at least 57%, or at least 58%, or at least 59%, or at least 60%;

preferably with at least said NPV and/or said sensitivity, more particularly with at least said NPV and said sensitivity and said specificity,
and/or

in which said multivariate classification model has an area under the ROC curve (AUC) of at least 0.60, at least 0.61, at least 0.66, more particularly at least 0.69, still more particularly at least 0.70, at least 0.71, at least 0.72, at least 0.73, at least 0.74, more particularly at least 0.75, still more particularly at least 0.76, still more particularly at least 0.77, in particular at least 0.78, at least 0.79, or at least 0.80.

5. The method according to any of claims 1 to 4, in which said genes selected in step i) comprise A2M and/or IL8, and/or
in which:

- the total number of said genes selected in step i) is four or five; and/or in which
- said genes selected in step i) comprise only one or two gene(s) from said list of sixteen genes, or comprise none at all.

6. The method according to any one of claims 1 to 5, in which said genes selected in step i) comprise, or are:

- SPP1, A2M, IL8, CHI3L1 and IL6ST (combination No.1); or
- SPP1, A2M, IL8, ANGPT2 and IL6ST (combination No.2); or
- SPP1, A2M, IL8, IL6ST and MMP2 (combination No.3); or
- SPP1, A2M, IL8, VIM and CXC10 (combination No.4); or
- SPP1, A2M, IL8, IL6ST and MMP9 (combination No.5); or
- SPP1, A2M, IL8, IL6ST and MMP1 (combination No.6); or
- SPP1, A2M, IL8, VIM, and ENG (combination No.7); or
- SPP1, A2M, IL8, CXCL10 and IL6ST (combination No.8); or
- SPP1, A2M, IL8, CXCL1 and IL6ST (combination No.9); or

- SPP1, A2M, IL8 and VIM (combination No.10); or
- SPP1, A2M, IL8, COL1A1 and IL6ST (combination No.11); or
- SPP1, A2M, IL8, CXCL11 and IL6ST (combination No.12); or
- SPP1, A2M, IL8, CXCL10 and ENG (combination No.13); or
- SPP1, A2M, IL8, IL6ST and TIMP1 (combination No.14); or
- SPP1, A2M, IL8, IHH and IL6ST (combination No.15); or
- SPP1, A2M, IL8, CXCL10 and S100A4 (combination No.16); or
- SPP1, A2M, IL8, IL6ST and MMP7 (combination No.17); or
- SPP1, A2M, IL8, ENG and CXCL11 (combination No.18); or
- SPP1, A2M, ENG and MMP9 (combination No.19); or
- SPP1, A2M, CXCL10 and ENG (combination No.20); or
- SPP1, A2M, CXCL10, p14ARF and MMP9 (combination No.21); or
- SPP1, A2M, IL8, CXCL6 and IL6ST (combination No.22); or
- SPP1, A2M, IL8 and S100A4 (combination No.23); or
- SPP1, A2M, IL8, ANGPT2 and MMP7 (combination No.24); or
- SPP1, A2M, IL8, CXCL10 and p14ARF (combination No.25); or
- SPP1, A2M, IL8 and TIMP1 (combination No.26); or
- SPP1, A2M, IL8 and p14ARF (combination No.27); or
- SPP1, A2M, IL8, CXCL10 and IRF9 (combination No.28); or
- SPP1, IL8, VIM and MMP2 (combination No.29).

**7.** The method according to any one of claims 1 to 6, in which, in addition to SSP1, said genes selected in step i) comprise:

- A2M, and
- IL8 and/or MMP9, preferably IL8,
  or
- A2M and/or IL8, and
- zero genes from said list of sixteen genes, or one or more genes from among said list of sixteen genes, wherein at least one or two genes are from among IL6ST, MMP9, SI00A4, p14ARF and CHI3L1, more particularly zero genes from among said list of sixteen genes or one or more genes from among said list of sixteen genes, wherein at least one or two genes are from among IL6ST, MMP9 and S100A4.

**8.** The method according to any one of claims 1 to 7, in which said genes selected in step i) comprise, or are:

- SPP1, A2M, IL8, CHI3L1 and IL6ST (combination No.1); or
- SPP1, A2M, IL8, VIM and CXCL10 (combination No.4); or
- SPP1, A2M, IL8, VIM, and ENG (combination No.7); or
- SPP1, A2M, IL8 and VIM (combination No.10); or
- SPP1, A2M, IL8, CXCL10 and ENG (combination No.13); or
- SPP1, A2M, ENG and MMP9 (combination No.19); or
- SPP1, A2M, CXCL10, p14ARF and MMP9 (combination No.21); or
- SPP1, A2M, IL8 and S100A4 (combination No.23).

**9.** The method according to any one of claims 6 to 8, in which:

- the genes selected in step i) are the genes of one of said combination No. 1 to No. 29,
- in step i), the levels to which each of those selected genes are transcribed are measured, and
- the comparison of step ii) is made by combining said measurement values obtained in step i) into the linear Z function which is indicated for this combination of genes in Table 4, and, optionally, by comparison of the output value obtained thereby with the threshold $\delta$ indicated for this function Z in Table 5,
  or

in which:

- the genes selected in step i) are SPP1, A2M, IL8, CXCL10 and S100A4 (combination No. 16),
- in step i), the levels to which these selected genes are translated are measured, and
- the comparison of step ii) is made by combining said measurement values obtained in step i) into the linear Z function which is indicated for this combination of genes in Table 6, and, optionally, by comparison of the output value obtained thereby with the threshold $\delta$ indicated for this function Z in Table 7.

10. The method according to any one of claims 1 to 9, in which:

- in addition to measuring the level at which the genes selected in step i) are transcribed or translated, the value of the following is measured, assayed or determined for said subject:

- one or more clinical factor (s) such as one or more clinical factor(s) selected from: sex, age at the date of sampling, body mass index, insulin sensitivity index, diabetes, alcohol consumption, degree of steatosis, mode of contamination, Metavir activity; and/or
- one or more virological factor (s), such as one or more virological factor(s) selected from: viral genotype, duration of infection, viral load assayed for patient at treatment start date, viral load assayed for patient at sampling date; and/or
- one or more biological factor(s) other than the levels of transcription or translation of said selected genes, such as one or more biological factor(s) selected from: concentration of haptoglobin, concentration of apolipoprotein A1, total bilirubin content, concentration of gamma glutamyl transpeptidase, concentration of aspartate aminotransferase, concentration of alanine aminotransferase (ALT), platelet count, prothrombin level, quantity of cholesterol HDL, total cholesterol, concentration of ferritin, level of glycaemia, concentration of peptide C, insulin level, concentration of triglycerides, quantity of albumin, transferrin iron saturation coefficient, and concentration of alkaline phosphatase,

and in which

- in step (ii), the value (s) for this (these) factors and the measurement values for the level of transcription or translation of said genes selected in step i) are compared with their values or with the distribution of their values in reference cohorts which have been pre-established according to their hepatic fibrosis score in order to classify said subject in whichever of those reference cohorts to which it has the highest probability of belonging,

and/or
in which said sample which has already been obtained from said subject is:

- a biological sample which has already been taken from said subject, or
- a sample comprising nucleic acids and/or proteins and/or polypeptides and/or peptides extracted or purified from said biological sample, or
- a sample comprising cDNAs which may have been obtained by reverse transcription of said nucleic acids,

said nucleic acids preferably being RNAs, in particular mRNAs,
said biological sample preferably being

- a biological tissue or cell sample which has already been removed or collected from the liver of said subject, for example by prior hepatic biopsy puncture or by prior hepatic cytopuncture, or
- a sample of biological fluid from said subject, such as a sample of blood, serum, plasma or urine.

11. A manufactured article comprising reagents in a combined preparation for their simultaneous, separate or sequential use, said reagents consisting of:

- reagents that specifically detect each of the transcription or translation products of 3 to 22 human genes, said 3 to 22 human genes comprising said genes chosen in accordance with step i) of any of claims 1 to 10,

in which said reagents are nucleic acids which hybridize specifically to the RNA of said selected genes according to any one of claims 1 to 10, and/or to the cDNA obtained by reverse transcription of these RNAs, or are proteins, polypeptides or peptides which specifically bind to the proteins encoded by said selected genes, and in particular in which said reagents are amplification primers and/or nucleic acid probes, or are antibodies or fragments of antibodies or protein, polypeptide or peptide aptamers,
and
in which said reagents are contained in or more tubes, or in the wells of a nucleic acid amplification plate for receiving a sample containing nucleic acids and a reaction mixture for nucleic acid amplification, or in the wells of a protein titration plate, more particularly a protein microtitration plate, for example an ELISA plate, or on microbeads or on a chip for the detection and quantification of nucleic acids, proteins, polypeptides or peptides.

12. A manufactured article according to claim 11 for implementing the method of claim 10, further comprising other reagents in a combined preparation for their simultaneous, separate or sequential use, said other reagents making it possible to detect, measure or determine:

- one or more virological factor(s), such as one or more virological factor(s) selected from: viral genotype, duration of infection, viral load assayed for patient at treatment start date, viral load assayed for patient at sampling date; and/or
- one or more biological factor(s) other than the levels of transcription or translation of said selected genes, such as one or more biological factor(s) selected from: concentration of haptoglobin, concentration of apolipoprotein A1, total bilirubin content, concentration of gamma glutamyl transpeptidase, concentration of aspartate aminotransferase, concentration of alanine aminotransferase (ALT), platelet count, prothrombin level, quantity of cholesterol HDL, total cholesterol, concentration of ferritin, level of glycaemia, concentration of peptide C, insulin level, concentration of triglycerides, quantity of albumin, transferrin iron saturation coefficient, and concentration of alkaline phosphatase,

in which said reagents are nucleic acids that hybridise specifically to the RNA of said virological factor or factors and/or said other biological factor or factors chosen in accordance with claim 10, and/or to the cDNA obtained by reverse transcription of these RNAs, or are proteins, polypeptides or peptides that bind specifically to the proteins coded by the genes of said other biological factor or factors chosen, in particular in which said reagents are amplification primers and/or nucleic acid probes, or are antibodies or fragments of antibodies or protein, polypeptide or peptide aptamers,

and

in which said reagents are contained in one or more tubes, or in wells of a nucleic acid amplification plate intended to receive a sample containing nucleic acids and a nucleic acid amplification reaction mixture, or in the wells of a protein titration plate, more particularly a protein microtitration plate, for example an ELISA plate, or on microbeads or on a chip for the detection and quantification of nucleic acids, proteins, polypeptides or peptides.

13. Use of an article manufactured according to either claim 11 or claim 12, for implementing a method according to any of claims 1 to 10.

Données biologiques et cliniques $\Rightarrow$ RÈGLE $\Rightarrow$ Statut clinique prédit
$(x_{p1}, x_{p2}, ..., x_{pn})$ DÉCISIONNELLE $y_p$

**FIGURE 1**

seuil de décision

indice négatif | indice positif

sains | pathologiques

valeur de
0  5  10  15  20  25  30  35  40  45  50  55  60  l'indice

faux négatifs | vrais positifs
(FN) | (VP)

0  5  10  15  20  25  30  35  40  45  50  55  60

vrais négatifs | faux positifs
(VN) | (FP)

0  5  10  15  20  25  30  35  40  45  50  55  60

**FIGURE 2**

**FIGURE 3**

**FIGURE 4**

**FIGURE 5**

EP 2 673 375 B1

**EP 2 673 375 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0216949 A1
- WO 2006103570 A2
- WO 2006082522 A1
- US 4376110 A
- US 4683202 A
- US 4683195 A
- US 4965188 A
- US 6569627 B
- US 376110 A
- FR 1151022
- US 61440986 B

**Littérature non-brevet citée dans la description**

- **HASTIE ; TIBISHIRANI ; FRIEDMAN.** *Leave-One-Out-Cross-Validation,* 2009
- **ANASTASIADIS et al.** New globally convergent training scheme based on the resilient propagation algorithm. *Neurocomputing,* 2005, vol. 64, 253-270
- An algorythm for the grading of activity in chronic hepatitis C. **BEDOSSA ; POYNARD.** Hepatology. groupe Metavir français, 1996, vol. 24, 289-93
- **BOX ; COX.** An analysis of transformations. *Journal of the Royal Statistical Society,* 1964, vol. 26, 211-243
- **BREIMAN.** Random Forests. *Machine Learning,* 2001, vol. 45, 5-32
- **CASTERA et al.** Prospective comparison of transient elastography, Fibrotest, APRI, and liver biopsy for the assessment of fibrosis in chronic hepatitis C. *Gastroenterology,* 2005, vol. 128, 343-50
- **CHAMBERS.** Software for data analysis : programming with R. Springer, 2008
- **COLE et al.** *Proc. Natl. Acad. Sci. USA,* 1983, vol. 80, 2026-2030
- **COLE et al.** Monoclonal Antibodies And Cancer Therapy. Alan R. Liss, Inc, 1985, 77-96
- **DALGAARD.** Introductory statistics with R. Springer, 2008
- **FALISSARD.** Comprendre et utiliser les statistiques dans les sciences de la vie. *Masson,* 2005
- **GOODMAN.** Grading and staging systems for inflammation and fibrosis in chronic liver diseases. *Journal of Hepatology,* 2007, vol. 47, 598-607
- **HECHENBICHLER ; SCHLIEP.** weighed k-nearest-neighbor techniques and ordinal classification. *Sonderforschungsbereich,* 2004, vol. 386
- **HASTIE ; TIBISHIRANI ; FRIEDMAN.** The Elements of Statistical Learning: Data Mining, Inference and Prediction. Springer, 2009
- **IKATA ; GENTLEMAN.** R : a language for data analysis and graphics. *Journal of computational and graphical statistics,* 1996, vol. 5, 299-314
- Using Neural Nets for Interpretation of Nonlinear Models. **INTRATOR ; INTRATOR.** Proceedings of the Statistical Computing Section. 1993, 244-249
- **KÖHLER ; MILSTEIN.** *Nature,* 1975, vol. 256, 495-497
- **KOSBOR et al.** *Immunology Today,* 1983, vol. 4, 72
- **KRAMAR et al.** Critères ROC généralisés pour l'évaluation de plusieurs marqueurs tumoraux. *Revue d'Epidémiologie et Santé Publique,* 1999, vol. 47, 376-383
- **KRAMAR et al.** mROC: a computer program for combining tumour markers in predicting disease states. *Computer methods and programs in biomedicine,* 2001, vol. 66, 199-207
- **LIAW ; WIENER.** Classification and regression by Random Forest. *R. News,* 2002, vol. 2 (3), 18-22
- **LIVAK ; SCHMITTGEN.** Analysis of relative gene expression data using real-time quantitative PCR and the 2(-Delta Delta C(T)) Method. *Methods,* 2001, vol. 25, 402-408
- Rprop - Description and Implementation Details. **RIEDMILLER.** Technical Report. University of Karlsruhe, 1994
- **RIEDMILLER ; BRAUN.** A direct adaptive method for faster backpropagation learning: the RPROP algorithm. *Proceedings of the IEEE International Conference on Neural Networks (ICNN),* 1993, 586-591
- **REISER ; FARAGGI.** Confidence intervals for the generalized ROC criterion. *Biometrics,* 1997, vol. 53, 644-652

111

- **SCHMITTEN ; LIVAK.** Analyzing real-time PCR data by the comparative Ct method. *Nature Protocols,* 2008, vol. 3 (6), 1101-1108
- **SHAHEEN et al.** FibroTest and FibroScan for the prediction of hepatitis C-related fibrosis : a systematic review of diagnostic test accuracy. *Am. J. Gastroenterol.,* 2007, vol. 102 (11), 2589-2600
- **SHAPIRO.** The interprétation of diagnostic tests. *Statistical Methods in Medical Research,* 1999, vol. 8, 113-134
- **SU ; LIU.** Linear combinations of multiple diagnostic markers. *Journal of the American Statistical Association,* 1993, vol. 88, 1350-1355
- **SWETS.** Measuring the accuracy of diagnostic systems. *Science,* 1988, vol. 240, 1285-1293
- **THEODORIDIS ; KOUTROUMBOS.** Pattern Recognition. Academic Press, Elsevier, 2009

SEQUENCE LISTING

<110> BIO-RAD PASTEUR
ARIANA PHARMACEUTICALS S.A.
INSTITUT NATIONAL DE LA SANTE ET DE LA RECHERCHE MEDICALE
ASSISTANCE PUBLIQUE - HOPITAUX DE PARIS
CENTRE NATIONAL DE LA RECHERCHE SCIENTIFIQUE

<120> Combinaison de biomarqueurs pour la détection et l'évaluation d'une fibrose hépatique

<130> B08651B - FP/BA

<150> FR1151022
<151> 2011-02-09

<150> US61440986
<151> 2011-02-09

<160> 46

<170> PatentIn version 3.3

<210> 1
<211> 24
<212> DNA
<213> Homo sapiens

<400> 1
gcaagtaaaa accaaggtct tcca          24

<210> 2
<211> 23
<212> DNA
<213> Homo sapiens

<400> 2
tccagtcaat tccaccactg ttc          23

<210> 3
<211> 23
<212> DNA
<213> Homo sapiens

<400> 3
tcgcagacct gacatccagt acc          23

<210> 4
<211> 23
<212> DNA
<213> Homo sapiens

<400> 4
ccattcaact cctcgctttc cat          23

<210> 5
<211> 23
<212> DNA
<213> Homo sapiens

<400> 5
ctccctctgg ttgataccca ctc          23

<210> 6
<211> 25
<212> DNA
<213> Homo sapiens

<400> 6
agaagtttcg ttgataacct gtcca          25

<210> 7
<211> 25
<212> DNA
<213> Homo sapiens

<400> 7
ctgactctaa gtggcattca aggag          25

<210> 8
<211> 25
<212> DNA
<213> Homo sapiens

<400> 8
ggttgattac taatgctgat gcagg          25

<210> 9
<211> 24
<212> DNA
<213> Homo sapiens

<400> 9
caccggaagg aaccatctca ctgt          24

<210> 10
<211> 23
<212> DNA
<213> Homo sapiens

<400> 10
tccttggcaa aactgcacct tca          23

<210> 11
<211> 24
<212> DNA
<213> Homo sapiens

<400> 11
cacaacatgc agatctggac cact          24

<210> 12
<211> 20
<212> DNA
<213> Homo sapiens

<400> 12
tgggagcttg aagccacgaa          20

<210> 13
<211> 19
<212> DNA
<213> Homo sapiens

<400> 13
acgtgaggat ggcagcgtt          19

<210> 14
<211> 25
<212> DNA
<213> Homo sapiens

<400> 14
gaagggttac caaatcccac tttat          25

<210> 15
<211> 21

<212> DNA
<213> Homo sapiens

<400> 15
ggttttcgtg gttcacatcc c          21

<210> 16
<211> 22
<212> DNA
<213> Homo sapiens

<400> 16
cccatcatca tgacctggtc tt          22

<210> 17
<211> 21
<212> DNA
<213> Homo sapiens

<400> 17
gaccacaggc catcacagtc c          21

<210> 18
<211> 25
<212> DNA
<213> Homo sapiens

<400> 18
tgtaccccac agcatagtca gtgtt          25

<210> 19
<211> 21
<212> DNA
<213> Homo sapiens

<400> 19
cctccggctc ctgctcctct t          21

<210> 20
<211> 22
<212> DNA
<213> Homo sapiens

<400> 20
ggcagttctt ggtctcgtca ca          22

<210> 21
<211> 24
<212> DNA
<213> Homo sapiens

<400> 21
tcgaaaagat gctgaacagt gaca          24

<210> 22
<211> 22
<212> DNA
<213> Homo sapiens

<400> 22
cttcaggaac agccaccagt ga          22


<210> 23
<211> 25
<212> DNA
<213> Homo sapiens


<400> 23
gtttacgcgt tacgctgaga gtaaa          25


<210> 24
<211> 21
<212> DNA
<213> Homo sapiens


<400> 24
cgttcttcag ggaggctacc a          21


<210> 25
<211> 24
<212> DNA
<213> Homo sapiens


<400> 25
gtgtgctaca gttgttcaag gctt          24


<210> 26
<211> 25
<212> DNA
<213> Homo sapiens


<400> 26
ctcaatatct gccactttca ctgct          25


<210> 27
<211> 24
<212> DNA
<213> Homo sapiens


<400> 27
aggccggctt tgactgggtg tatt          24


<210> 28
<211> 20
<212> DNA
<213> Homo sapiens


<400> 28
gcggccgagt gctcggactt          20


<210> 29
<211> 24
<212> DNA
<213> Homo sapiens


<400> 29
cctgcctgtg actttcaagc tact          24

<210> 30
<211> 24
<212> DNA
<213> Homo sapiens

<400> 30
cattccaccc aaagcatgtt atct          24

<210> 31
<211> 21
<212> DNA
<213> Homo sapiens

<400> 31
ggccgcatgg atgttgctga g          21

<210> 32
<211> 22
<212> DNA
<213> Homo sapiens

<400> 32
tctgagtccc tggctggcca ga          22

<210> 33
<211> 23
<212> DNA
<213> Homo sapiens

<400> 33
ggcttgaagc tgcttacgaa ttt          23

<210> 34
<211> 25
<212> DNA
<213> Homo sapiens

<400> 34
acagcccagt acttattccc tttga          25

<210> 35
<211> 21
<212> DNA
<213> Homo sapiens

<400> 35
actgcggttt tctcgaatcc a          21

<210> 36
<211> 20
<212> DNA
<213> Homo sapiens

<400> 36
ggtatccatc gccatgctcc          20

<210> 37
<211> 24

<212> DNA
<213> Homo sapiens

<400> 37
agtgggaaca ggctcaggac tatc        24

<210> 38
<211> 24
<212> DNA
<213> Homo sapiens

<400> 38
ggtaggccaa agaatttttg catc        24

<210> 39
<211> 19
<212> DNA
<213> Homo sapiens

<400> 39
cggcttgccc tggtgcagt        19

<210> 40
<211> 23
<212> DNA
<213> Homo sapiens

<400> 40
cgtcccgggt gtagagtctc tcg        23

<210> 41
<211> 21
<212> DNA
<213> Homo sapiens

<400> 41
ctcgggcaaa gagggtgaca a        21

<210> 42
<211> 23
<212> DNA
<213> Homo sapiens

<400> 42
gcttcatctg tccttttccc caa        23

<210> 43
<211> 19
<212> DNA
<213> Homo sapiens

<400> 43
gagcccctgg cttctggca        19

<210> 44
<211> 21
<212> DNA
<213> Homo sapiens

<400> 44
gccctgatga cgaggtcgga a        21

<210> 45
<211> 20
<212> DNA
<213> Homo sapiens

<400> 45
ggcgacctgg aagtccaact        20

<210> 46
<211> 19
<212> DNA
<213> Homo sapiens

<400> 46
ccatcagcac cacagcctt        19